# EUROPEAN PATENT APPLICATION

(11) **EP 4 675 354 A1**
(43) Date of publication of application: **07.01.2026**
(21) Application number: 25185268.7
(22) Date of filing: 25.06.2025
(51) Int. Cl.: G03F 7/039, G03F 7/004

(54) **CHEMICALLY AMPLIFIED POSITIVE RESIST COMPOSITION AND RESIST PATTERN FORMING PROCESS**

(30) Priority: 03.07.2024 JP 2024107164
(71) Applicant: Shin-Etsu Chemical Co., Ltd., Tokyo 100-0005 (JP)
(72) Inventor: FUKUSHIMA, Masahiro, Joetsu-shi (JP)
(74) Representative: Ter Meer Steinmeister & Partner

(57) **Abstract**

An onium salt consisting of a sulfonic acid anion having an aromatic sulfonic acid structure substituted with an aromatic ring-containing hydrocarbyl group and a triarylsulfonium cation containing iodine and fluorine generates an acid with limited diffusion. A chemically amplified positive resist composition comprising the onium salt as an acid generator and a polymer adapted to be decomposed under the action of acid to increase its solubility in alkaline developer is processed by lithography to form a pattern of rectangular profile.

## Description

### TECHNICAL FIELD

This invention relates to a chemically amplified positive resist composition and a resist pattern forming process.

### BACKGROUND ART

Pattern formation to a smaller feature size is required to meet the recent demand for higher integration in integrated circuits. Acid-catalyzed chemically amplified resist compositions are most often used in forming resist patterns with a feature size of 0.2 µm or less. High-energy radiation such as UV, deep-UV or EB is used as the energy source for exposure of these resist compositions. In particular, the EB lithography, which is utilized as the ultra-fine microfabrication technique, is also indispensable in processing a photomask blank into a photomask for use in the fabrication of semiconductor devices. Resist compositions for use in the EB lithography include positive ones wherein exposed regions are dissolved away to form a pattern and negative ones wherein exposed regions are retained to form a pattern. Either one which is easier to use is chosen in accordance with the morphology of the necessary resist pattern.

In general, the EB lithography is by writing an image with EB, without using a mask. In the case of positive resist, those regions of a resist film other than the regions to be retained are successively irradiated with EB having a minute area. In the case of negative resist, those regions of a resist film to be retained are successively irradiated with EB. The operation of successively scanning all finely divided regions on the work surface takes a long time as compared with full wafer exposure through a photomask. To prevent any throughput decline, a resist film having a high sensitivity is required. Because of a long image writing time, it is likely that a difference arises between an initially imaged portion and a lately imaged portion. The stability with time of the exposed portion in vacuum is one of the important performance factors. One of the important applications of chemically amplified resist material resides in processing of photomask blanks. Some photomask blanks have a surface material that can have an impact on the pattern profile of the overlying chemically amplified resist film, for example, a layer of a chromium compound, typically chromium oxide deposited on a photomask substrate. For high resolution and profile retention after etching, it is one important performance factor to maintain the profile of a resist film pattern rectangular independent of the type of substrate.

Attempts were made to ameliorate resist sensitivity and pattern profile in a controlled way by properly selecting and combining components used in resist compositions and adjusting processing conditions. One outstanding problem is the diffusion of acid, which has a significant impact on the resolution of a chemically amplified resist film. In the processing of photomasks, it is required that the profile of the resist pattern resulting from exposure does not change depending on the time taken until PEB. The major cause for time-dependent changes is the diffusion of acid generated upon exposure. Since the problem of acid diffusion has large impacts on sensitivity and resolution not only in the photomask processing, but also in general resist compositions, many studies are made thereon.

Patent Documents 1 and 2 describe acid generators capable of generating bulky acids upon exposure, for thereby controlling acid diffusion and reducing roughness. Since these acid generators are still insufficient to control acid diffusion, it is desired to have an acid generator with more controlled diffusion.

Patent Document 3 discloses a resist composition comprising a base polymer having bound thereto an acid generator capable of generating a sulfonic acid upon light exposure whereby acid diffusion is controlled. This approach of controlling acid diffusion by binding repeat units capable of generating acid upon exposure to a base polymer is effective in forming a pattern with reduced LER. However, the base polymer having bound therein repeat units capable of generating acid upon exposure encounters a problem with respect to its solubility in organic solvent, depending on the structure and proportion of the relevant units.

Polymers comprising a major proportion of aromatic structure having an acidic side chain, for example, polyhydroxystyrene are useful in resist materials for the KrF lithography. These polymers are not used in resist materials for the ArF lithography since they exhibit strong absorption at a wavelength of around 200 nm. These polymers, however, are expected to form useful resist materials for the EB and EUV lithography for forming patterns of smaller size than the processing limit of ArF lithography because they offer high etching resistance.

Often used as the base polymer in positive resist compositions for EB and EUV lithography is a polymer having an acidic functional group on phenol side chain masked with an acid labile group (or acid-decomposable protective group). Upon exposure to high-energy radiation, the acid labile group is deprotected by the catalysis of an acid generated from a photoacid generator so that the polymer may turn soluble in alkaline developer. Typical of the acid labile group are tertiary alkyl, tert-butoxycarbonyl, and acetal groups. The use of protective groups (e.g., acetal groups) requiring a relatively low level of activation energy for deprotection offers the advantage that a resist film having a high sensitivity is obtainable. However, if the diffusion of generated acid is not fully controlled, deprotection reaction can occur even in the unexposed region of the resist film, giving rise to problems like a degradation of LER and a lowering of in-plane uniformity (CDU) of pattern line width.

Patent Documents 4 and 5 describe photoacid generators capable of generating a non-fluorinated aromatic sulfonic acid having a plurality of bulky alkyl substituents.

Since a plurality of alkyl substituents are introduced, the generated acid has a higher molecular weight, which is effective for suppressing acid diffusion. The control of acid diffusion is insufficient for the purpose of forming small-size patterns. There remains room for further improvement.

In forming small-size patterns by the EB lithography, there is the problem that the profile of a resist pattern is inversely tapered by the influence of back scattering resulting from reflection of EB to the substrate. The inversely tapered profile may permit the resist pattern to collapse during development. For meeting the demand for further miniaturization, it is desired to solve the outstanding problem and to have a photoacid generator capable of suppressing acid diffusion while correcting the pattern profile.

### Citation List

Patent Document 1: JP-A 2009-053518
Patent Document 2: JP-A 2010-100604
Patent Document 3: JP-A 2011-022564
Patent Document 4: JP 6248882
Patent Document 5: JP-A 2019-202974

### DISCLOSURE OF INVENTION

Resist compositions are recently demanded which are capable of forming not only line-and-space (LS), isolated line (IL) and isolated space (IS) patterns of satisfactory profile, but also hole patterns of satisfactory profile. The acid generator described in Patent Document 4 generates a bulky acid, indicating that acid diffusion is controlled to some extent. This acid generator, however, gives rise to a problem that when a small-size pattern is formed, the pattern takes an inversely tapered profile and collapses during development in alkaline developer.

An object of the invention is to provide a chemically amplified positive resist composition comprising an onium salt capable of generating an acid with controlled diffusion, and a resist pattern forming process using the composition.

The inventors have found that an onium salt consisting of a sulfonic acid anion of an aromatic sulfonic acid structure substituted with a hydrocarbyl group containing at least one aromatic ring and a triarylsulfonium cation containing iodine and fluorine has satisfactory solvent solubility. When a resist film is formed from a positive resist composition comprising the onium salt as a photoacid generator and processed by lithography, a pattern with reduced LER and improved CDU is obtained. By virtue of proper dissolution inhibition, a pattern of rectangular profile is obtained.

In one aspect, the invention provides a chemically amplified positive resist composition comprising (A) a photoacid generator and (B) a base polymer containing a polymer adapted to be decomposed under the action of acid to increase its solubility in alkaline developer. The photoacid generator (A) contains an onium salt consisting of an anion having the formula (A1) and a cation having the formula (A2).

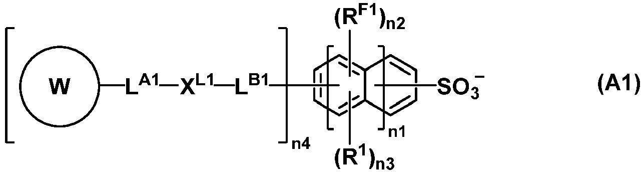

Herein n1 is 0 or 1, n2 is 0, 1, 2, 3 or 4, n3 is 0, 1, 2, 3 or 4, with the proviso that when n1=0, 0 ≤ n2+n3 ≤ 4, when n1=1, 0 ≤ n2+n3 ≤ 6, and n4 is 0 or 1,
W is a C₆-C₄₀ hydrocarbyl group containing at least one aromatic ring, which may contain a heteroatom,
R^{F1} is fluorine, a C₁-C₆ fluorinated saturated hydrocarbyl group, C₁-C₆ fluorinated saturated hydrocarbyloxy group, or C₁-C₆ fluorinated saturated hydrocarbylthio group,
R¹ is halogen exclusive of fluorine, nitro group, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom; when n3 is 2, 3 or 4, a plurality of R¹ may be identical or different, and a plurality of R¹ may bond together to form a ring with the carbon atoms to which they are attached,
L^{A1} and L^{B1} are each independently a single bond, ether bond, ester bond, amide bond, sulfonate ester bond, sulfonamide bond, carbonate bond or carbamate bond,
X^{L1} is a single bond or a C₁-C₄₀ hydrocarbylene group which may contain a heteroatom.

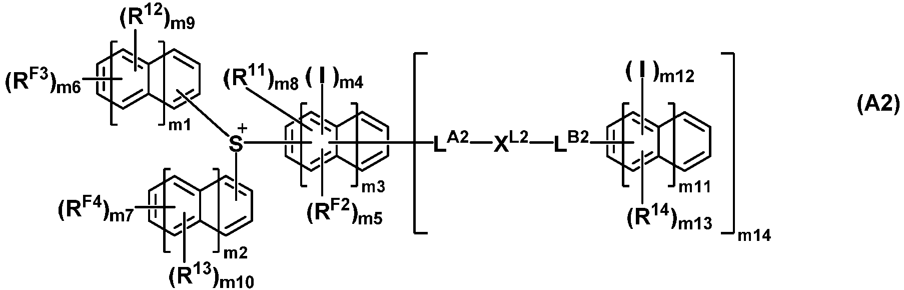

Herein m1 is 0 or 1, m2 is 0 or 1, m3 is 0 or 1, m4 is 0, 1, 2, 3 or 4, m5 is 0, 1, 2, 3 or 4, m6 is 0, 1, 2, 3, 4, 5 or 6, m7 is 0, 1, 2, 3, 4, 5 or 6, m8 is 0, 1 or 2, m9 is 0, 1 or 2, m10 is 0, 1 or 2, m11 is 0 or 1, m12 is 0, 1, 2, 3 or 4, m13 is 0, 1 or 2, m14 is 0, 1 or 2, with the proviso that when m1=0, 0 ≤ m6+m9 ≤ 4, and when m1=1, 0 ≤ m6+m9 ≤ 6; when m2=0, 0 ≤ m7+m10 ≤4, and when m2=1, 0 ≤ m7+m10 ≤ 6; when m3=0, 1 ≤ m4+m5+m8+m14 ≤ 4, and when m3=1, 1 ≤ m4+m5+m8+m14 ≤ 6; when m11=0, 0 ≤ m12+m13 ≤ 4, and when m11 =1, 0 ≤ m12+m13 ≤ 6; m4+m12 ≥ 1,
R^{F2} to R^{F4} are each independently fluorine, a C₁-C₆ fluorinated saturated hydrocarbyl group, C₁-C₆ fluorinated saturated hydrocarbyloxy group, or C₁-C₆ fluorinated saturated hydrocarbylthio group; when m5 is 2 or more, a plurality of R^{F2} may be identical or different; when m6 is 2 or more, a plurality of R^{F3} may be identical or different; when m7 is 2 or more, a plurality of R^{F4} may be identical or different,
R¹¹ to R¹⁴ are each independently halogen exclusive of iodine and fluorine, nitro group, cyano group, a C₁-C₂₀ hydrocarbyl group which may contain a heteroatom, C₁-C₂₀ hydrocarbyloxy group which may contain a heteroatom, C₁-C₂₀ hydrocarbylthio group which may contain a heteroatom; when m8=2, two R¹¹ may be identical or different and two R¹¹ may bond together to form a ring with the carbon atoms to which they are attached; when m9=2, two R¹² may be identical or different and two R¹² may bond together to form a ring with the carbon atoms to which they are attached; when m10=2, two R¹³ may be identical or different and two R¹³ may bond together to form a ring with the carbon atoms to which they are attached; when m13=2, two R¹⁴ may be identical or different and two R¹⁴ may bond together to form a ring with the carbon atoms to which they are attached;
the aromatic rings directly bonded to S⁺ in the sulfonium cation may bond together to form a ring with S⁺,
L^{A2} and L^{B2} are each independently a single bond, ether bond, ester bond, amide bond, sulfonate ester bond, sulfonamide bond, carbonate bond or carbamate bond,
X^{L2} is a single bond or a C₁-C₄₀ hydrocarbylene group which may contain a heteroatom.

The polymer comprises repeat units having the formula (B 1).

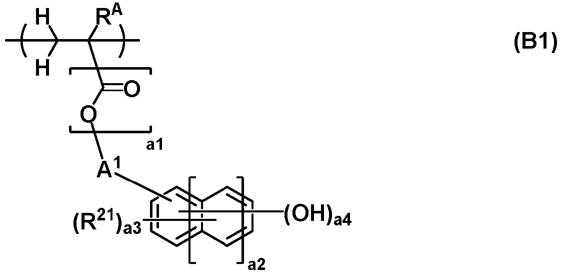

Herein a1 is 0 or 1, a2 is 0, 1 or 2, a3 is an integer meeting 0 ≤ a3 ≤ 5+2(a2)-a4, a4 is 1, 2 or 3,
R^{A} is hydrogen, fluorine, methyl or trifluoromethyl,
R²¹ is halogen, nitro group, carboxy group, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, and
A¹ is a single bond or C₁-C₁₀ saturated hydrocarbylene group in which some -CH₂-may be replaced by -O-.

In a preferred embodiment, W is a group having the formula (W-1) or (W-2).

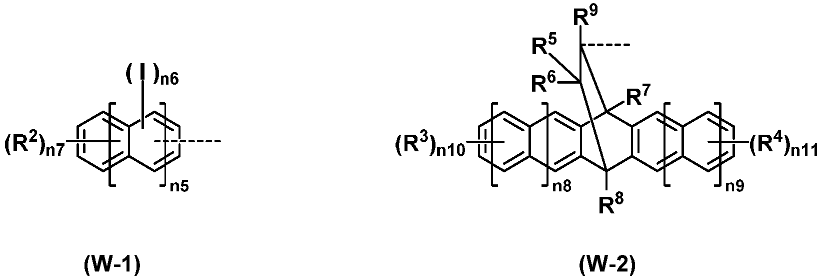

Herein n5 is 0 or 1, n6 is 0, 1, 2, 3 or 4, n7 is 0, 1, 2, 3 or 4, with the proviso that when n5=0, 1 ≤ n6+n7 ≤ 5, and when n5 =1, 1 ≤ n6+n7 ≤ 7, n8 is 0 or 1, n9 is 0 or 1, n10 is 0, 1, 2, 3 or 4, n11 is 0, 1, 2, 3 or 4,
R² is each independently hydrogen, halogen exclusive of iodine, hydroxy, or a C₁-C₂₀ hydrocarbyl group which may contain a heteroatom,
R³ and R⁴ are each independently hydrogen, halogen, or a C₁-C₂₀ hydrocarbyl group which may contain a heteroatom,
R⁵ to R⁹ are each independently hydrogen, halogen, or a C₁-C₄₀ hydrocarbyl group which may contain a heteroatom,
the broken line designates a point of attachment to L^{A1}

In a preferred embodiment, the anion having formula (A1) has the formula (A1-1): 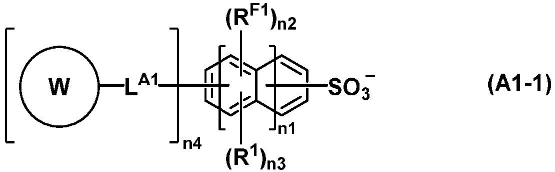 wherein n1 to n4, W, R^{F1}, R¹, and L^{A} are as defined above.

In a preferred embodiment, the cation having formula (A2) has the formula (A2-1): 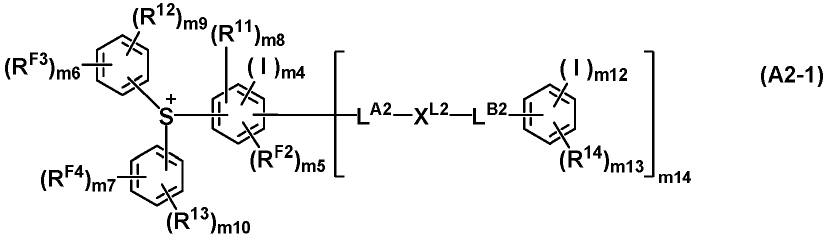 wherein m4 to m10, m12 to m14, R^{F2} to R^{F4} , R¹¹ to R¹⁴, L^{A2}, L^{B2} and X^{L2} are as defined above.

More preferably, the cation having formula (A2-1) has the formula (A2-2): 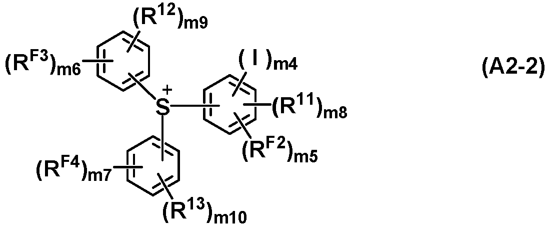 wherein m4 to m10, R^{F2} to R^{F4}, and R¹¹ to R¹³ are as defined above.

In a preferred embodiment, the polymer further comprises repeat units having the formula (B2-1).

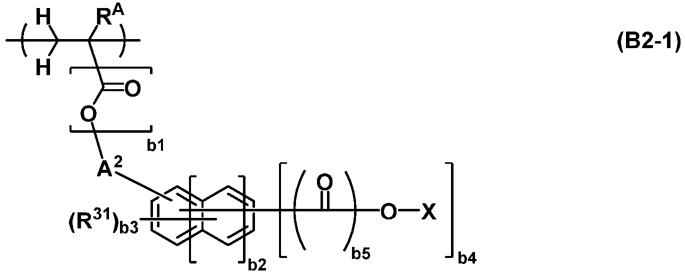

Herein R^{A} is hydrogen, fluorine, methyl or trifluoromethyl,
b1 is 0 or 1, b2 is 0, 1 or 2, b3 is an integer meeting 0 ≤ b3 ≤ 5+2(b2)-b4, b4 is 1, 2 or 3, b5 is 0 or 1,
R³¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
A² is a single bond or C₁-C₁₉ saturated hydrocarbylene group in which some -CH₂-may be replaced by -O-,
X is an acid labile group when b4=1, and X is hydrogen or an acid labile group, at least one being an acid labile group, when b4=2 or 3.

In a preferred embodiment, the polymer further comprises repeat units having the formula (B2-2).

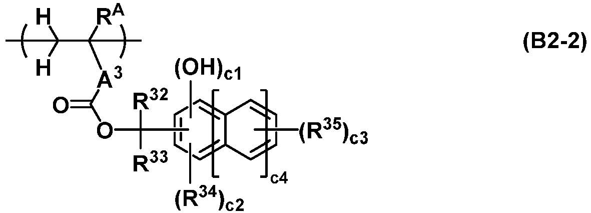

Herein c1 is 0, 1 or 2, c2 is 0, 1 or 2, c3 is 0, 1, 2, 3, 4 or 5, c4 is 0, 1 or 2,
R^{A} is hydrogen, fluorine, methyl or trifluoromethyl,
R³² and R³³ are each independently a C₁-C₁₀ hydrocarbyl group which may contain a heteroatom, R³² and R³³ may bond together to form a ring with the carbon atom to which they are attached,
R³⁴ is each independently fluorine, a C₁-C₅ fluorinated alkyl group or C₁-C₅ fluorinated alkoxy group,
R³⁵ is each independently a C₁-C₁₀ hydrocarbyl group which may contain a heteroatom, and
A³ is a single bond, phenylene, naphthylene or *-C(=O)-O-A³¹-, A³¹ is a C₁-C₂₀ aliphatic hydrocarbylene group which may contain a hydroxy moiety, ether bond, ester bond or lactone ring, a phenylene group or a naphthylene group, * designates a point of attachment to the carbon atom in the backbone.

In a preferred embodiment, the polymer further comprises repeat units of at least one type selected from repeat units having the formula (B3), repeat units having the formula (B4), and repeat units having the formula (B5):

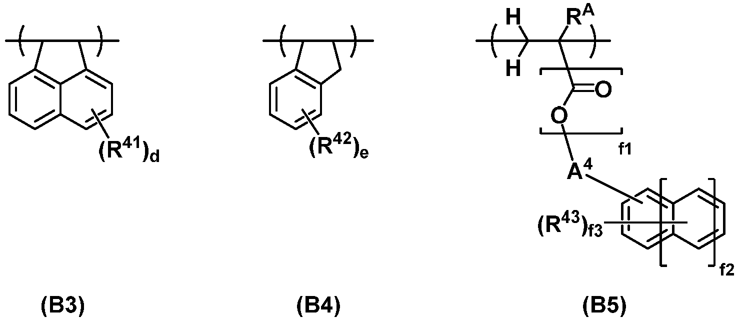

Herein d is 0, 1, 2, 3, 4, 5 or 6, e is 0, 1, 2, 3 or 4, f1 is 0 or 1, f2 is 0, 1 or 2, f3 is 0, 1, 2, 3, 4 or 5,
R^{A} is hydrogen, fluorine, methyl or trifluoromethyl,
R⁴¹ and R⁴² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
R⁴³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro, or cyano, R⁴³ may also be hydroxy when f2=1 or 2, and
A⁴ is a single bond or C₁-C₁₀ saturated hydrocarbylene group in which some -CH₂-may be replaced by -O-.

The polymer may further comprise repeat units of at least one type selected from repeat units having the formula (B6), repeat units having the formula (B7), repeat units having the formula (B8), repeat units having the formula (B9), and repeat units having the formula (B10).

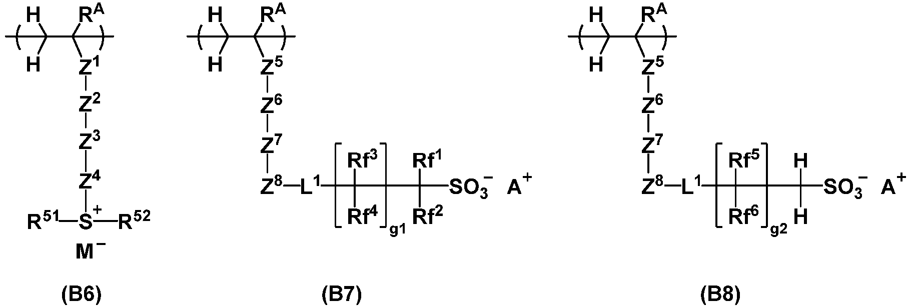

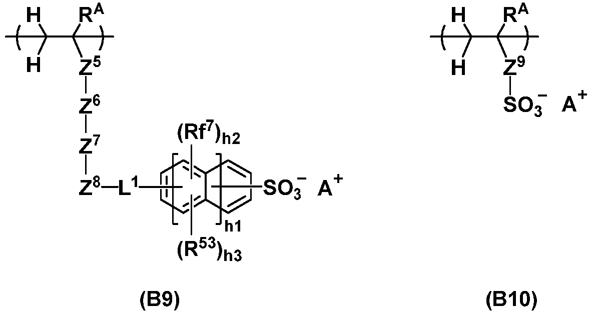

Herein g1 and g2 are each independently 0, 1, 2 or 3, h1 is 0 or 1, h2 is 0, 1, 2, 3 or 4, h3 is 0, 1, 2, 3 or 4, with the proviso that when h1=0, 0 ≤ h2+h3 ≤ 4, and when h1=1, 0 ≤ h2+h3 ≤ 6,
R^{A} is hydrogen, fluorine, methyl or trifluoromethyl,
Z₁ is a single bond or an optionally substituted phenylene group,
Z² is a single bond, **-C(=O)-O-Z²¹-, **-C(=O)-NH-Z²¹-, or **-O-Z²¹-, Z²¹ is a C₁-C₆ aliphatic hydrocarbylene group, phenylene group or a divalent group obtained by combining the foregoing, which may contain halogen, carbonyl moiety, ester bond, ether bond or hydroxy moiety,
Z³ is a single bond, ether bond, ester bond, amide bond, sulfonate ester bond, sulfonamide bond, carbonate bond or carbamate bond,
Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety,
Z⁵ is each independently a single bond, an optionally substituted phenylene, naphthylene, or *-C(=O)-O-Z⁵¹-, Z⁵¹ is a C₁-C₁₀ aliphatic hydrocarbylene group which may contain halogen, hydroxy moiety, ether bond, ester bond or lactone ring, or phenylene or naphthylene group,
Z⁶ is a single bond, ether bond, ester bond, amide bond, sulfonate ester bond, sulfonamide bond, carbonate bond or carbamate bond,
Z⁷ is each independently a single bond, ***-Z⁷¹-C(=O)-O-, ***-C(=O)-NH-Z⁷¹-, or ***-O-Z⁷¹-, Z⁷¹ is a C₁-C₂₀ hydrocarbylene group which may contain a heteroatom,
Z⁸ is each independently a single bond, ****-Z⁸¹-C(=O)-O-, ****-C(=O)-NH-Z⁸¹-, or ****-O-Z⁸¹-, Z⁸¹ is a C₁-C₂₀ hydrocarbylene group which may contain a heteroatom,
Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety,
* is a point of attachment to the carbon atom in the backbone, ** is a point of attachment to Z¹, *** is a point of attachment to Z⁶, **** is a point of attachment to Z⁷,
L¹ is a single bond, ether bond, ester bond, carbonyl group, sulfonate ester bond, sulfonamide bond, carbonate bond or carbamate bond,
Rf¹ and Rf² are each independently fluorine or a C₁-C₆ fluorinated saturated hydrocarbyl group,
Rf³ and Rf⁴ are each independently hydrogen, fluorine, or a C₁-C₆ fluorinated saturated hydrocarbyl group,
Rf⁶ and Rf⁶ are each independently hydrogen, fluorine, or a C₁-C₆ fluorinated saturated hydrocarbyl group, excluding that all Rf⁶ and Rf⁶ are hydrogen at the same time,
Rf⁷ is fluorine, a C₁-C₆ fluorinated alkyl group, C₁-C₆ fluorinated alkoxy group, or C₁-C₆ fluorinated alkylthio group,
R³¹ and R³² are each independently a C₁-C₂₀ hydrocarbyl group which may contain a heteroatom, R³¹ and R³² may bond together to form a ring with the sulfur atom to which they are attached,
R⁵³ is halogen exclusive of fluorine, or a C₁-C₂₀ hydrocarbyl group which may contain a heteroatom; when h3 is 2, 3 or 4, a plurality of R¹³ may bond together to form a ring with the carbon atoms to which they are attached,
M⁻ is a non-nucleophilic counter ion,
A⁺ is an onium cation.

In a preferred embodiment, repeat units having an aromatic ring structure account for at least 60 mol% of the overall repeat units of the polymer in the base polymer.

The resist composition may further comprise (C) an organic solvent.

The resist composition may further comprise (D) a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6).

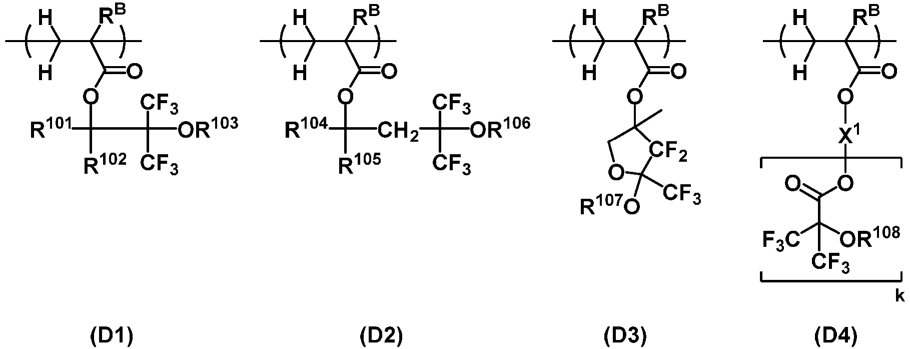

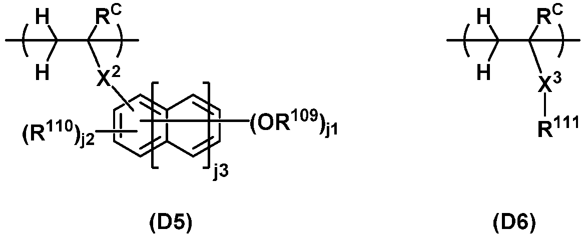

Herein j1 is 1, 2 or 3, j2 is an integer meeting 0 ≤ j2 ≤ 5+2(j3)j1, j3 is 0 or 1, k is 1, 2 or 3,
R^{B} is each independently hydrogen, fluorine, methyl or trifluoromethyl,
R^{C} is each independently hydrogen or methyl,
R¹⁰¹, R¹⁰², R¹⁰⁴ and R¹⁰⁵ are each independently hydrogen or a C₁-C₁₀ saturated hydrocarbyl group,
R¹⁰³, R¹⁰⁶ , R¹⁰⁷ and R¹⁰⁸ are each independently hydrogen, a C₁-C₁₅ hydrocarbyl group, C₁-C₁₅ fluorinated hydrocarbyl group, or acid labile group, and when R¹⁰³, R¹⁰⁶, R¹⁰⁷ and R¹⁰⁸ each are a hydrocarbyl or fluorinated hydrocarbyl group, an ether bond or carbonyl moiety may intervene in a carbon-carbon bond,
R¹⁰⁹ is hydrogen or a C₁-C₅ straight or branched hydrocarbyl group in which a heteroatom-containing moiety may intervene in a carbon-carbon bond,
R¹¹⁰ is a C₁-C₅ straight or branched hydrocarbyl group in which a heteroatom-containing moiety may intervene in a carbon-carbon bond,
R¹¹¹ is a C₁-C₂₀ saturated hydrocarbyl group in which at least one hydrogen is substituted by fluorine, and in which some constituent -CH₂- may be replaced by an ester bond or ether bond,
X¹ is a C₁-C₂₀ (k+1)-valent hydrocarbon group or C₁-C₂₀ (k+1)-valent fluorinated hydrocarbon group,
X² is a single bond, *-C(=O)-O- or *-C(=O)-NH-, * designates a point of attachment to the carbon atom in the backbone,
X³ is a single bond, -O-, *-C(=O)=O-X³¹-X³²- or *-C(=O)-NH-X³¹-X³²-, X³¹ is a single bond or C₁-C₁₀ saturated hydrocarbylene group, X³² is a single bond, ester bond, ether bond, or sulfonamide bond, and * designates a point of attachment to the carbon atom in the backbone.

The resist composition may further comprise (E) a quencher.

In a preferred embodiment, the photoacid generator (A) and the quencher (E) are present in a weight ratio of less than 3/1.

The resist composition may further comprise a photoacid generator other than the photoacid generator defined herein.

In another aspect, the invention provides a resist pattern forming process comprising the steps of:
applying the chemically amplified positive resist composition defined herein onto a substrate to form a resist film thereon,
exposing the resist film patternwise to high-energy radiation, and
developing the exposed resist film in an alkaline developer.

The high-energy radiation is typically EUV of wavelength 3 to 15 nm or EB.

The process is effective even when the substrate has the outermost surface of a chromium-containing material. Typically, the substrate is a photomask blank.

### ADVANTAGEOUS EFFECTS OF INVENTION

When processed by the microfabrication technology, especially EB and EUV lithography processes, a chemically amplified positive resist composition comprising an onium salt consisting of an anion having formula (A1) and a cation having formula (A2) as a photoacid generator can form a resist pattern having a very high resolution and reduced LER. A pattern of rectangular profile is obtainable by virtue of adequate dissolution inhibition.

### DESCRIPTION OF PREFERRED EMBODIMENTS

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group.

In chemical formulae, the broken line (---) and asterisk (*) each designate a point of attachment, namely valence bond. Me stands for methyl and Ac for acetyl. As used herein, the term "halogenated" refers to a halogen-substituted or halogen-containing compound or group. For example, "fluorinated" refers to a fluorine-substituted or fluorine-containing compound or group. The terms "group" and "moiety" are interchangeable.

The abbreviations and acronyms have the following meaning.
- PAG:: photoacid generator
- Mw:: weight average molecular weight
- Mn:: number average molecular weight
- Mw/Mn:: molecular weight distribution or dispersity
- GPC:: gel permeation chromatography
- PEB:: post-exposure baking
- LER:: line edge roughness
- CDU:: critical dimension uniformity

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Chemically amplified positive resist composition

One embodiment of the invention is a chemically amplified positive resist composition comprising (A) a photoacid generator in the form of an onium salt consisting of a sulfonic acid anion of an aromatic sulfonic acid structure substituted with a hydrocarbyl group containing at least one aromatic ring and a triarylsulfonium cation containing iodine and fluorine and (B) a base polymer containing a specific polymer.

### (A) Photoacid generator

The photoacid generator (A) is an onium salt containing an anion having the formula (A1).

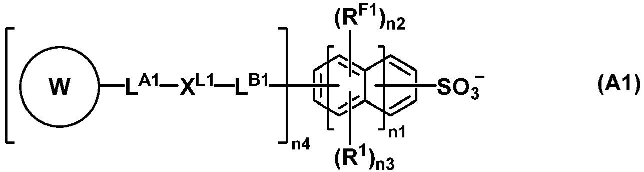

In formula (A1), n1 is 0 or 1. The relevant structure is a benzene ring when n1=0, and a naphthalene ring when n1=1. The benzene ring corresponding to n1=0 is preferred from the aspect of solvent solubility. The subscript n2 is 0, 1, 2, 3 or 4. It is preferred from the aspect of reactant availability that n2 be 4 when n2 is 1 or more. The subscript n3 is 0, 1, 2, 3 or 4. It is provided that when n1=0, 0 ≤ n2+n3 ≤ 4, and when n1=1, 0 ≤ n2+n3 ≤ 6. The subscript n4 is 0 or 1. It is preferred from the aspect of acid diffusion control that n4 be 1.

In formula (A1), R^{F1} is fluorine, a C₁-C₆ fluorinated saturated hydrocarbyl group, C₁-C₆ fluorinated saturated hydrocarbyloxy group, or C₁-C₆ fluorinated saturated hydrocarbylthio group. R^{F1} is preferably fluorine, trifluoromethyl, difluoromethyl, trifluoromethoxy, difluoromethoxy, trifluoromethylthio or difluoromethylthio, more preferably fluorine, trifluoromethyl or trifluoromethoxy. Since fluorine or a fluorinated substituent group is contained, the acid strength of the generated acid is so enhanced due to the electron attractive effect that deprotection reaction of acid labile groups such as tertiary ester or tertiary ether groups may smoothly take place. When n2 is 2, 3 or 4, a plurality of R^{F1} may be identical or different.

In formula (A1), R¹ is halogen exclusive of fluorine, nitro group, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom. Suitable halogen atoms exclusive of fluorine include chlorine, bromine and iodine, with iodine being preferred. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, icosyl; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl, adamantyl; C₂-C₂₀ alkenyl groups such as vinyl, allyl, propenyl, butenyl, hexenyl; C₃-C₂₀ cyclic unsaturated hydrocarbyl groups such as cyclohexenyl; C₆-C₂₀ aryl groups such as phenyl and naphthyl; C₇-C₂₀ aralkyl groups such as benzyl, 1-phenylethyl, 2-phenylethyl, and combinations thereof. Inter alia, aryl groups are preferred. In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy, cyano, fluorine, chlorine, bromine, iodine, carbonyl, ether bond, ester bond, sulfonate ester bond, carbonate bond, lactone ring, sultone ring, carboxylic anhydride (-C(=O)-O-C(=O)-) or haloalkyl moiety. When n3 is 2 or more, a plurality of R¹ may be identical or different. When n3 is 2 or more, a plurality of R¹ may bond together to form a ring with the carbon atoms to which they are attached. The ring is preferably 5 to 8-membered.

In formula (A1), W is a C₆-C₄₀ hydrocarbyl group containing at least one aromatic ring, which may contain a heteroatom, preferably a group having the formula (W-1) or (W-2).

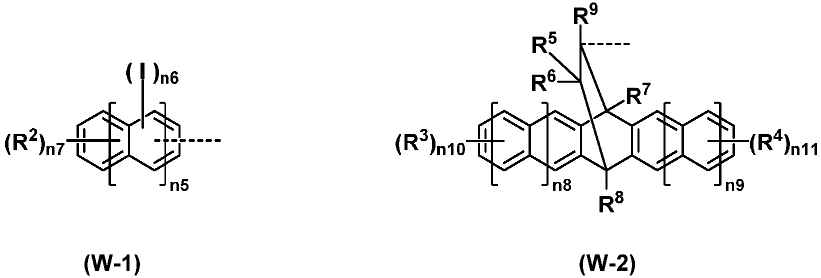

The broken line designates a point of attachment to L^{A1}.

In formula (W-1), n5 is 0 or 1. The relevant structure is a benzene ring when n5=0, and a naphthalene ring when n5=1. The benzene ring corresponding to n5=0 is preferred from the aspect of solvent solubility. The subscript n6 is 0, 1, 2, 3 or 4, and n7 is 0, 1, 2, 3 or 4. It is provided that when n5=0, 1 ≤ n6+n7 ≤ 5, and when n5 =1, 1 ≤ n6+n7 ≤ 7. When n5=0, n6 is preferably 2, 3 or 4 and from the aspect of EUV absorption, n6 is more preferably 3 or 4. It is preferred from the aspect of reactant availability that n7 be 1, 2 or 3, and more preferred from the aspect of acid diffusion control that n7 be 2 or 3.

In formula (W-1), R² is each independently hydrogen, halogen exclusive of iodine, hydroxy, or a C₁-C₂₀ hydrocarbyl group which may contain a heteroatom. Suitable halogen atoms exclusive of iodine include fluorine, chlorine and bromine, with fluorine being preferred from the aspect of solvent solubility. Examples of the hydrocarbyl group which may contain a heteroatom are as exemplified above for the hydrocarbyl group R¹, but not limited thereto. A group of branched or cyclic structure is preferred as R².

In formula (W-1), it is preferred that at least one selected from R² and iodine be attached to the carbon atom adjoining the carbon atom to which L^{A1} is attached. Then, the rotation of the aromatic ring to which R² and/or iodine is attached and the aromatic ring to which a sulfo group is attached, about the linking axis: -L^{A1}-X^{L1}-L^{B1}-, is restrained by steric hindrance, leading to a decline of acid diffusion.

In formula (W-2), n8 is 0 or 1. The relevant structure is a benzene ring when n8=0, and a naphthalene ring when n8=1. The benzene ring corresponding to n8=0 is preferred from the aspect of solvent solubility. The subscript n9 is 0 or 1. The relevant structure is a benzene ring when n9=0, and a naphthalene ring when n9=1. The benzene ring corresponding to n9=0 is preferred from the aspect of solvent solubility. The subscript n10 is 0, 1, 2, 3 or 4. From the aspect of reactant availability, n10 is preferably 0, 1 or 2. The subscript n11 is 0, 1, 2, 3 or 4. From the aspect of reactant availability, n11 is preferably 0, 1 or 2.

In formula (W-2), R³ and R⁴ are each independently hydrogen, halogen, or a C₁-C₂₀ hydrocarbyl group which may contain a heteroatom. Suitable halogen atoms include fluorine, chlorine, bromine and iodine, with fluorine and iodine being preferred. Examples of the hydrocarbyl group which may contain a heteroatom are as exemplified above for the hydrocarbyl group R¹, but not limited thereto. A group of branched or cyclic structure is preferred as R³ and R⁴.

In formula (W-2), R⁵ to R⁹ are each independently hydrogen, halogen, or a C₁-C₄₀ hydrocarbyl group which may contain a heteroatom. Suitable halogen atoms include fluorine, chlorine, bromine and iodine, with fluorine and iodine being preferred. Examples of the hydrocarbyl group which may contain a heteroatom are as exemplified above for the hydrocarbyl group R¹, but not limited thereto. A group of branched or cyclic structure is preferred as R⁵ to R⁹.

In formula (W-2), any two of R⁵ to R⁹ may bond together to form a ring with the carbon atoms to which they are attached. The ring is preferably 5 to 8-membered.

In formula (A1), L^{A1} and L^{B1} are each independently a single bond, ether bond, ester bond, amide bond, sulfonate ester bond, sulfonamide bond, carbonate bond or carbamate bond. L^{A1} and L^{B1} are preferably a single bond, ether bond or ester bond.

In formula (A1), X^{L1} is a single bond or a C₁-C₄₀ hydrocarbylene group which may contain a heteroatom. The hydrocarbylene group may be straight, branched or cyclic and examples thereof include alkanediyl groups, cyclic saturated hydrocarbylene groups, and arylene groups. Suitable heteroatoms include oxygen, nitrogen and sulfur.

Examples of the C₁-C₄₀ hydrocarbylene group which may contain a heteroatom, represented by X^{L1}, are shown below, but not limited thereto. Herein, * designates a point of attachment to L^{A1} or L^{B1}.

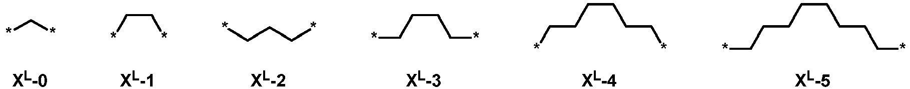

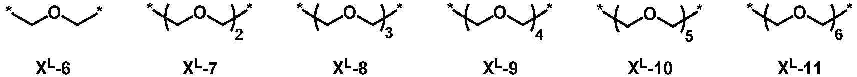

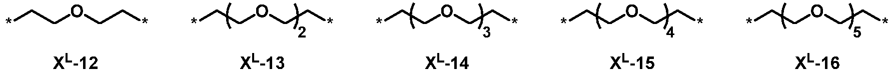

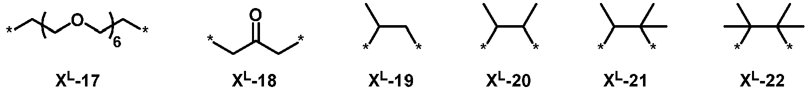

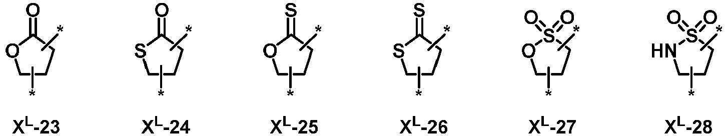

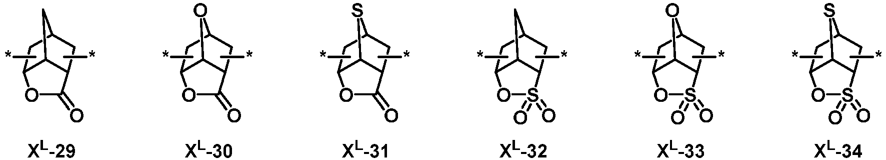

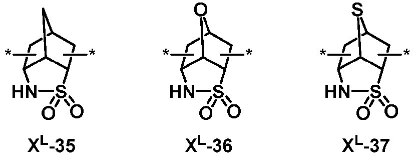

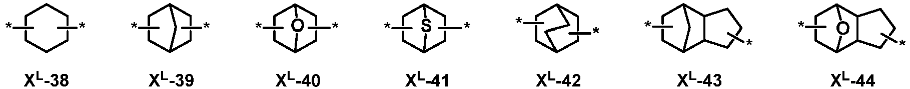

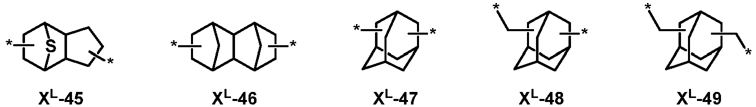

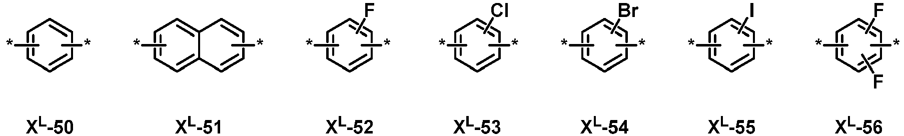

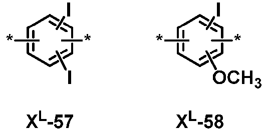

Of these, X^{L}-0 to X^{L}-22 and X^{L}-47 to X^{L}-58 are preferred.

Of the anions having formula (A1), those having the formula (A1-1) are preferred.

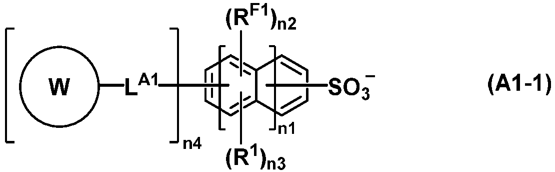

Herein n1 to n4, W, R^{F1}, R¹, and L^{A1} are as defined above.

Examples of the anion having formula (A1) are shown below, but not limited thereto.

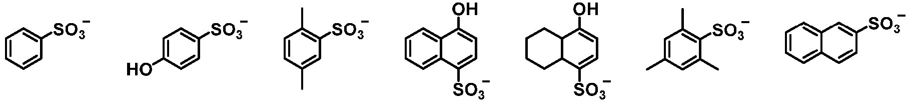

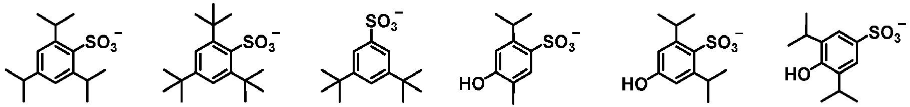

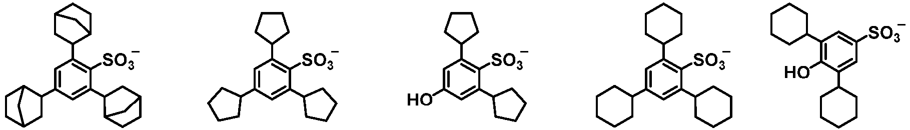

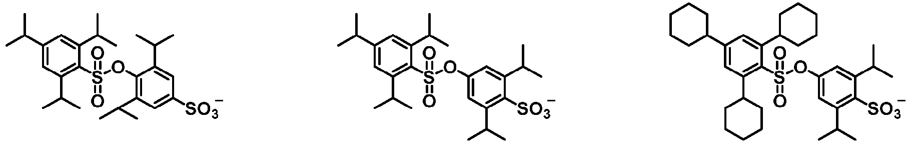

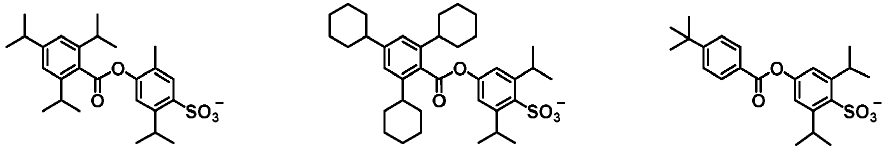

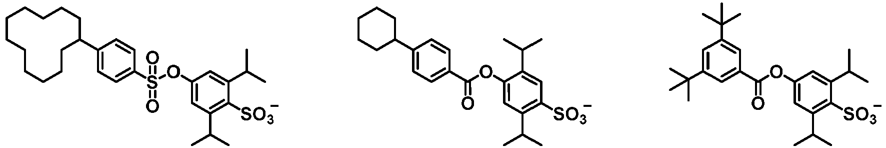

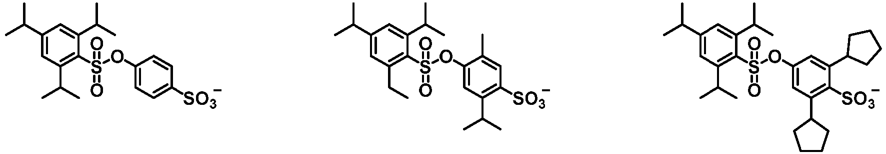

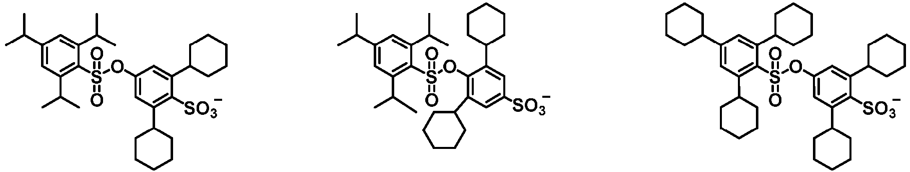

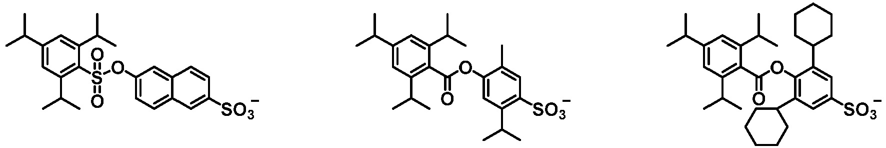

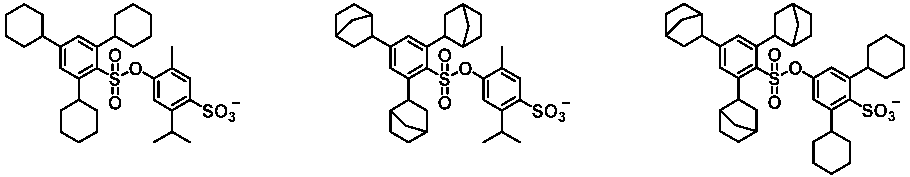

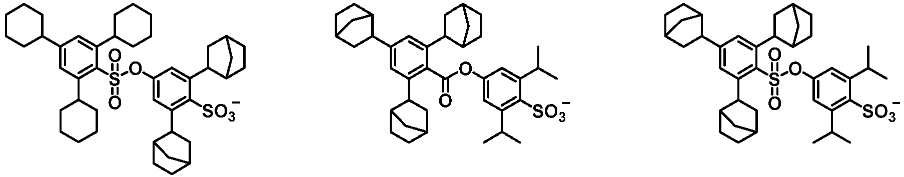

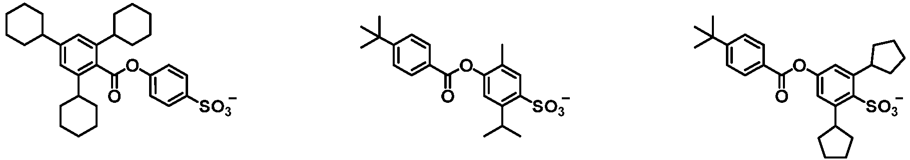

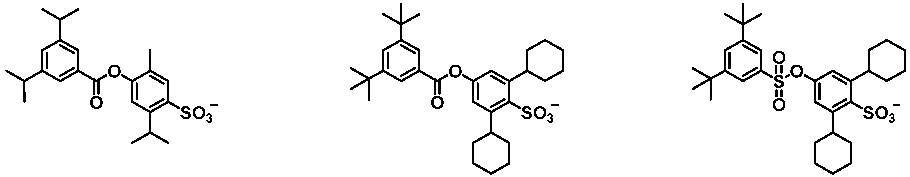

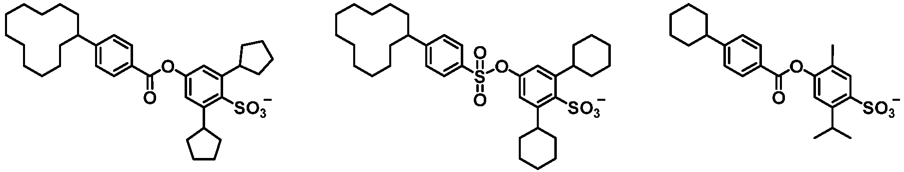

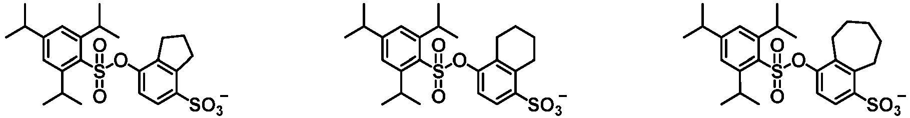

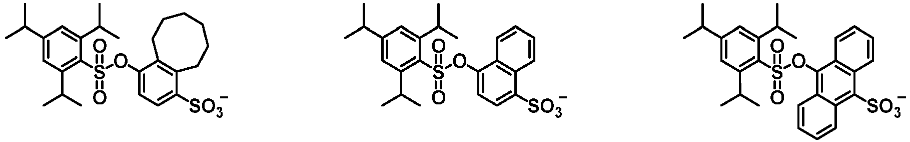

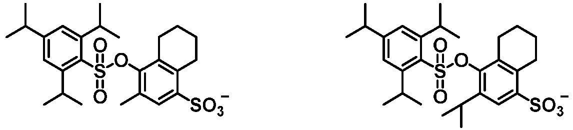

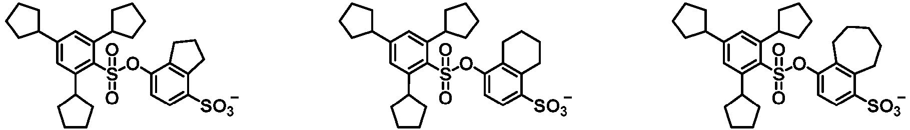

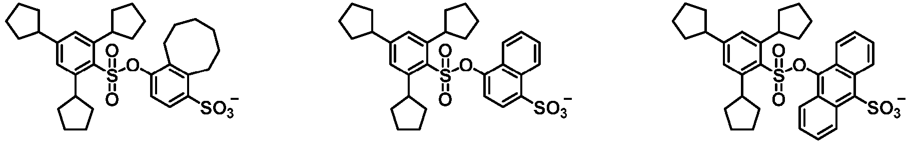

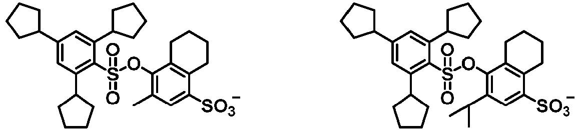

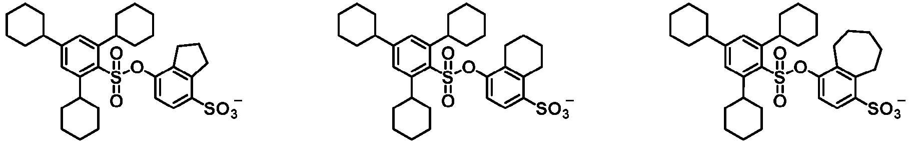

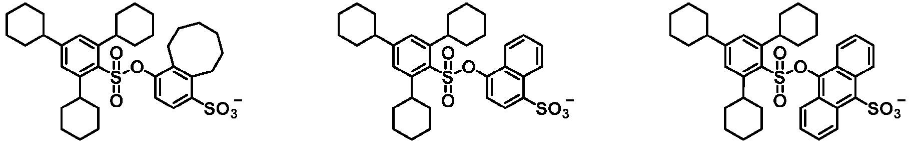

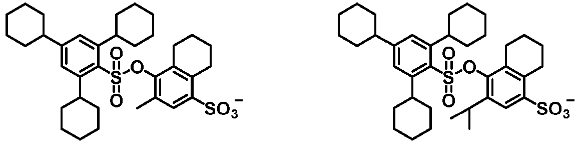

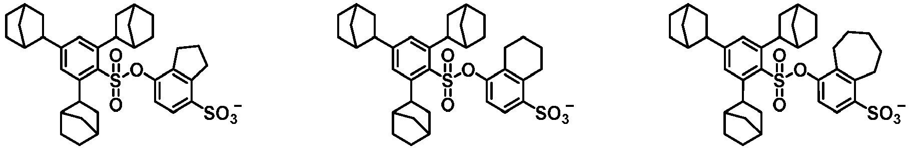

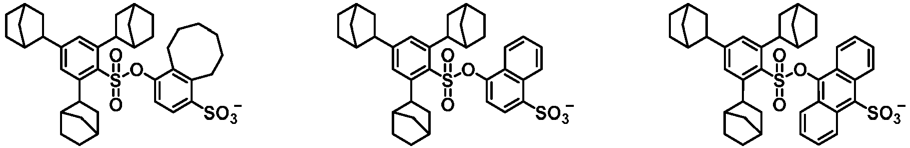

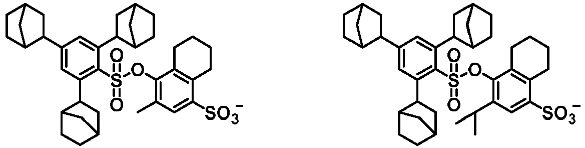

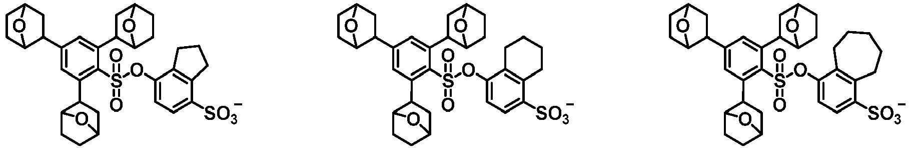

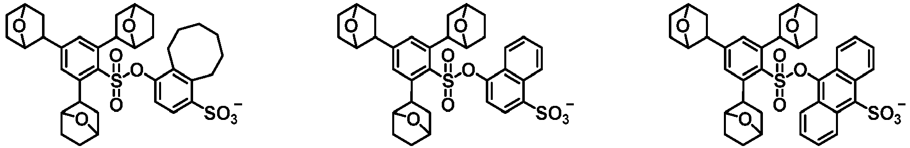

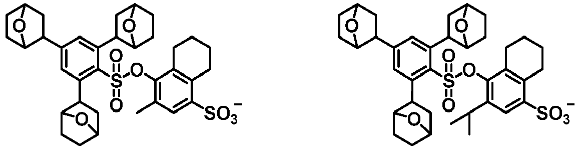

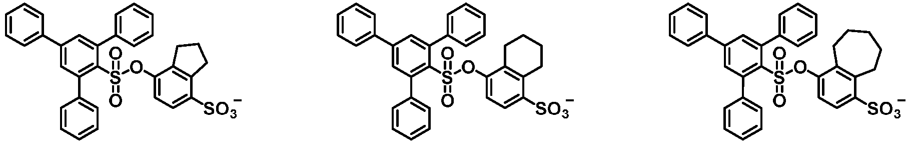

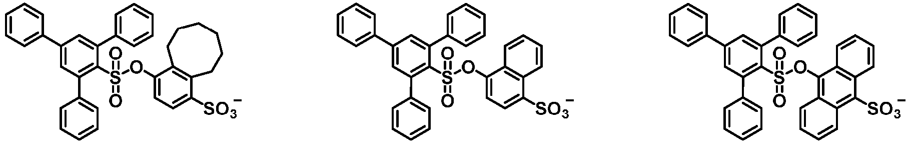

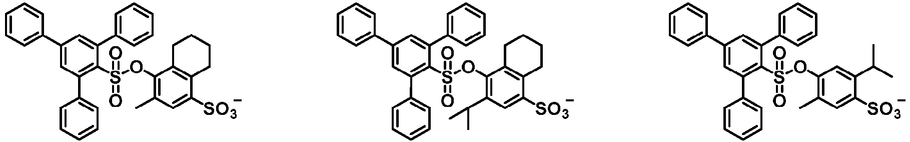

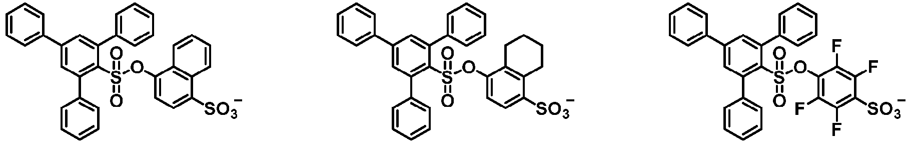

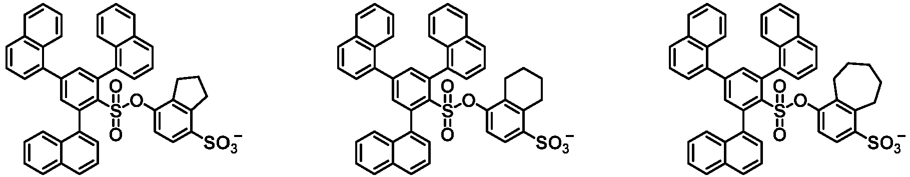

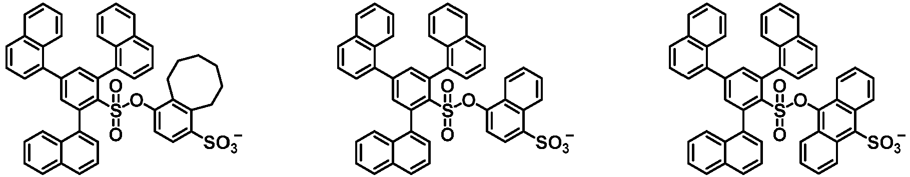

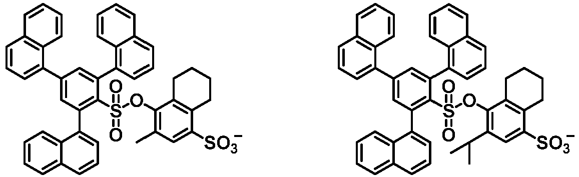

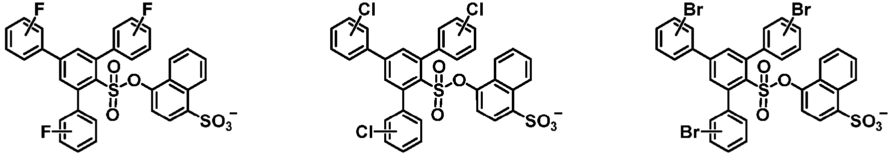

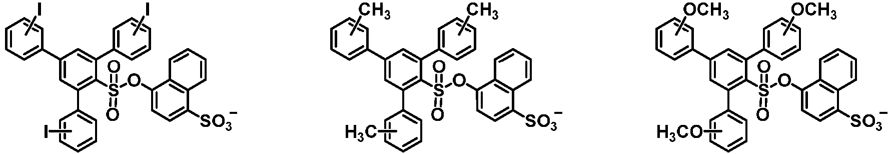

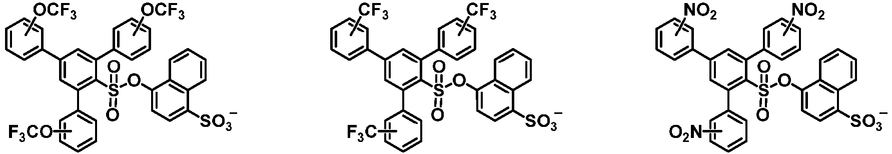

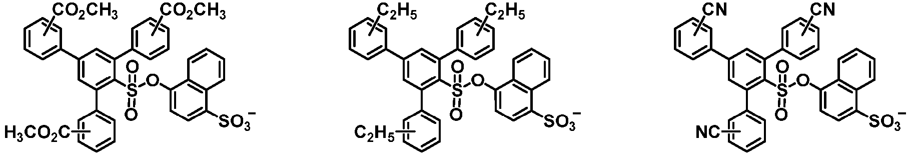

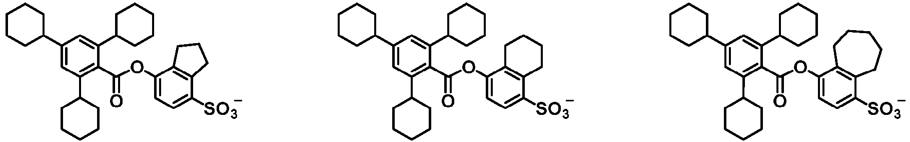

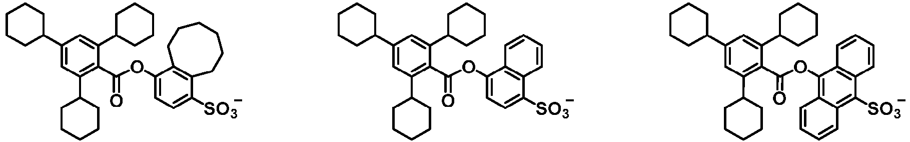

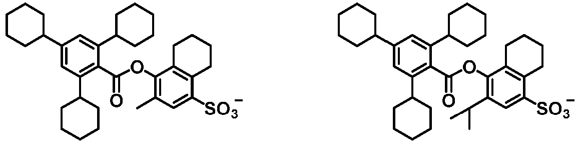

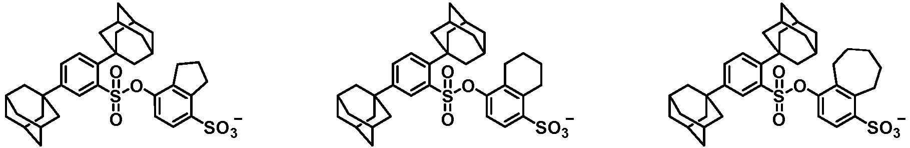

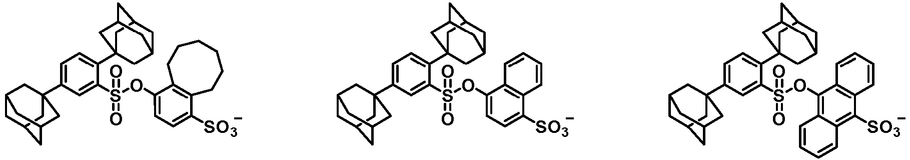

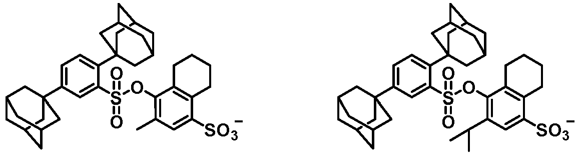

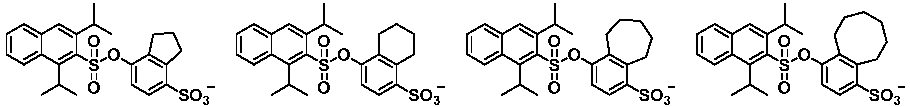

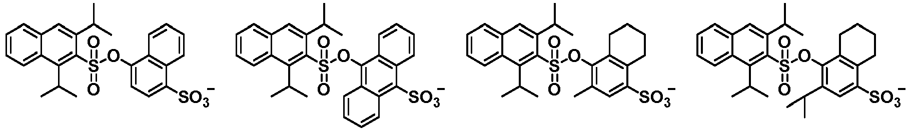

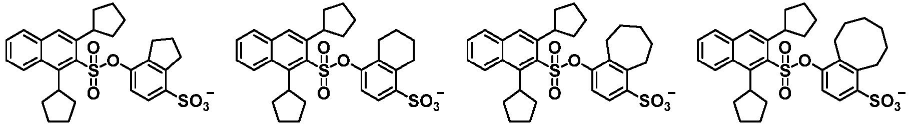

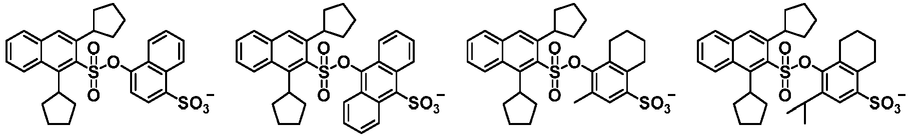

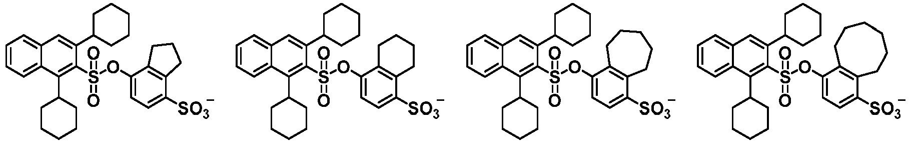

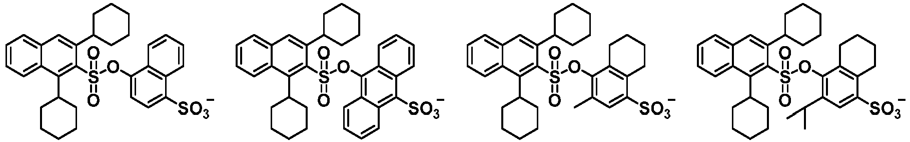

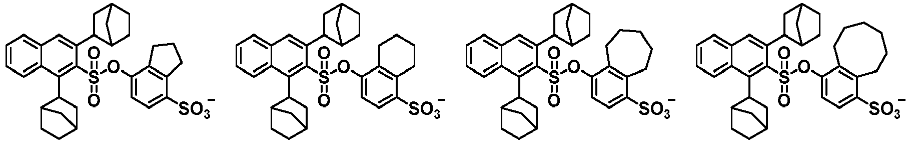

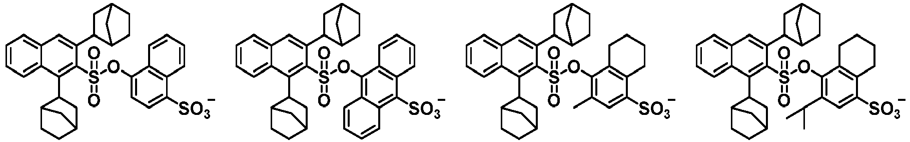

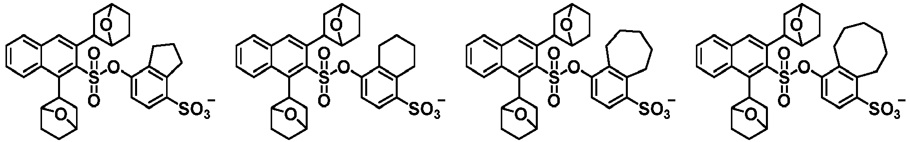

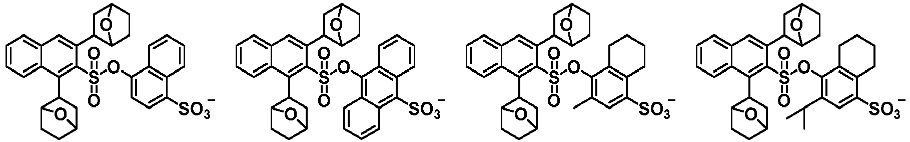

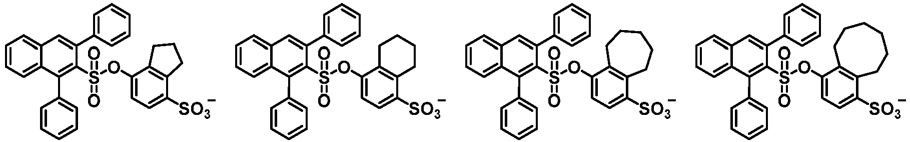

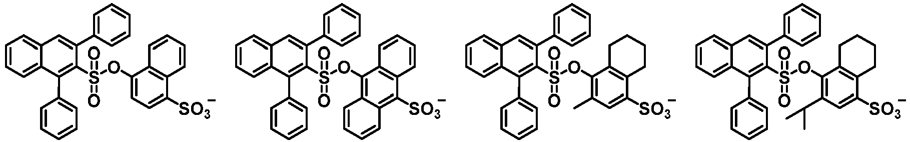

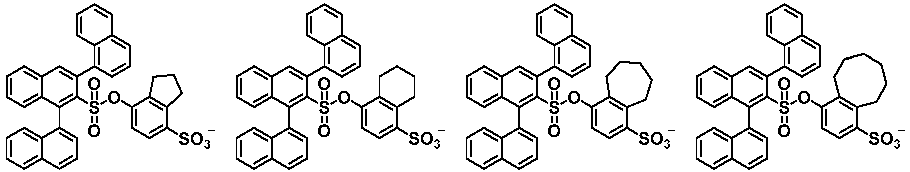

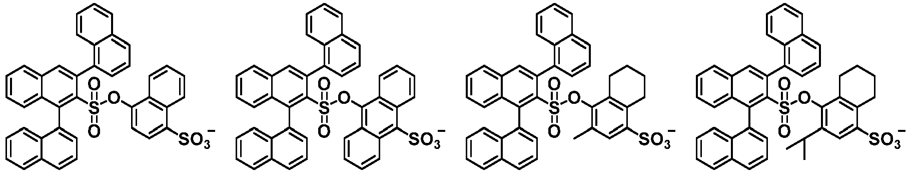

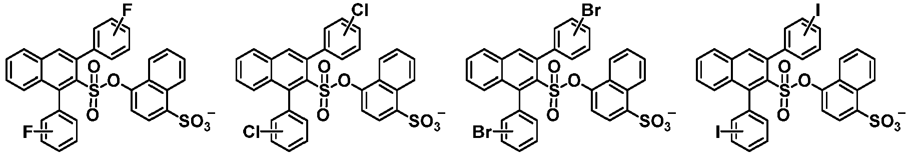

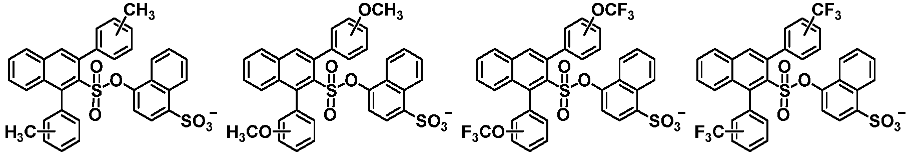

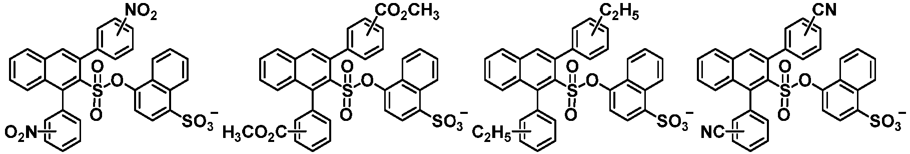

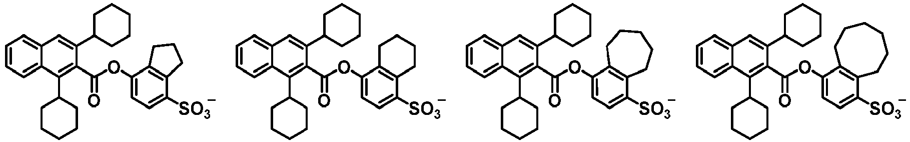

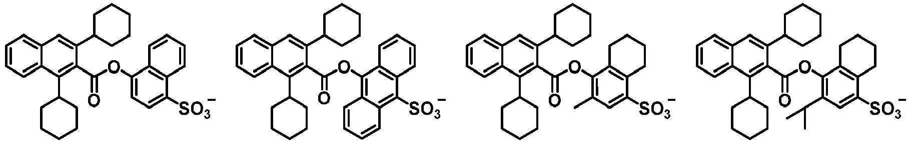

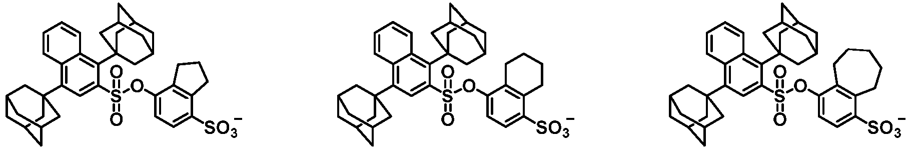

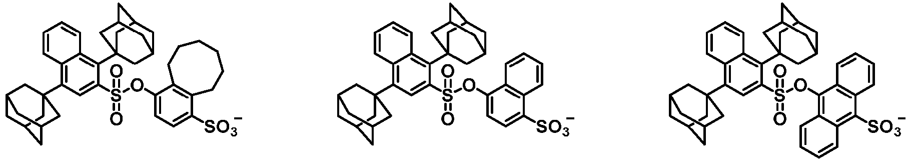

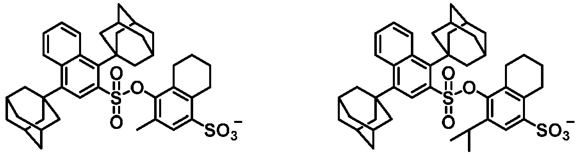

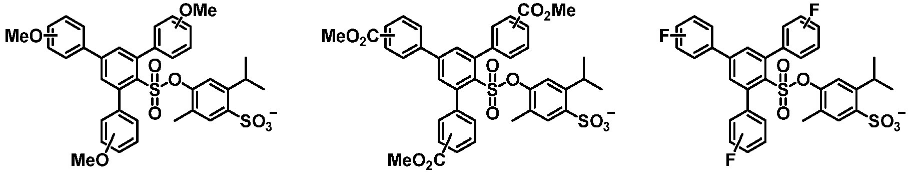

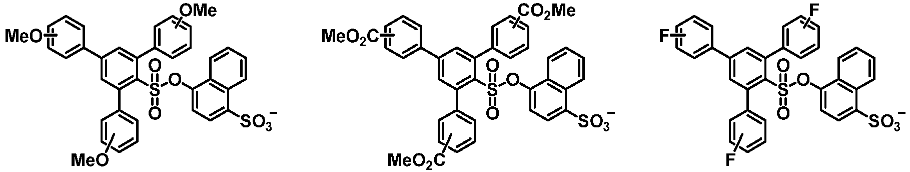

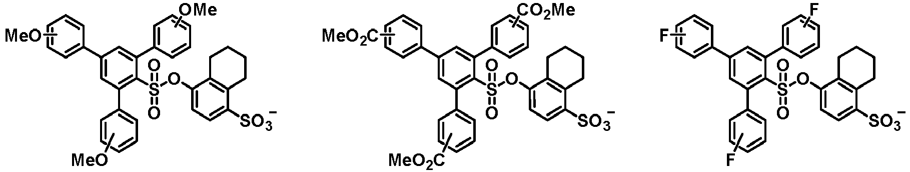

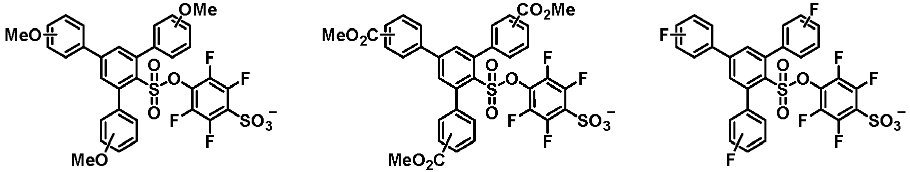

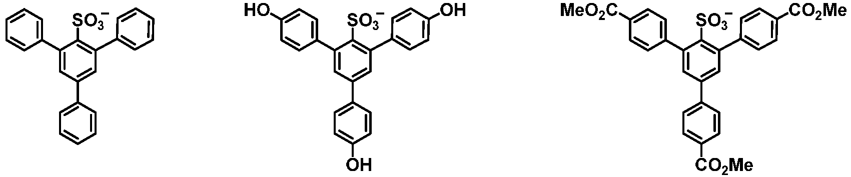

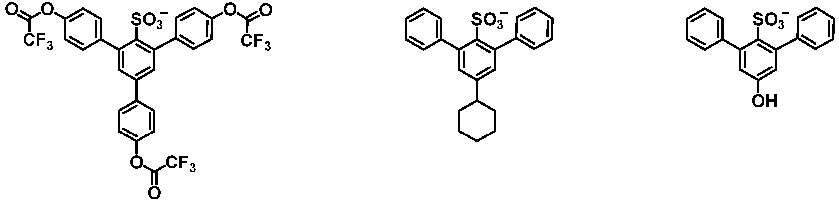

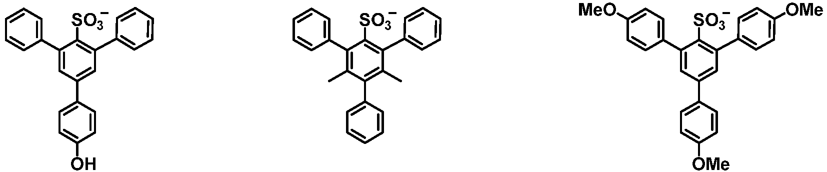

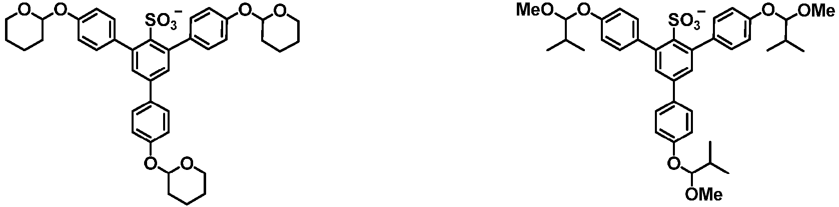

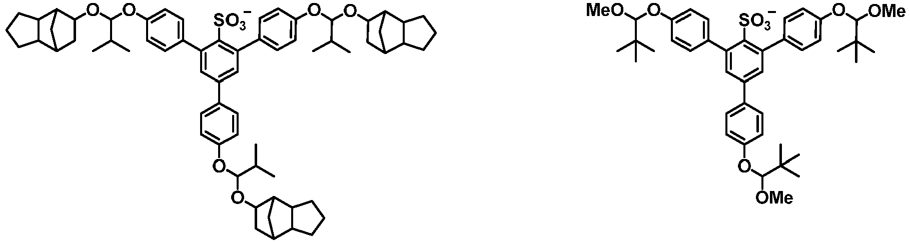

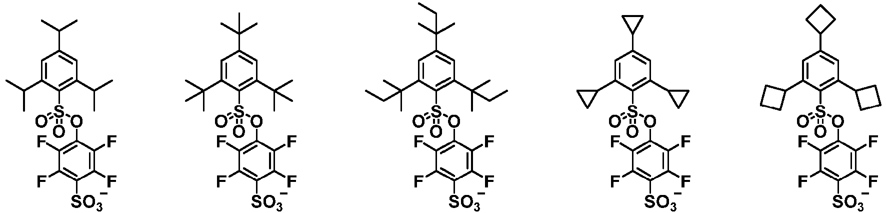

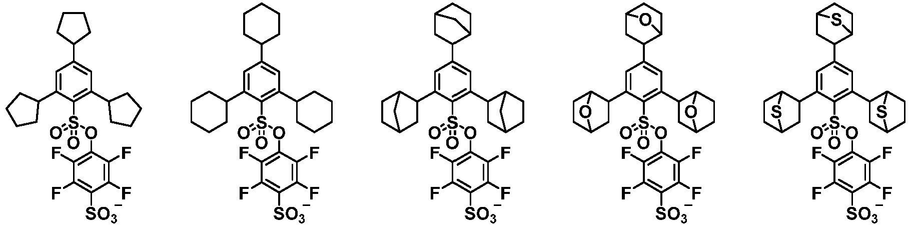

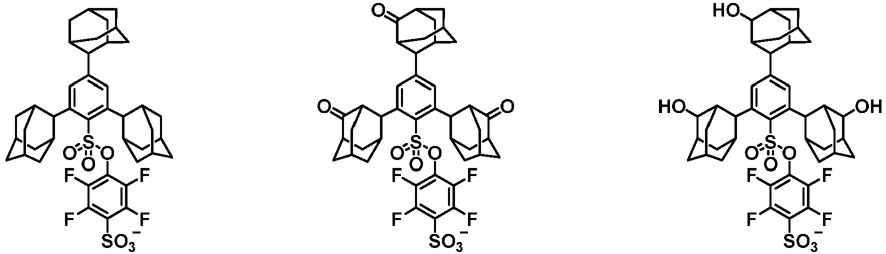

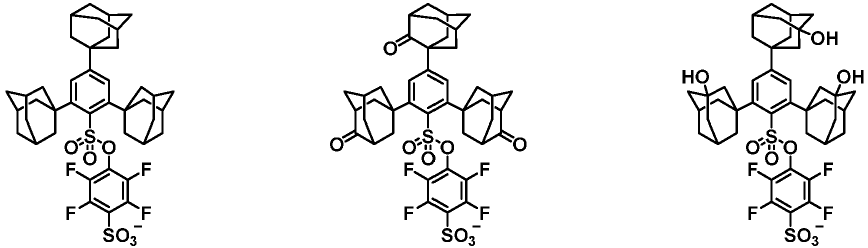

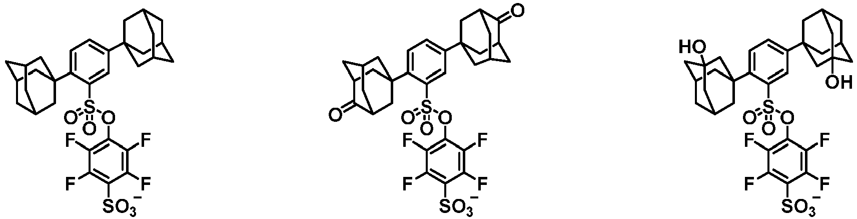

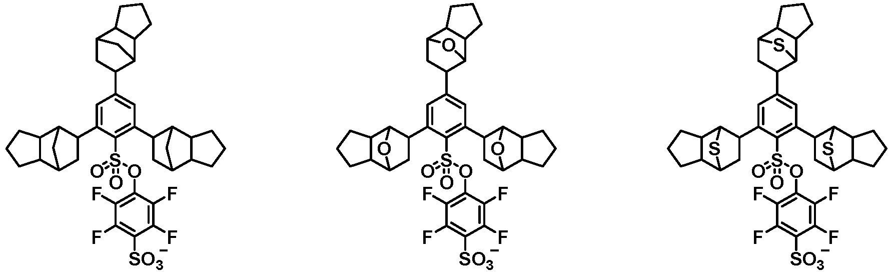

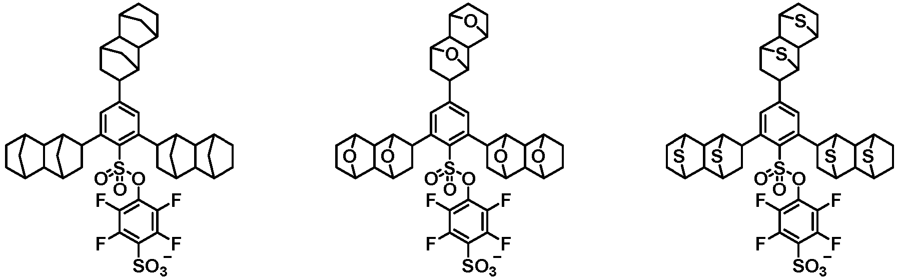

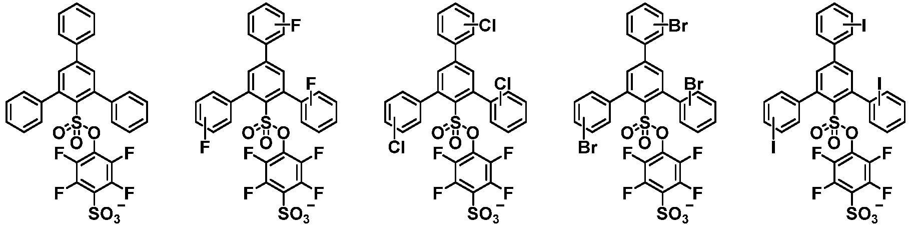

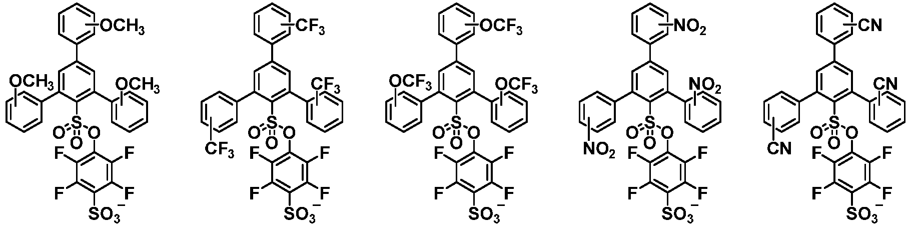

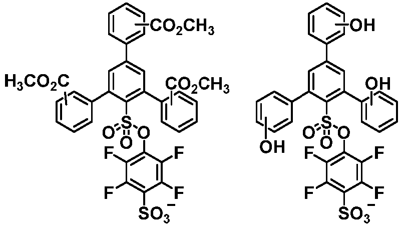

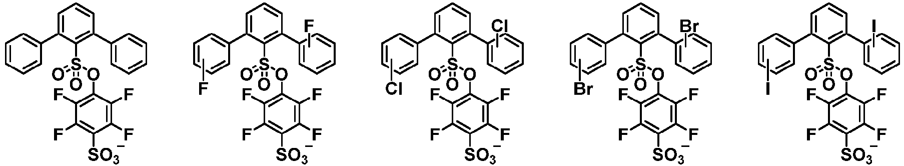

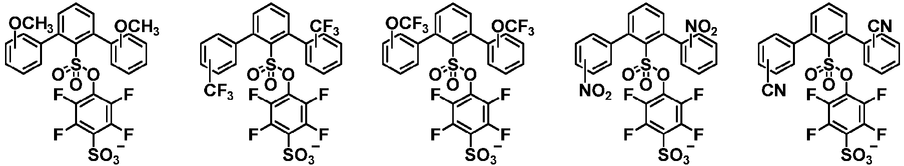

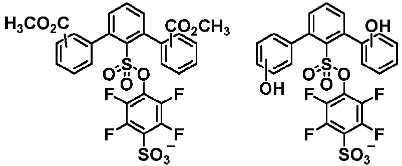

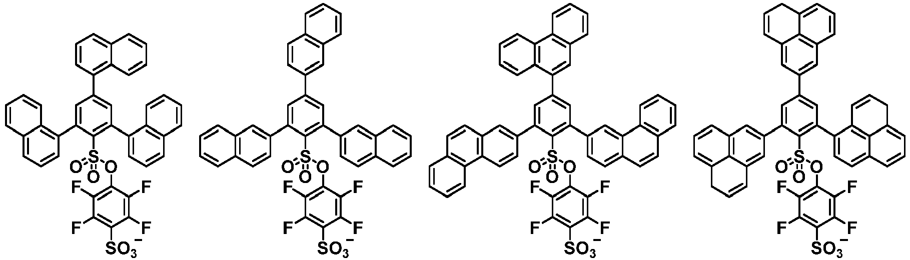

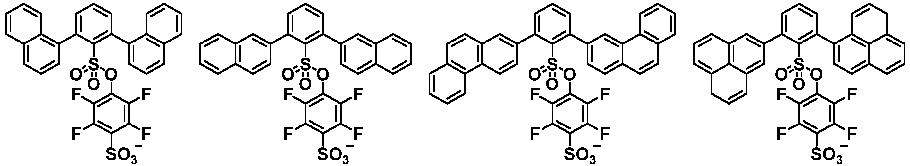

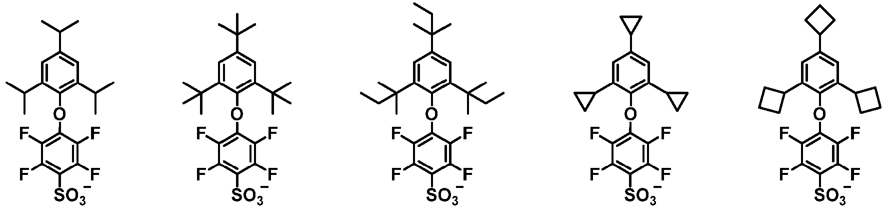

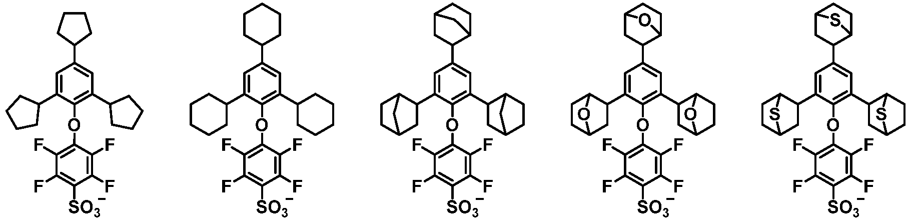

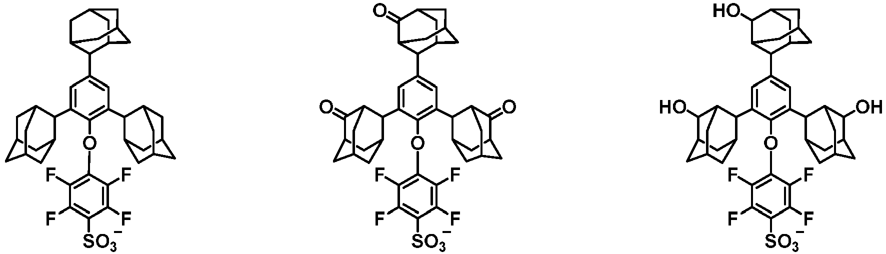

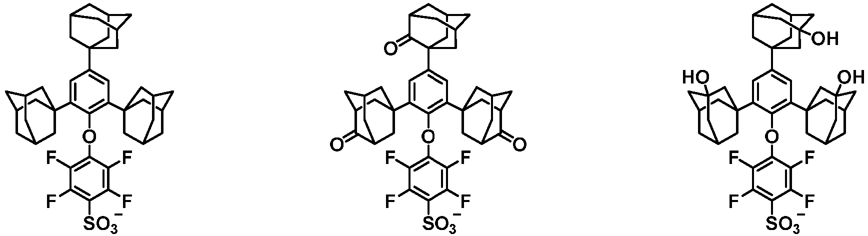

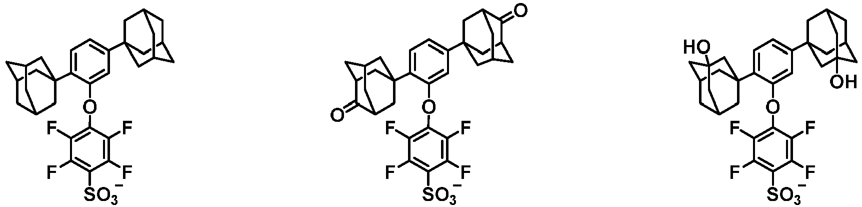

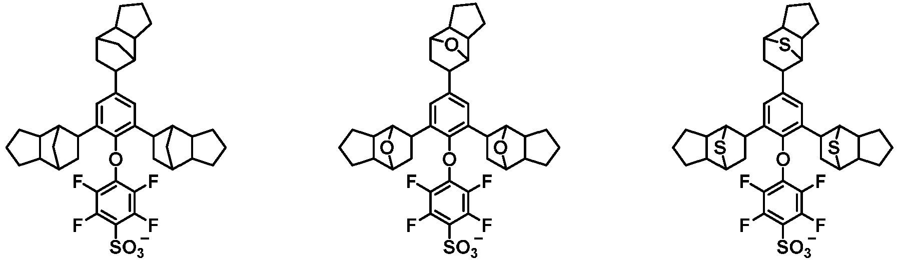

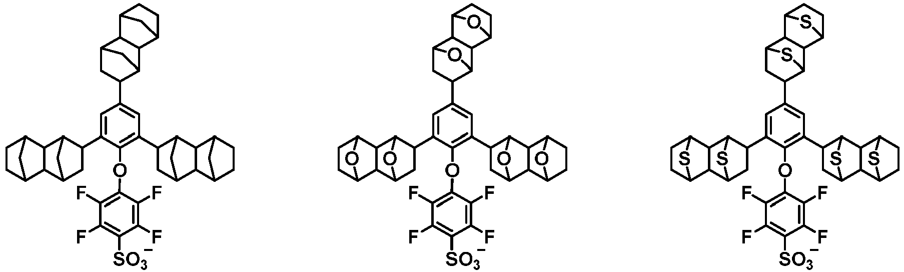

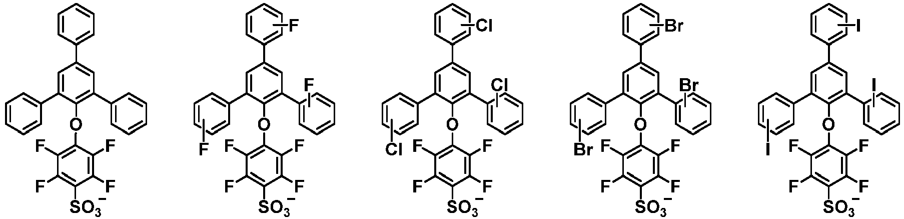

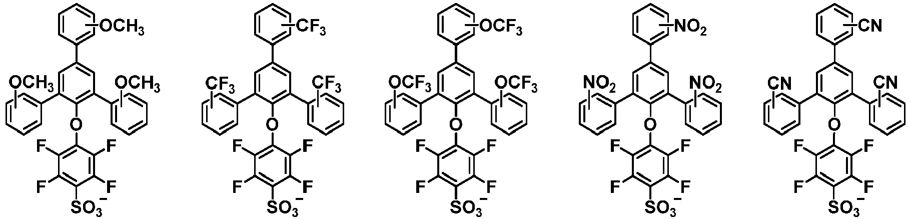

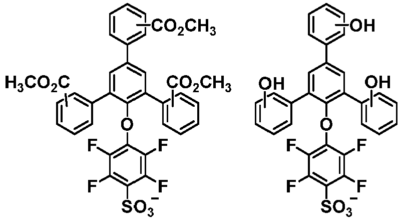

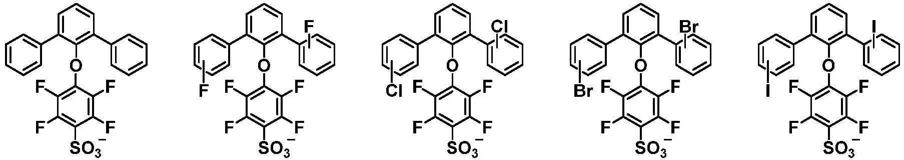

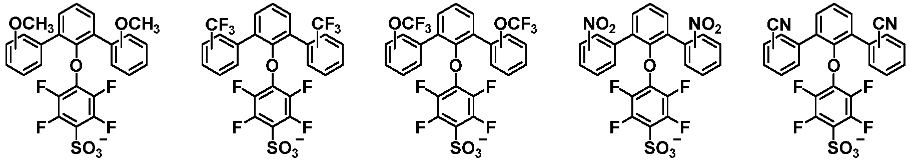

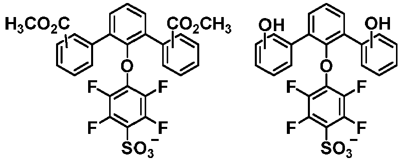

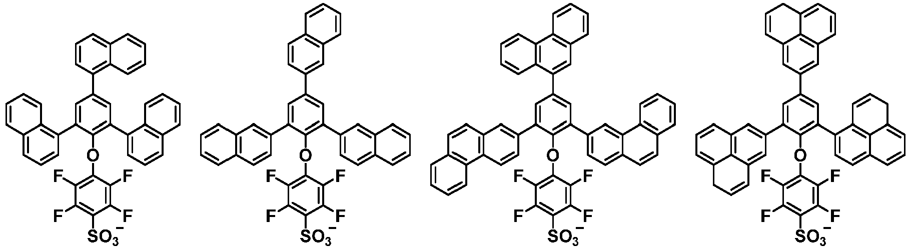

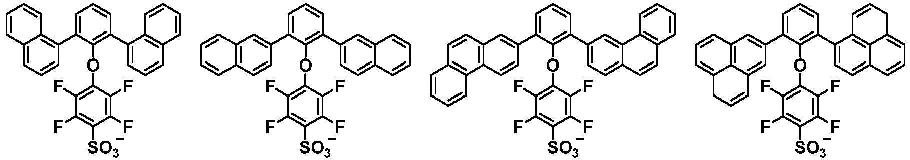

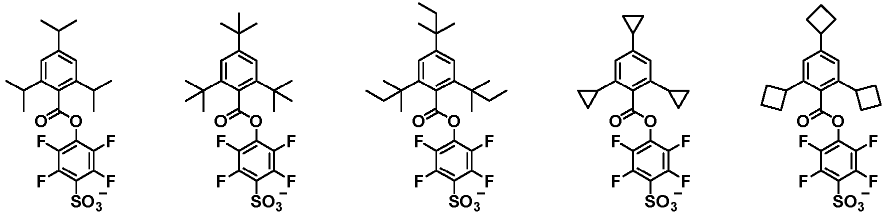

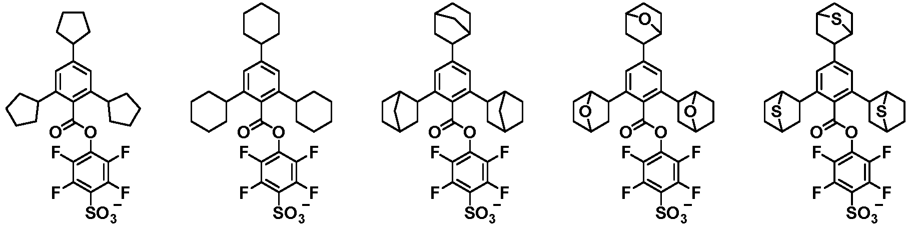

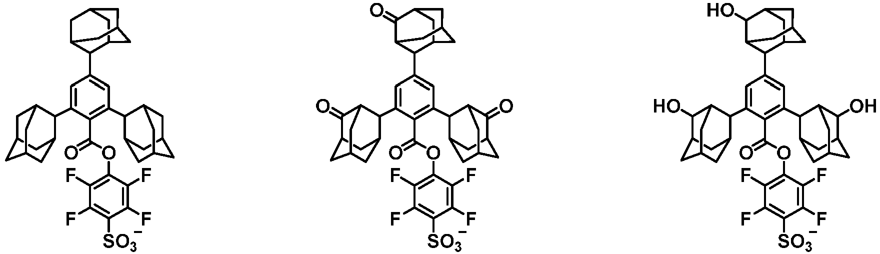

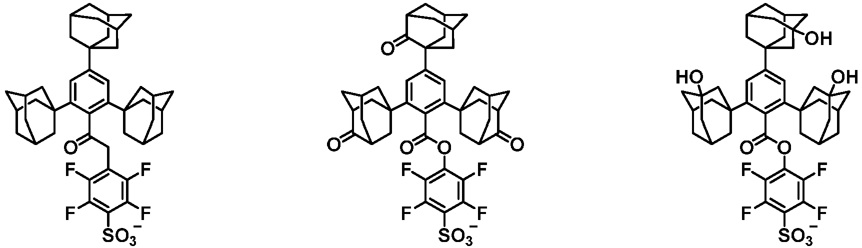

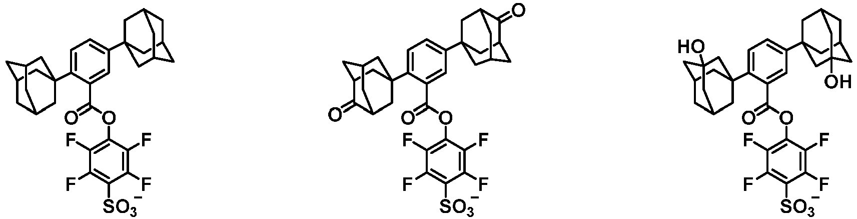

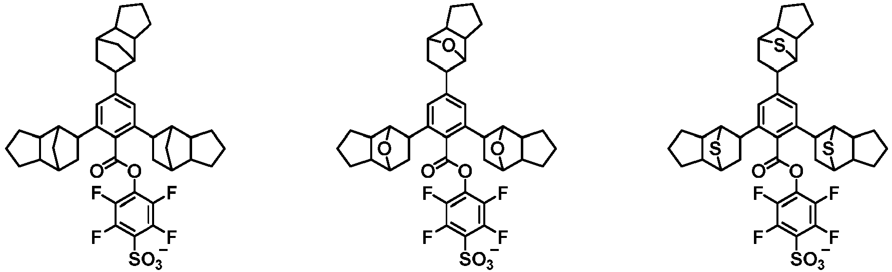

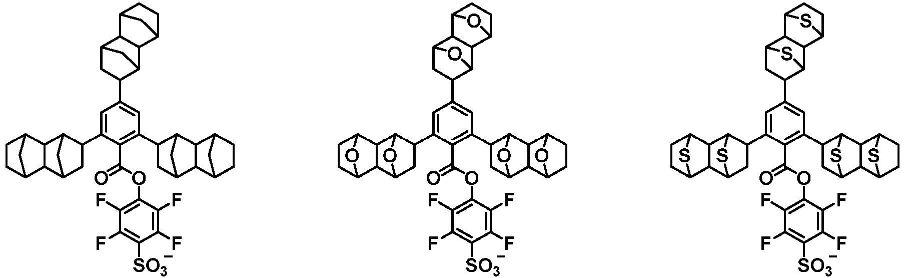

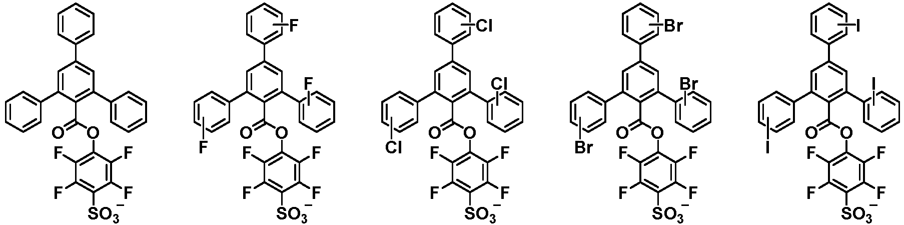

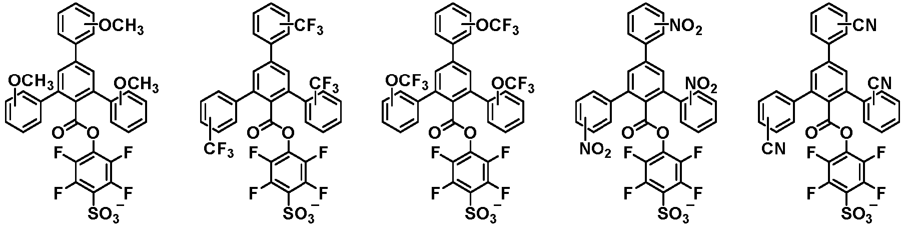

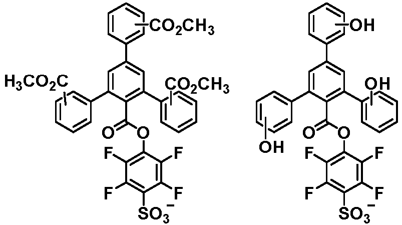

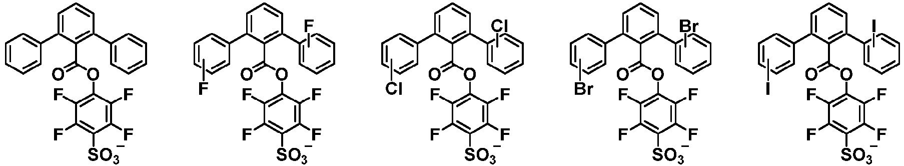

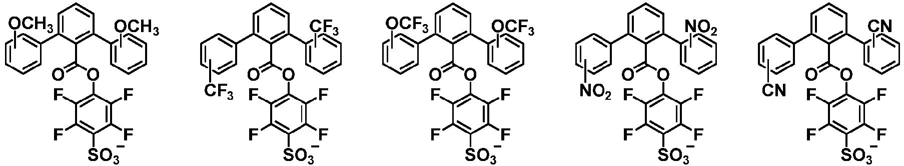

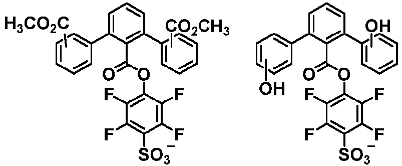

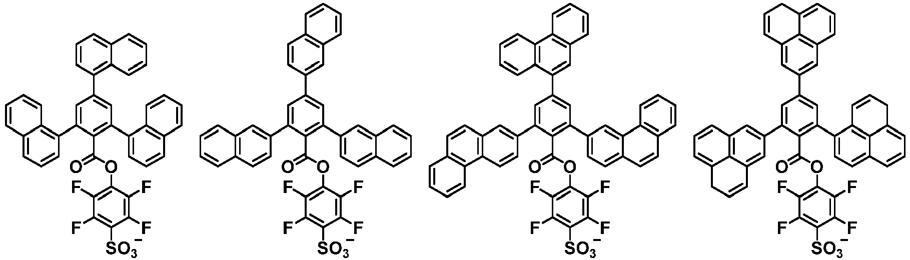

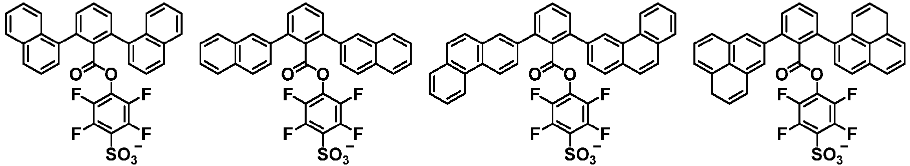

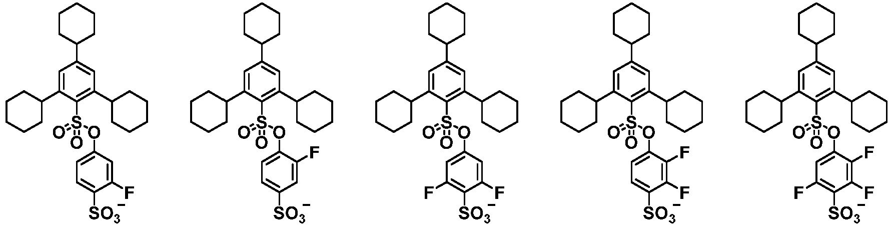

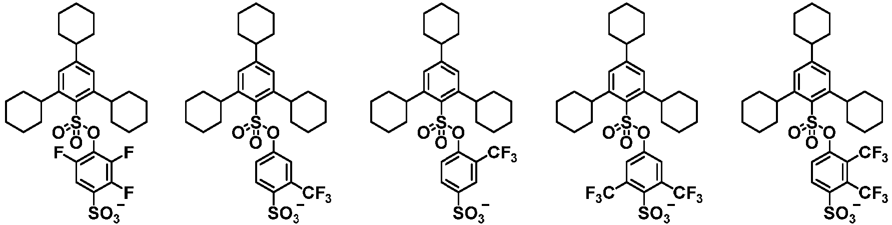

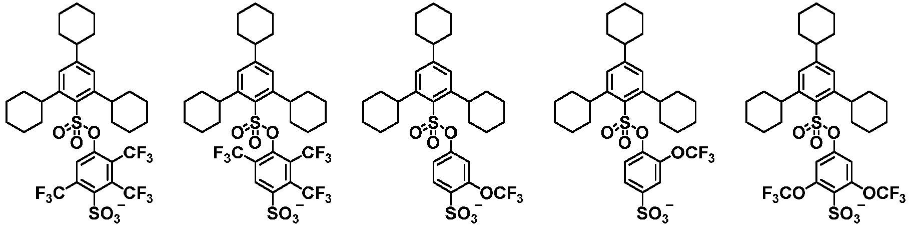

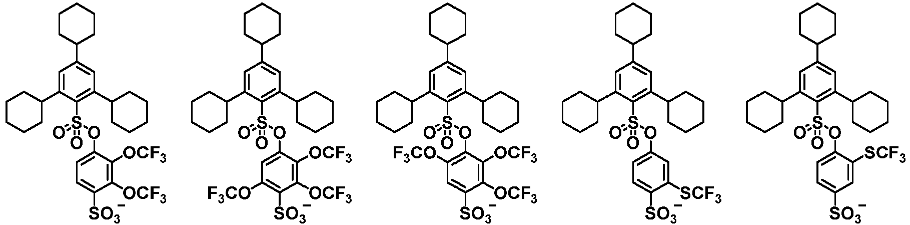

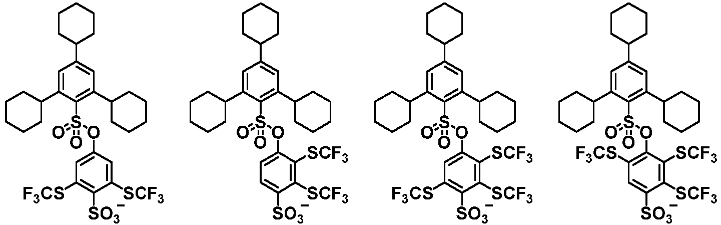

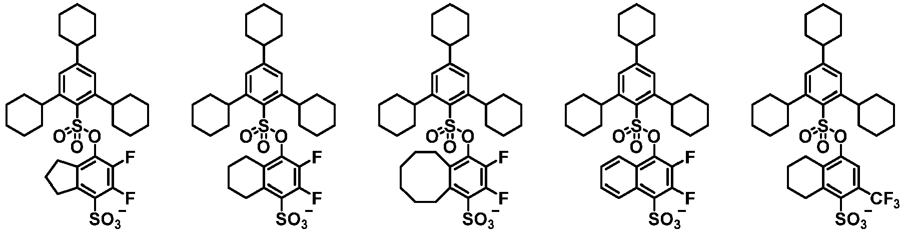

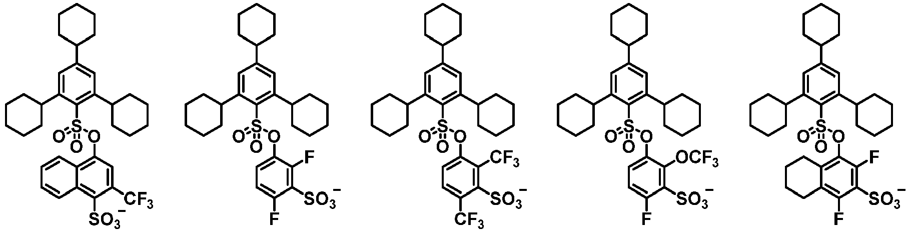

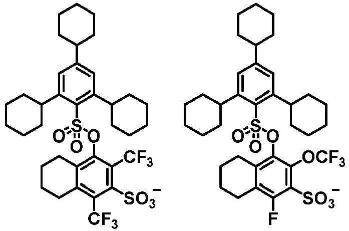

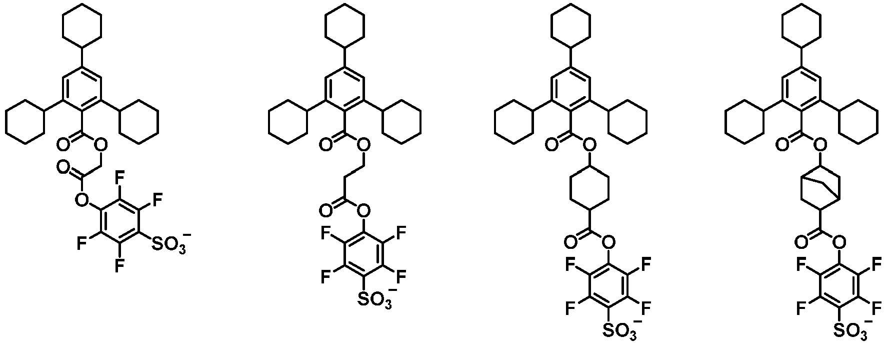

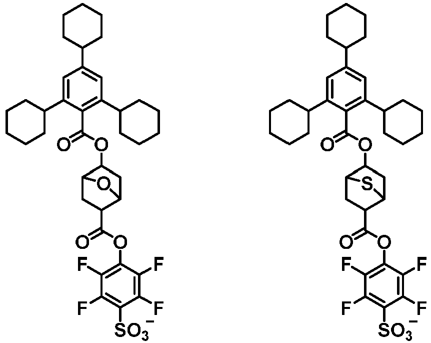

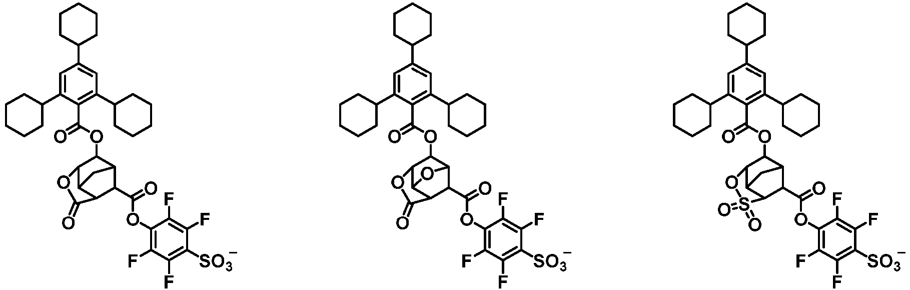

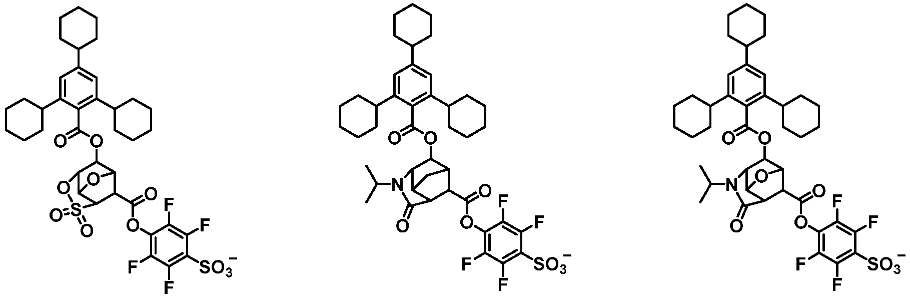

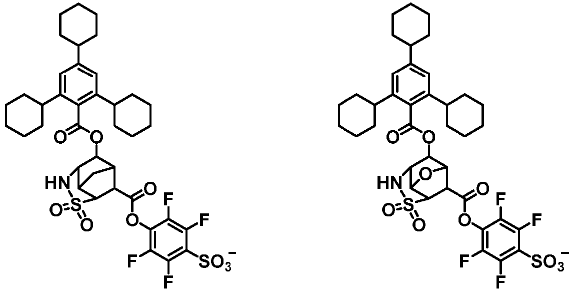

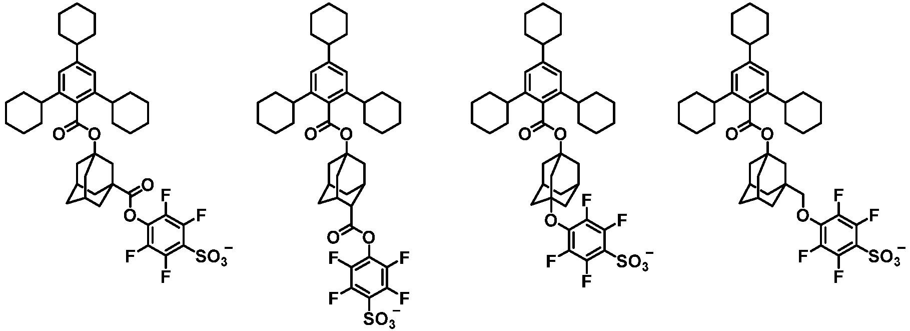

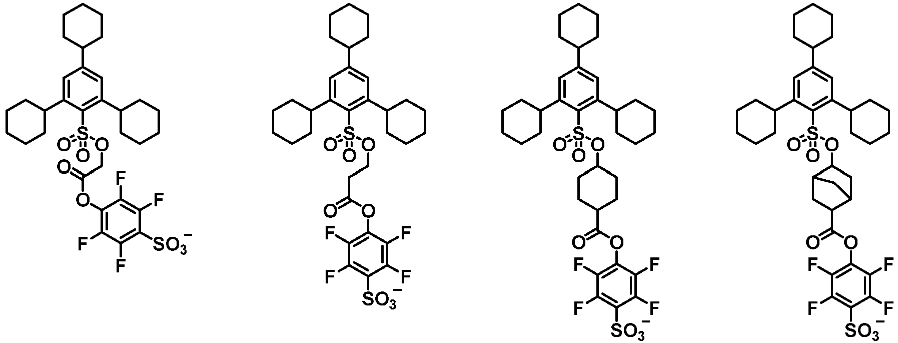

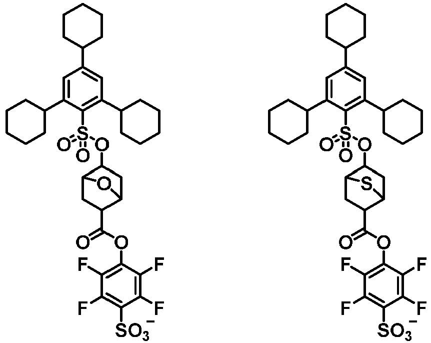

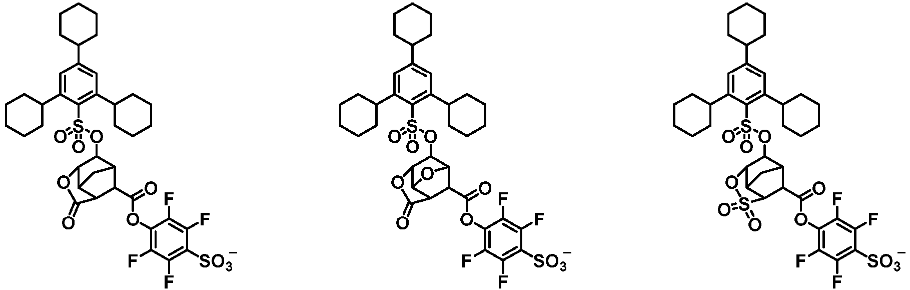

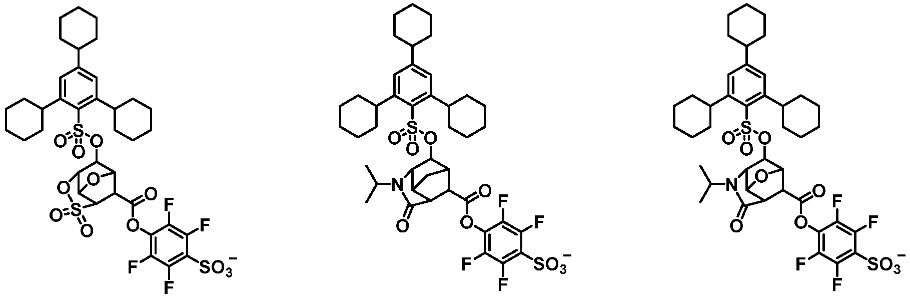

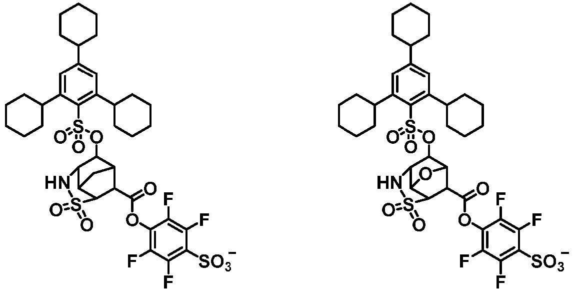

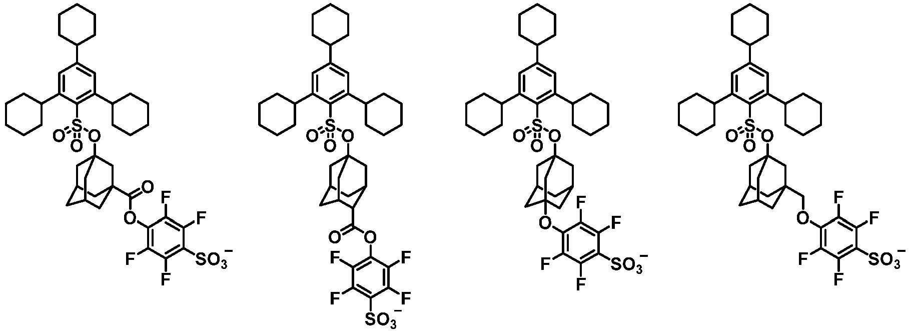

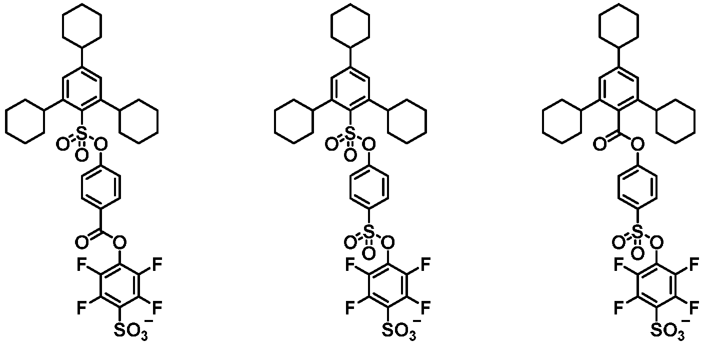

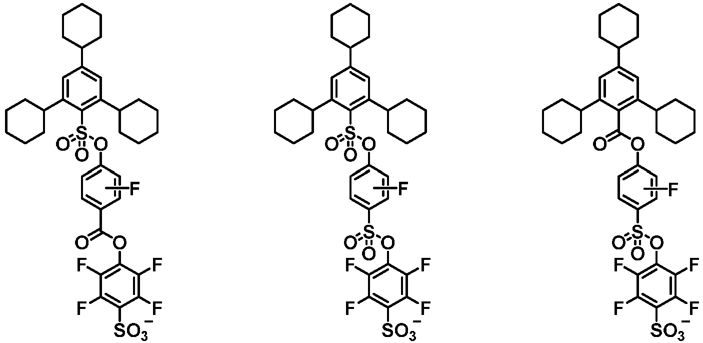

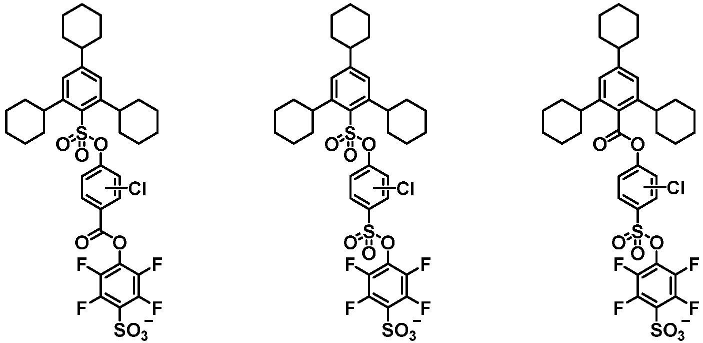

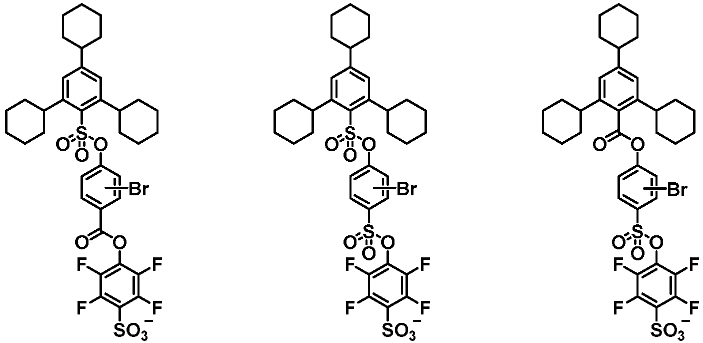

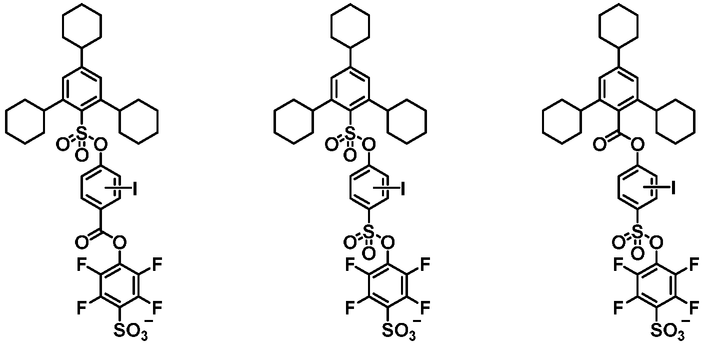

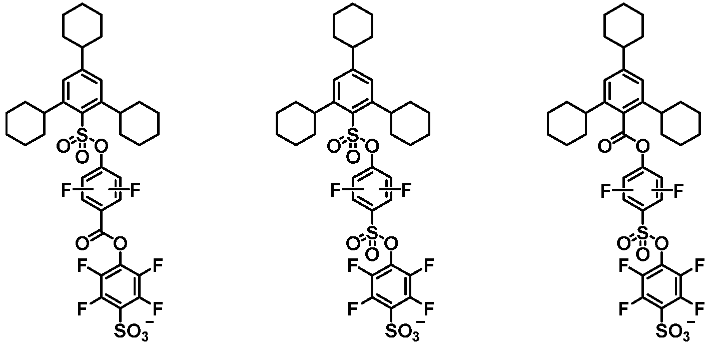

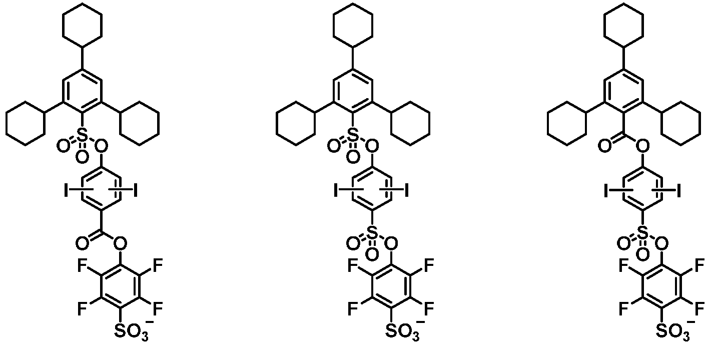

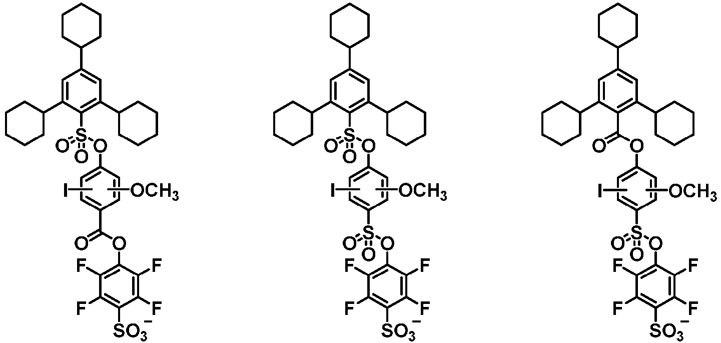

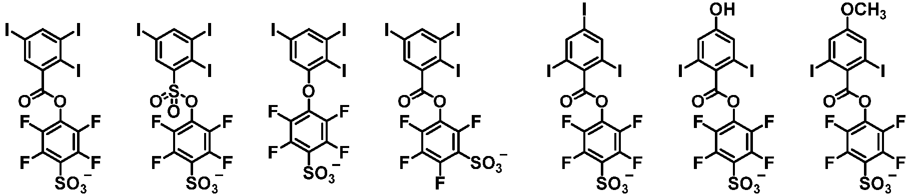

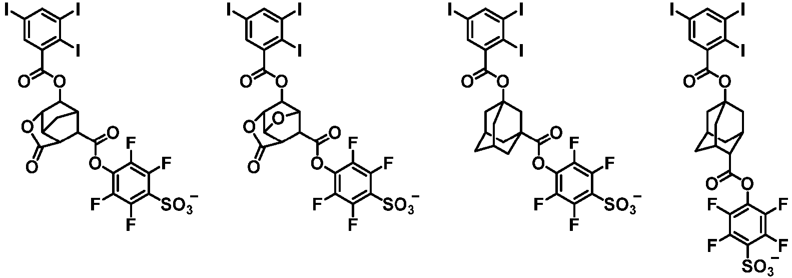

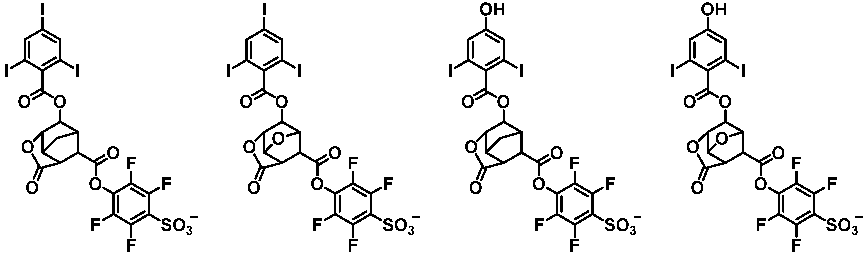

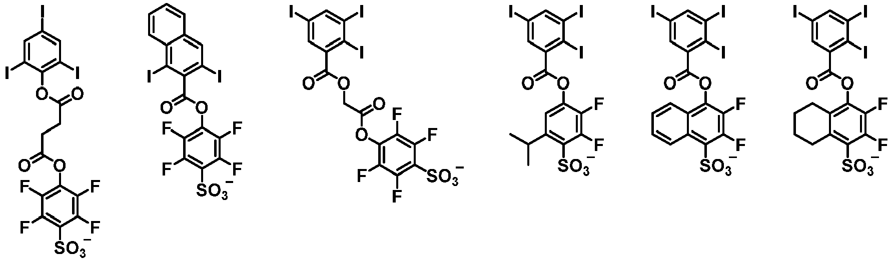

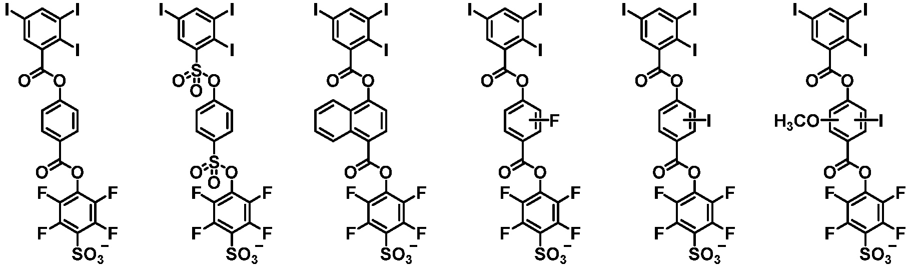

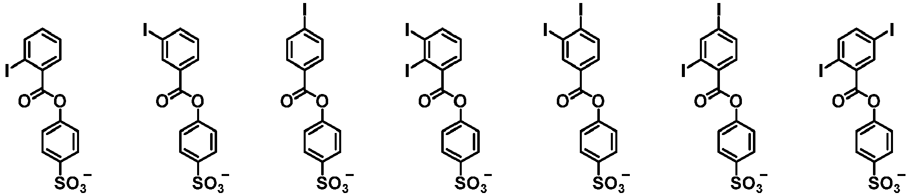

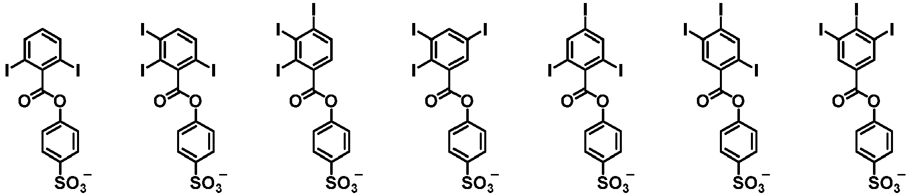

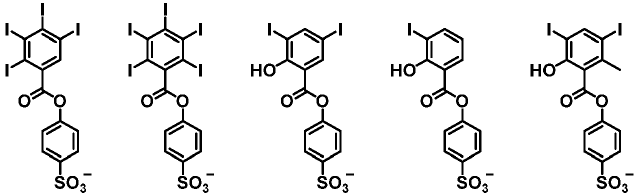

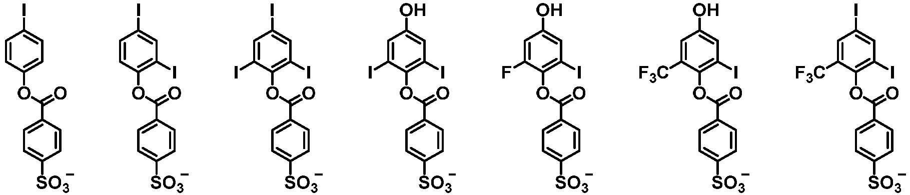

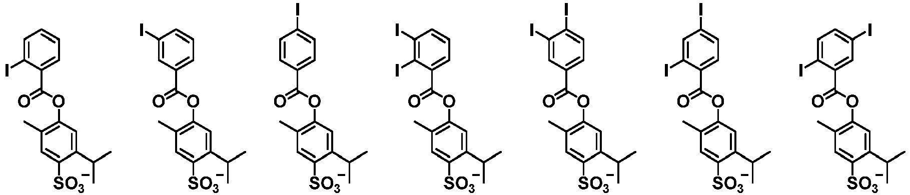

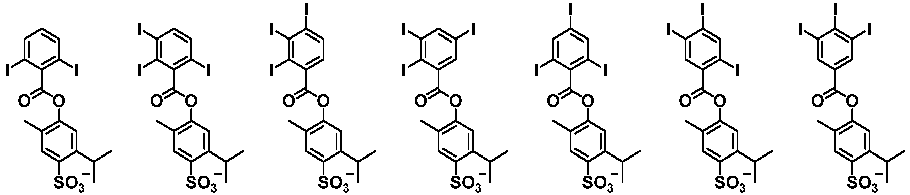

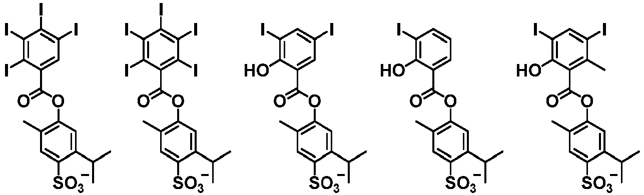

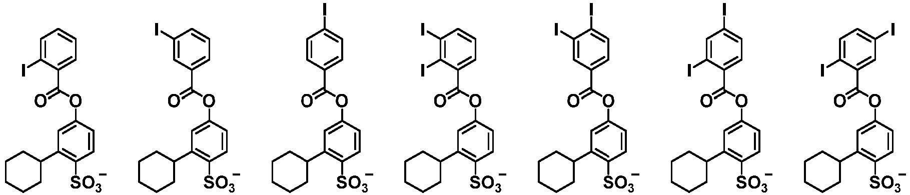

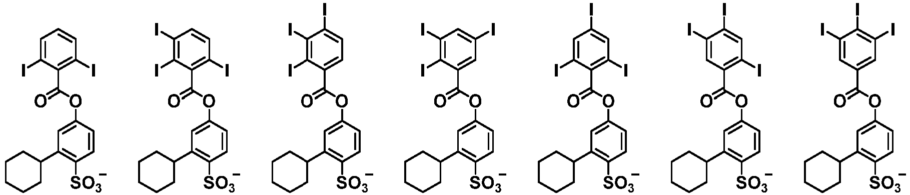

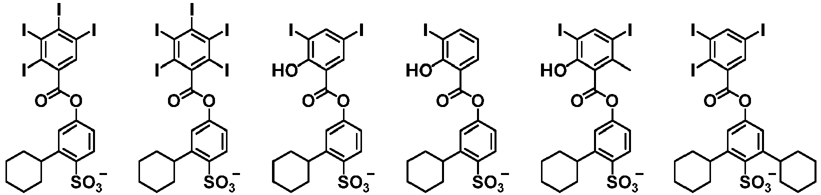

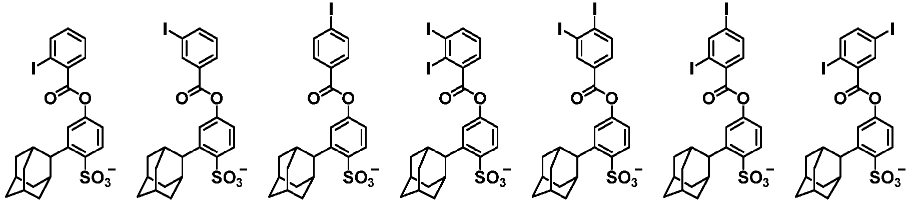

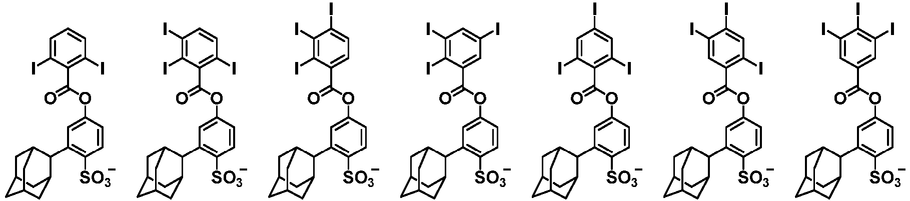

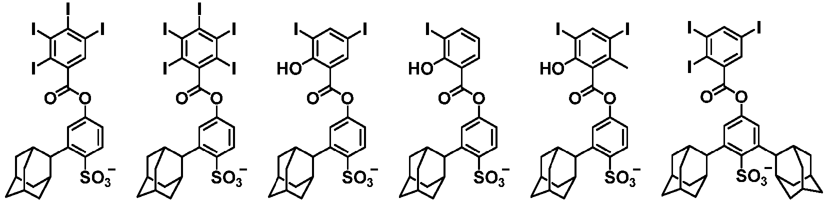

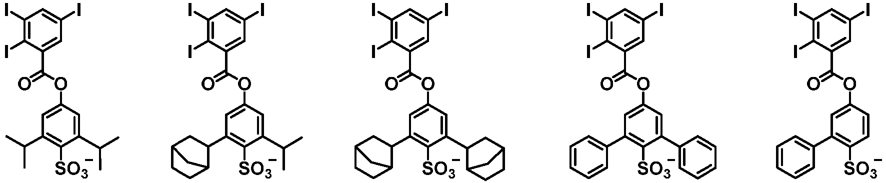

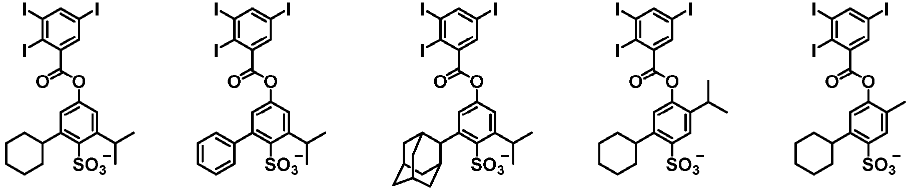

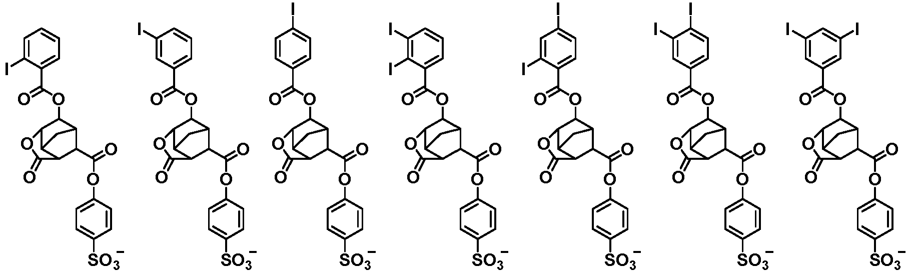

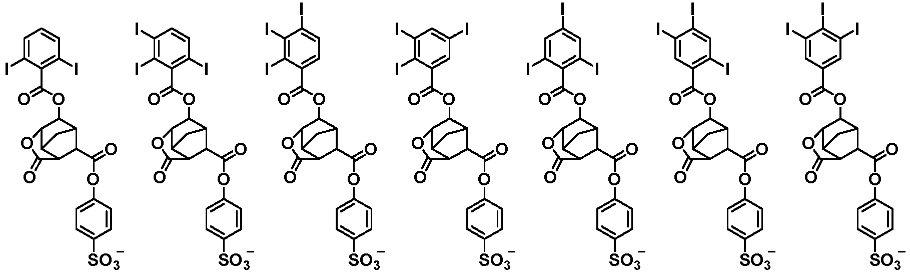

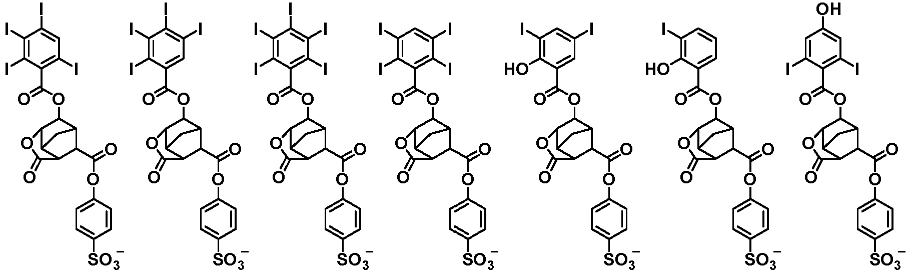

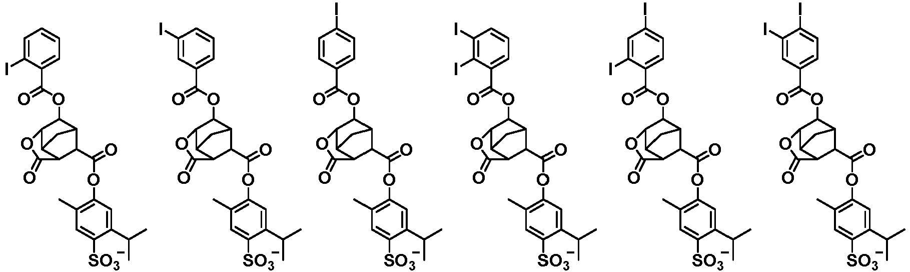

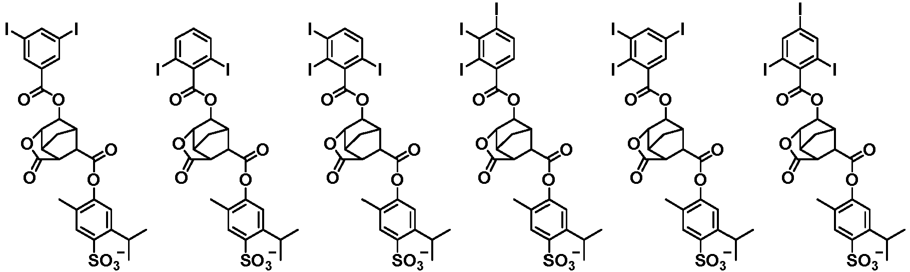

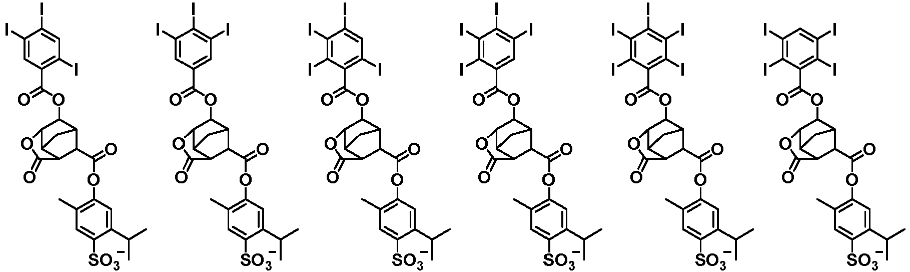

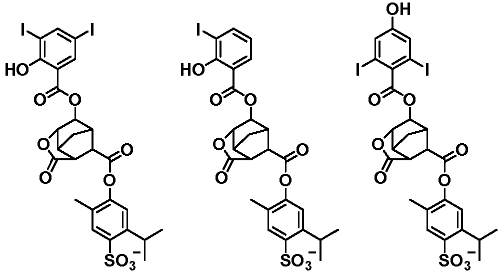

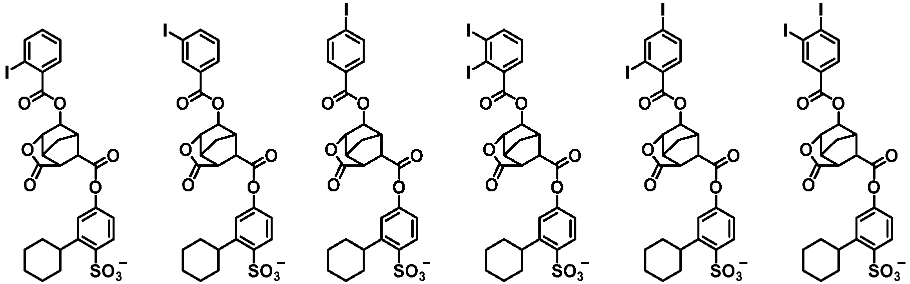

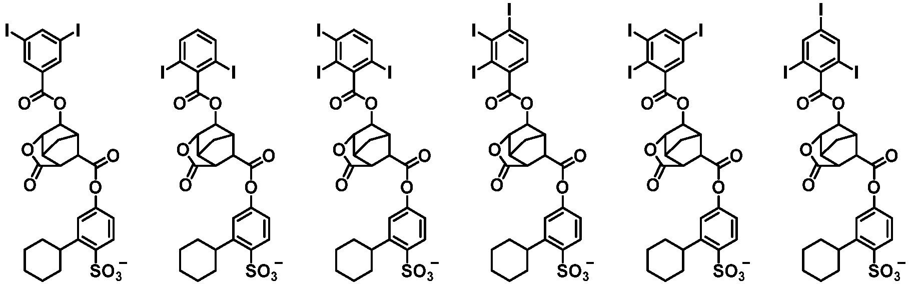

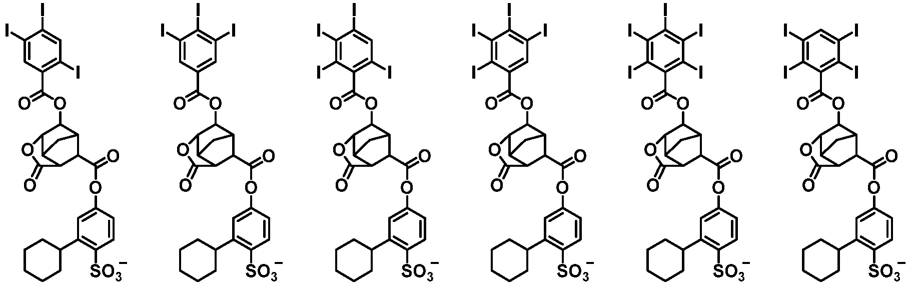

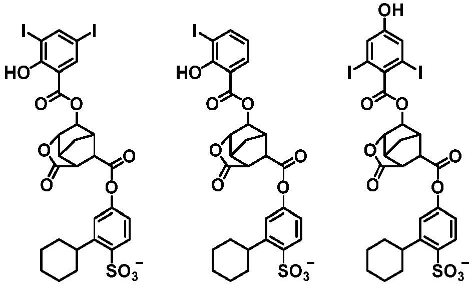

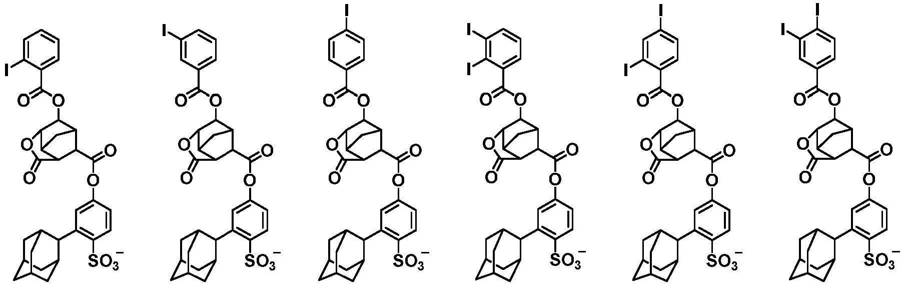

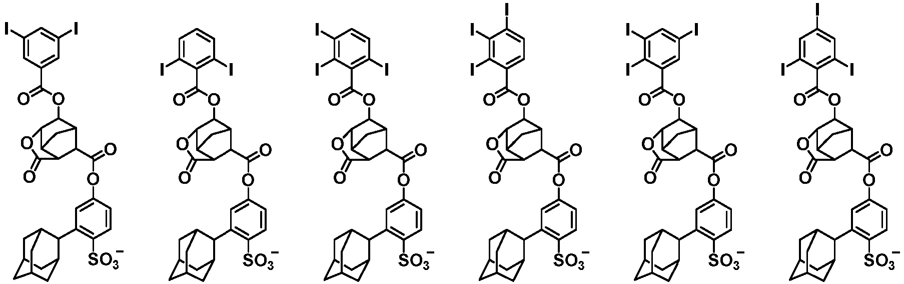

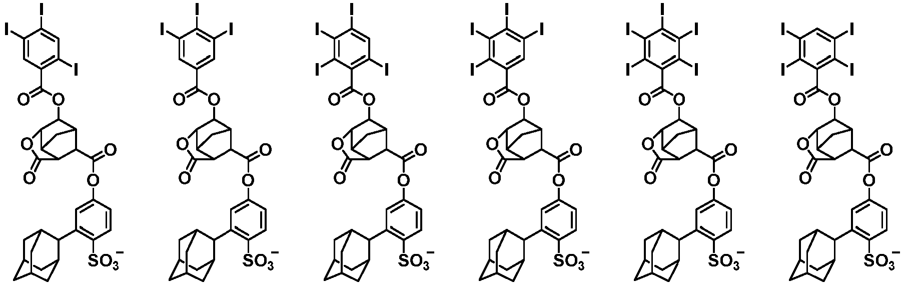

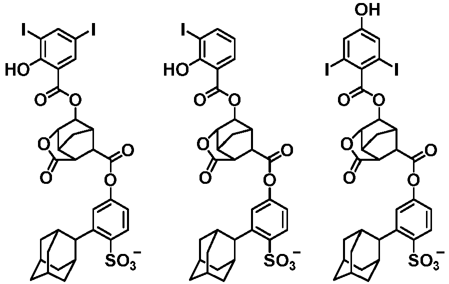

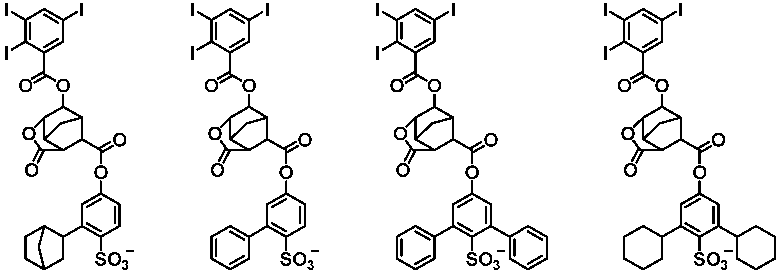

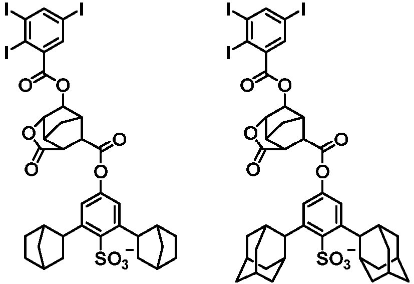

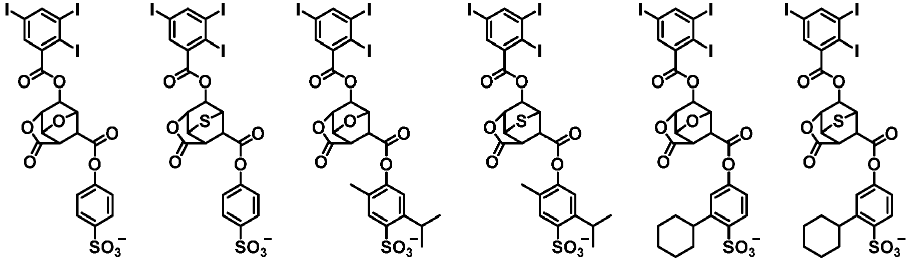

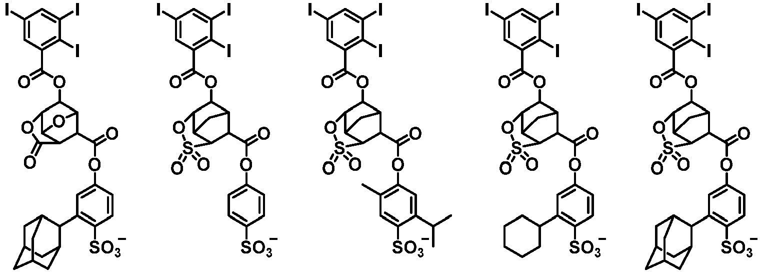

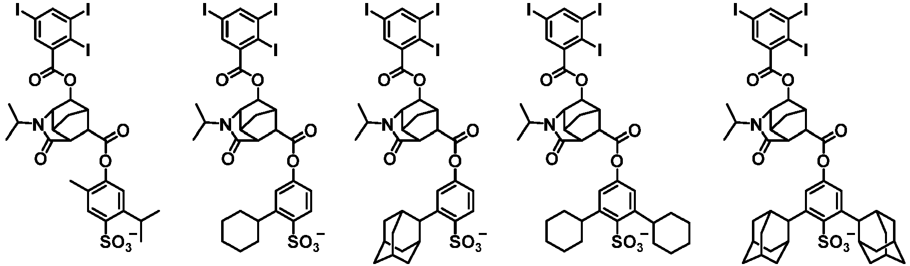

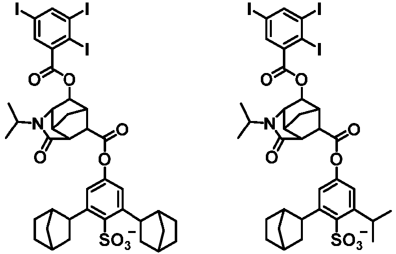

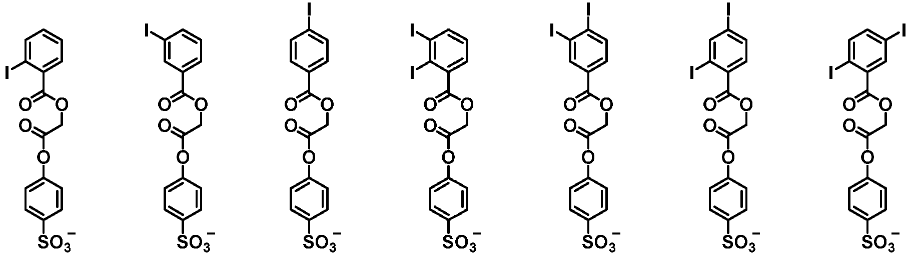

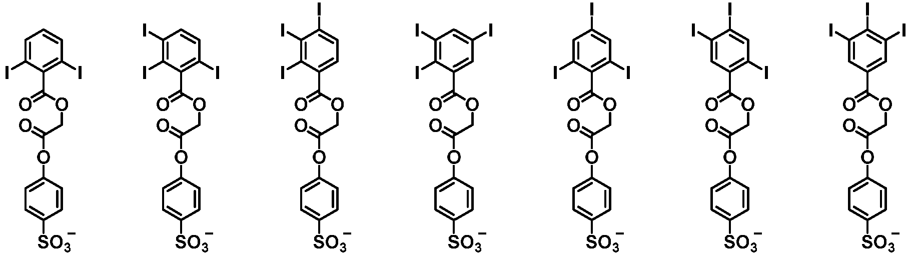

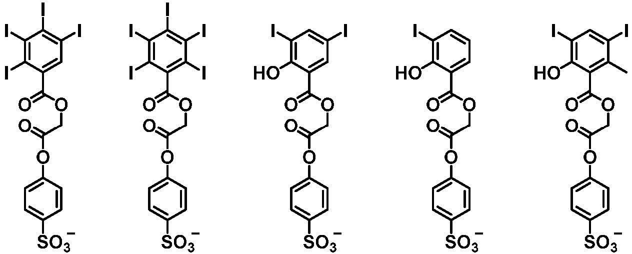

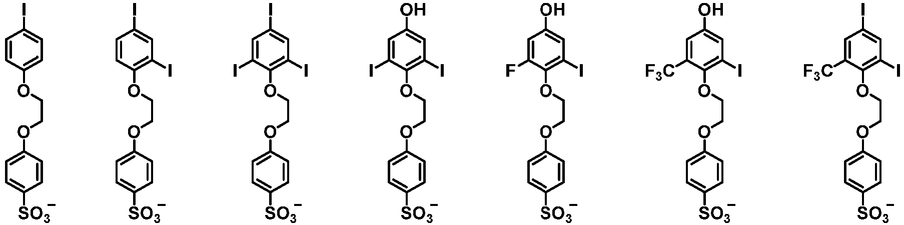

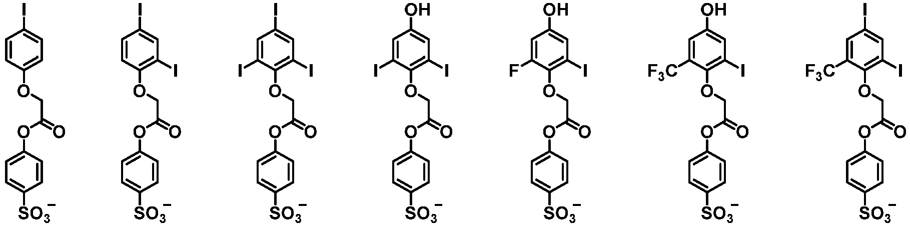

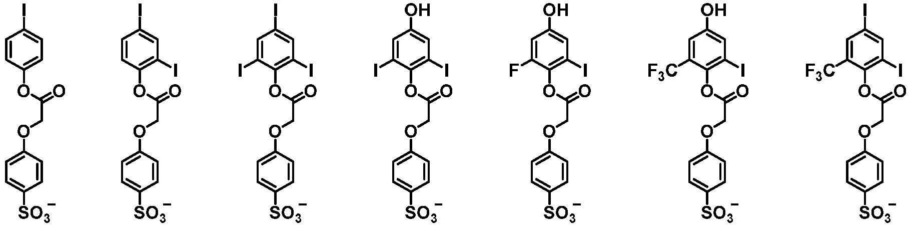

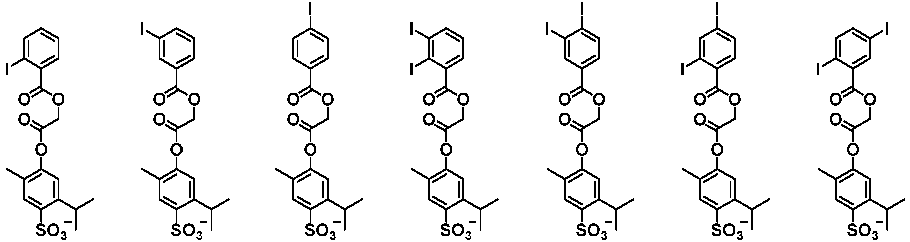

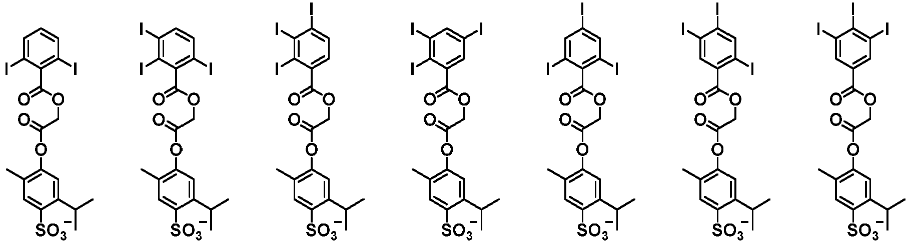

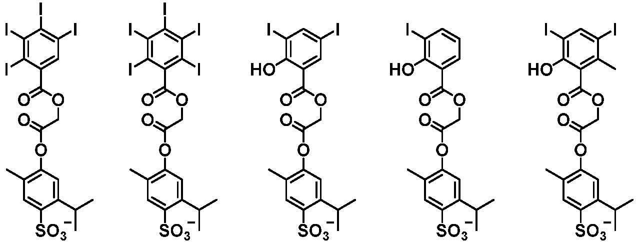

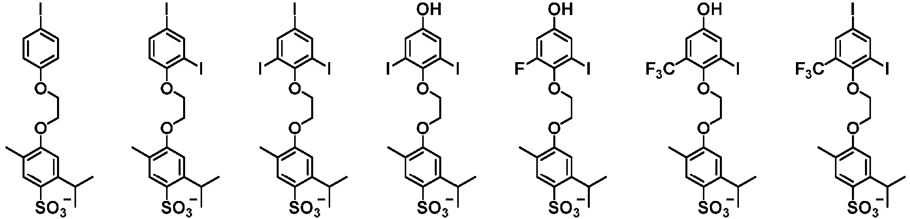

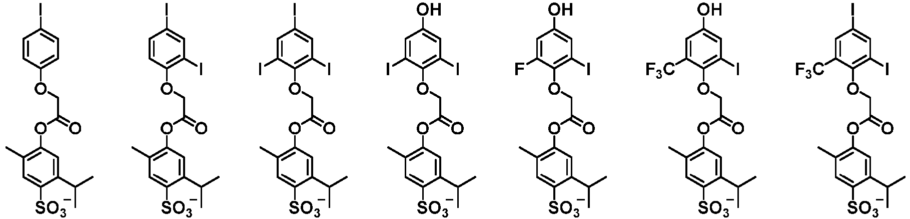

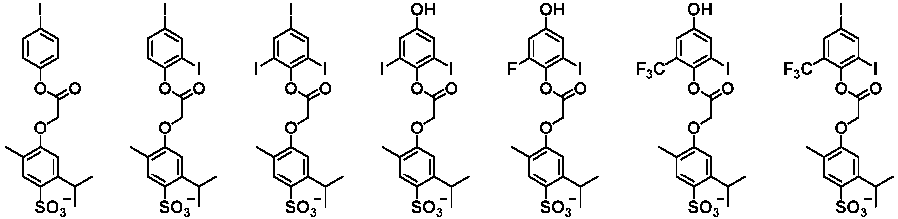

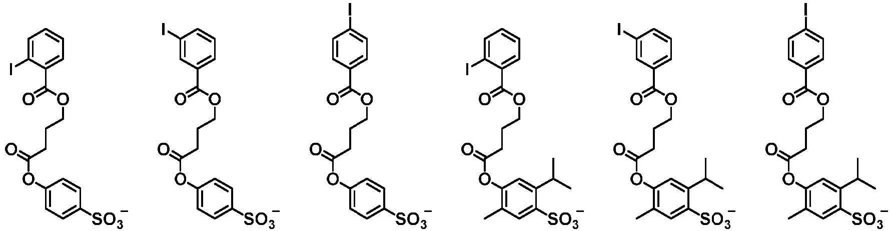

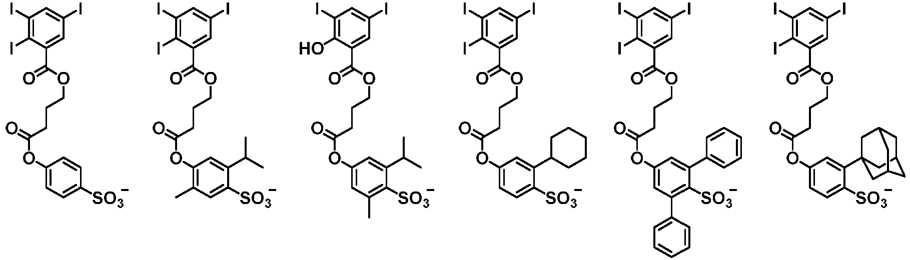

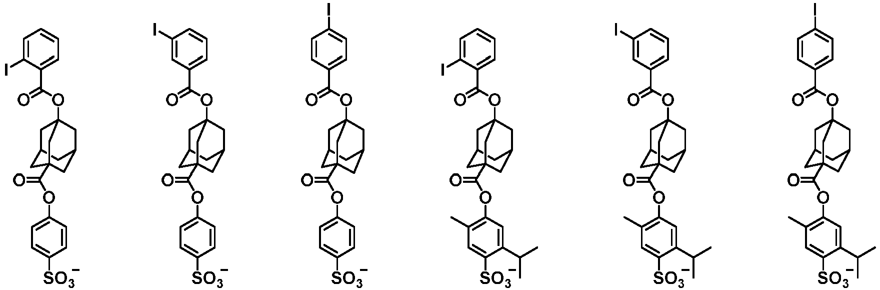

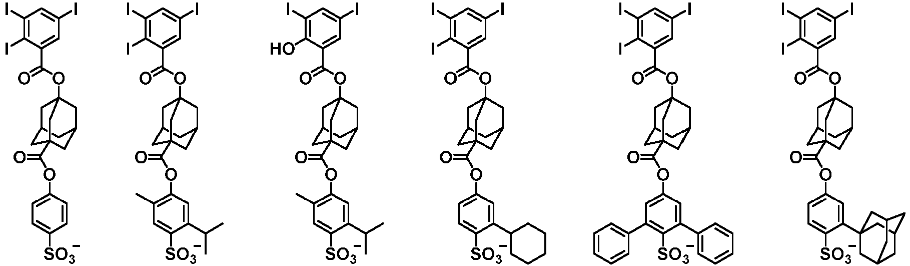

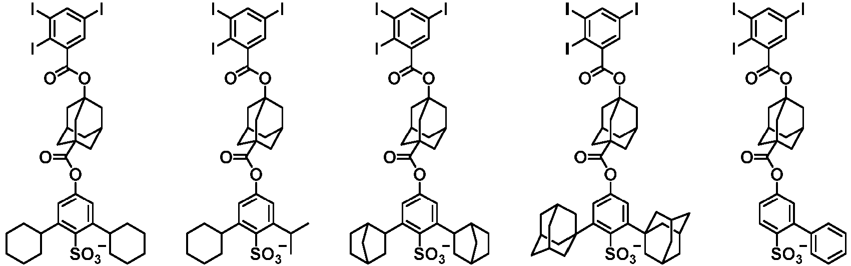

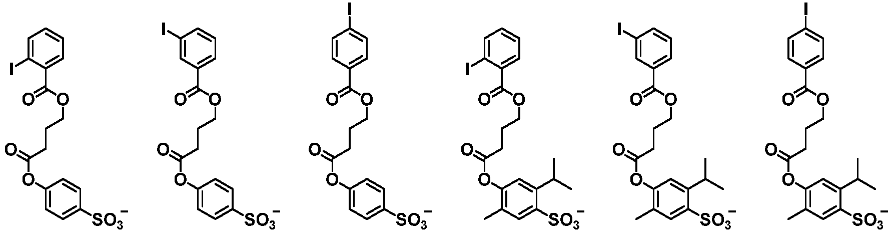

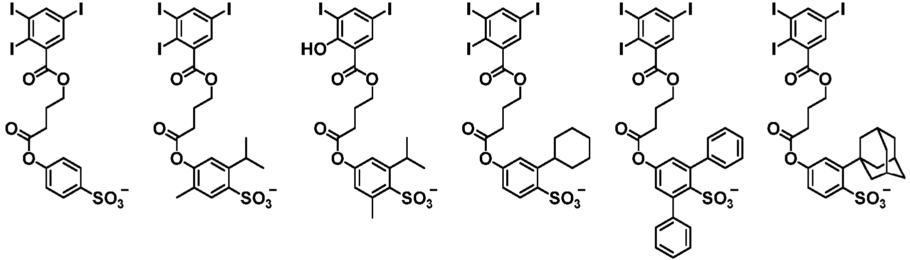

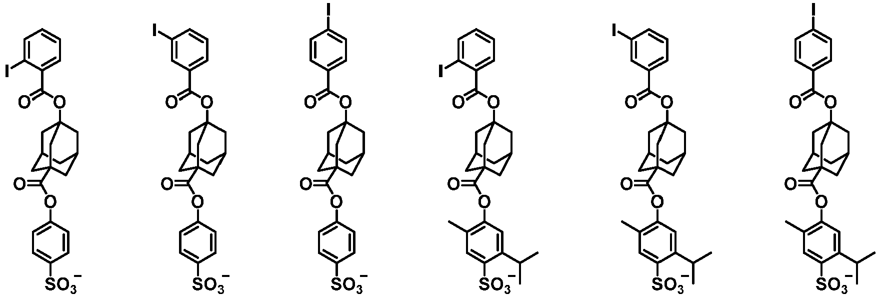

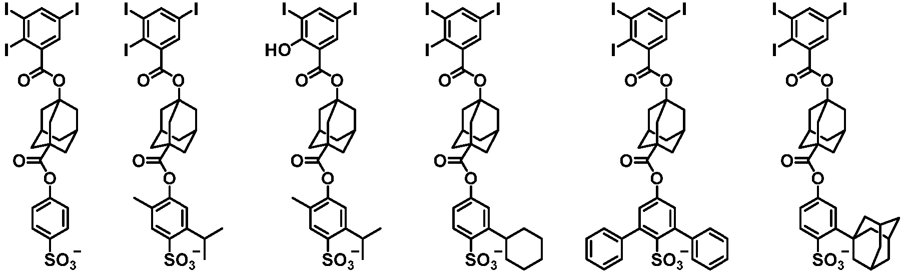

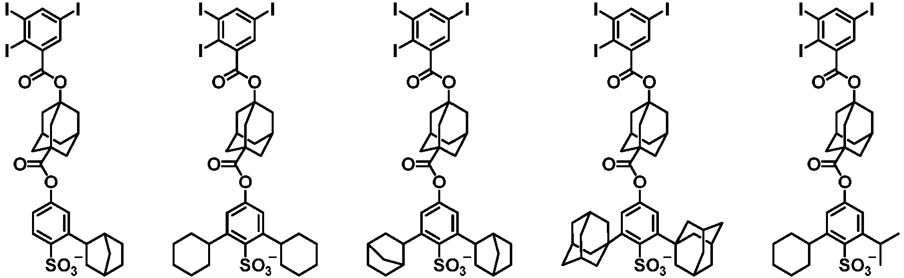

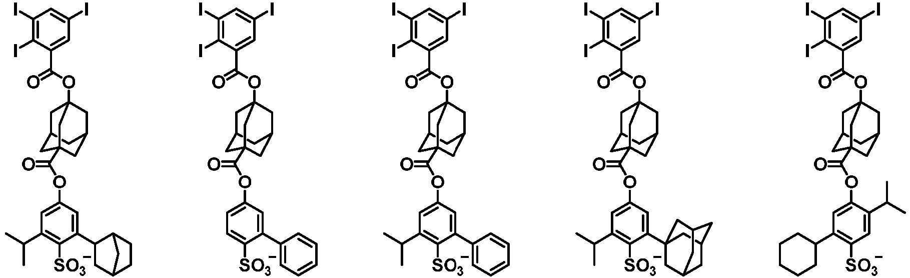

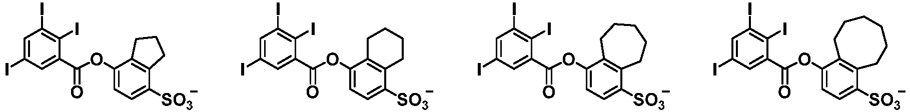

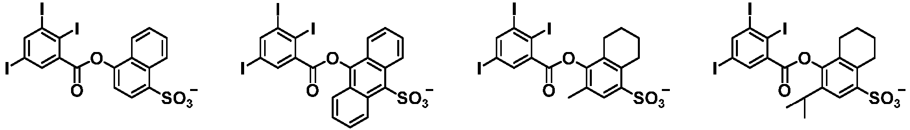

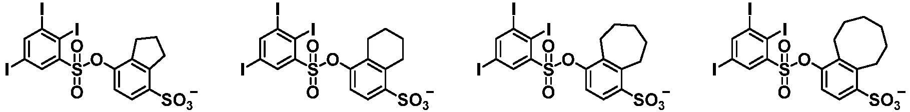

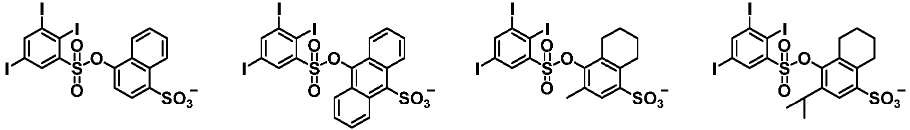

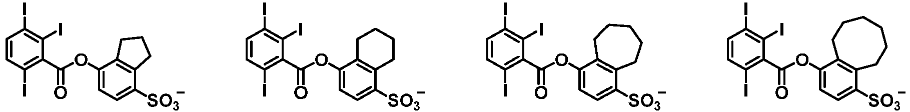

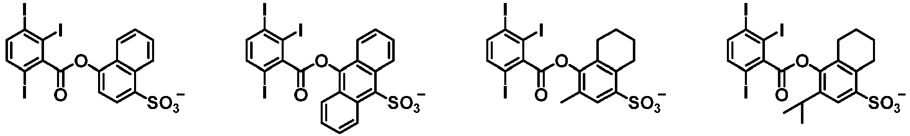

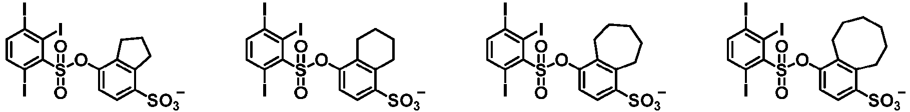

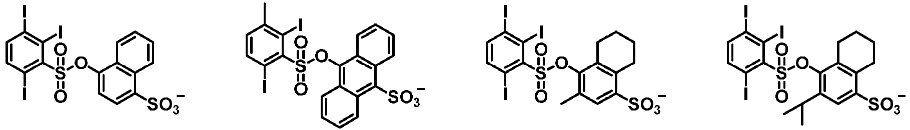

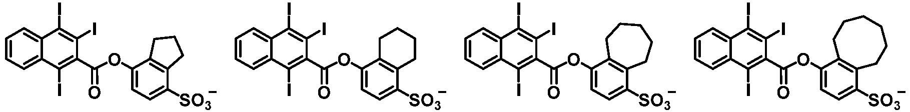

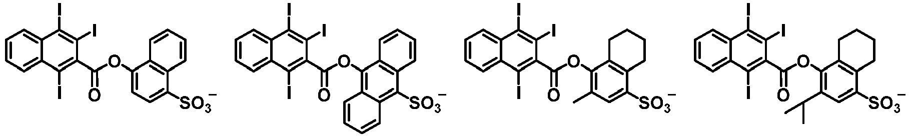

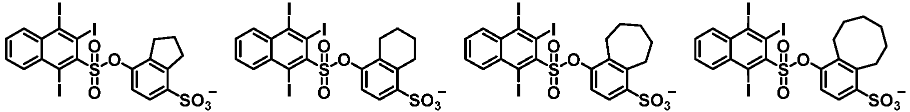

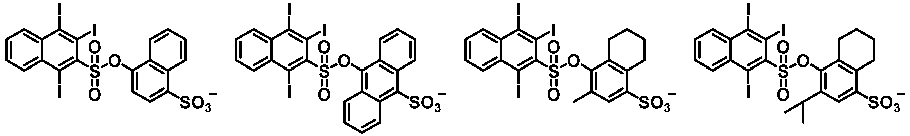

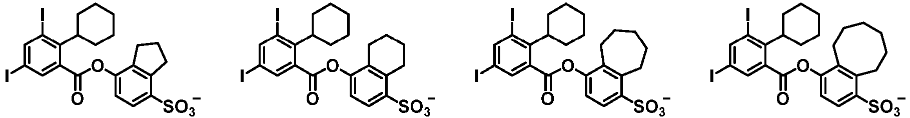

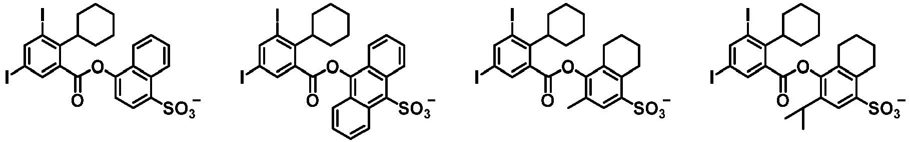

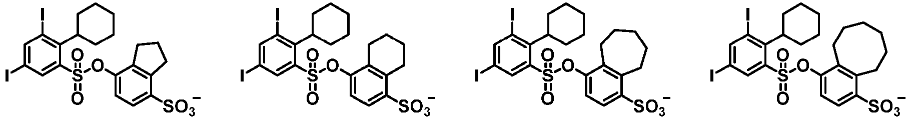

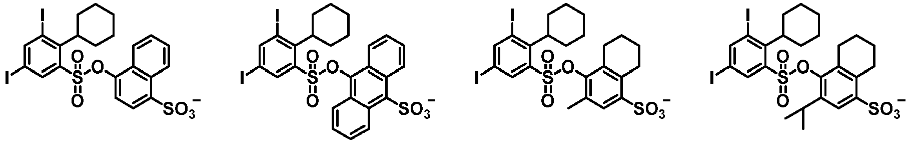

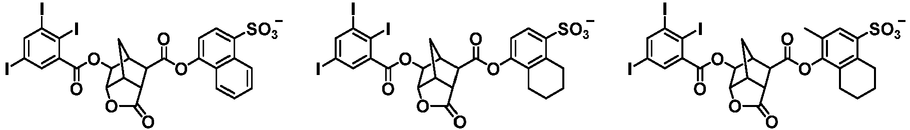

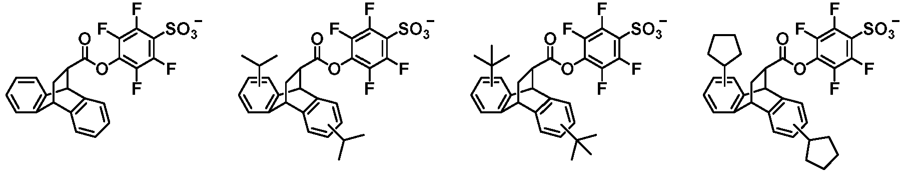

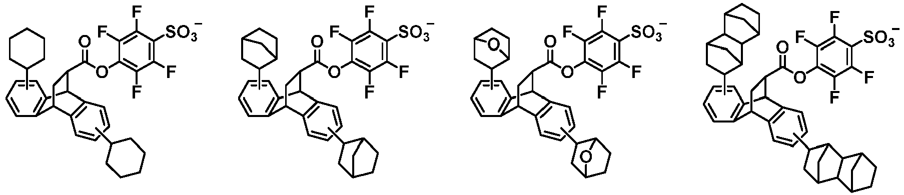

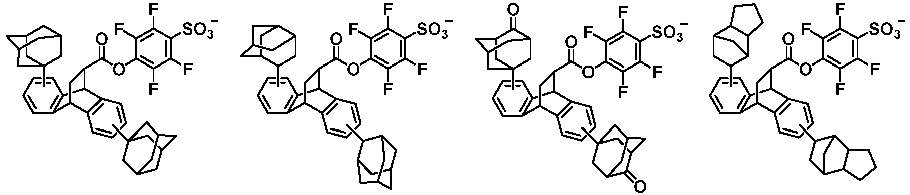

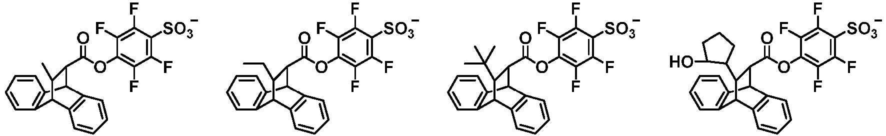

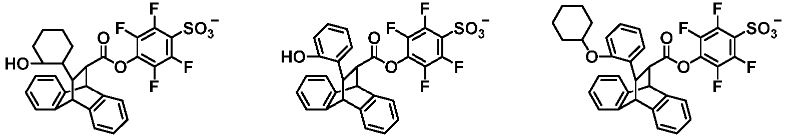

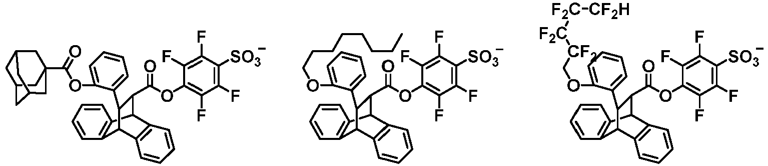

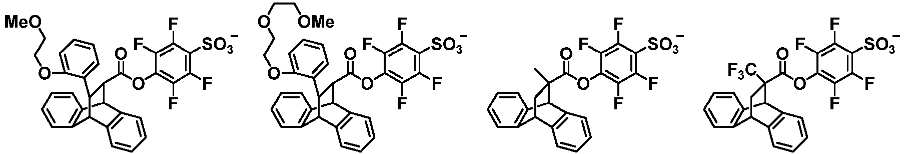

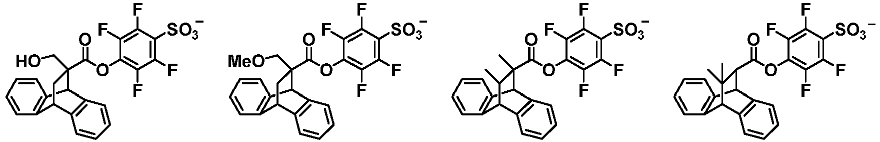

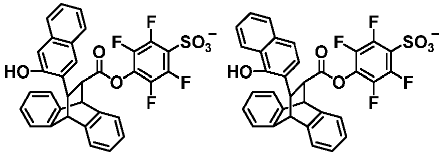

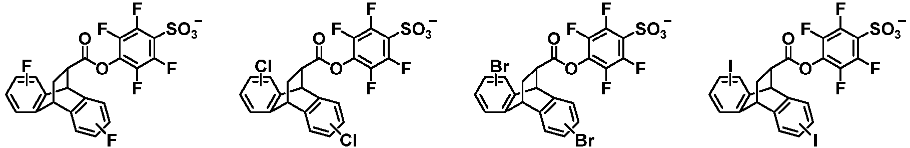

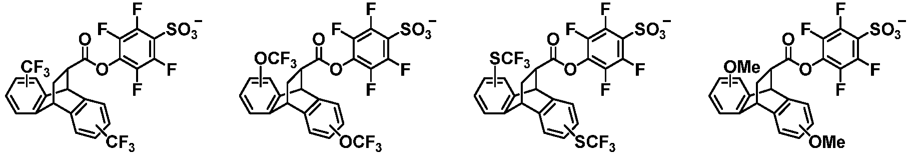

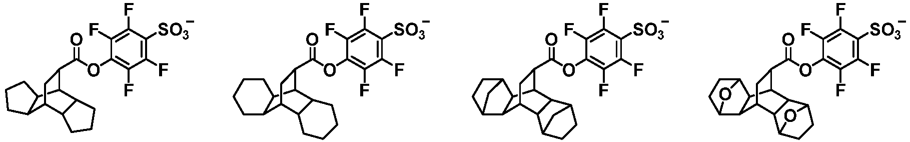

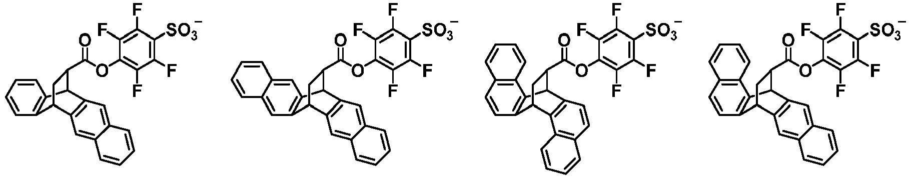

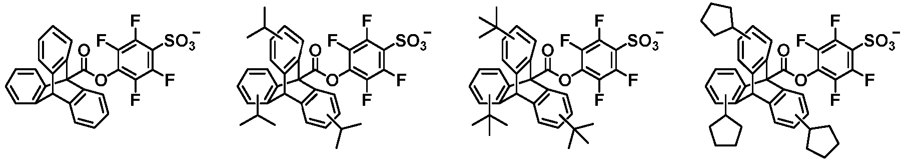

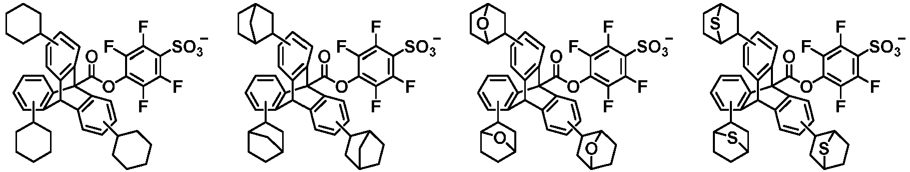

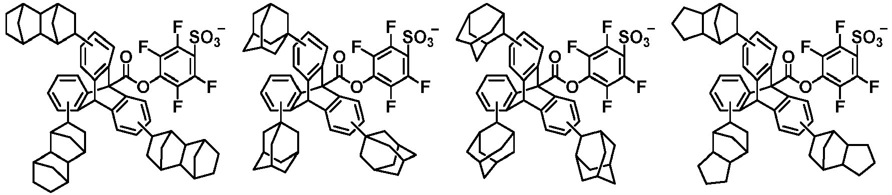

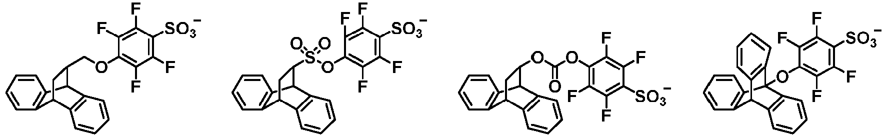

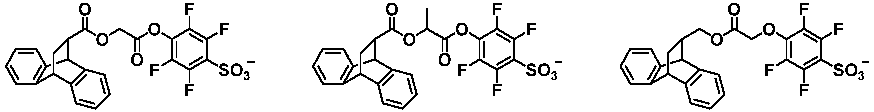

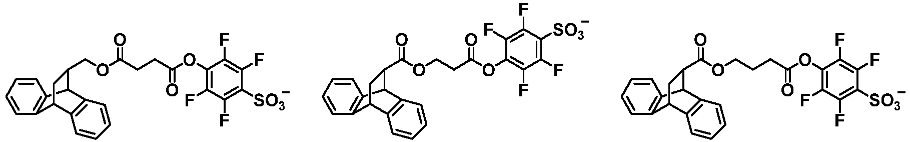

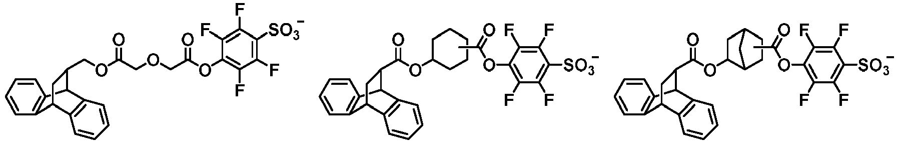

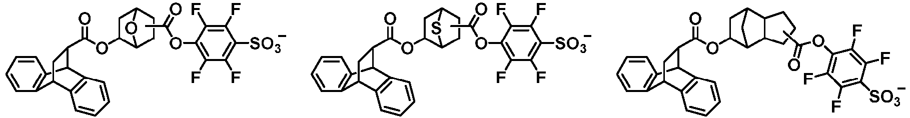

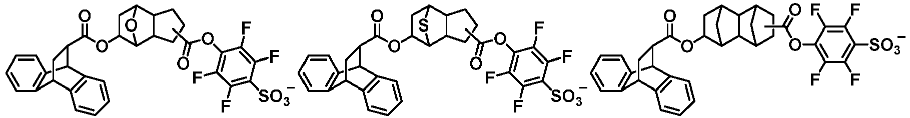

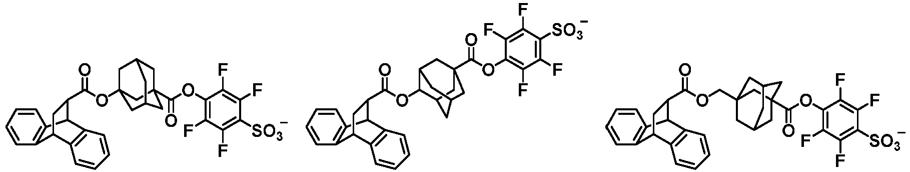

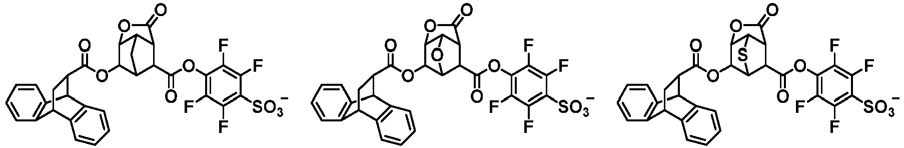

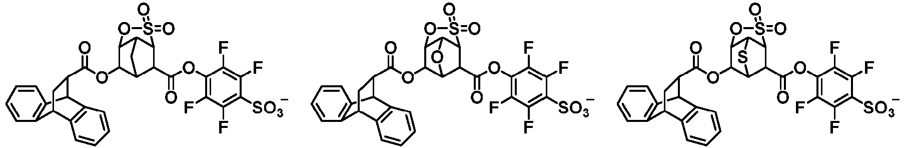

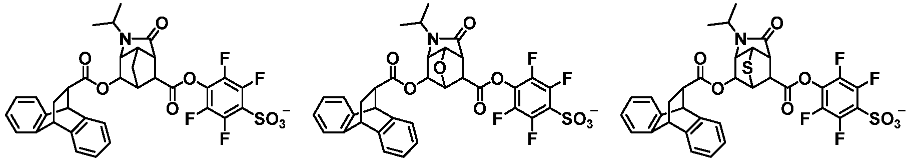

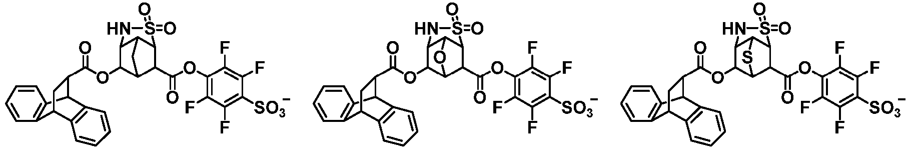

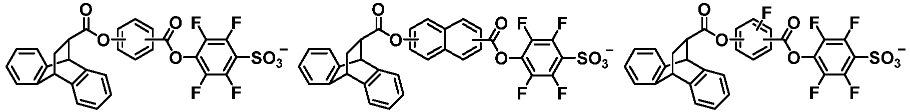

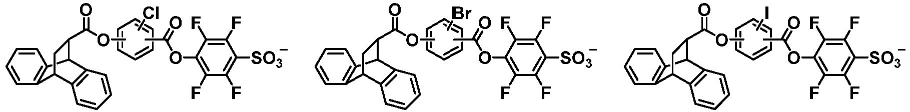

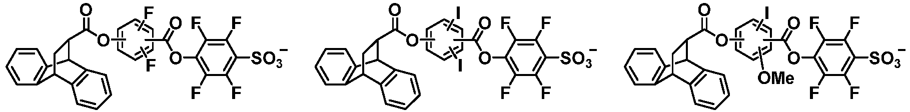

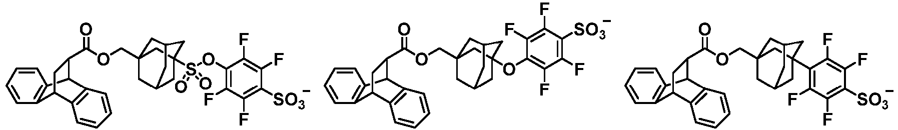

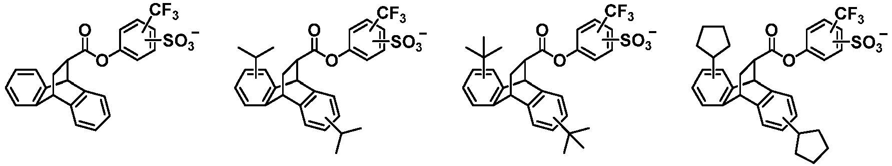

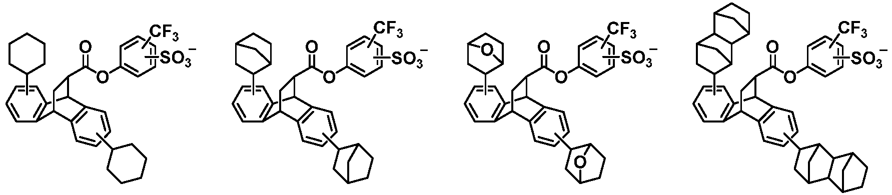

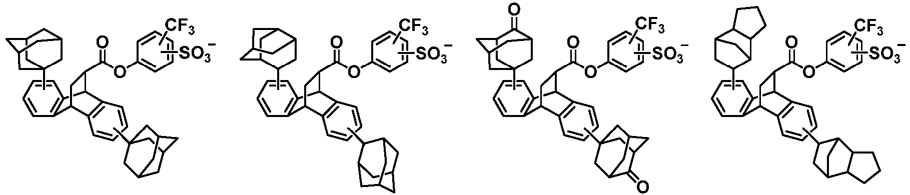

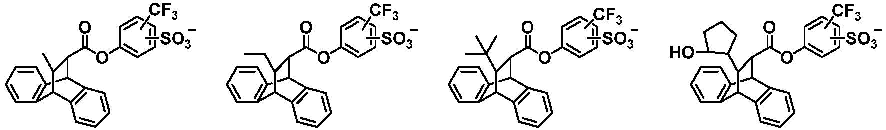

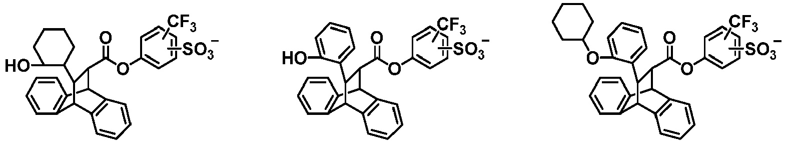

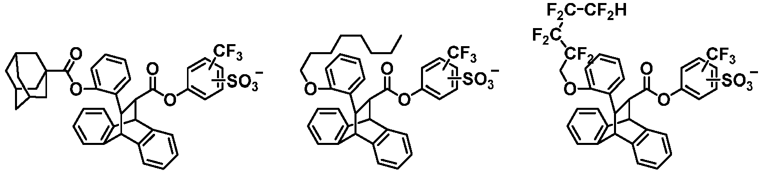

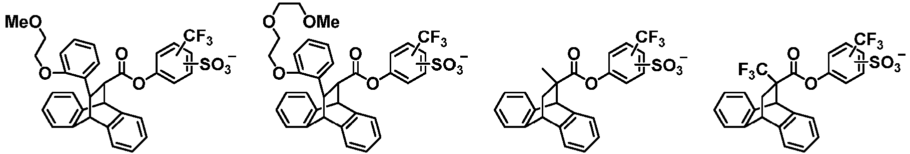

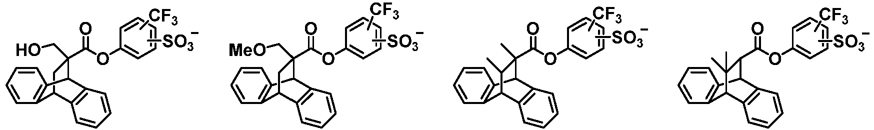

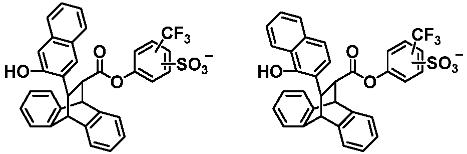

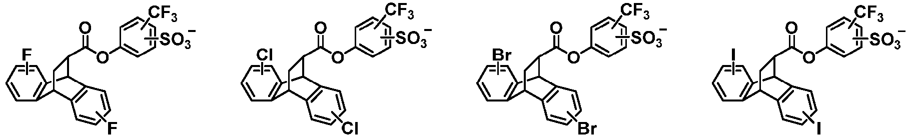

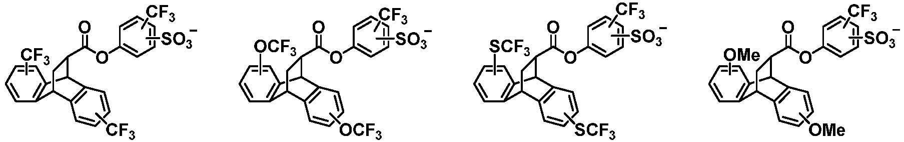

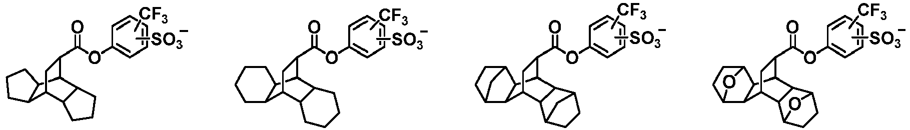

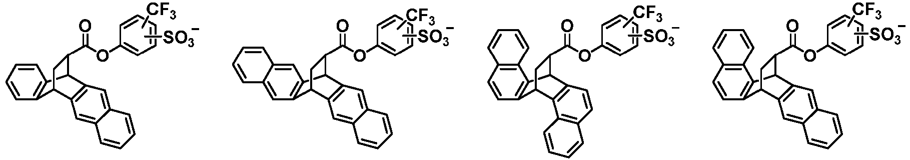

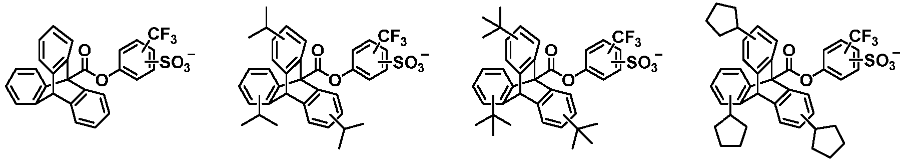

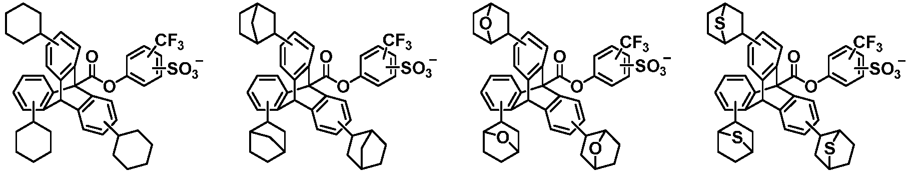

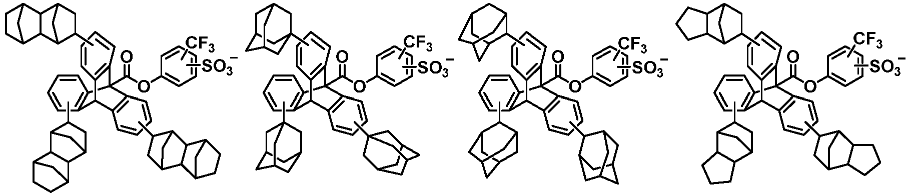

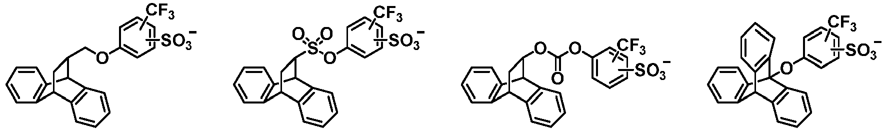

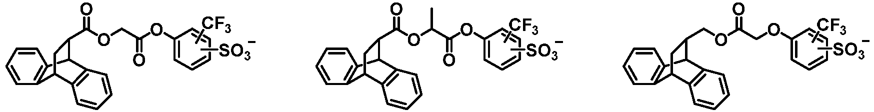

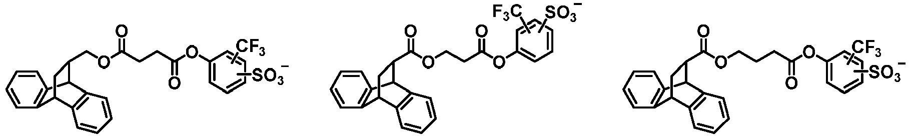

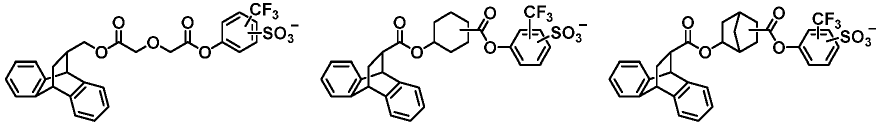

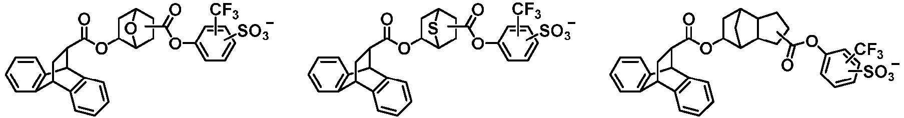

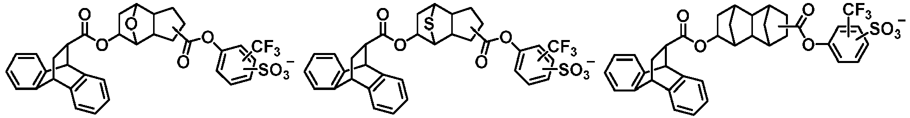

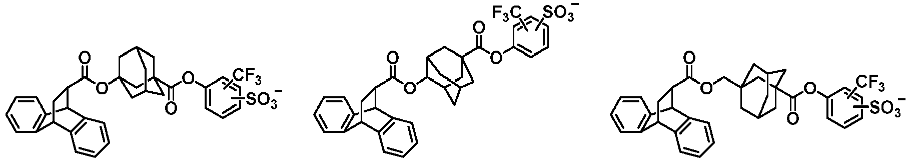

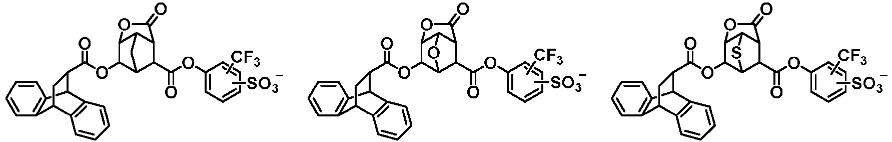

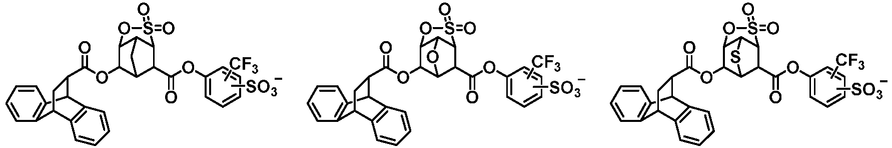

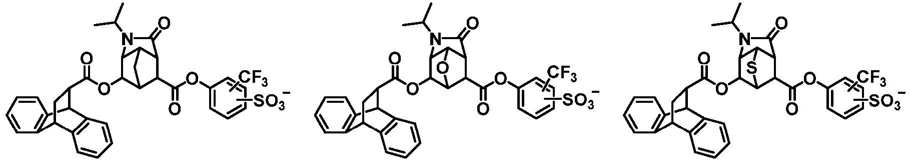

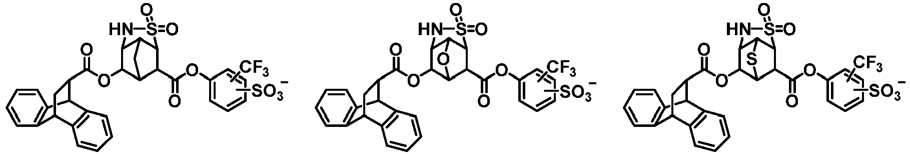

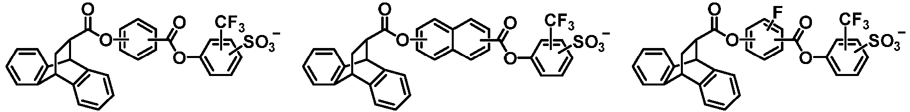

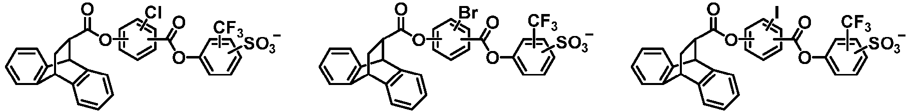

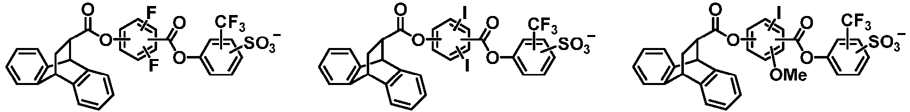

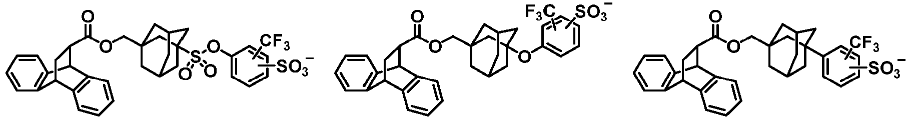

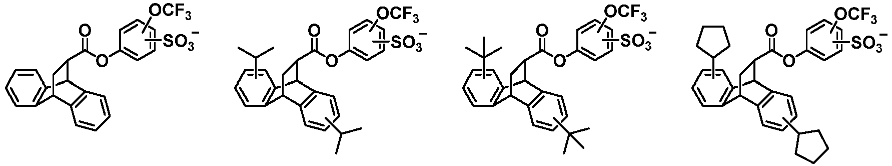

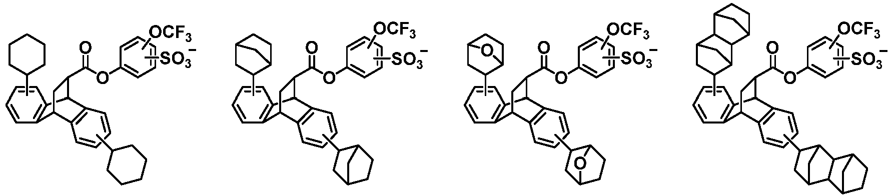

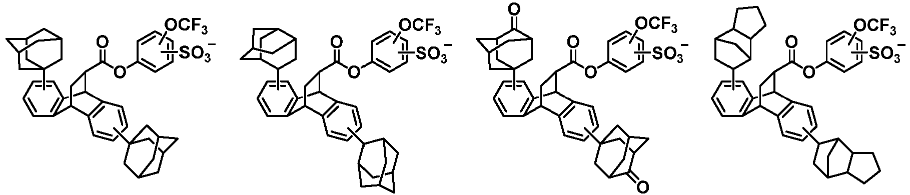

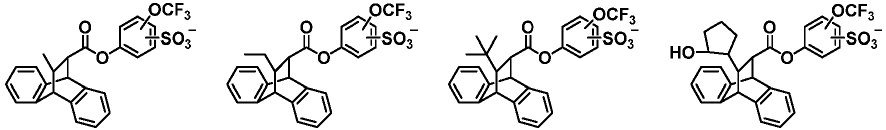

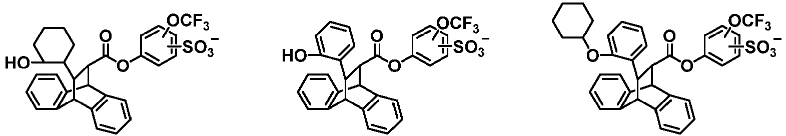

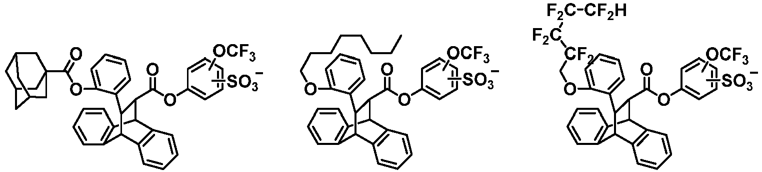

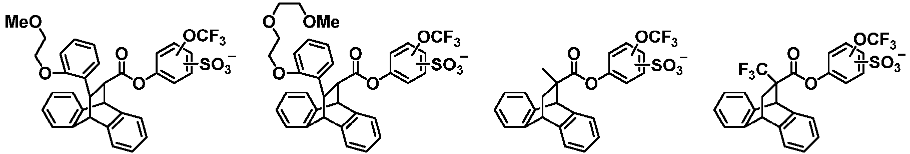

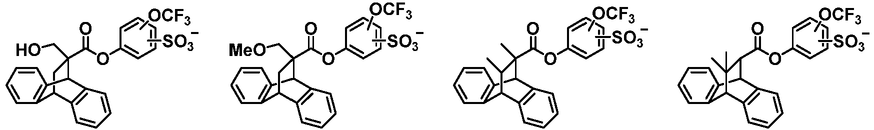

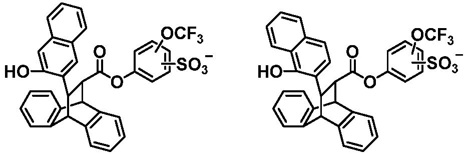

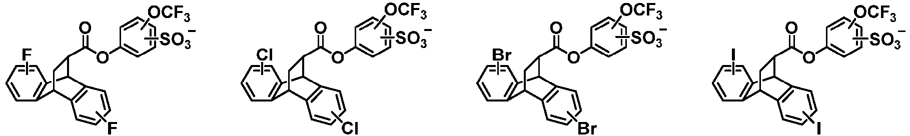

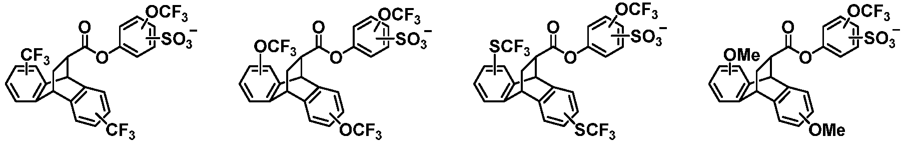

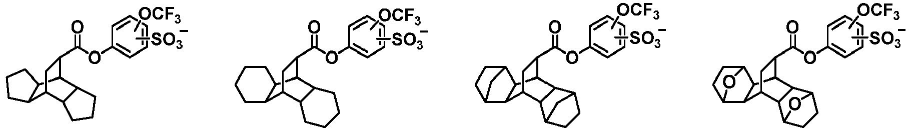

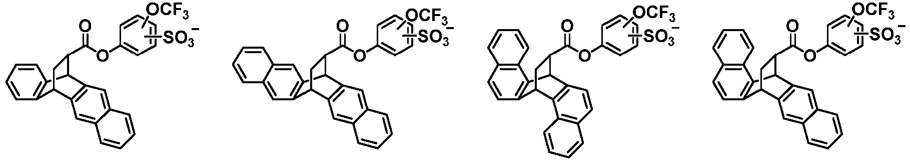

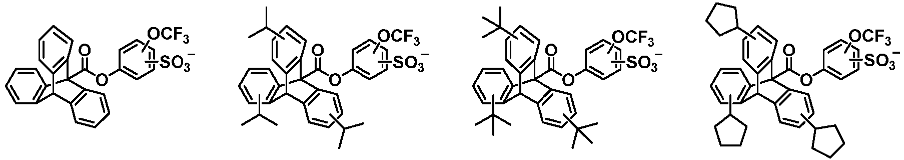

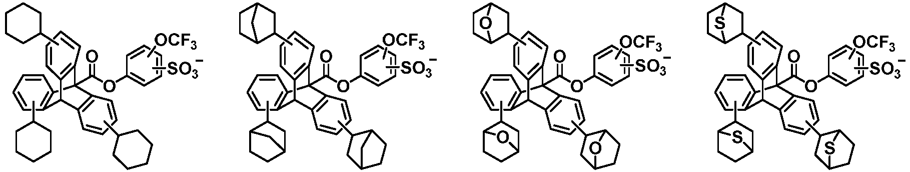

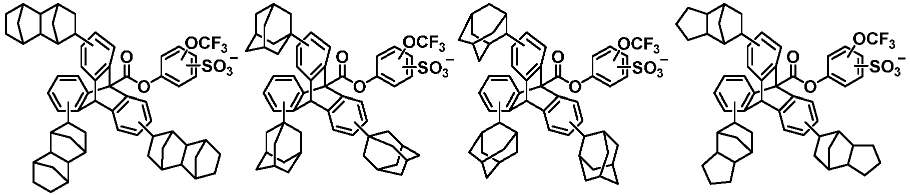

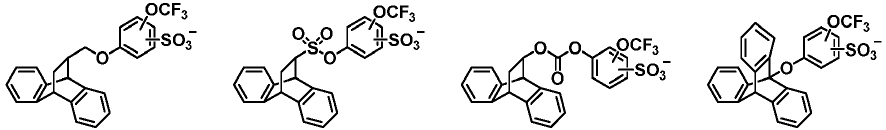

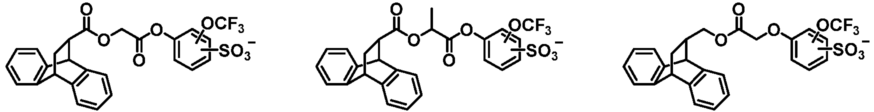

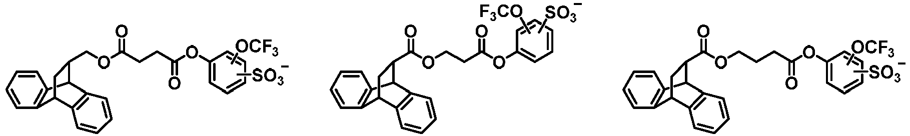

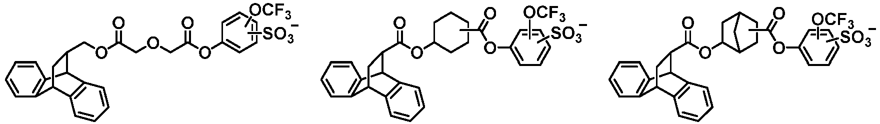

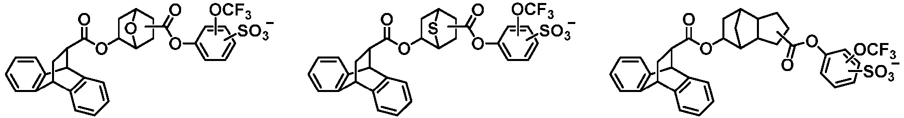

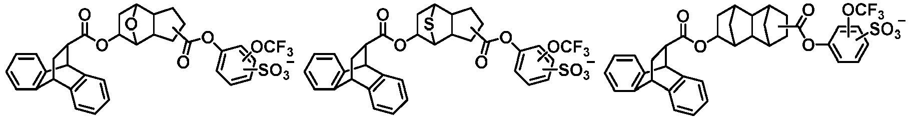

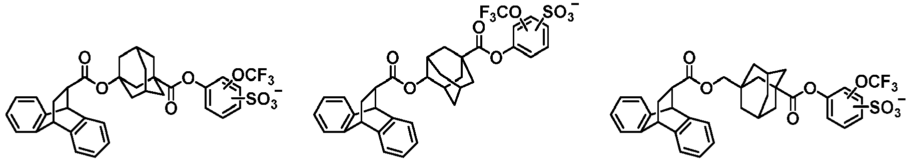

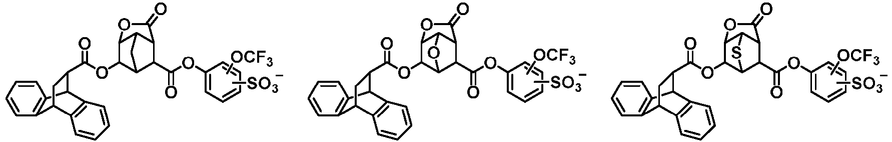

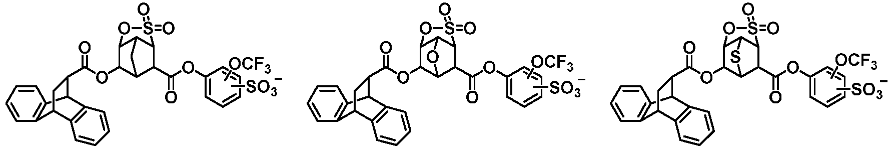

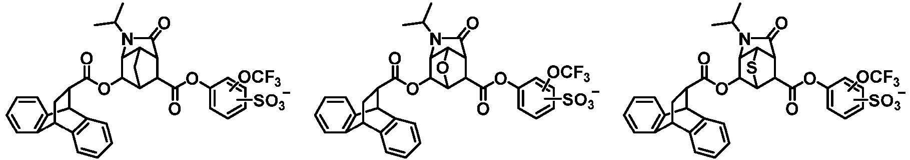

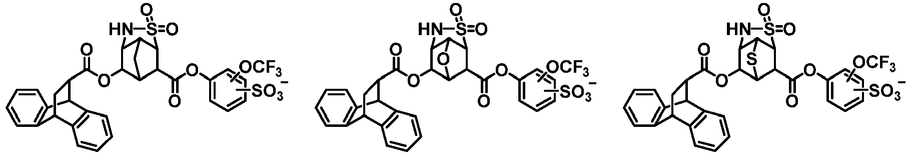

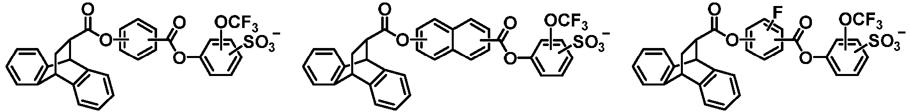

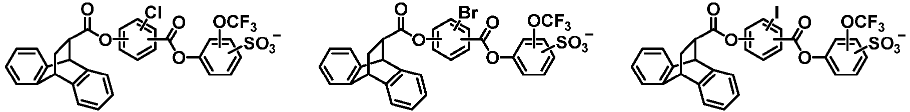

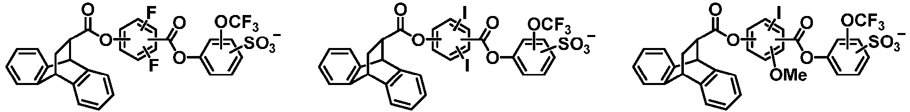

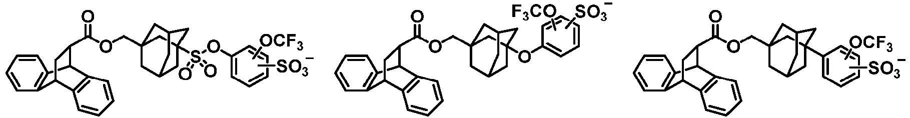

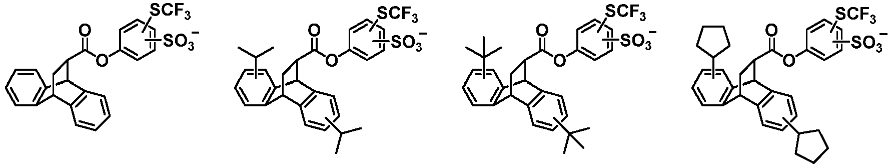

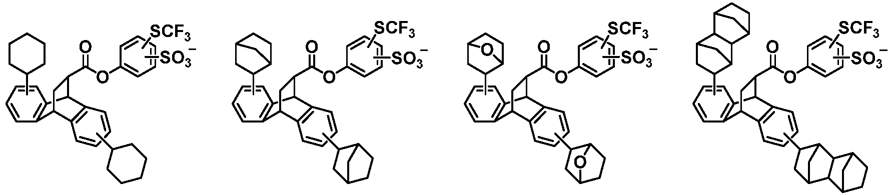

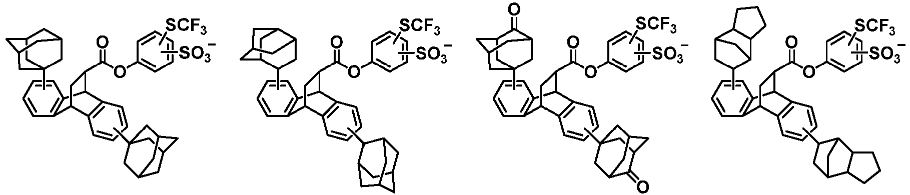

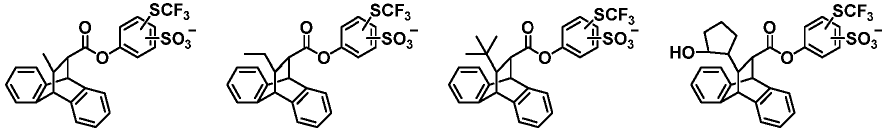

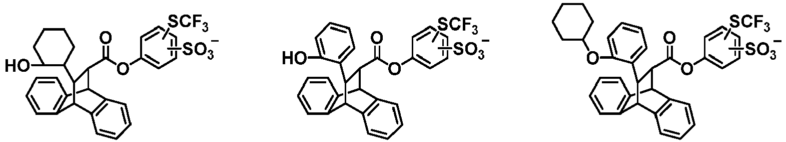

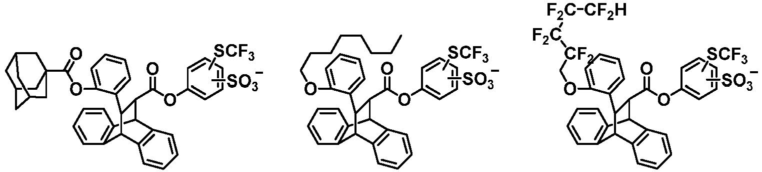

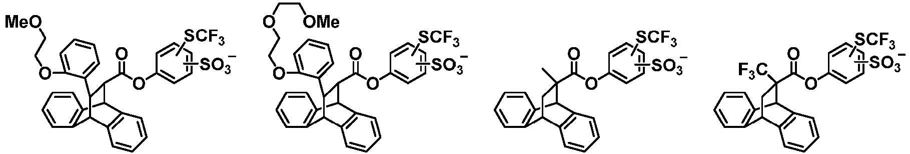

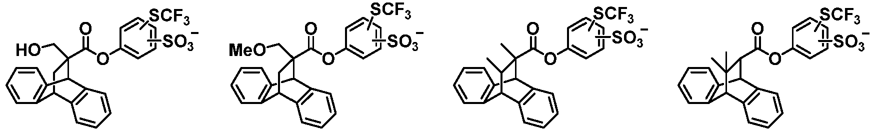

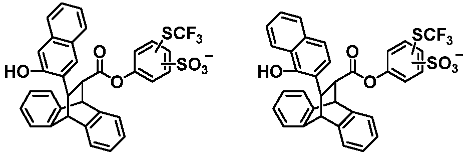

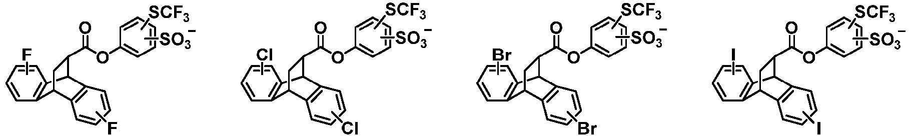

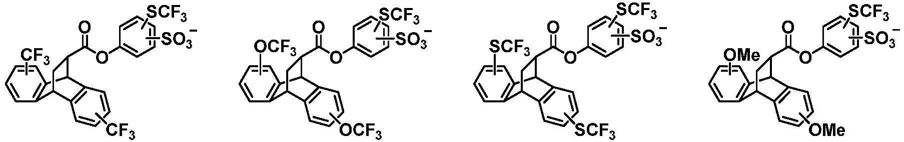

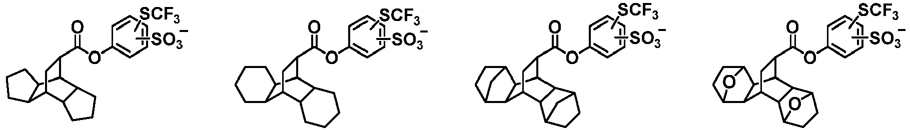

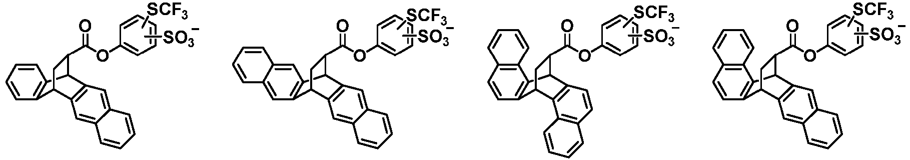

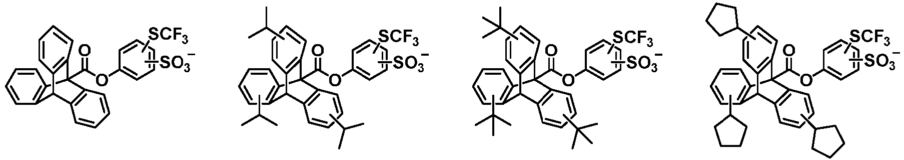

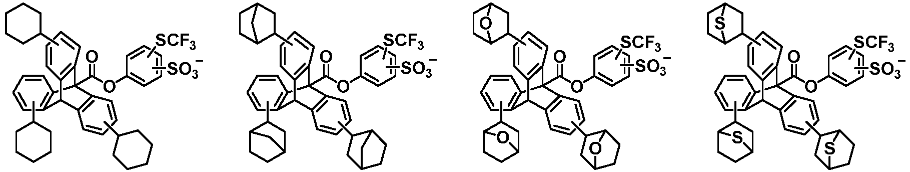

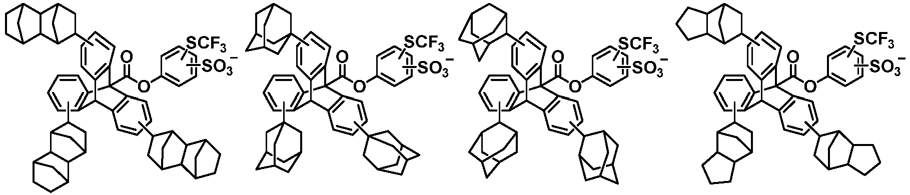

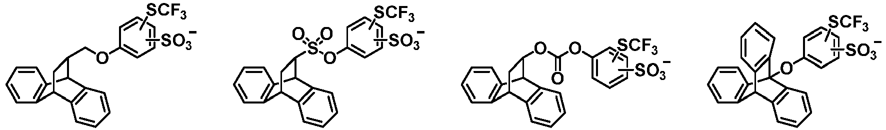

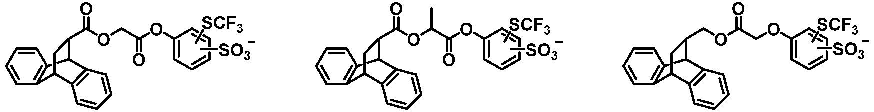

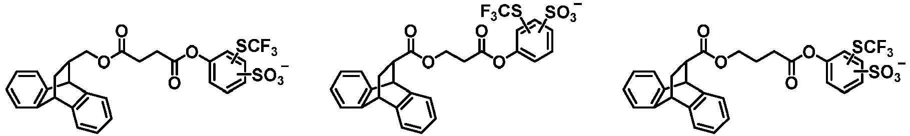

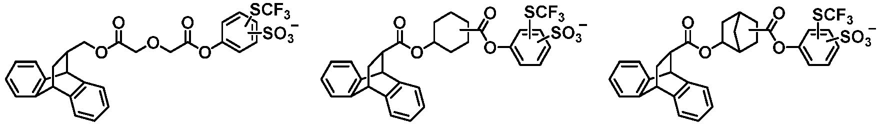

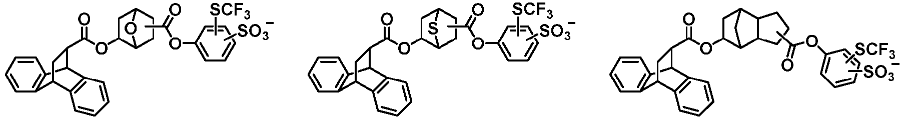

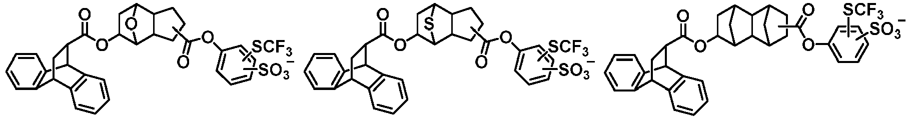

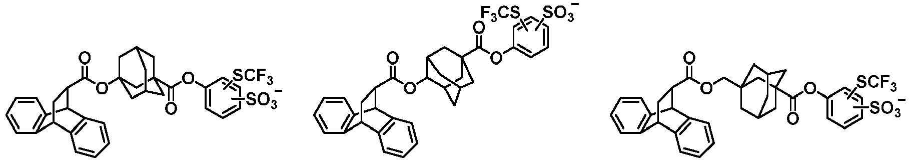

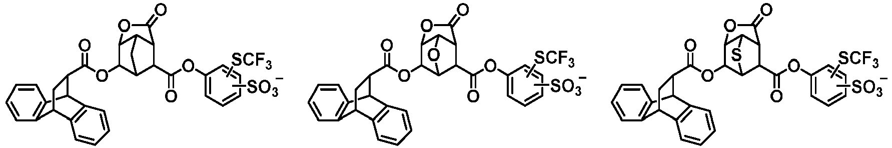

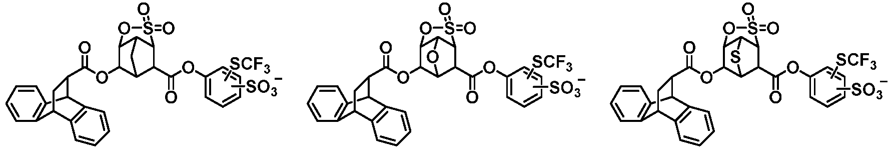

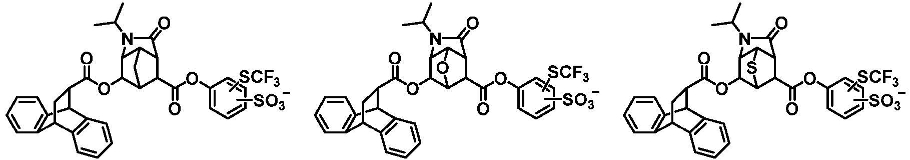

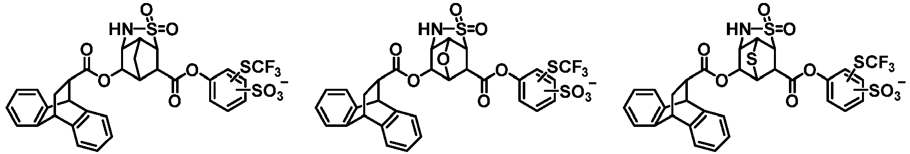

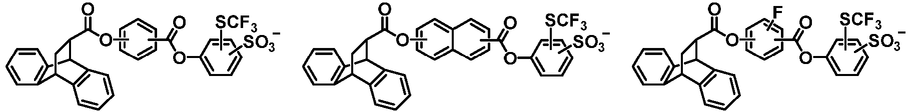

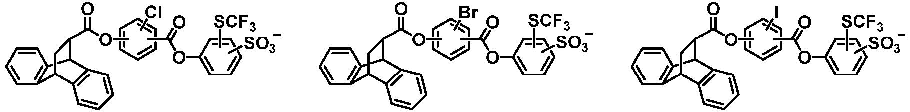

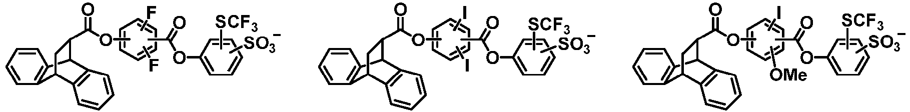

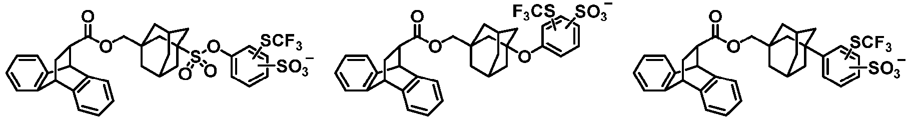

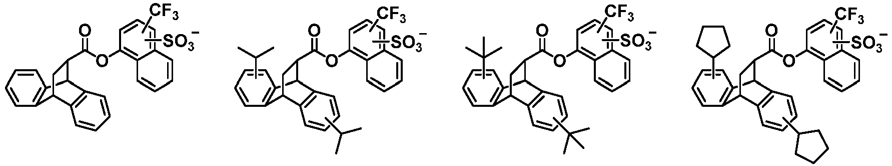

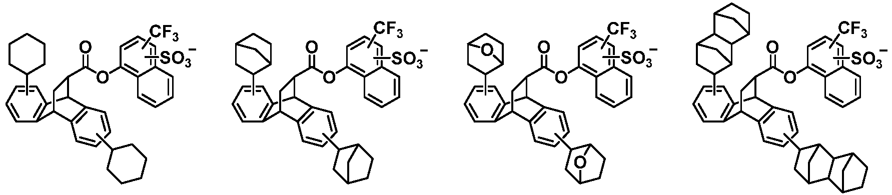

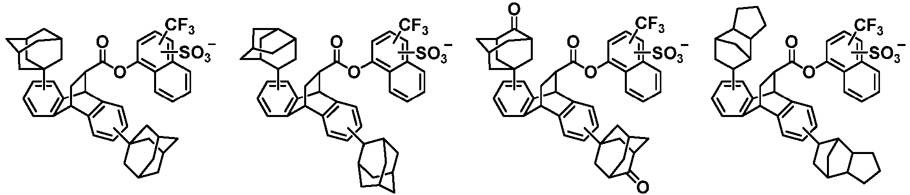

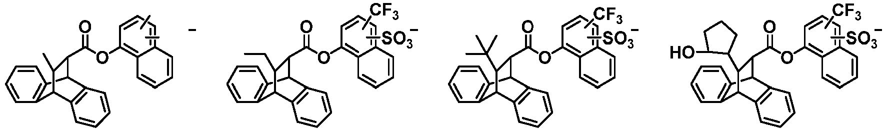

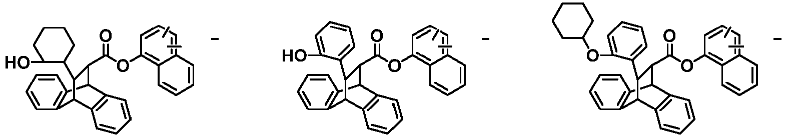

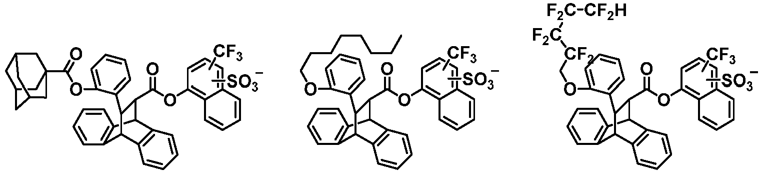

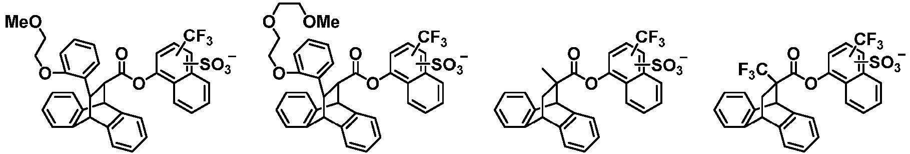

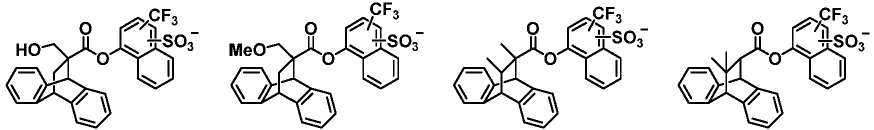

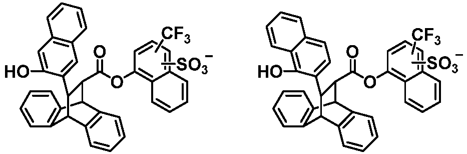

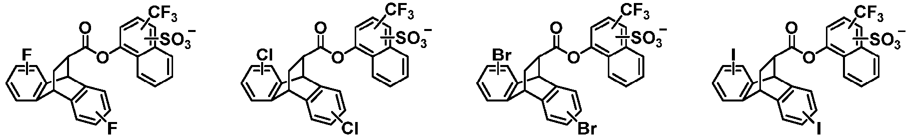

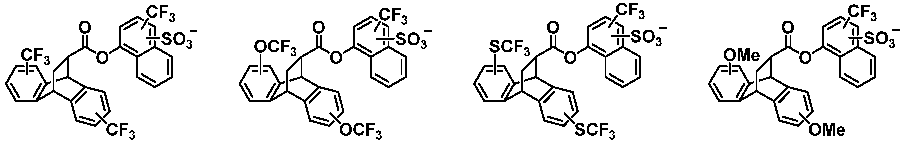

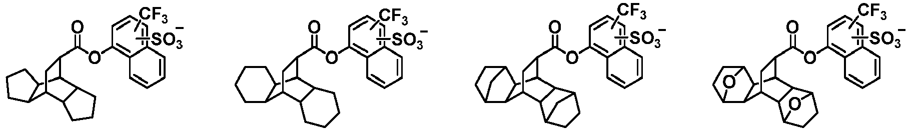

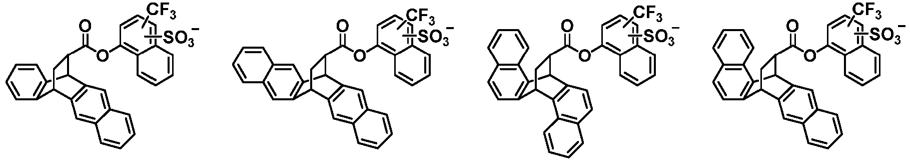

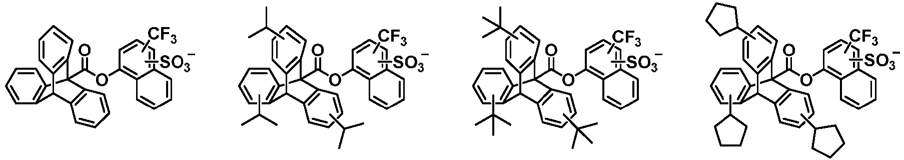

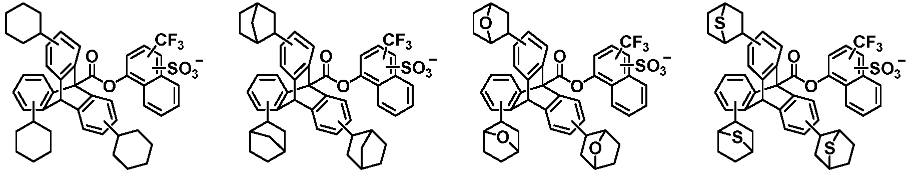

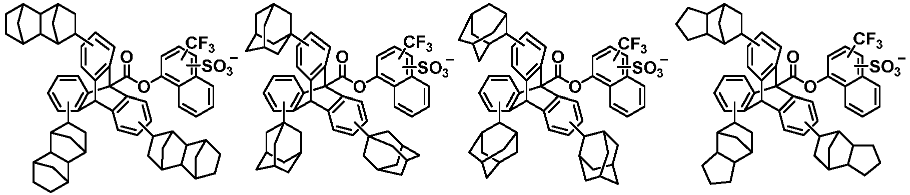

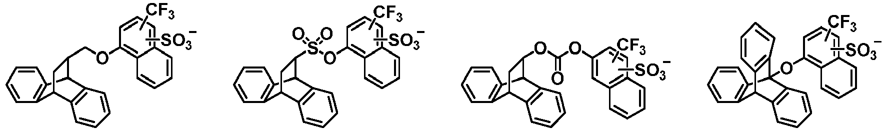

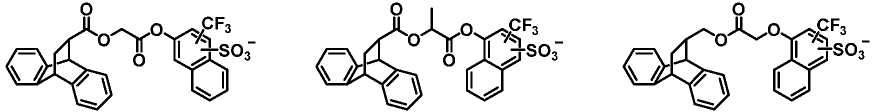

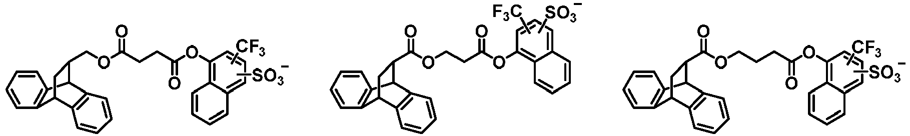

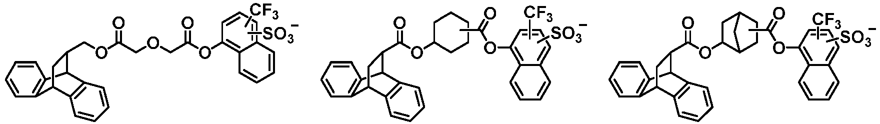

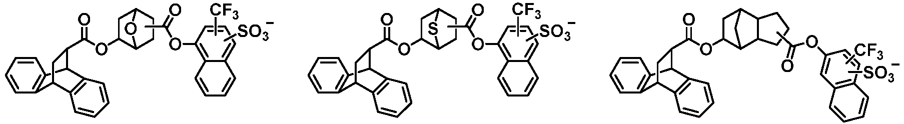

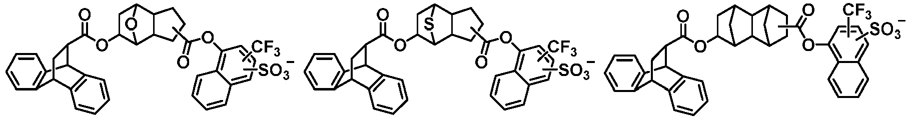

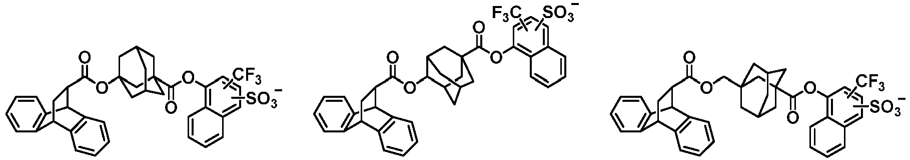

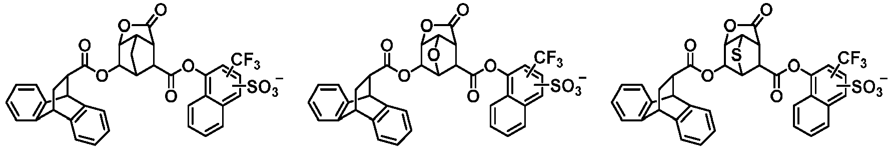

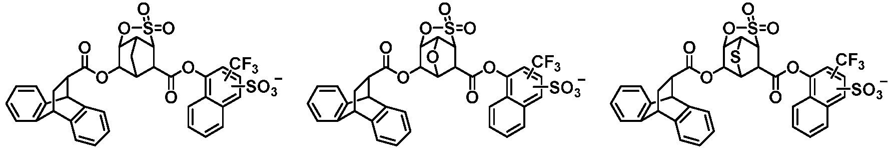

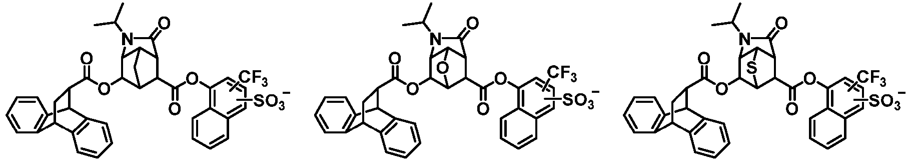

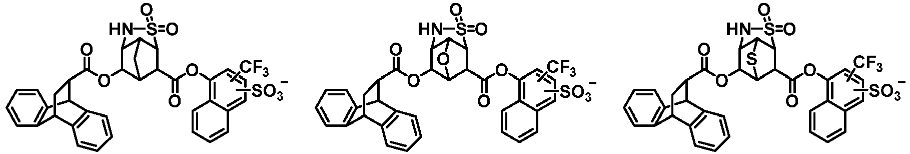

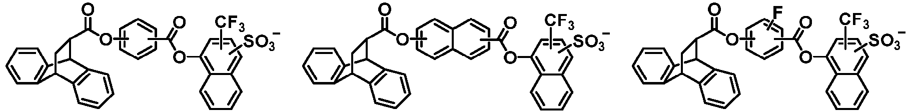

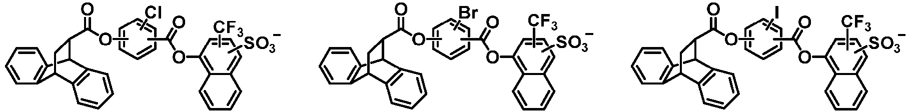

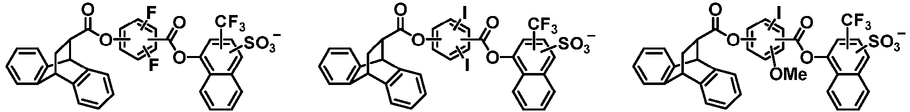

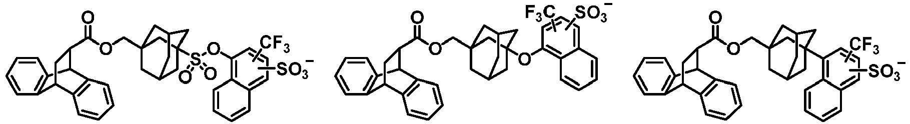

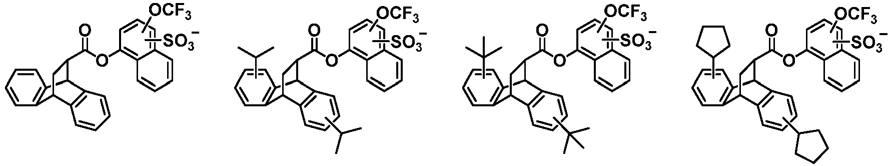

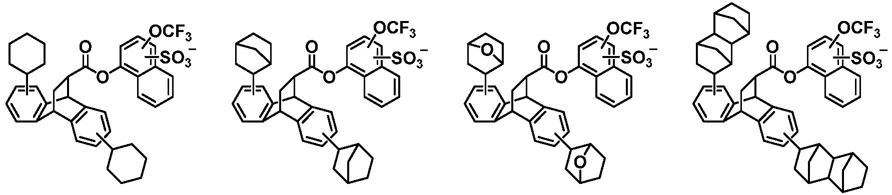

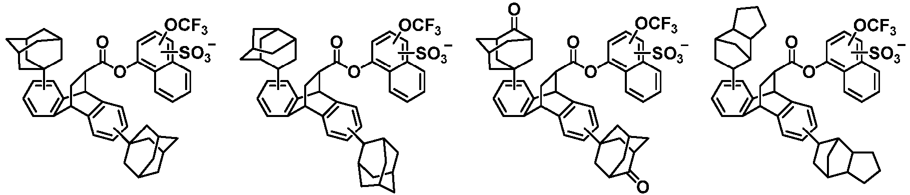

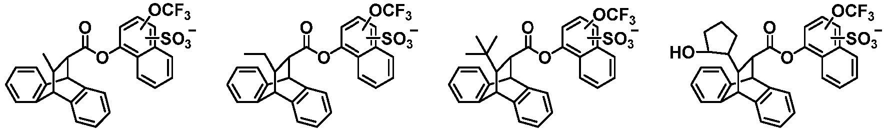

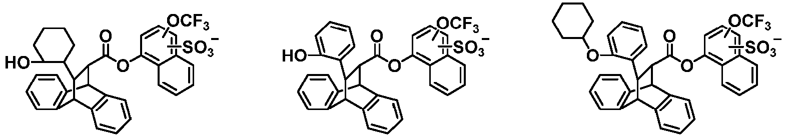

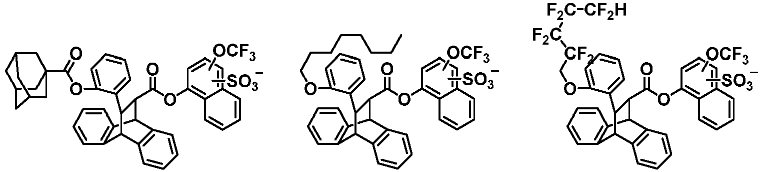

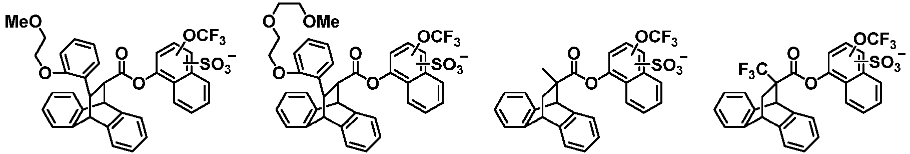

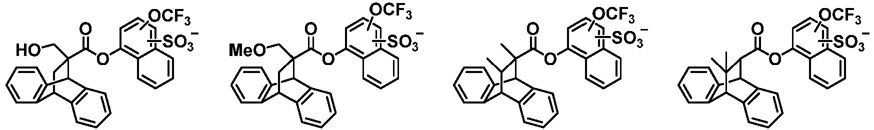

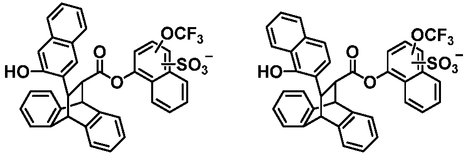

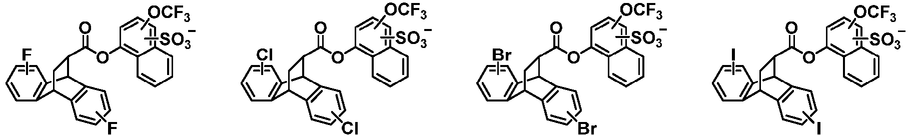

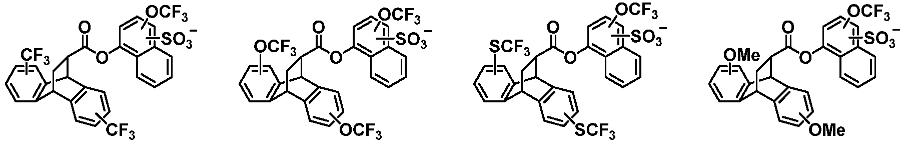

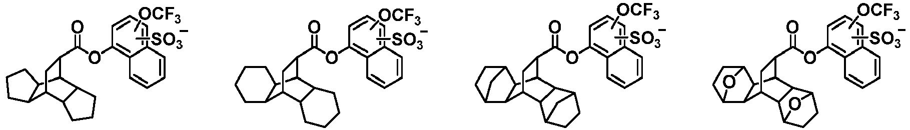

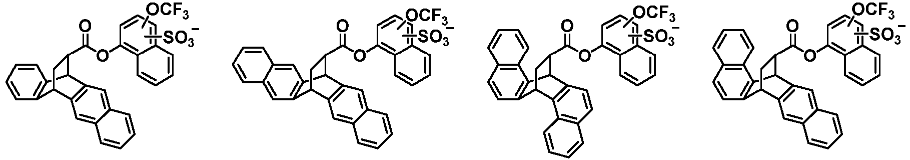

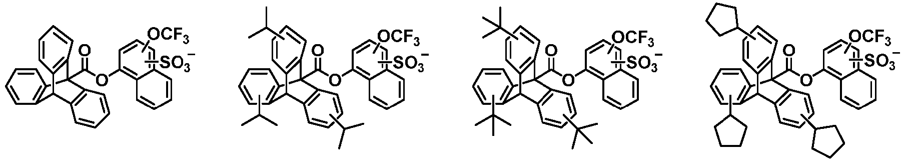

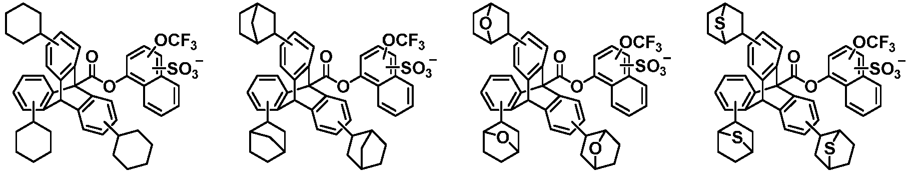

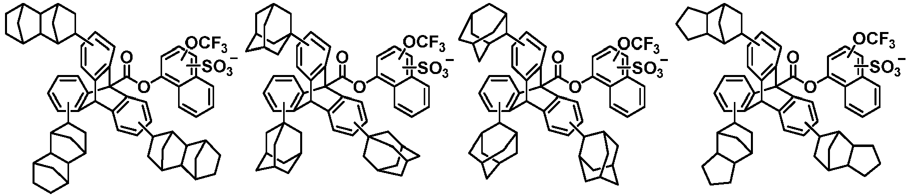

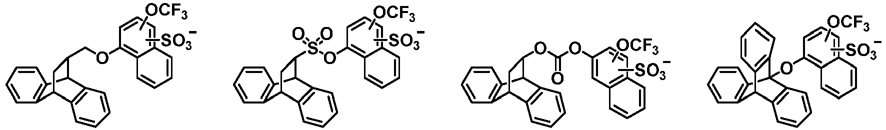

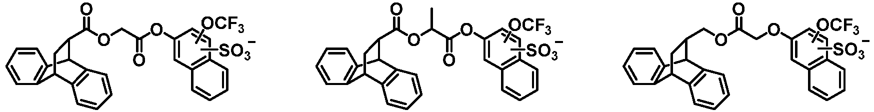

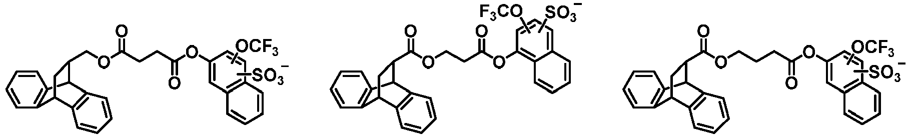

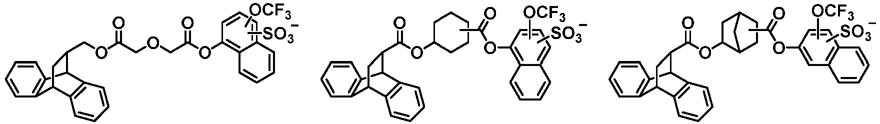

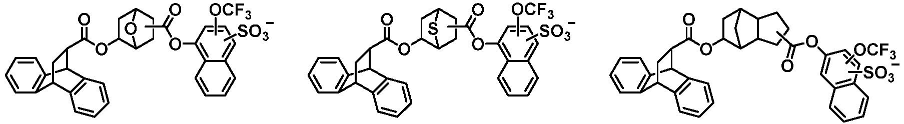

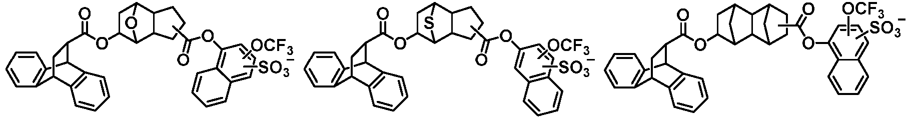

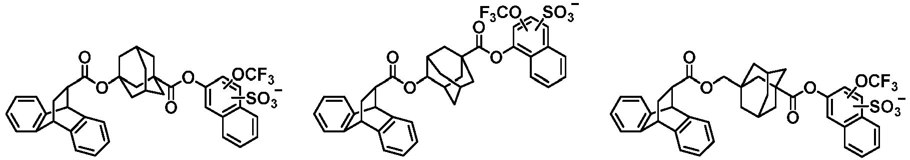

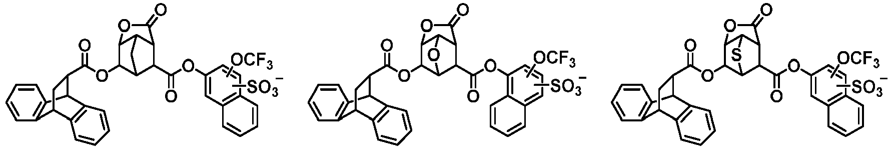

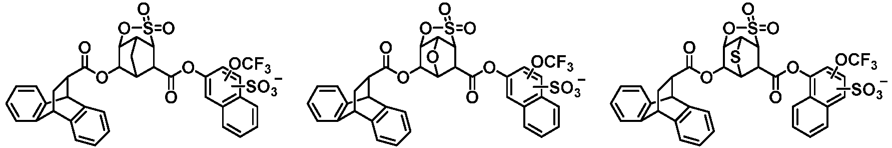

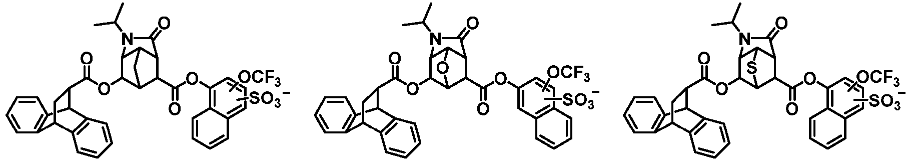

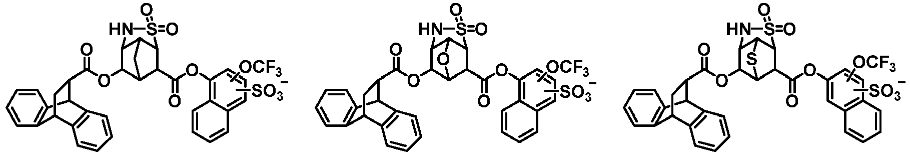

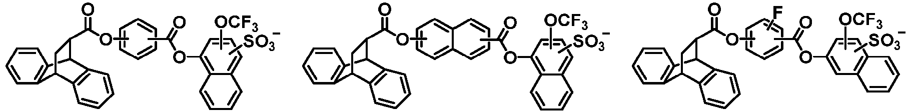

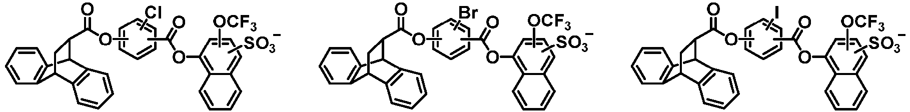

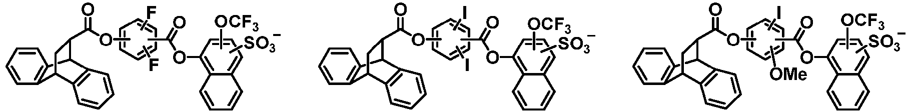

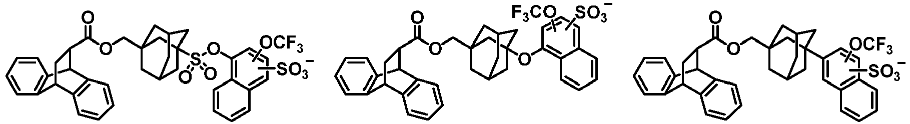

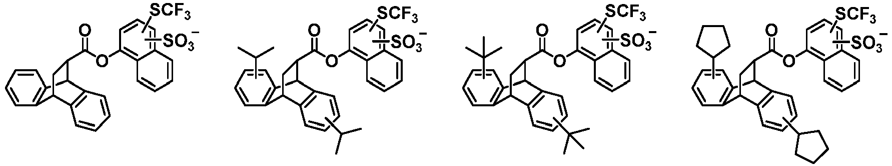

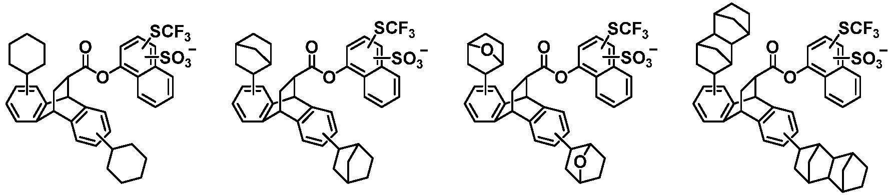

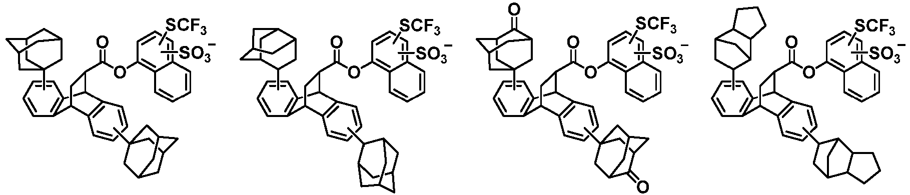

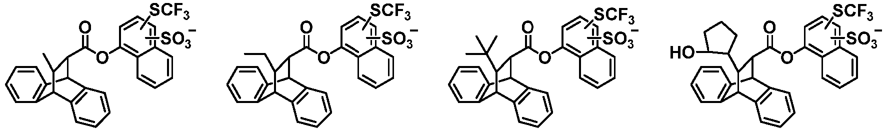

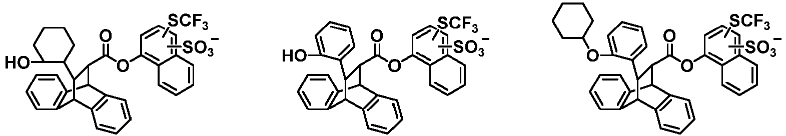

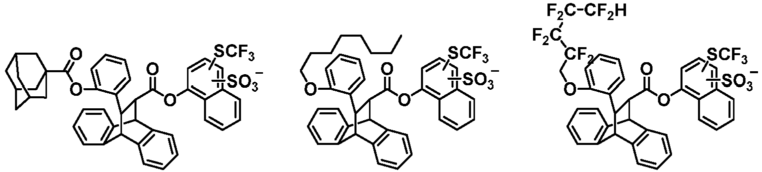

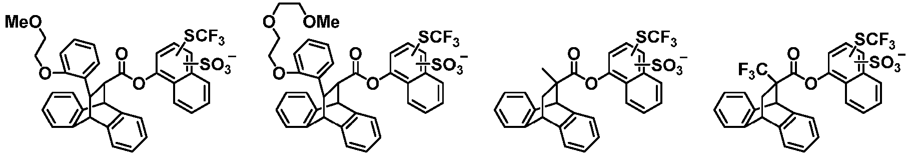

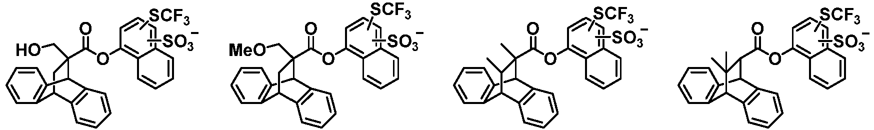

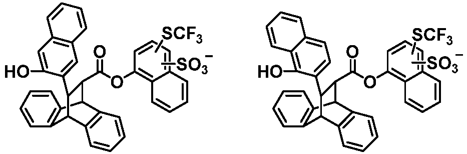

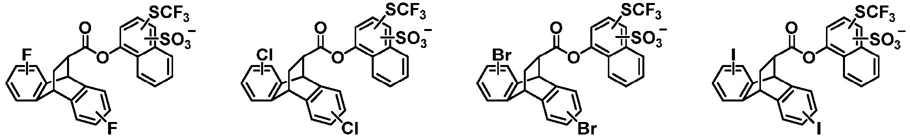

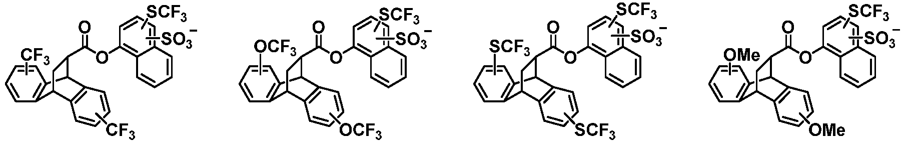

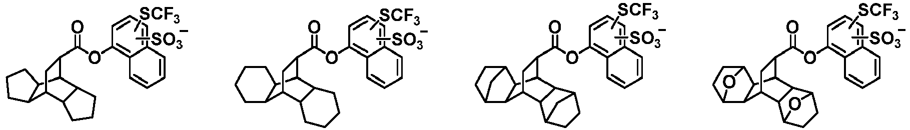

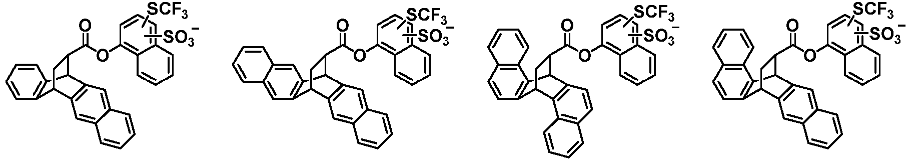

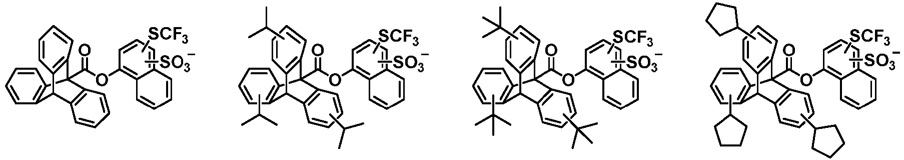

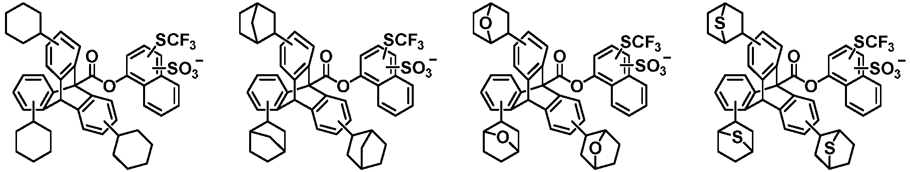

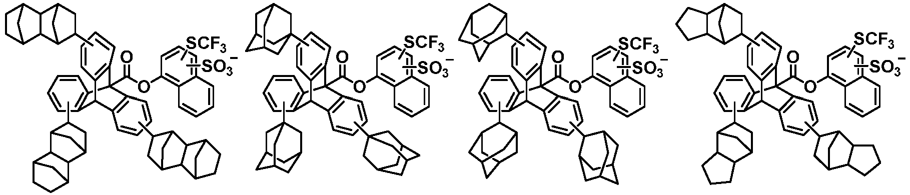

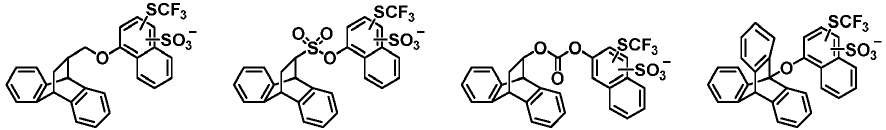

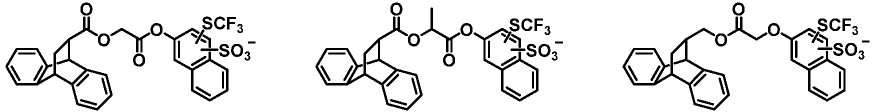

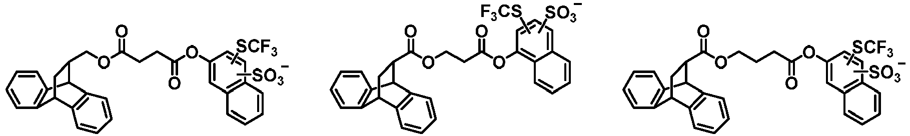

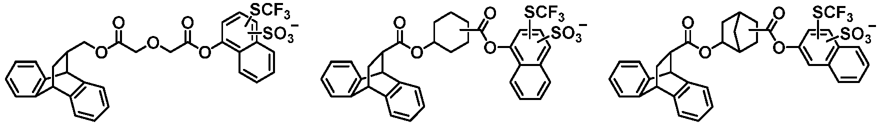

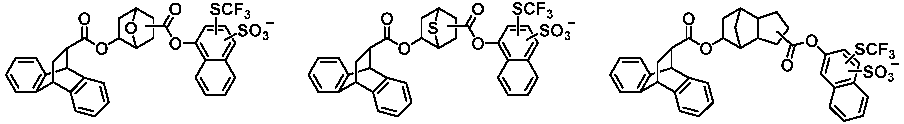

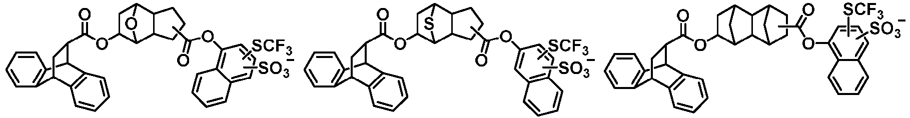

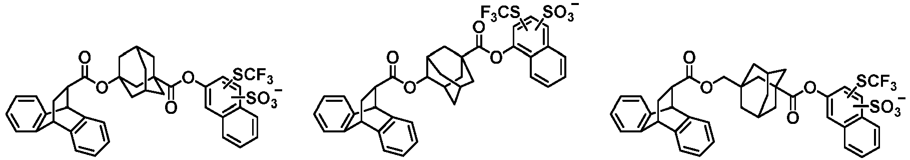

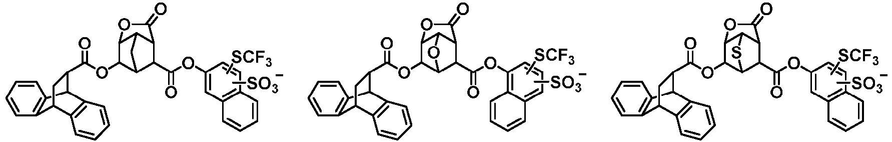

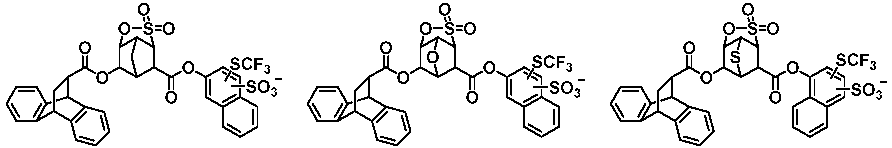

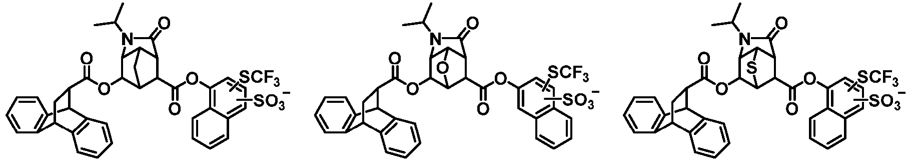

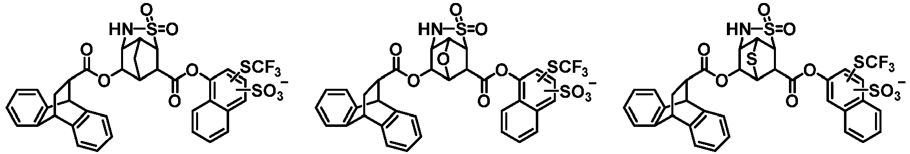

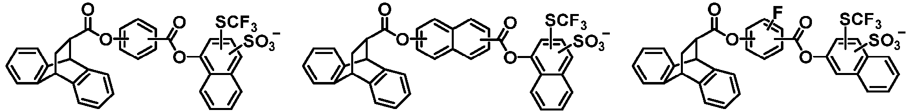

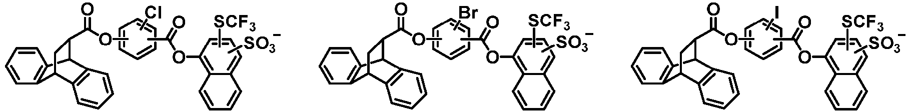

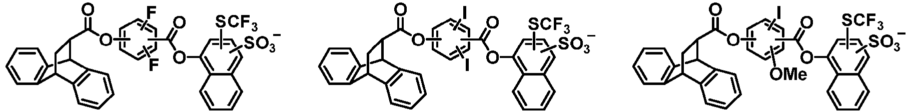

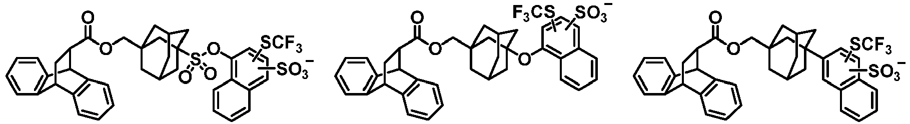

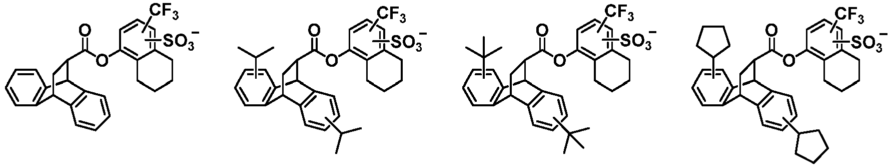

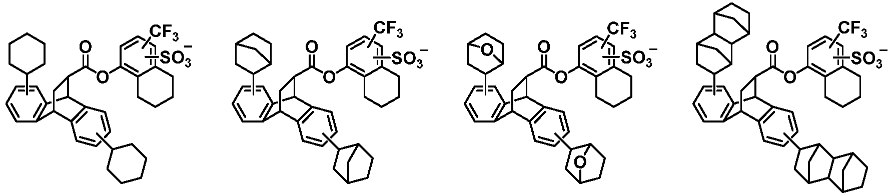

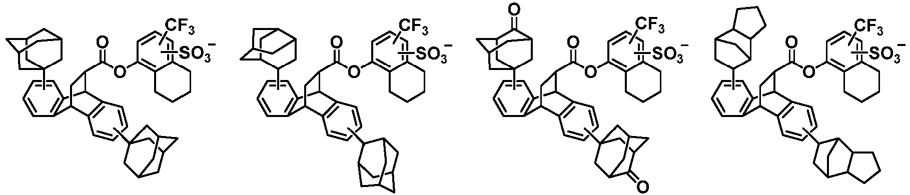

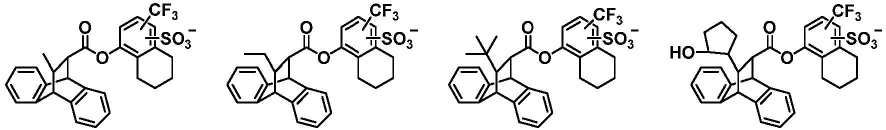

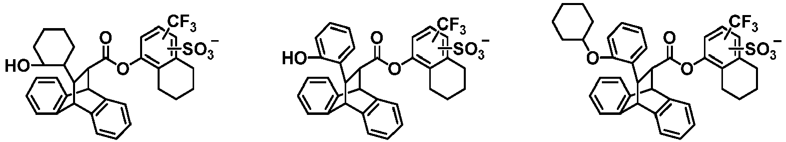

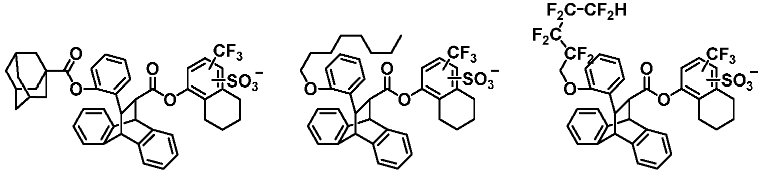

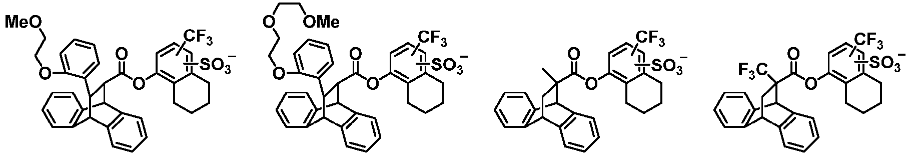

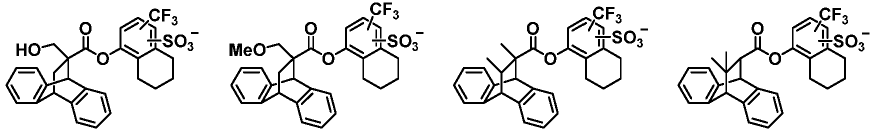

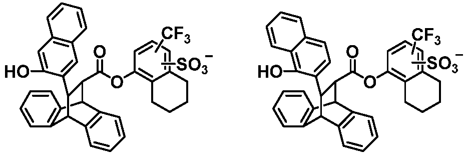

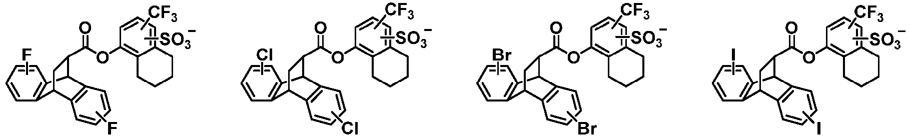

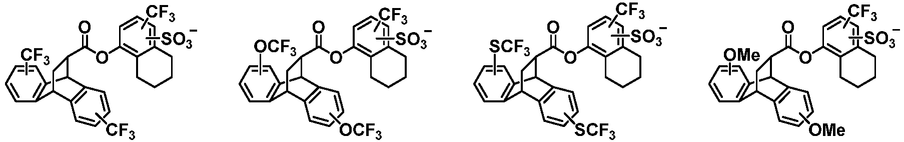

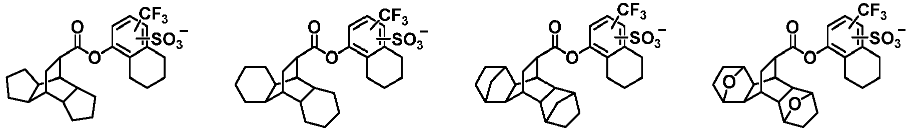

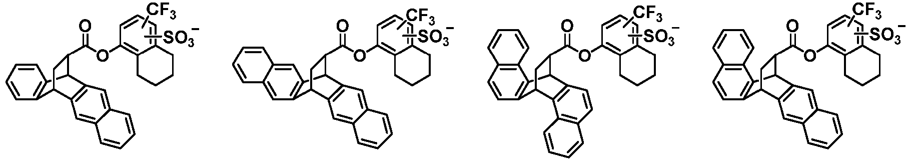

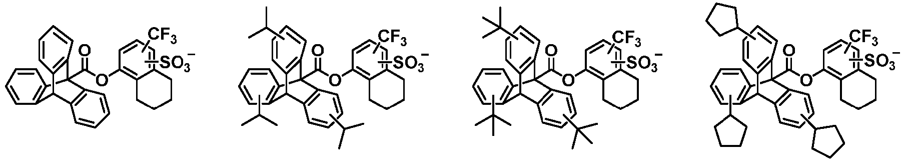

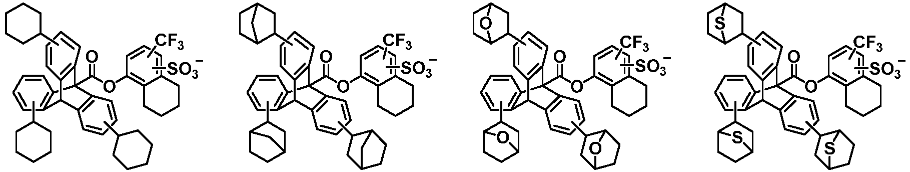

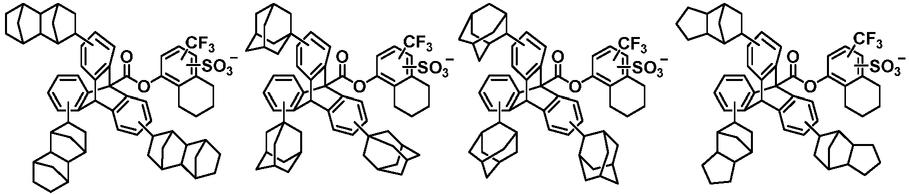

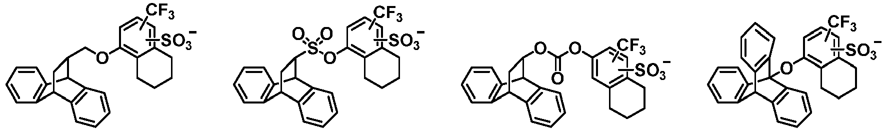

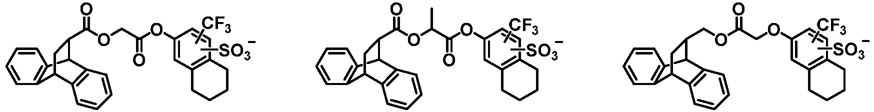

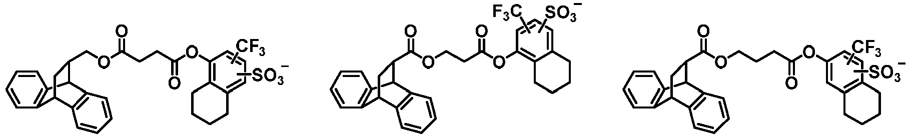

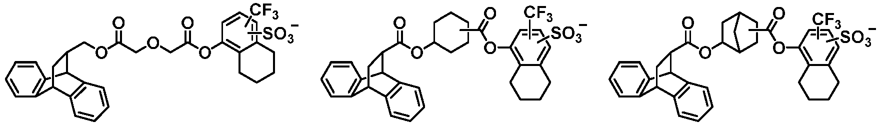

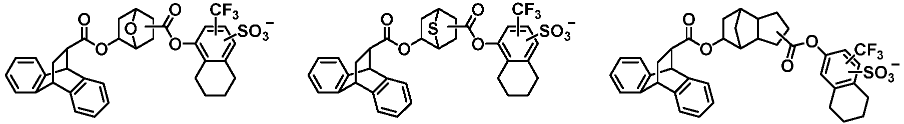

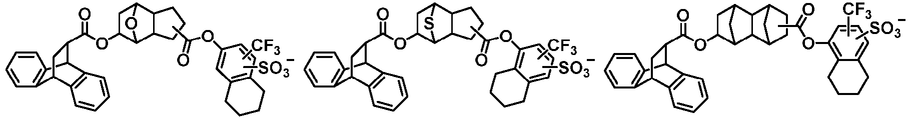

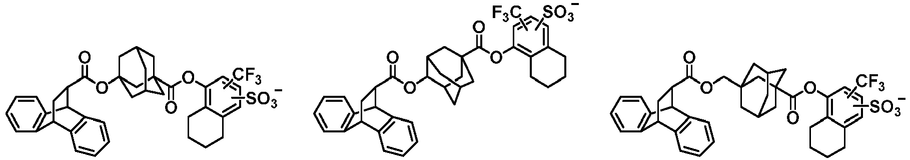

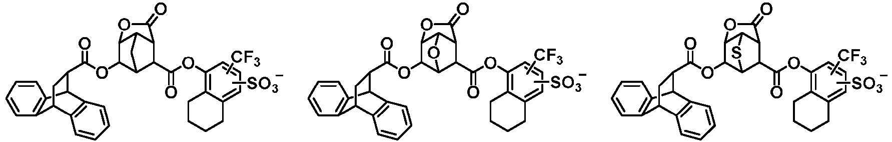

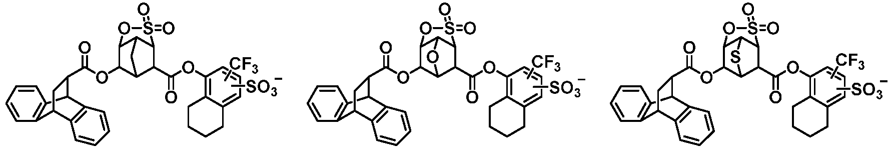

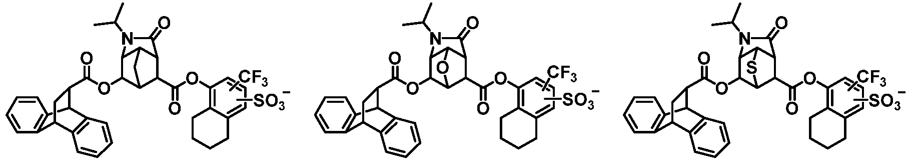

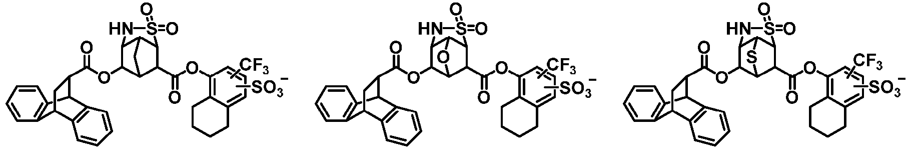

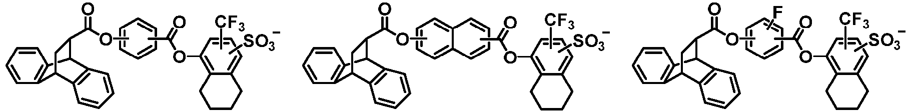

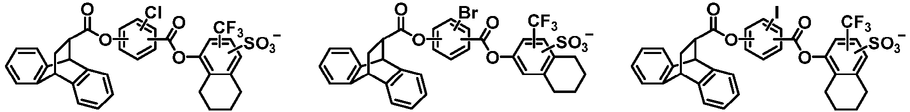

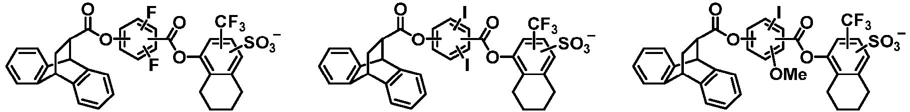

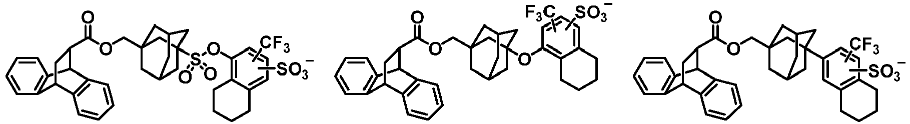

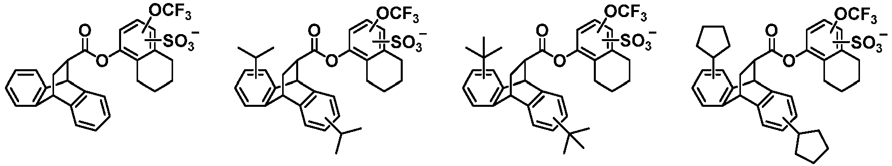

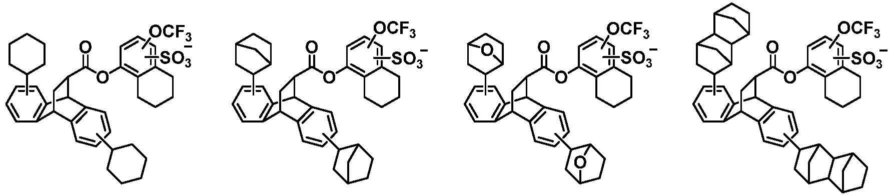

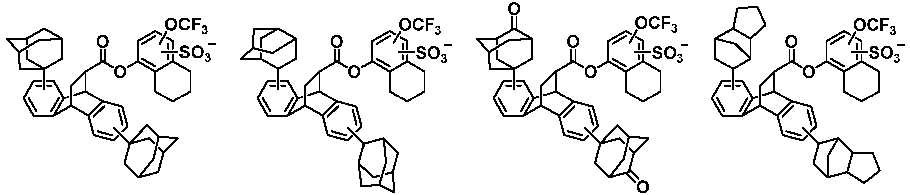

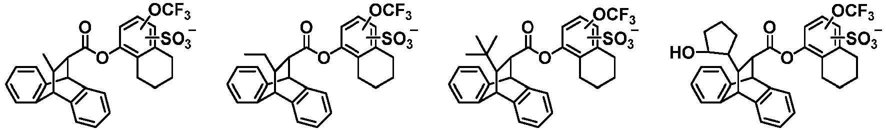

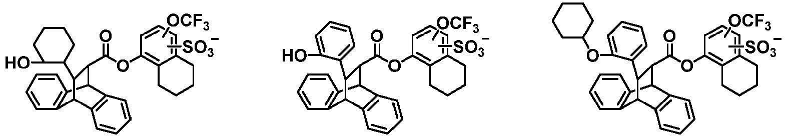

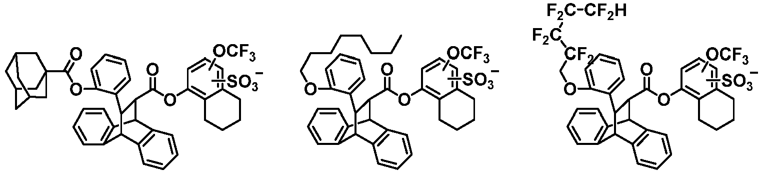

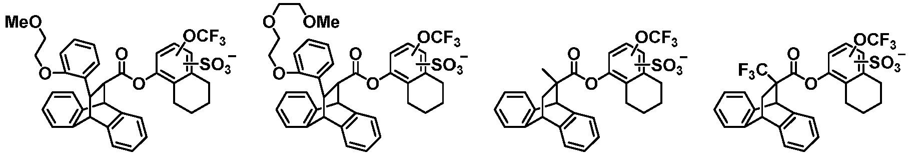

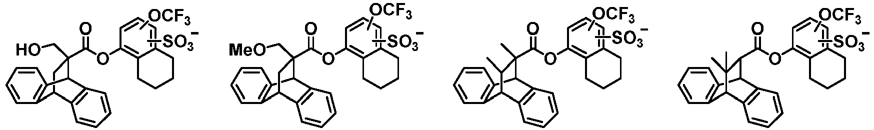

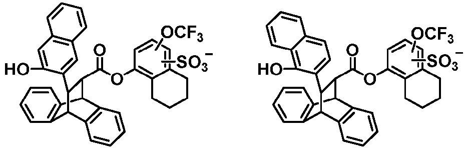

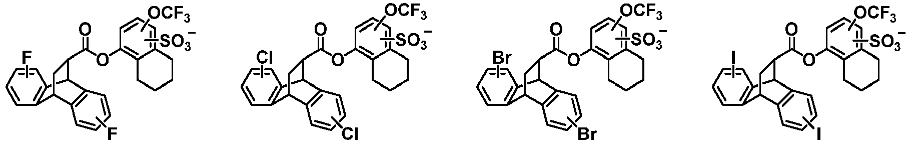

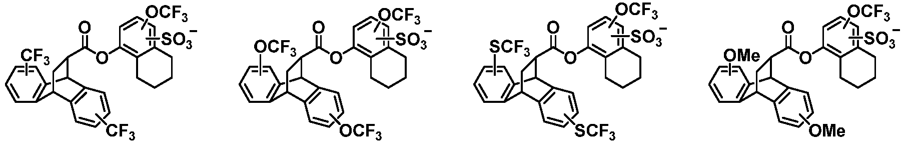

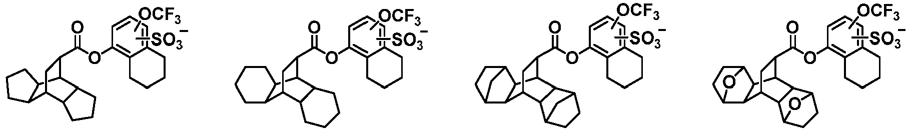

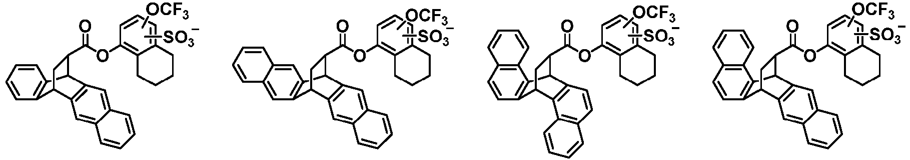

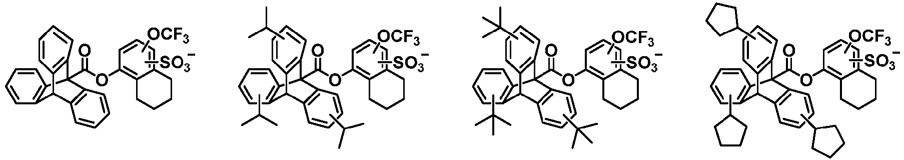

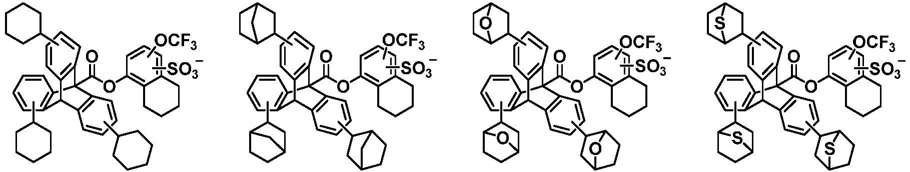

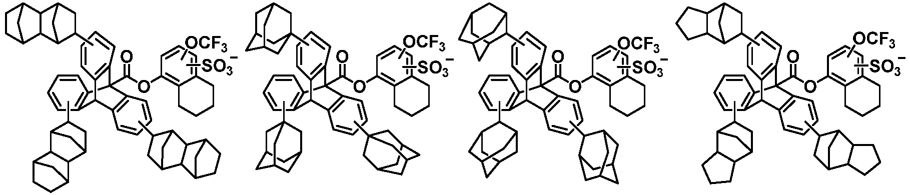

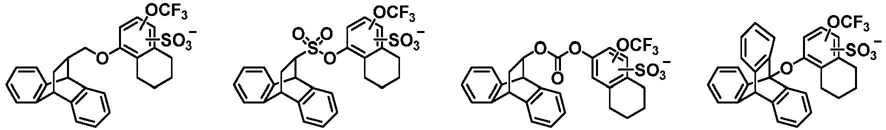

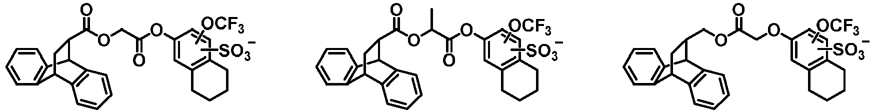

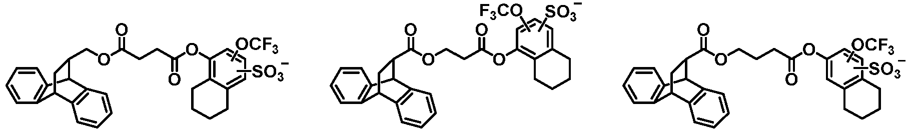

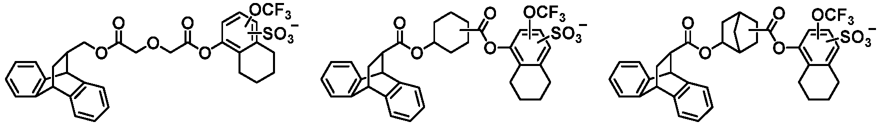

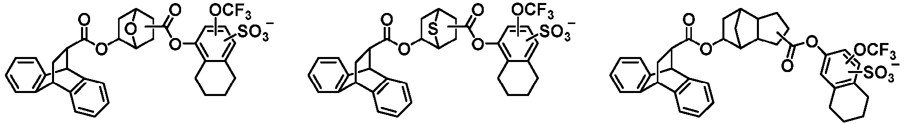

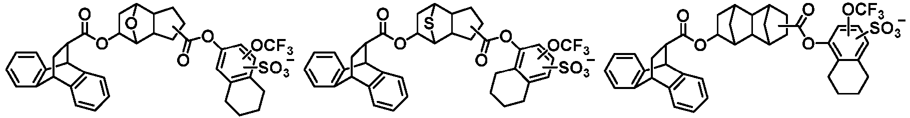

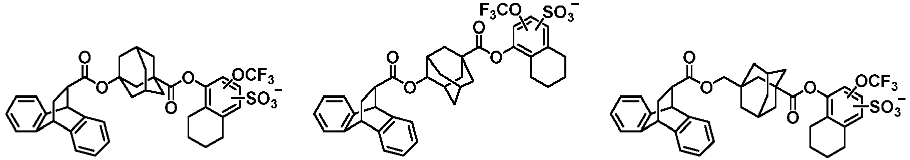

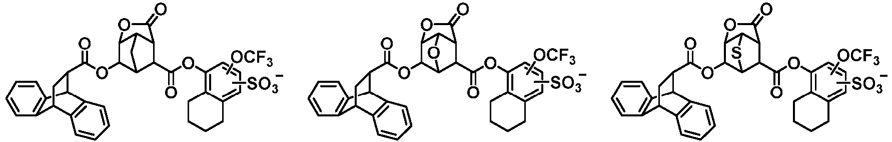

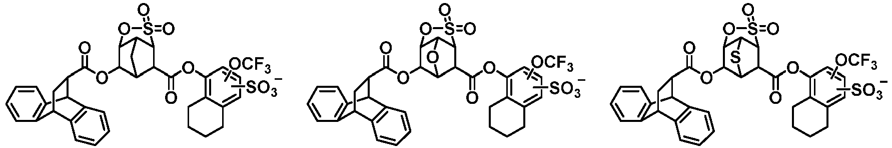

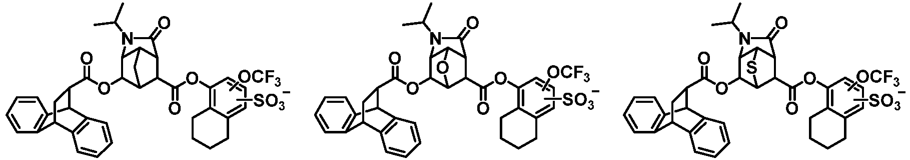

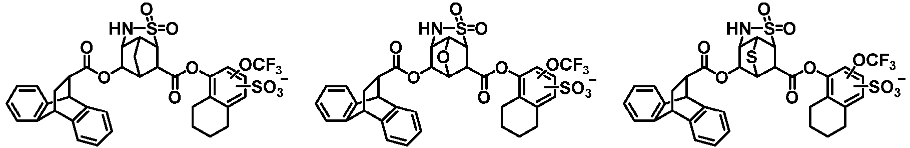

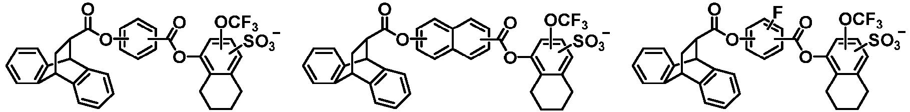

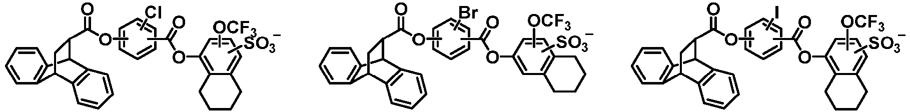

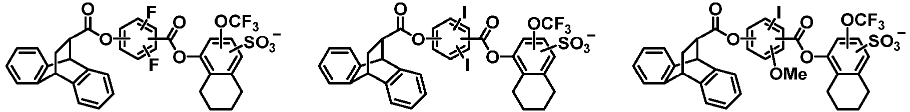

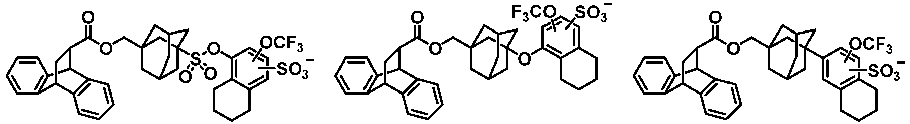

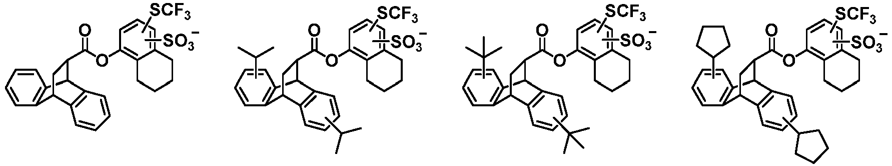

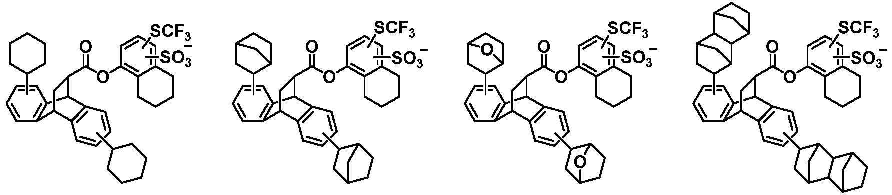

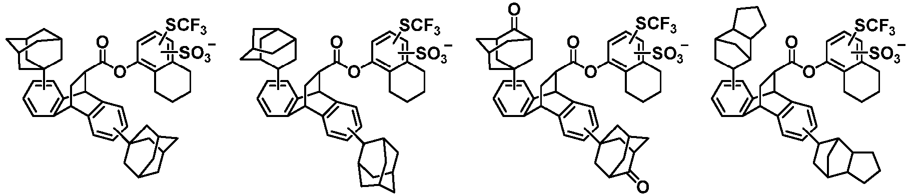

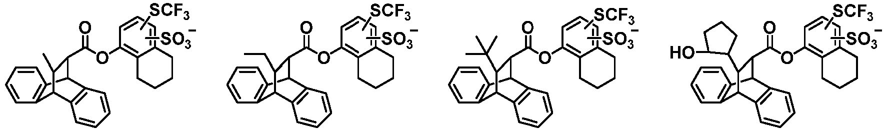

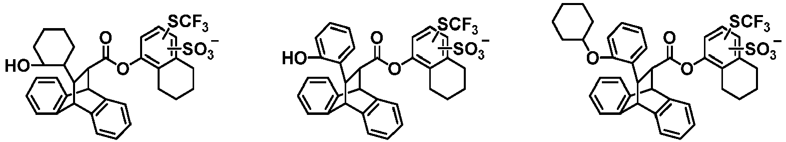

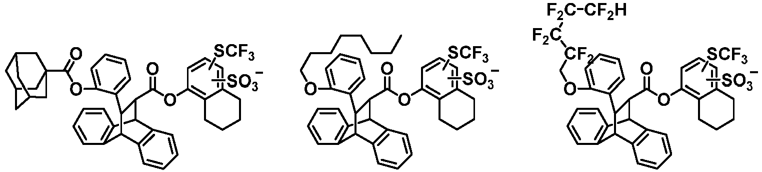

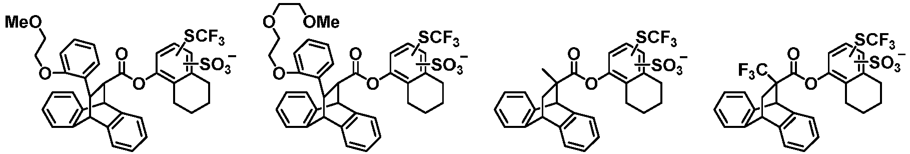

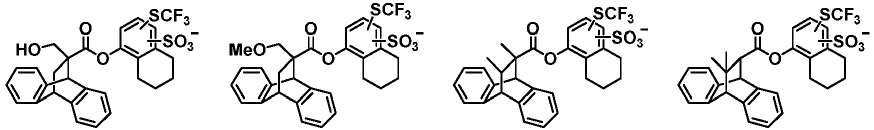

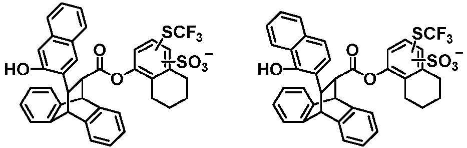

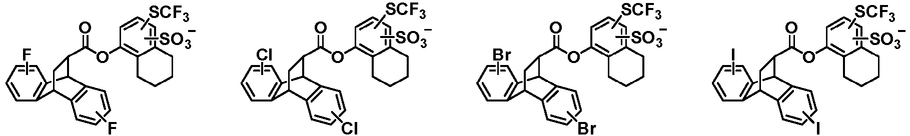

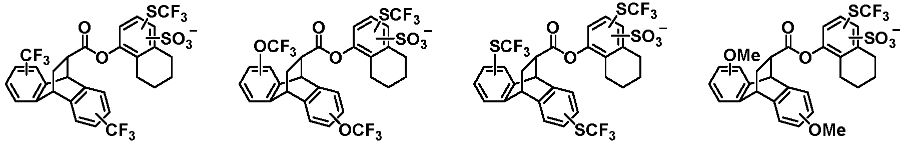

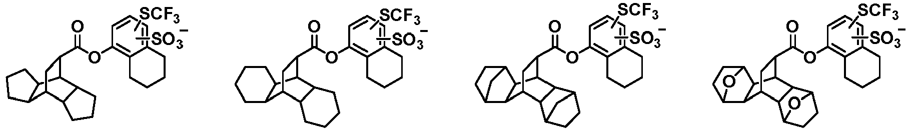

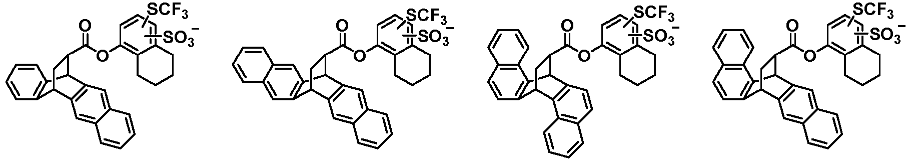

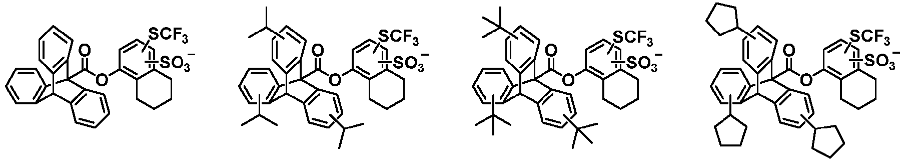

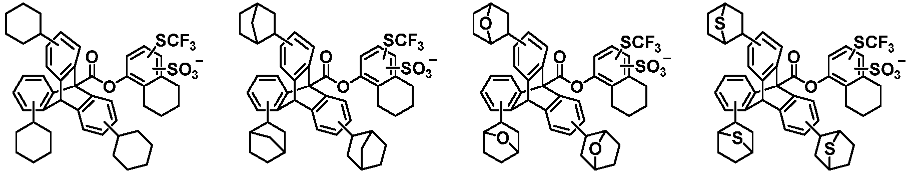

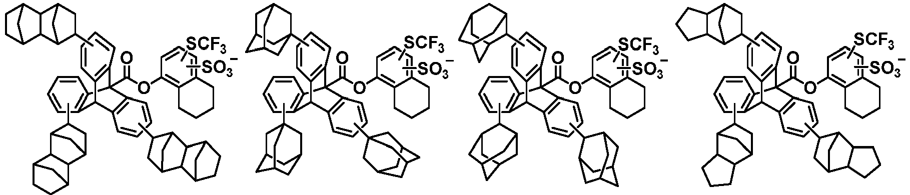

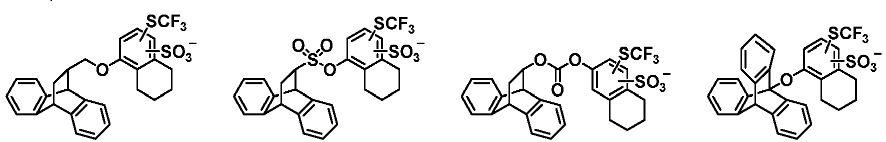

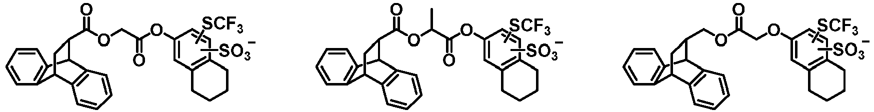

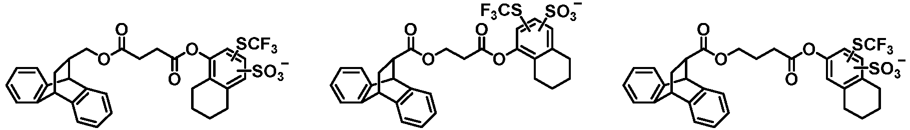

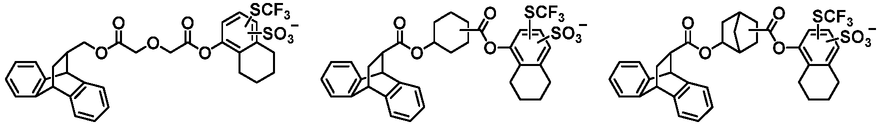

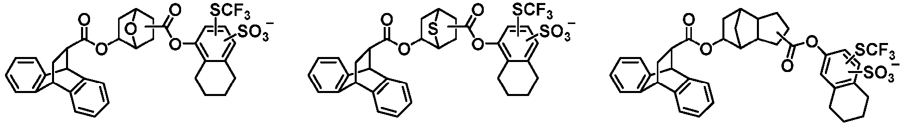

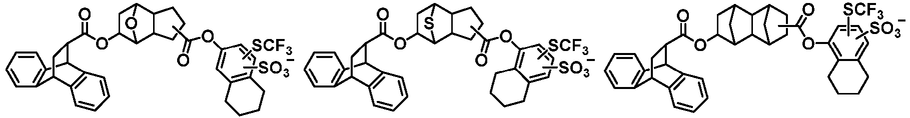

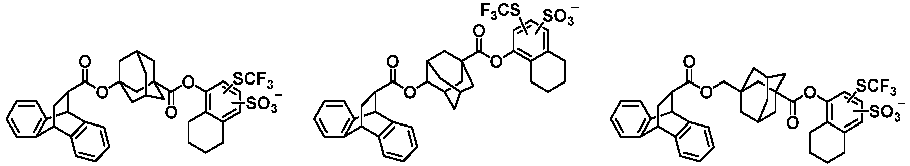

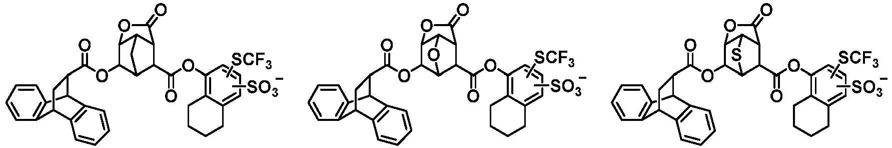

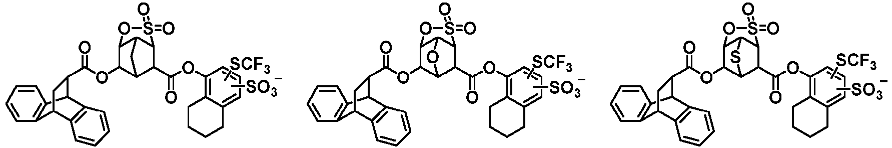

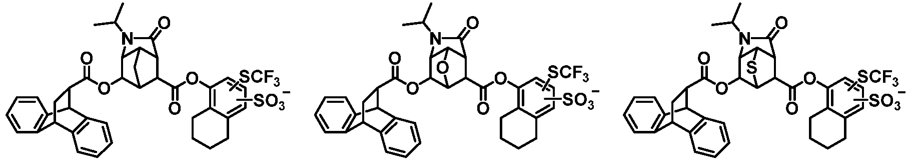

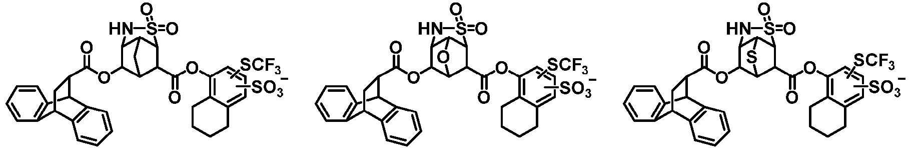

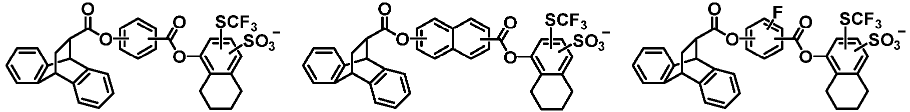

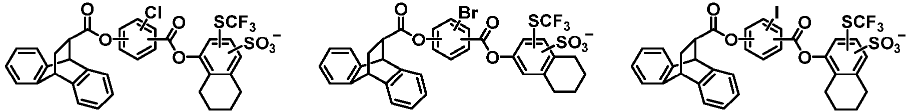

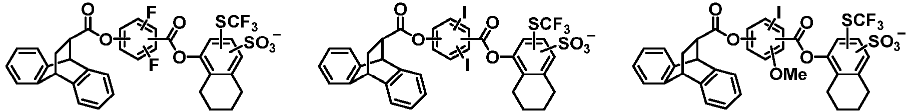

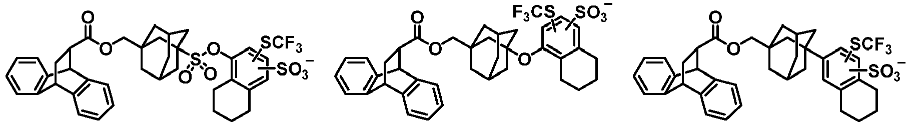

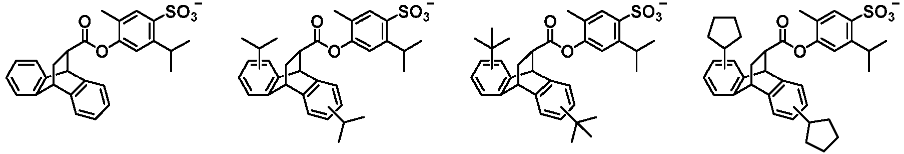

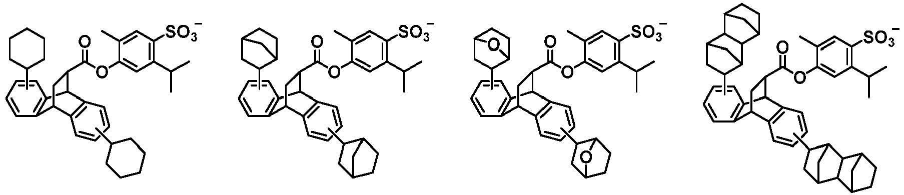

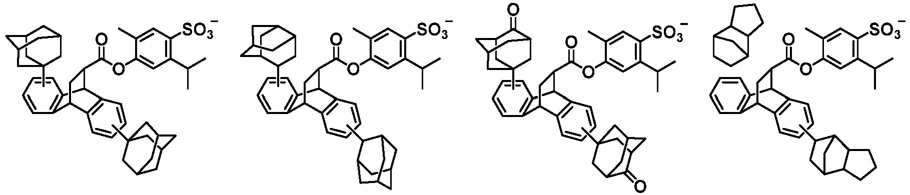

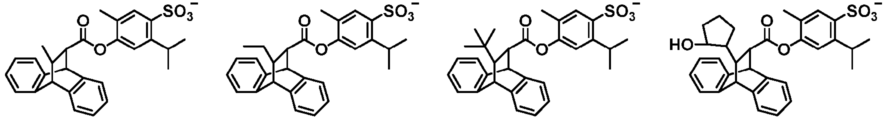

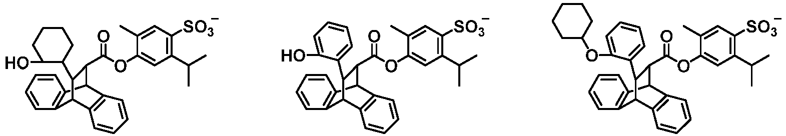

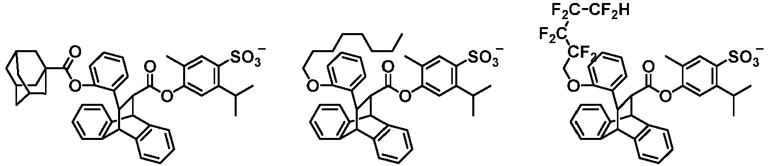

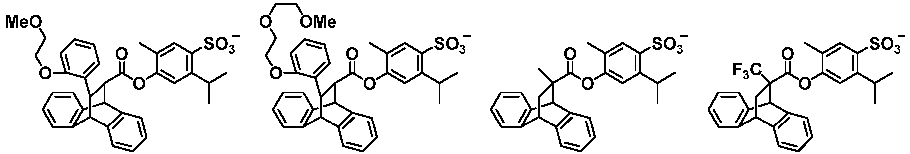

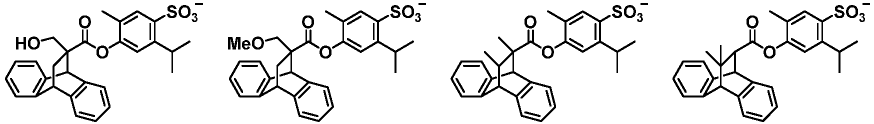

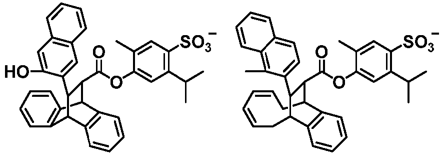

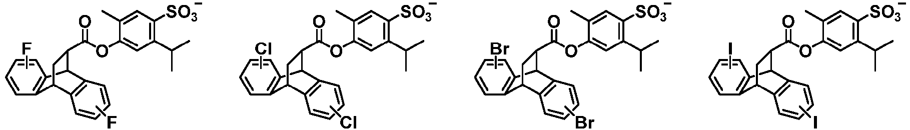

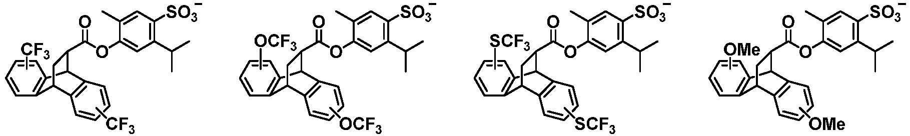

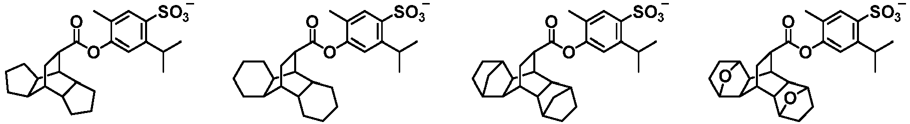

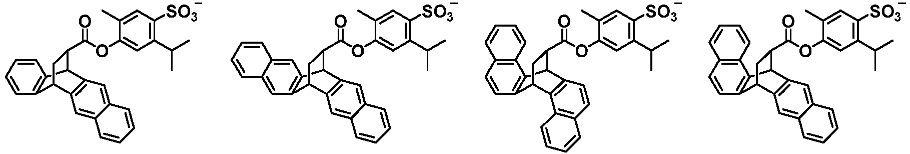

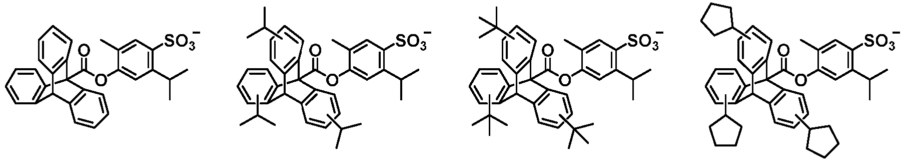

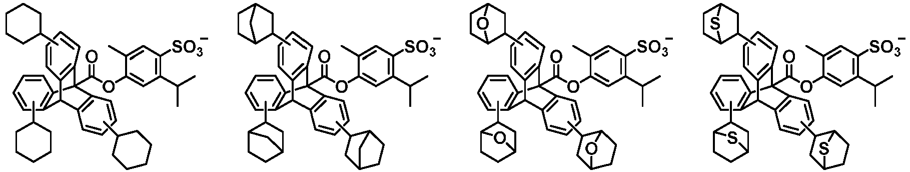

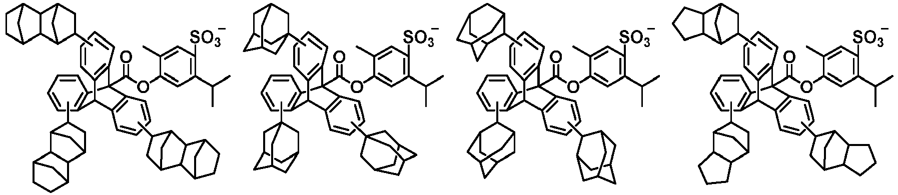

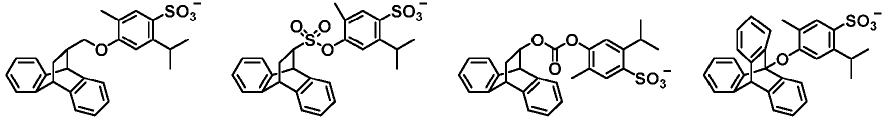

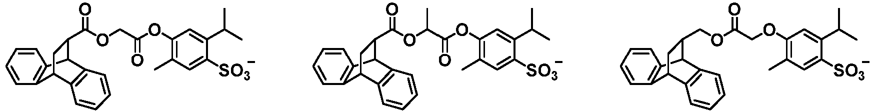

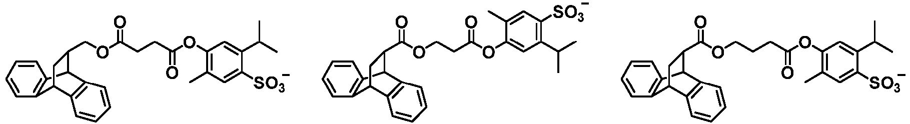

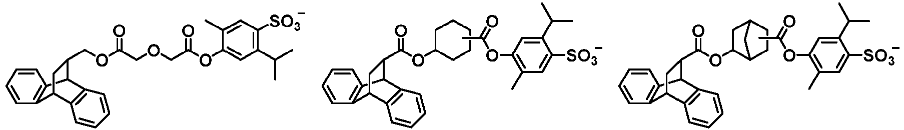

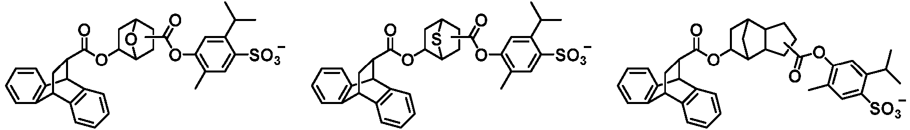

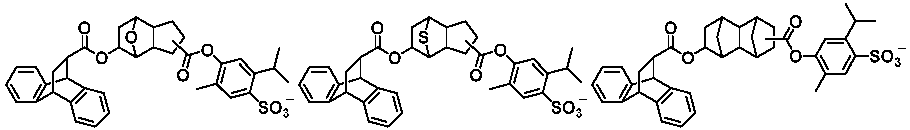

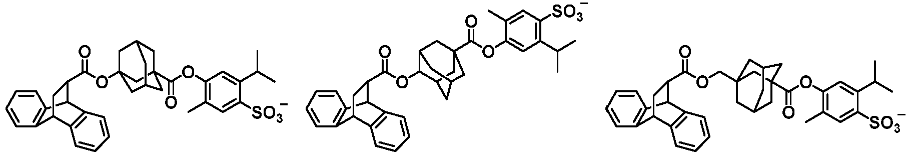

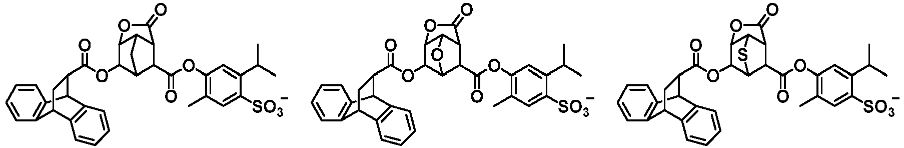

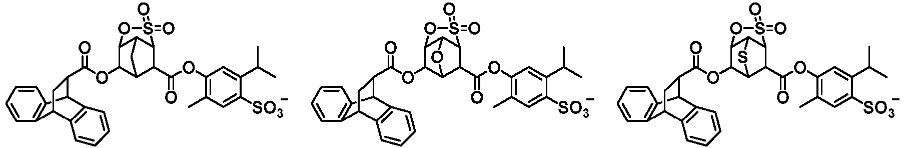

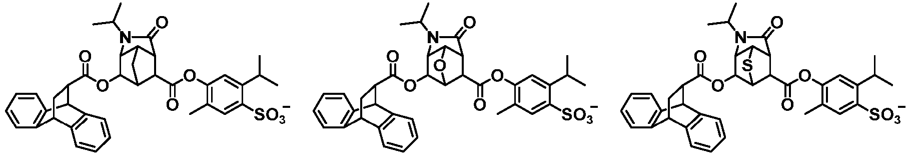

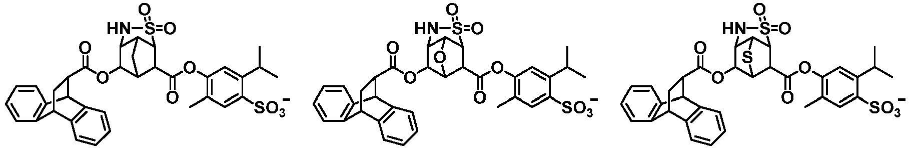

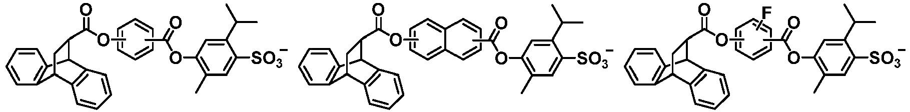

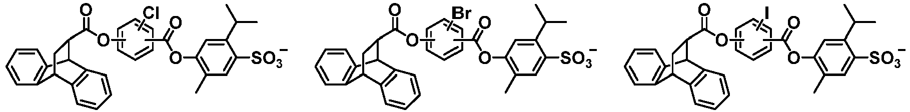

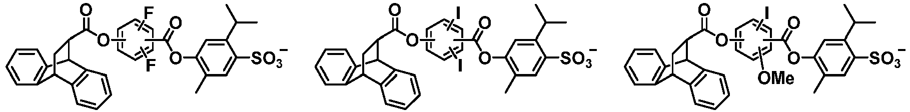

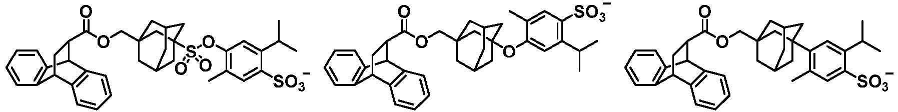

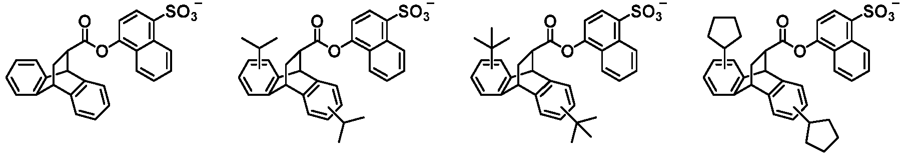

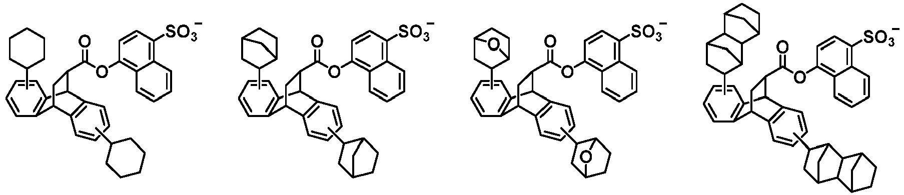

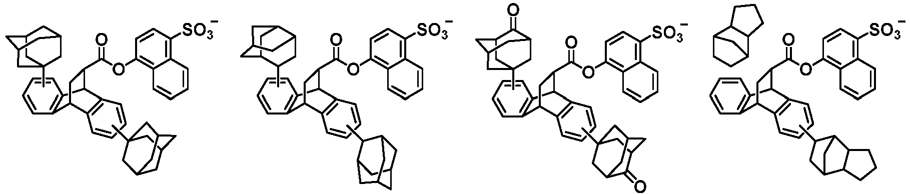

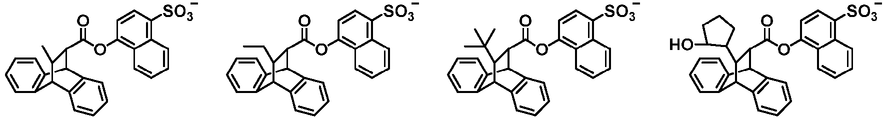

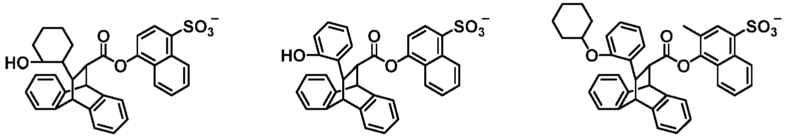

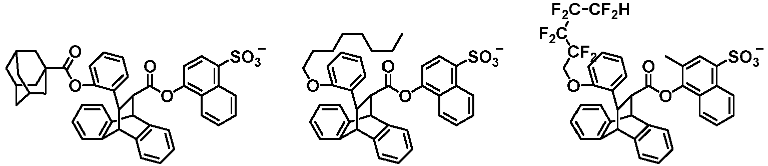

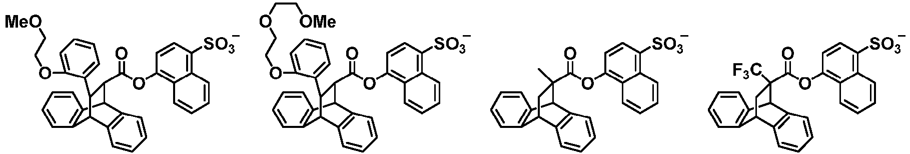

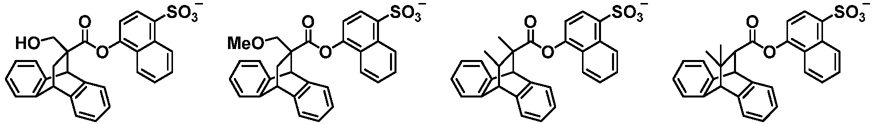

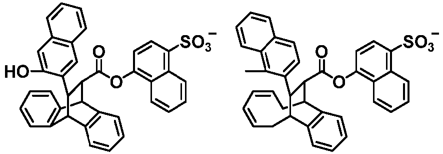

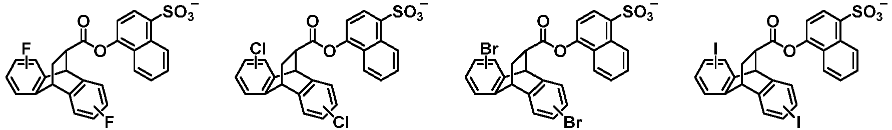

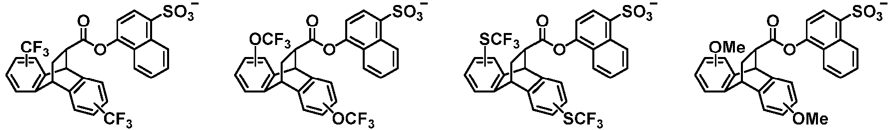

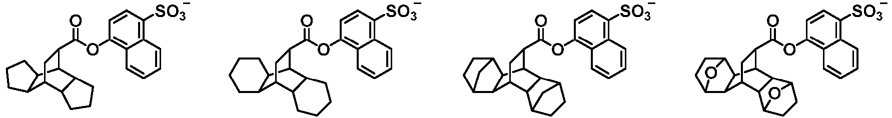

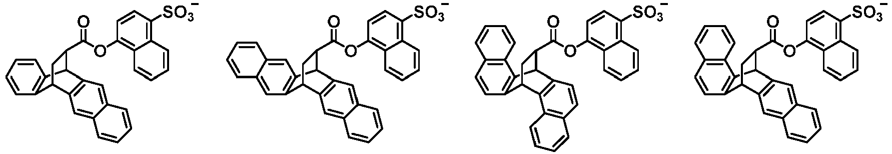

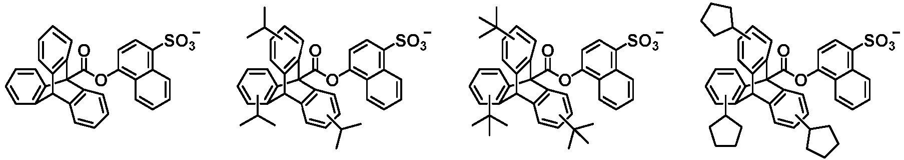

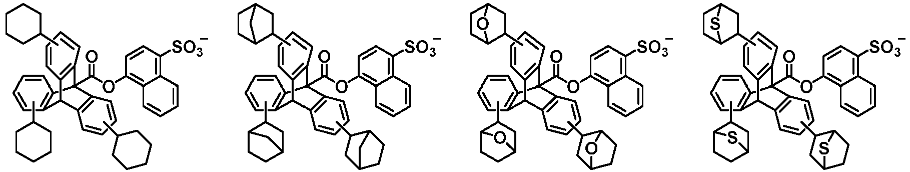

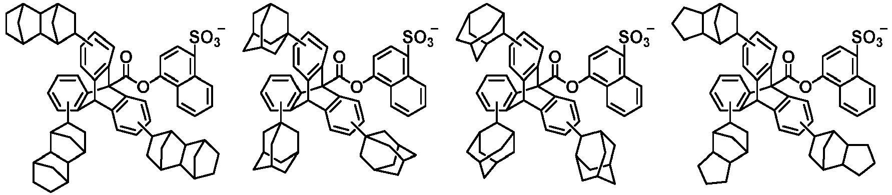

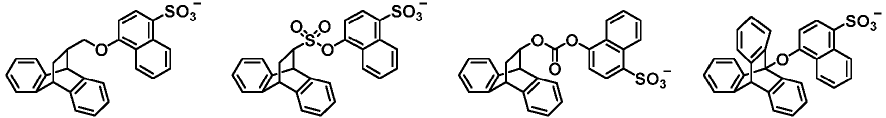

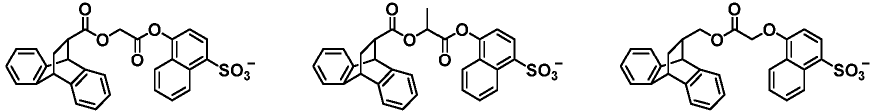

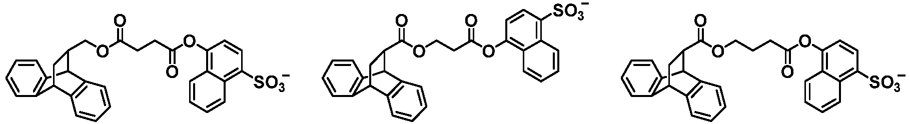

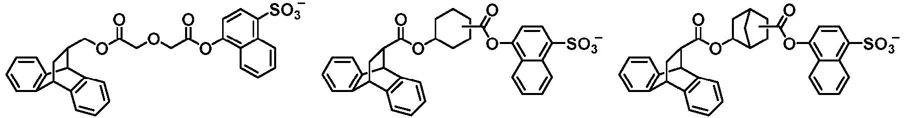

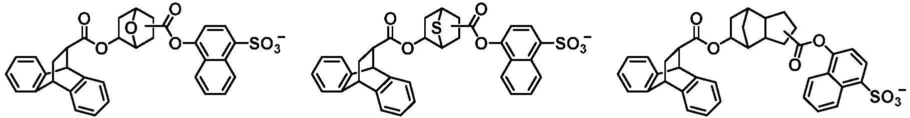

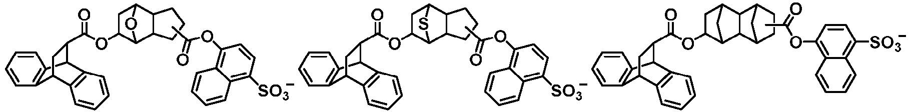

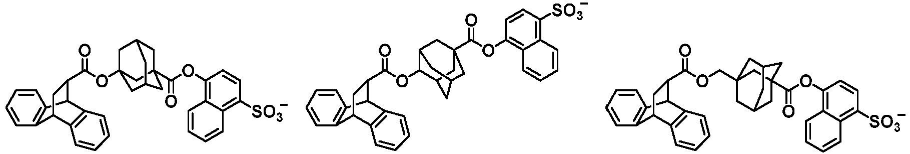

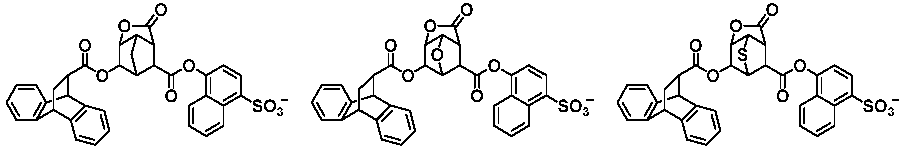

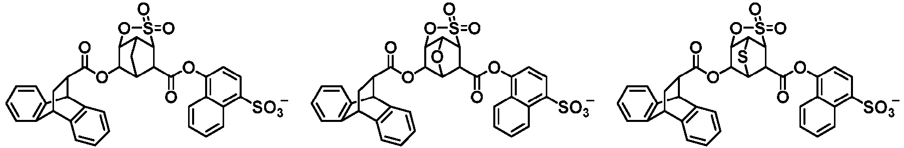

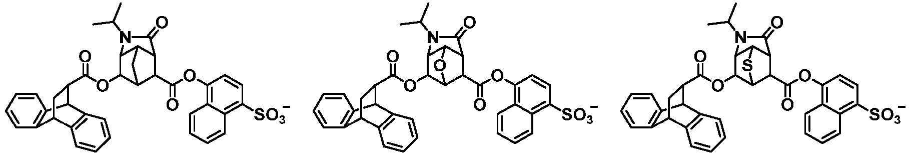

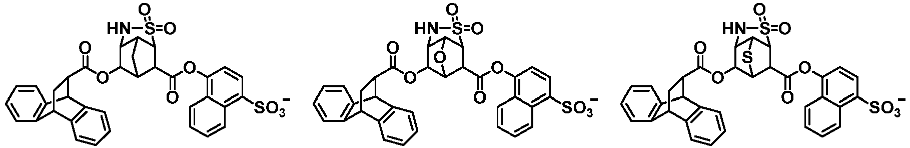

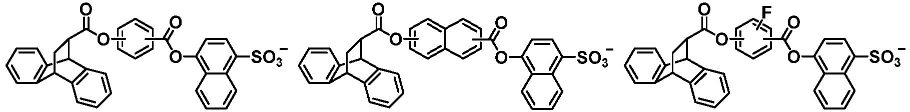

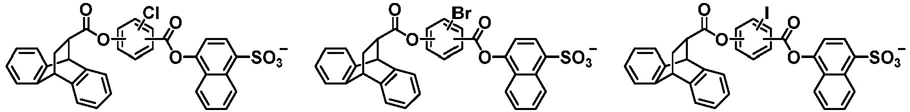

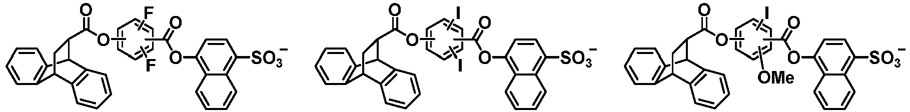

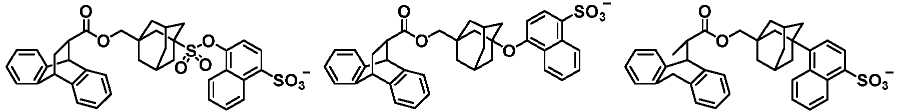

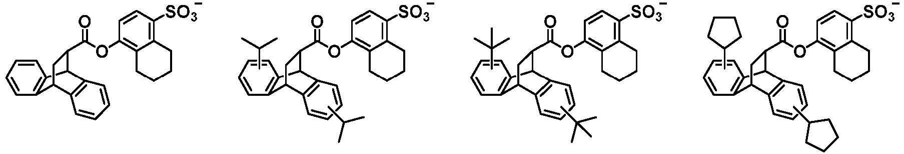

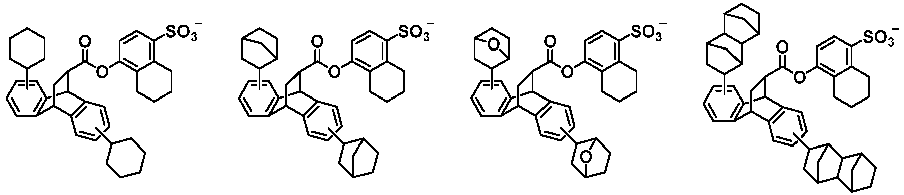

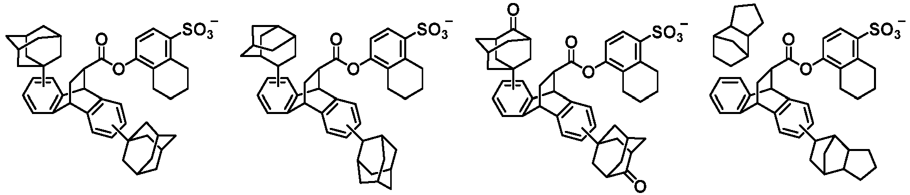

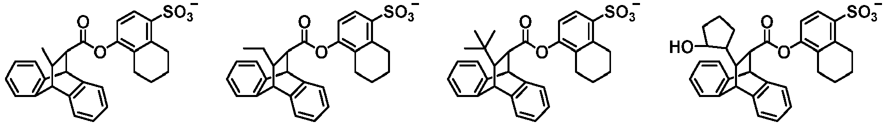

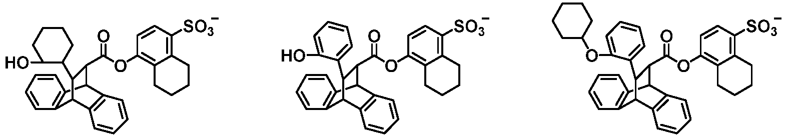

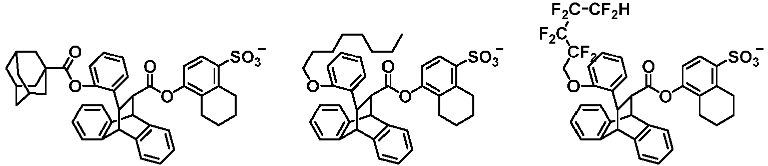

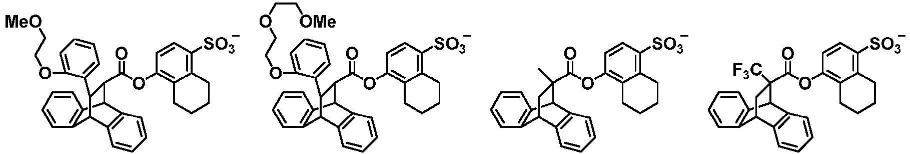

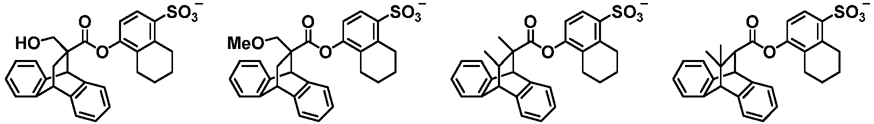

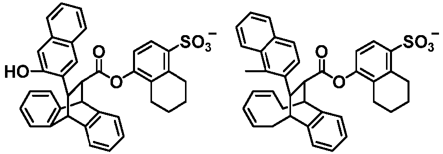

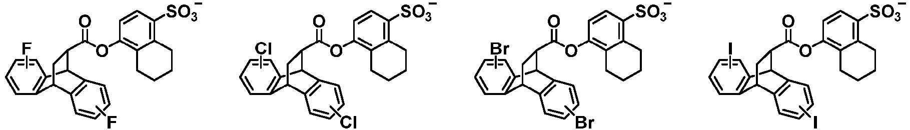

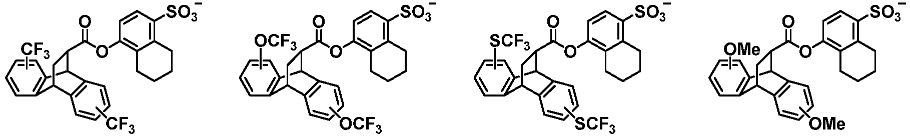

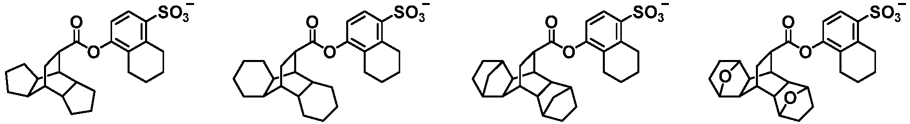

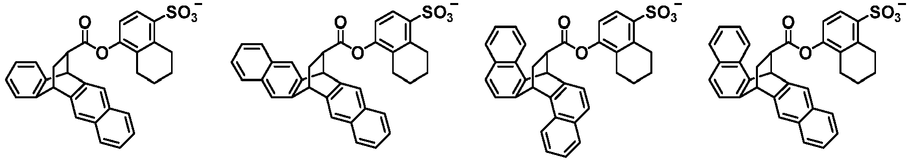

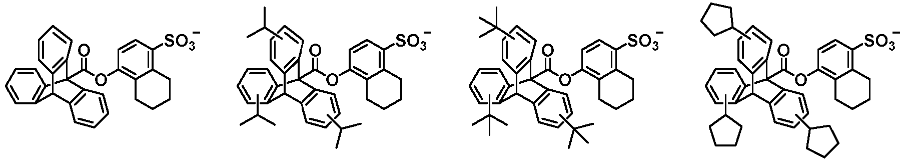

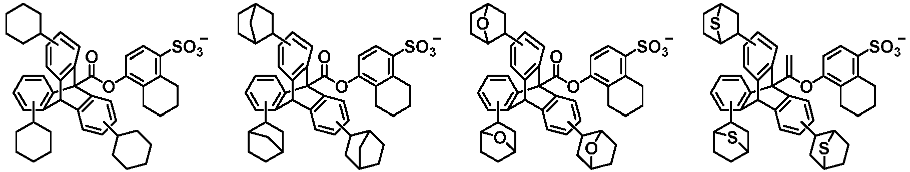

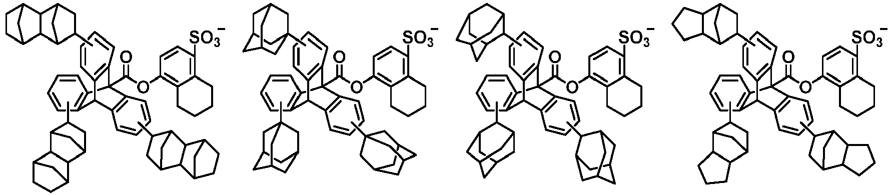

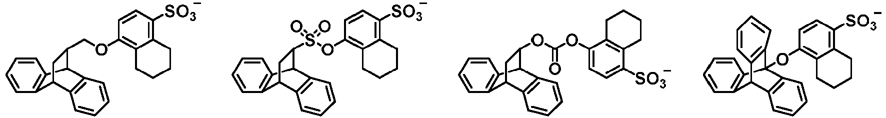

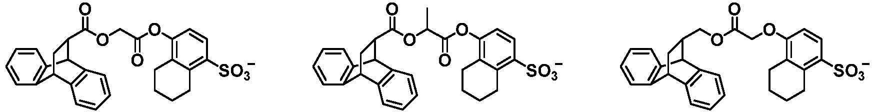

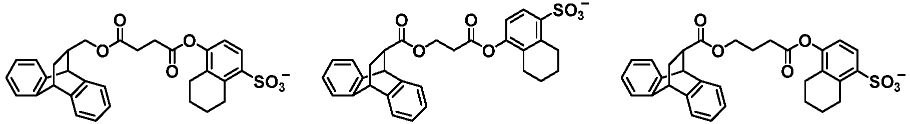

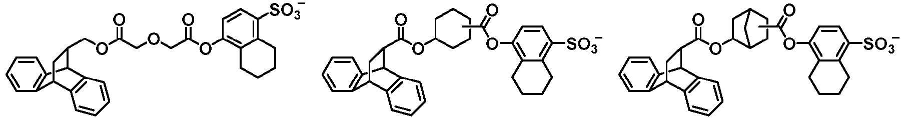

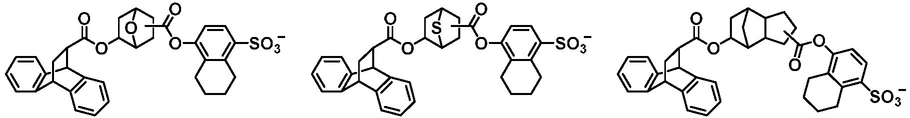

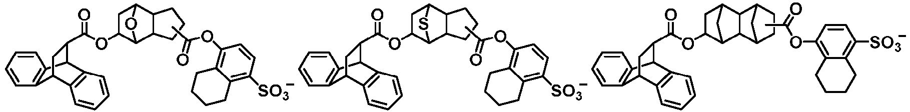

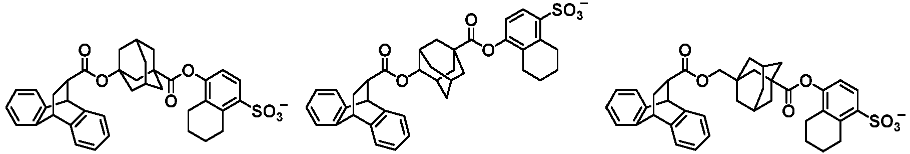

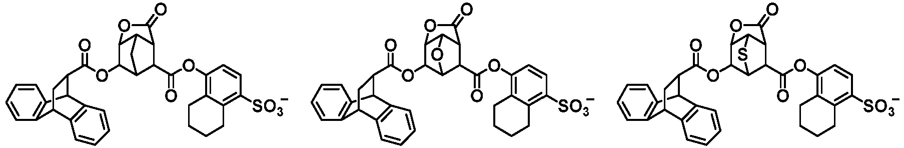

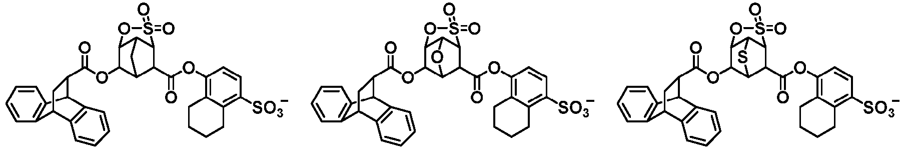

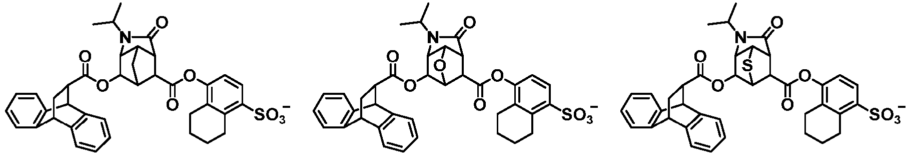

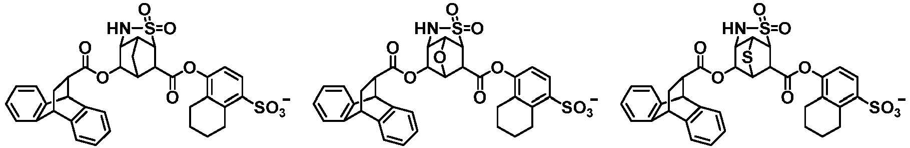

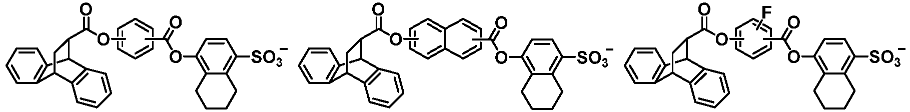

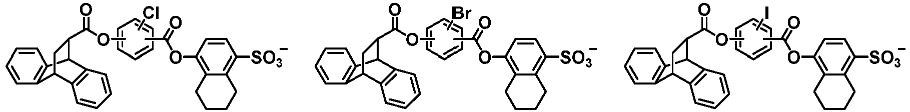

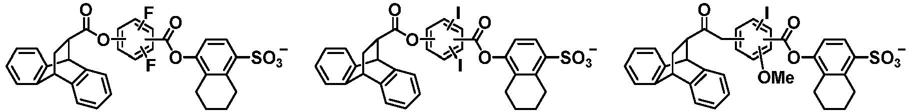

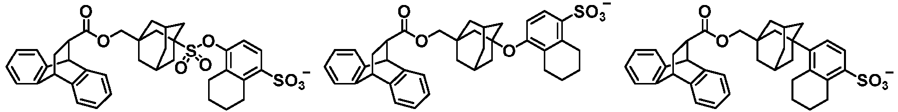

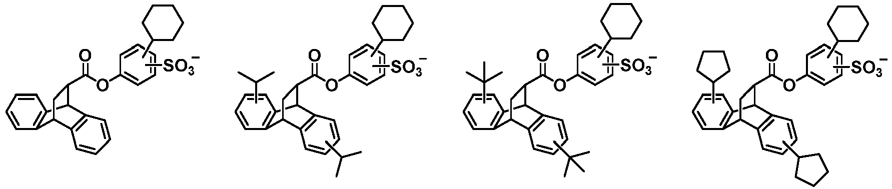

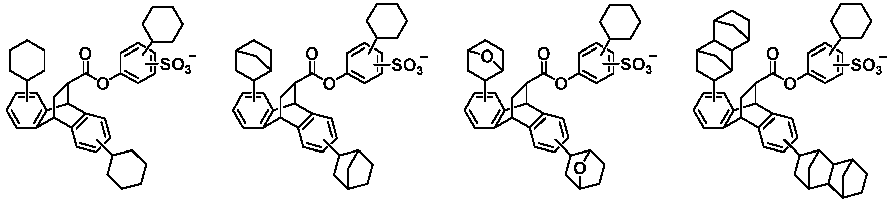

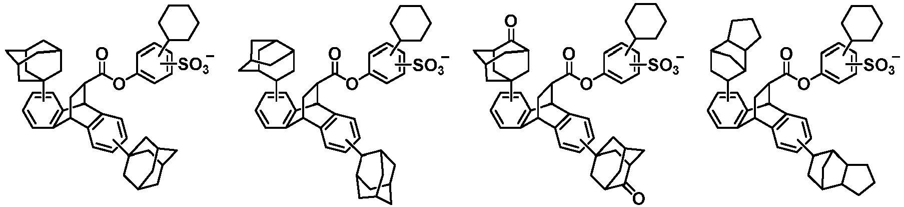

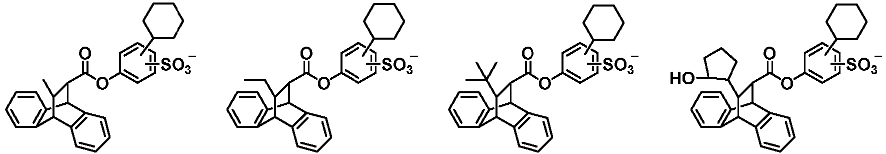

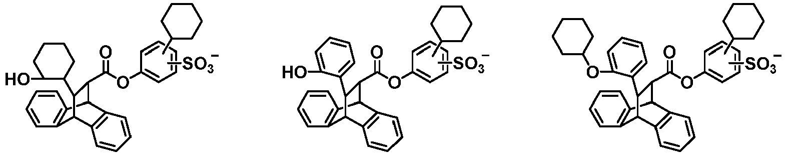

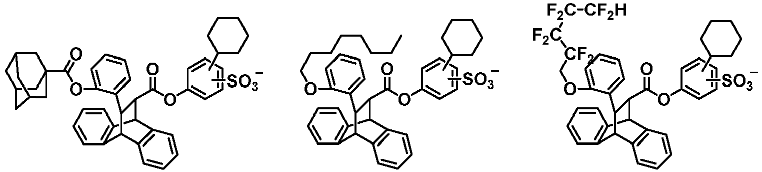

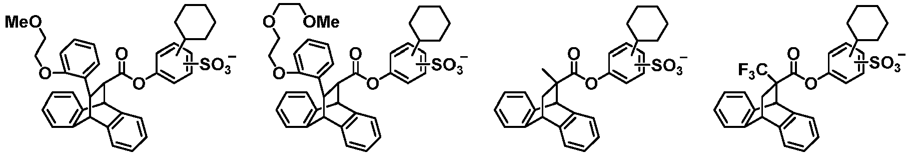

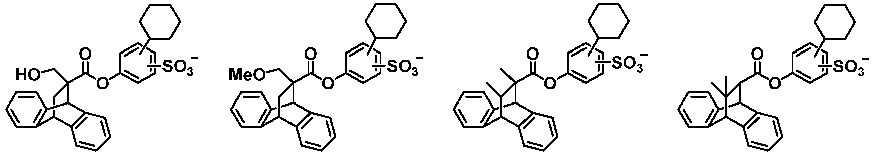

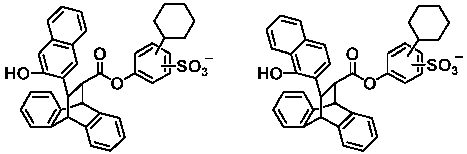

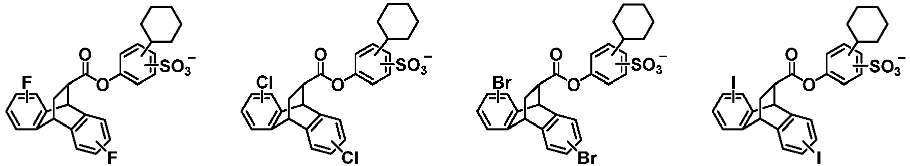

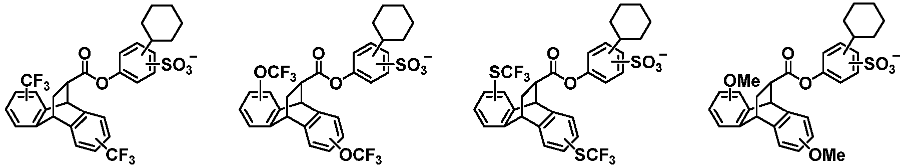

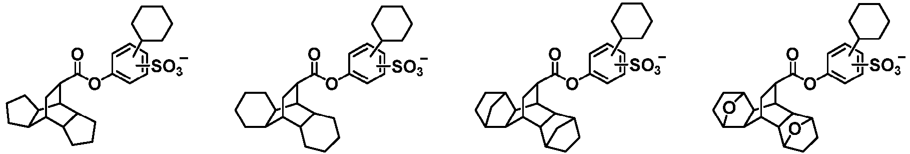

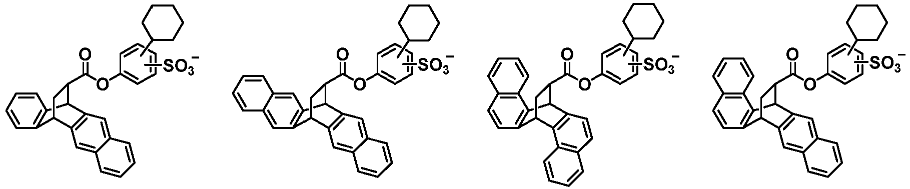

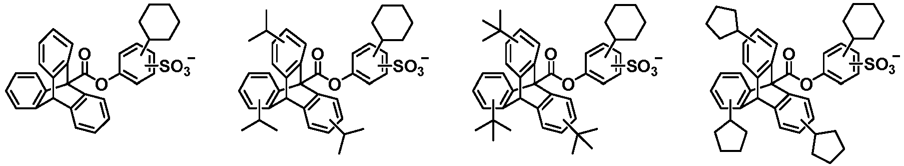

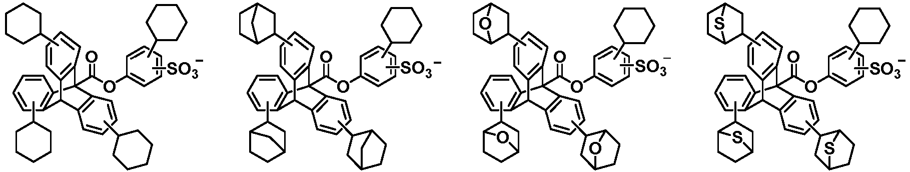

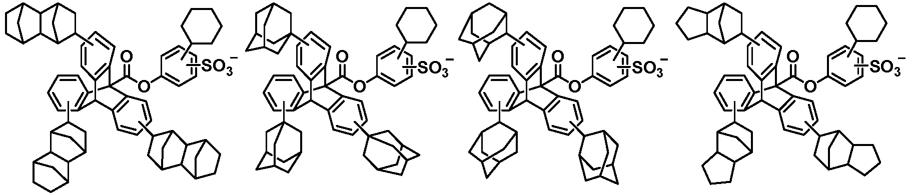

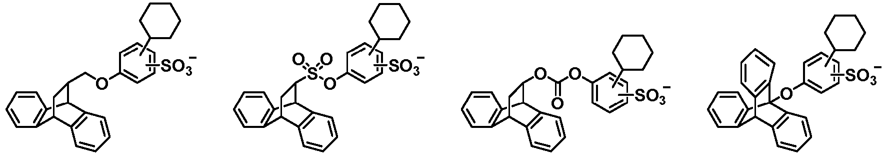

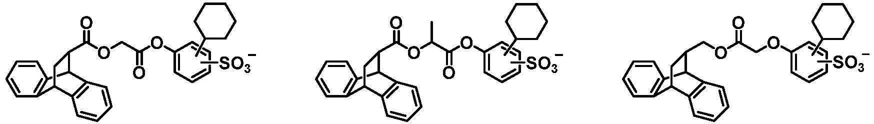

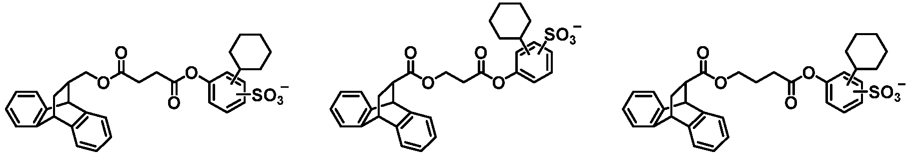

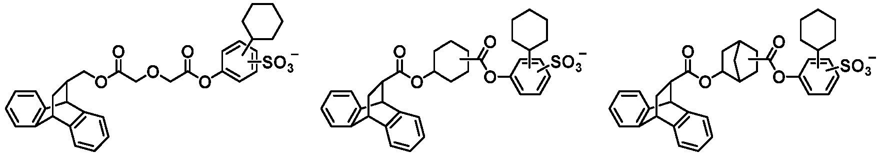

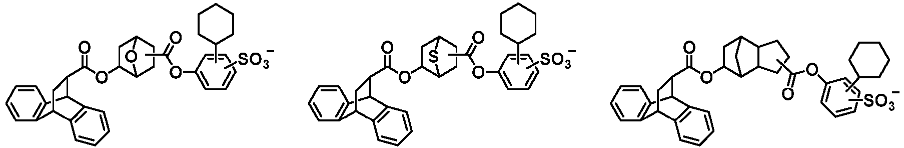

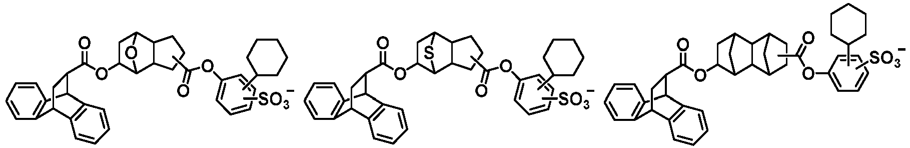

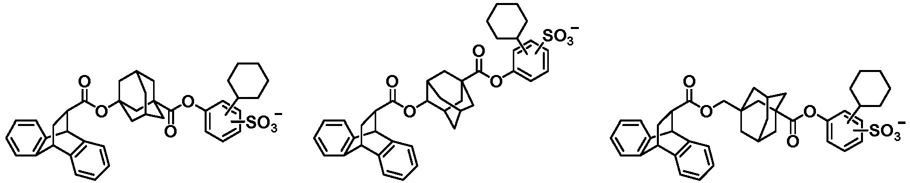

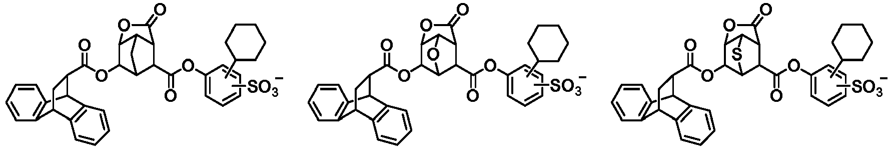

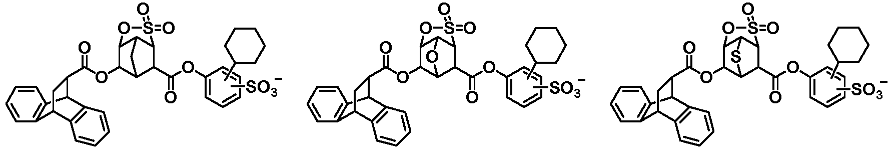

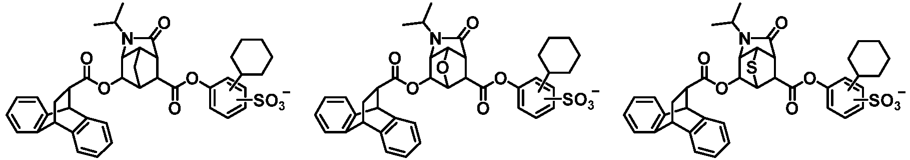

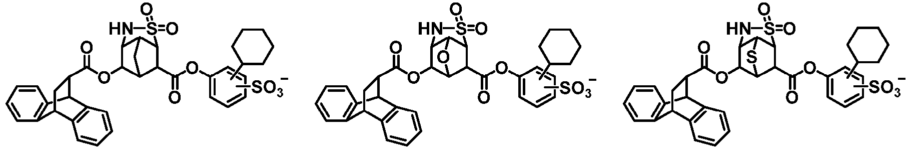

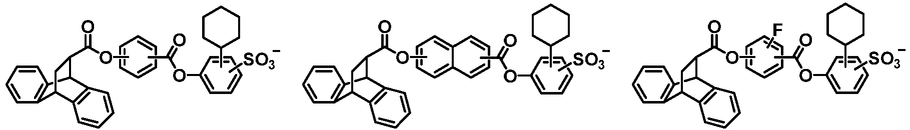

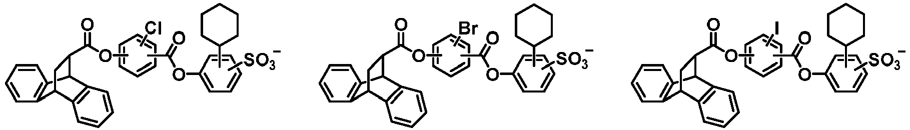

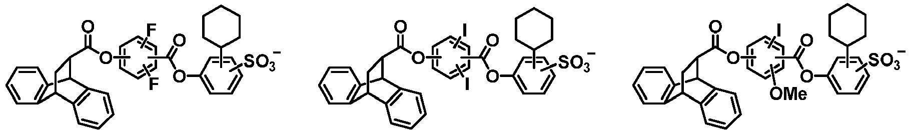

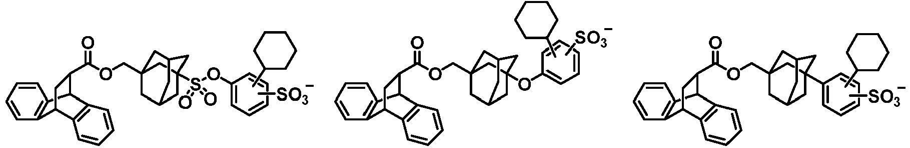

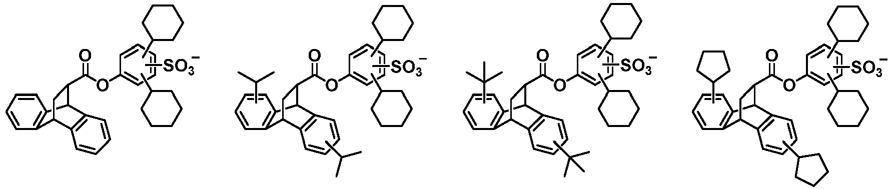

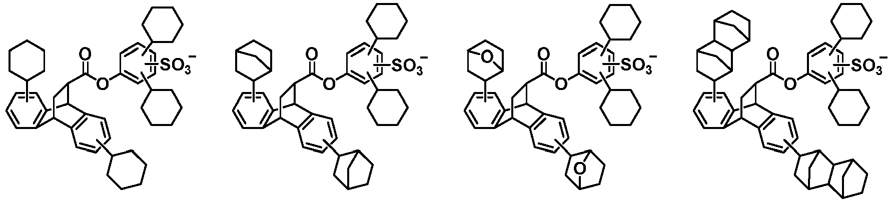

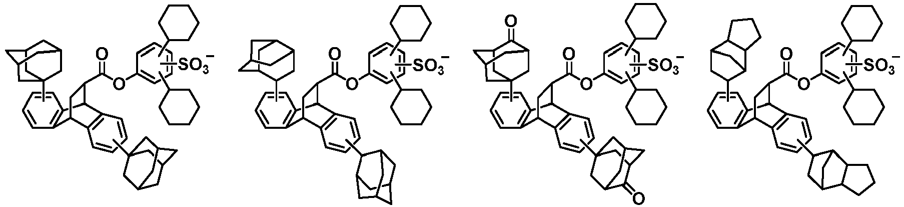

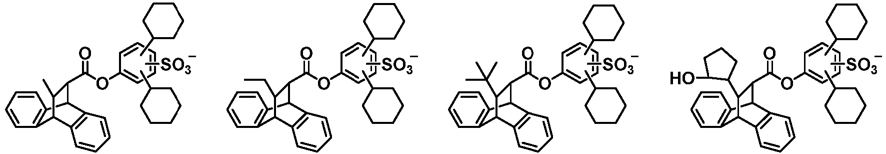

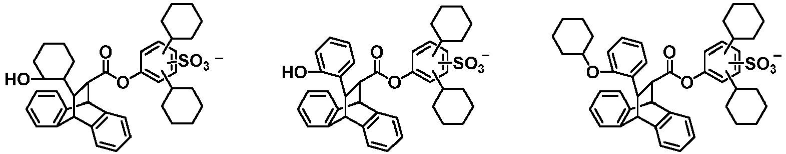

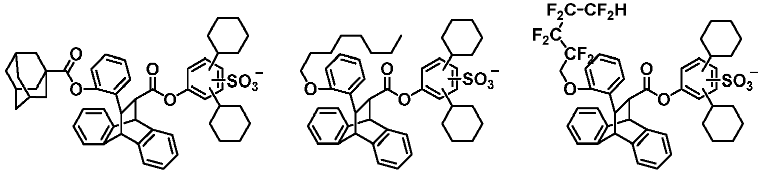

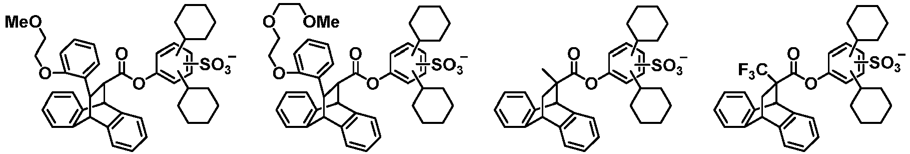

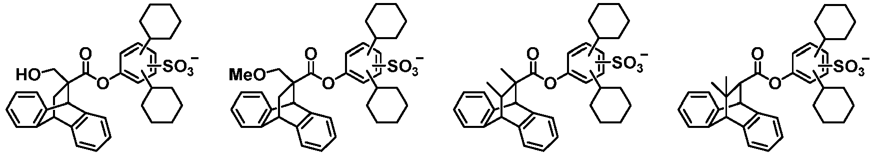

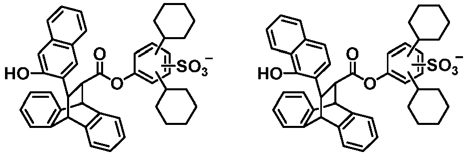

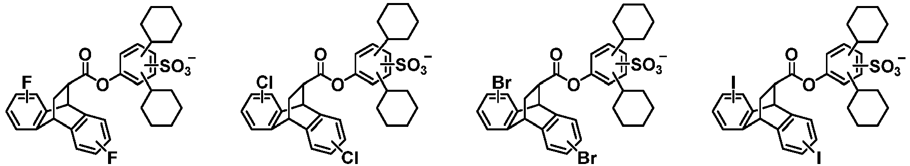

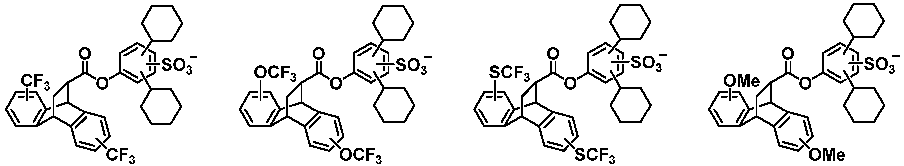

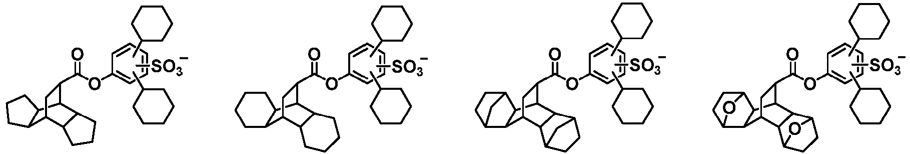

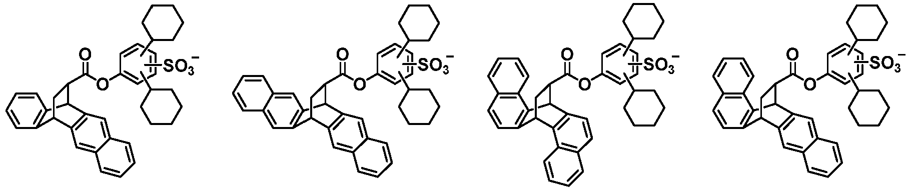

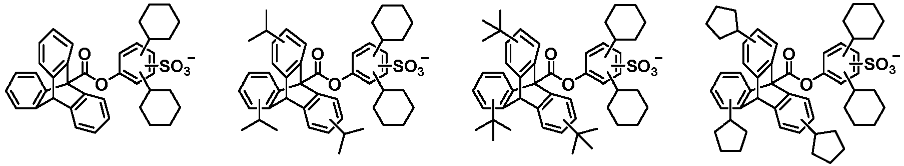

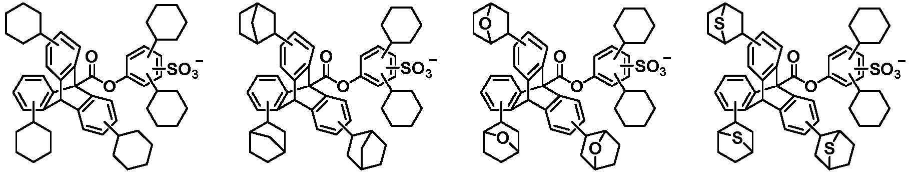

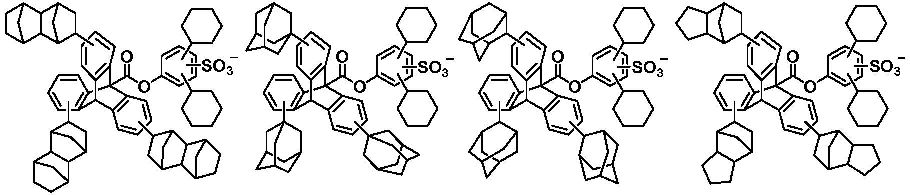

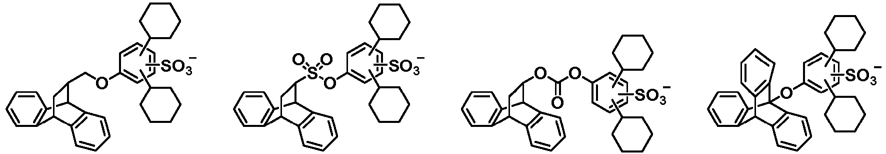

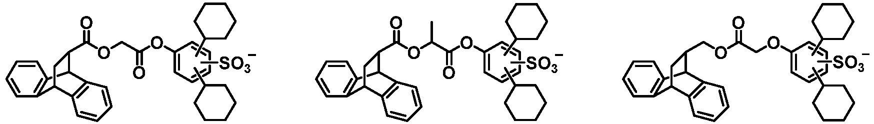

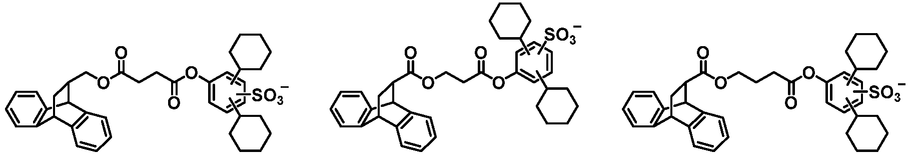

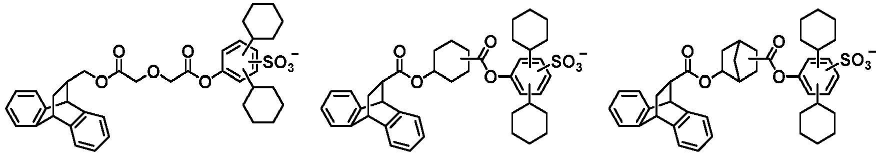

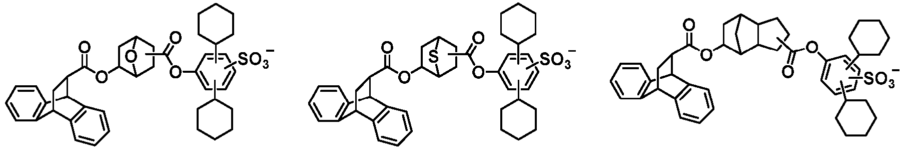

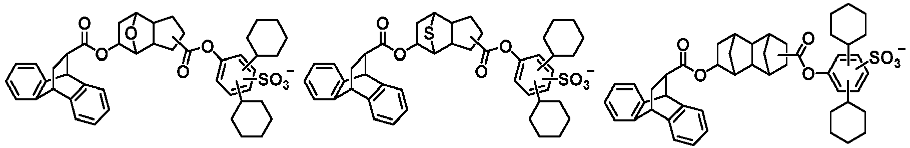

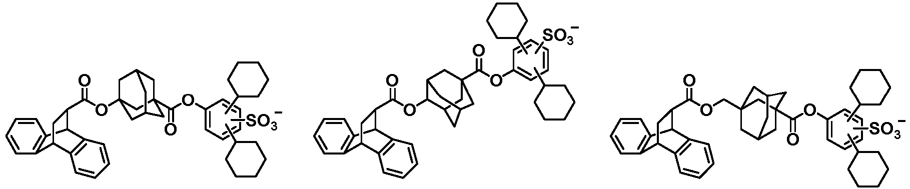

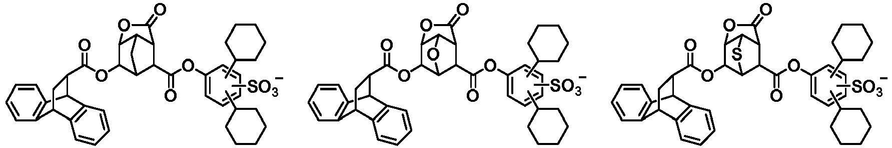

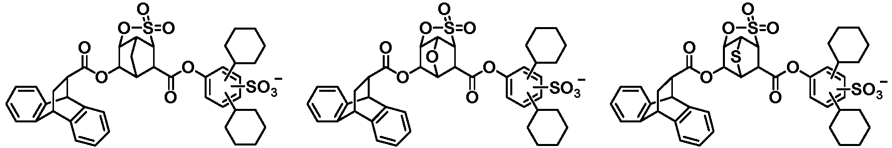

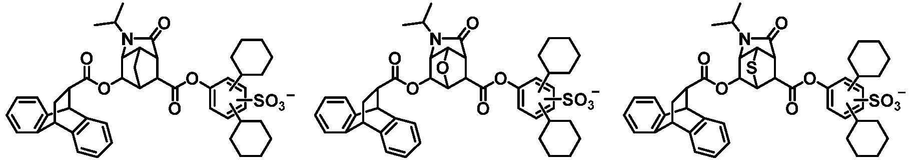

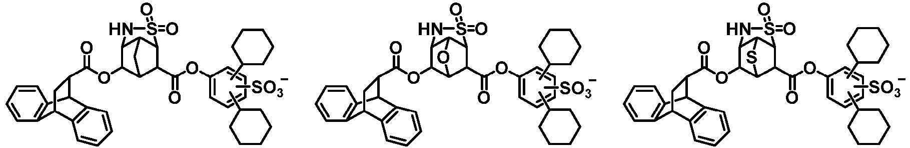

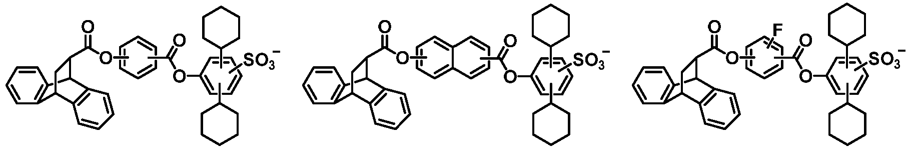

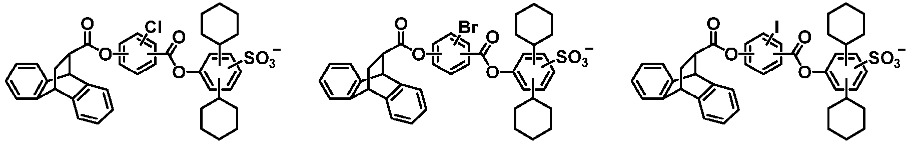

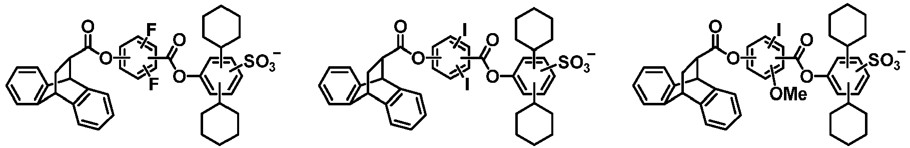

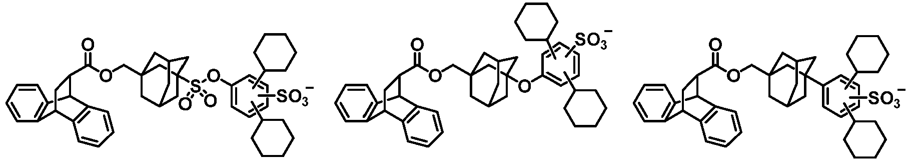

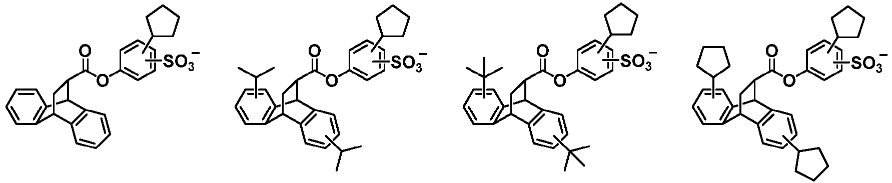

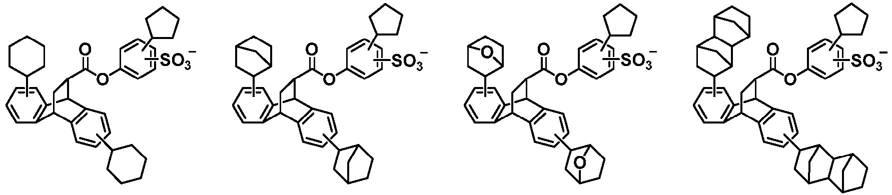

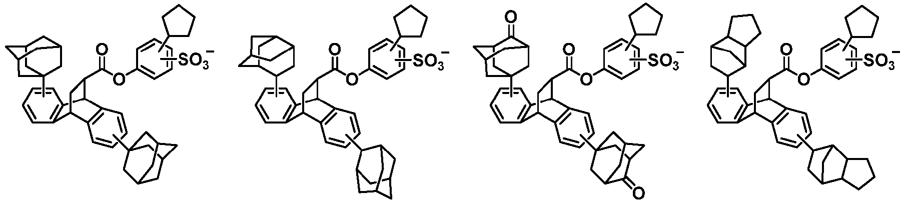

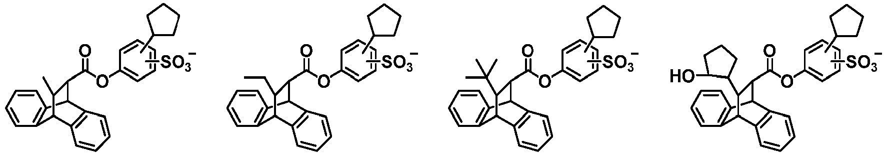

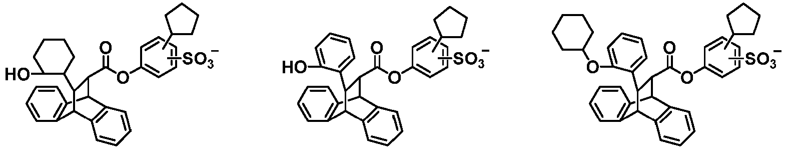

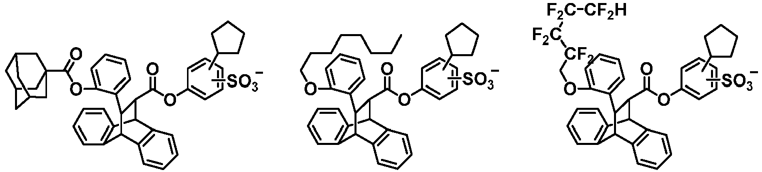

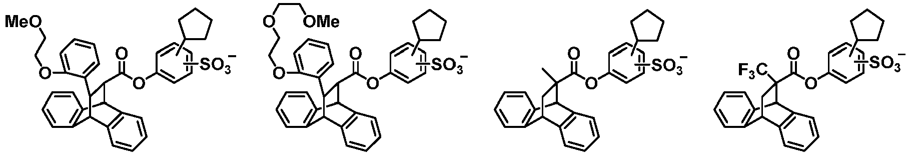

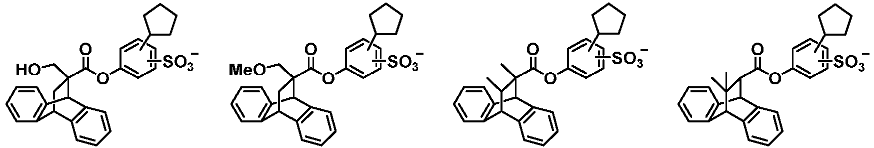

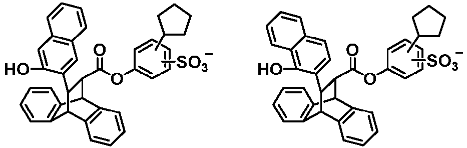

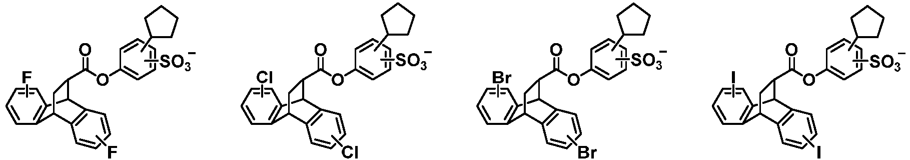

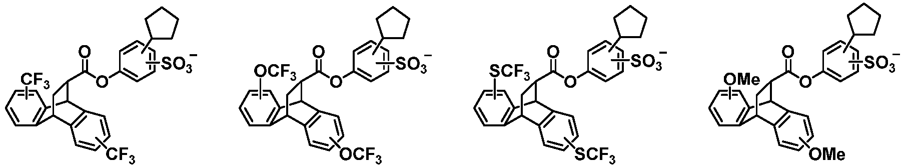

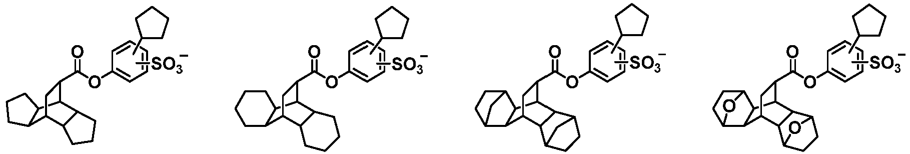

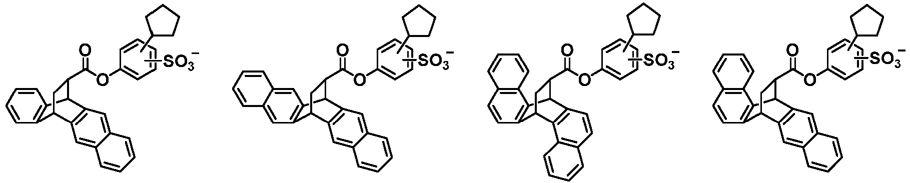

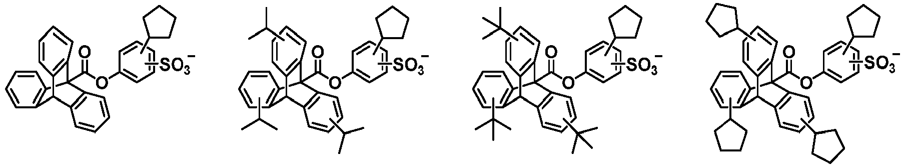

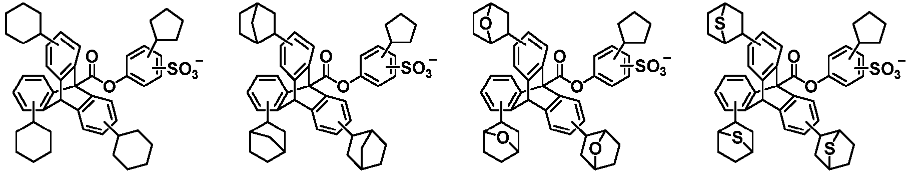

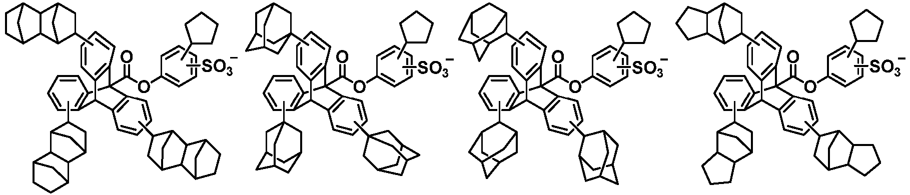

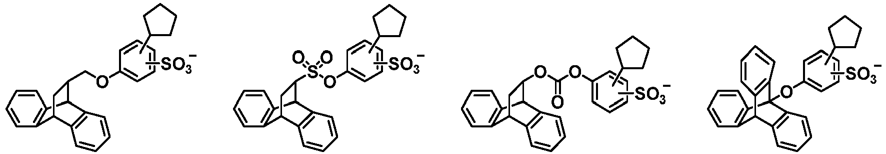

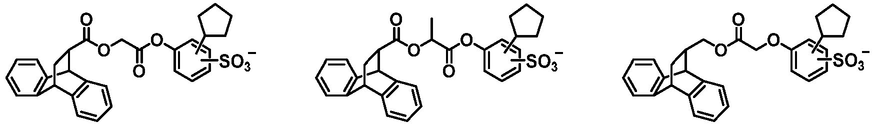

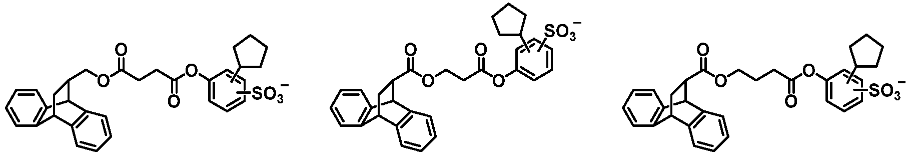

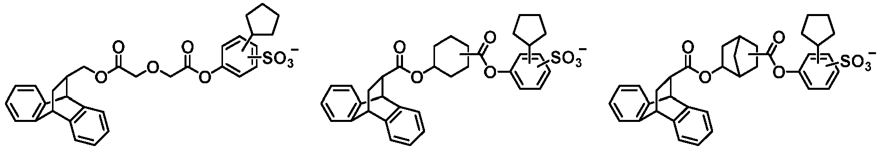

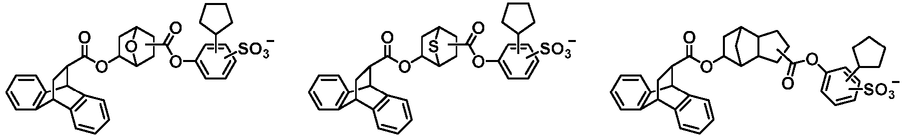

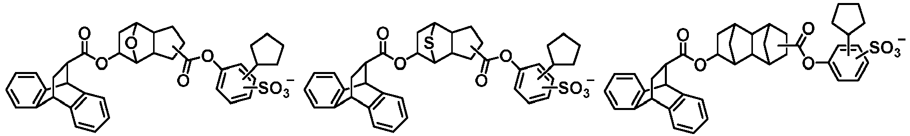

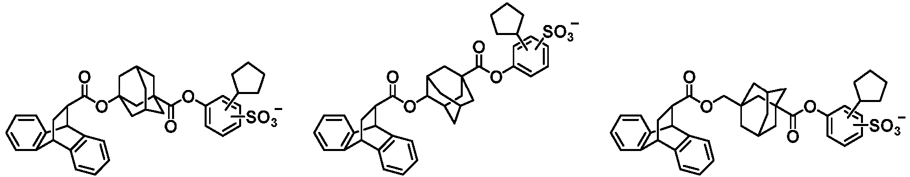

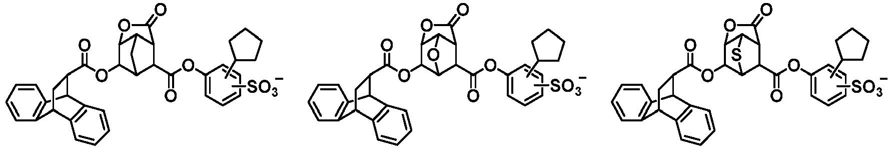

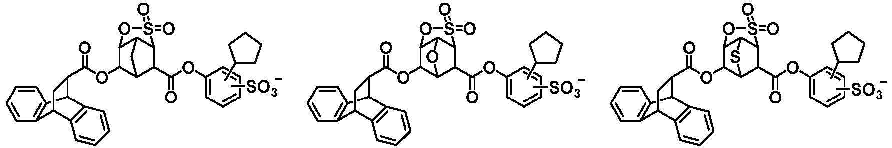

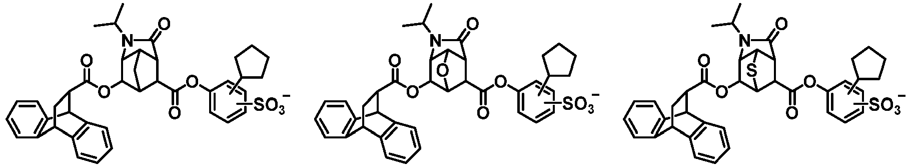

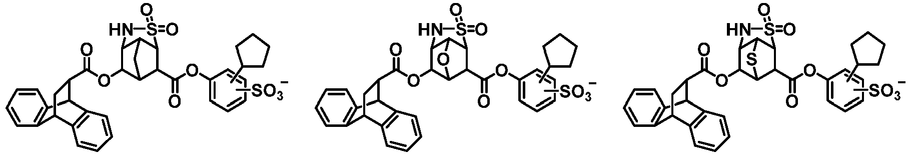

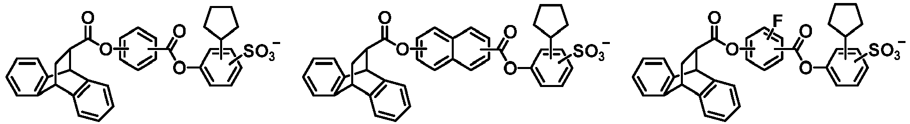

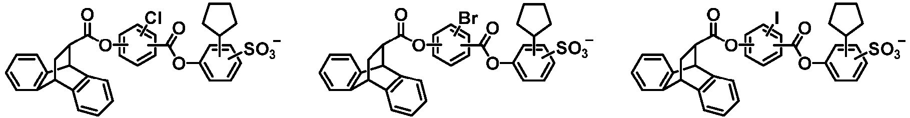

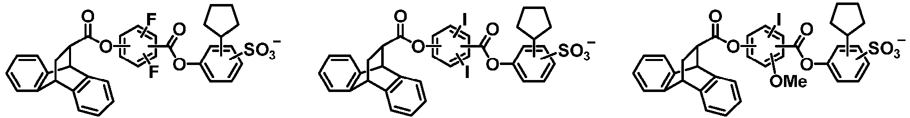

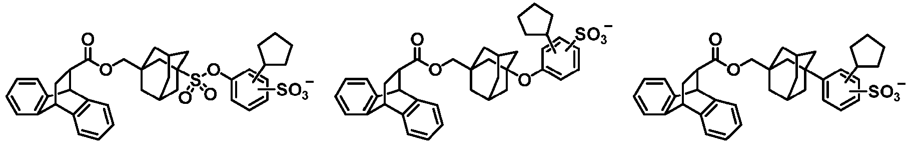

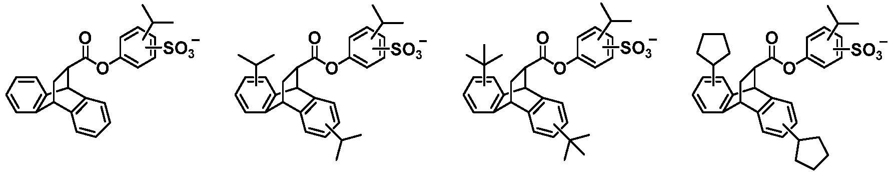

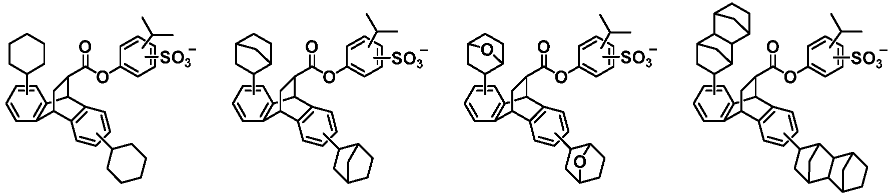

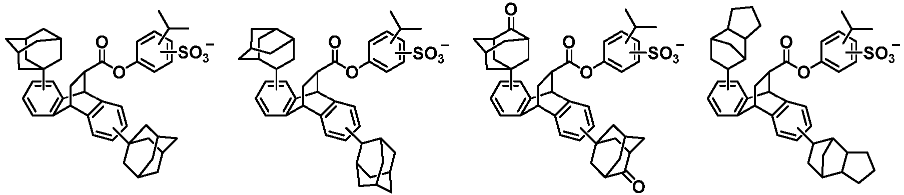

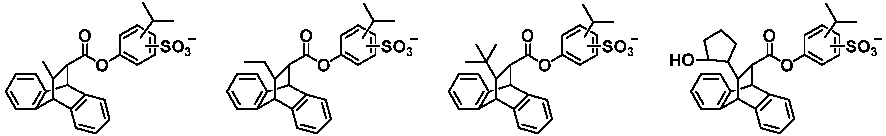

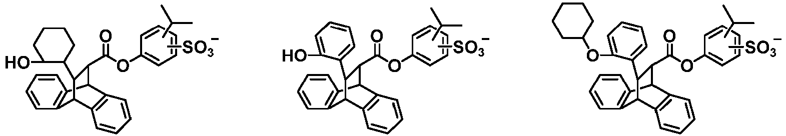

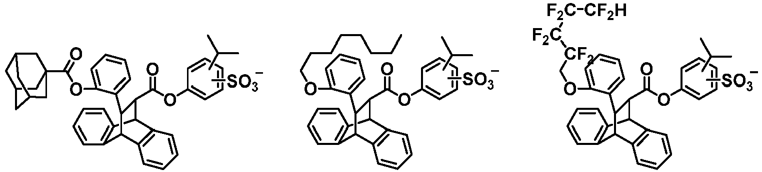

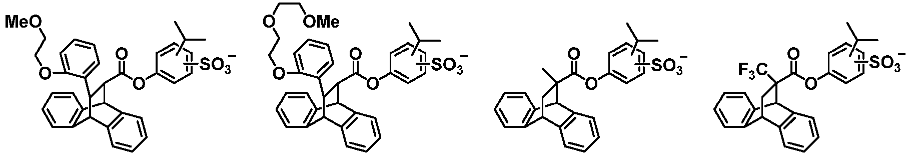

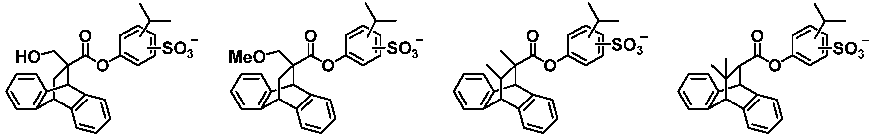

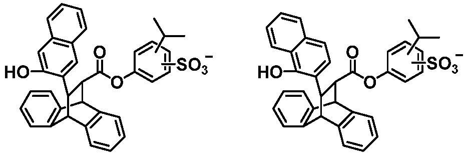

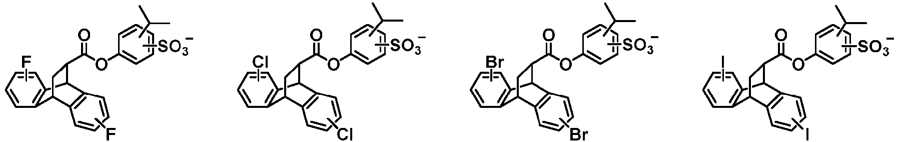

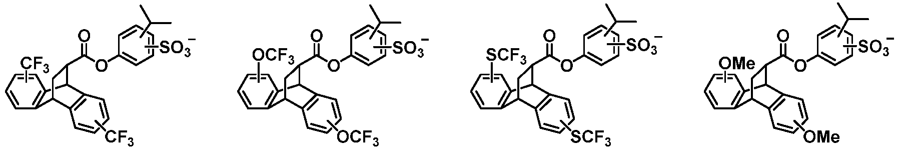

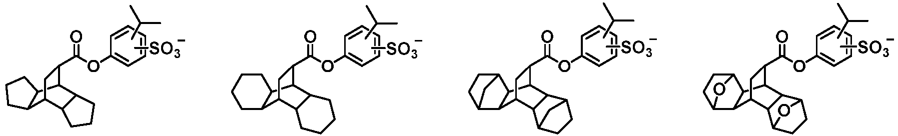

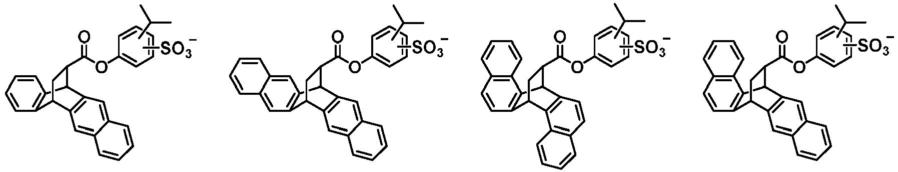

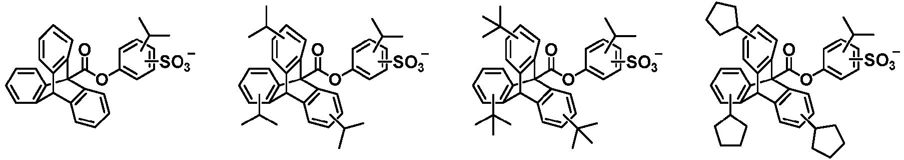

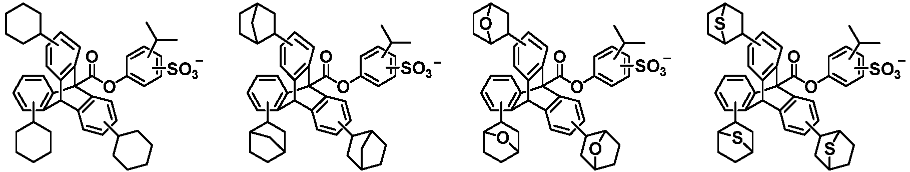

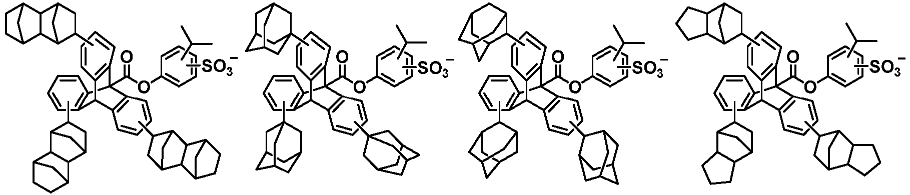

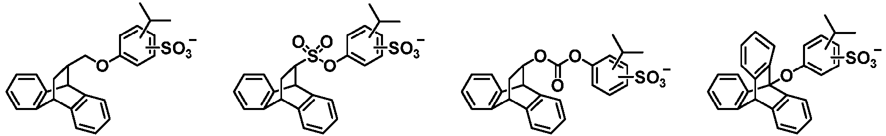

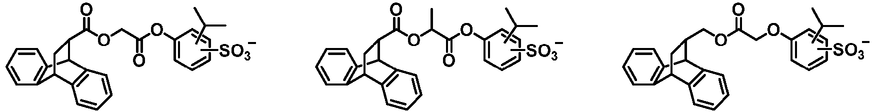

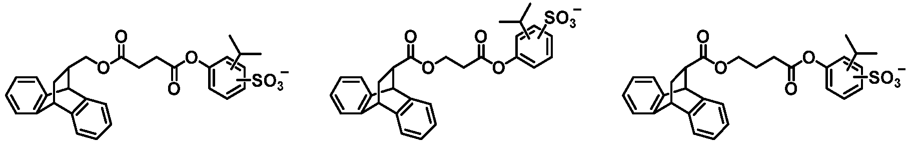

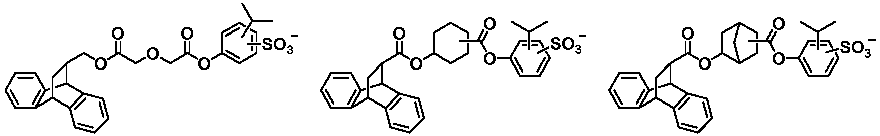

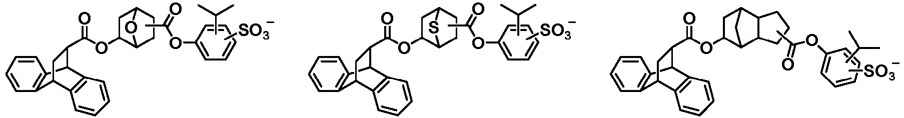

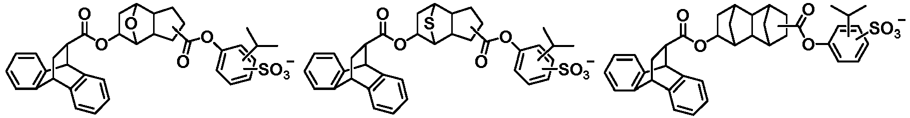

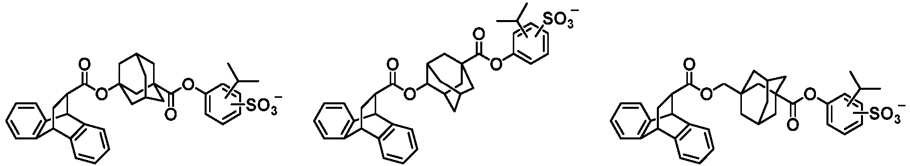

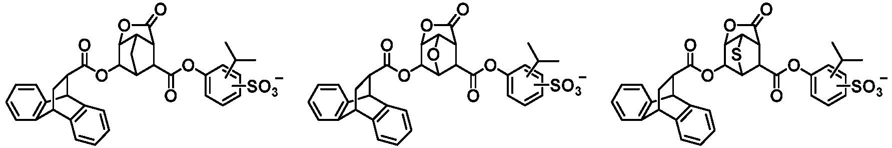

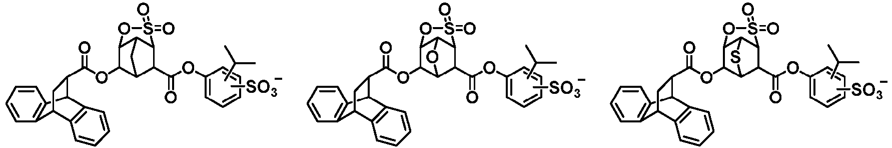

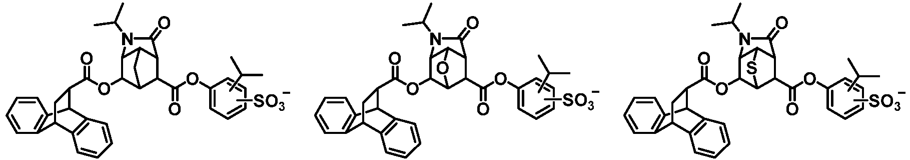

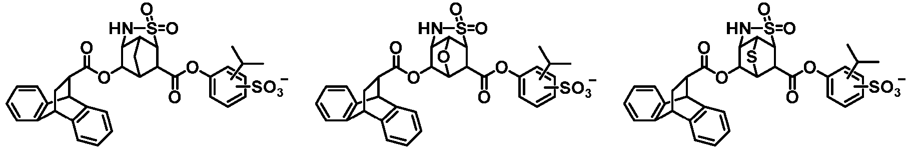

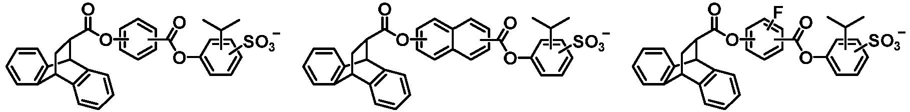

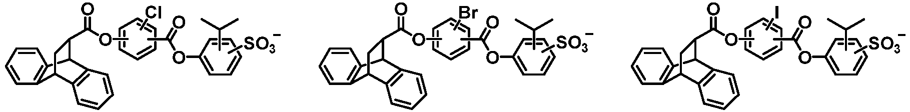

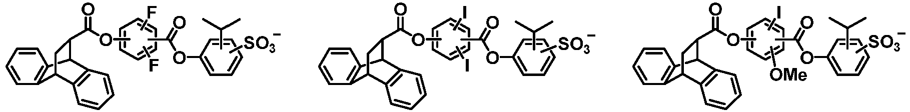

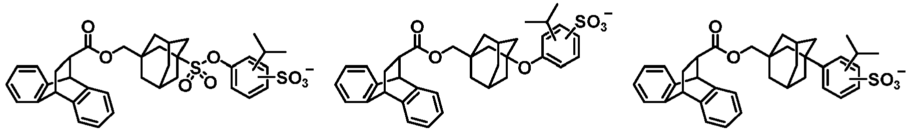

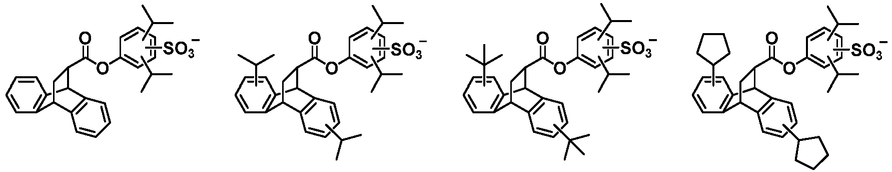

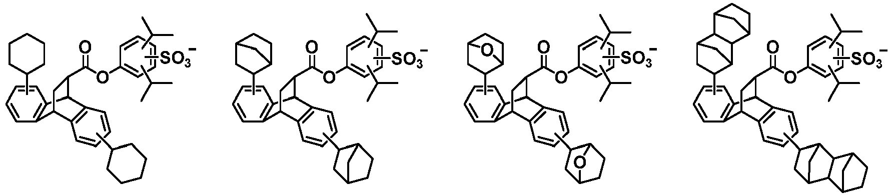

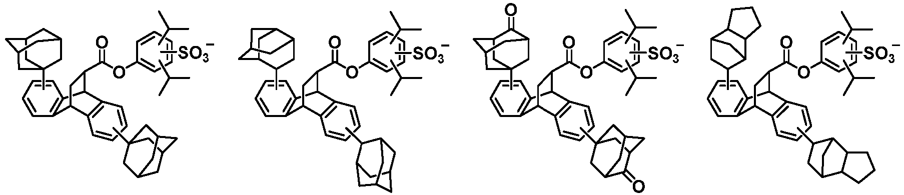

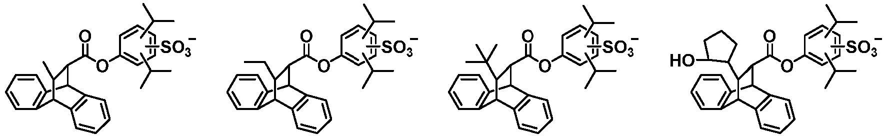

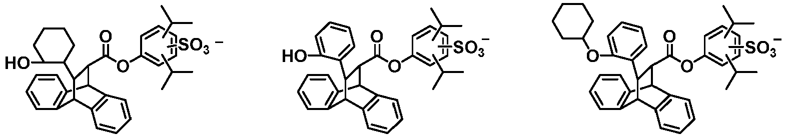

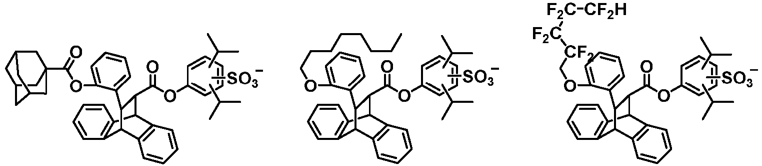

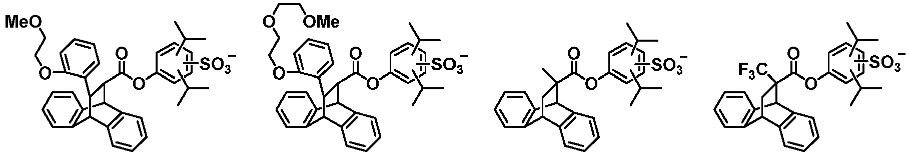

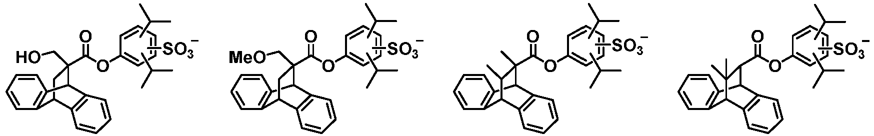

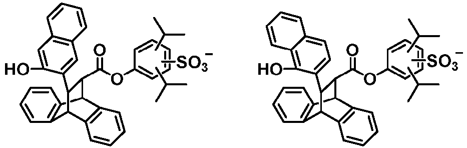

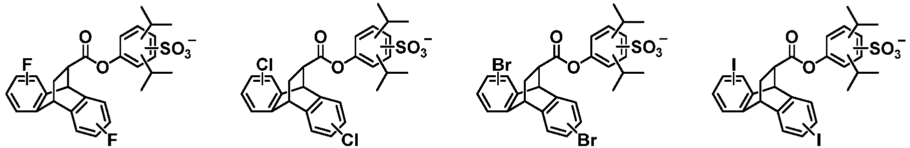

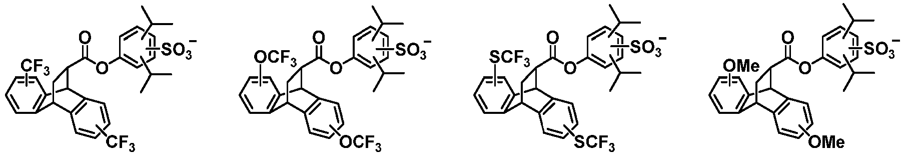

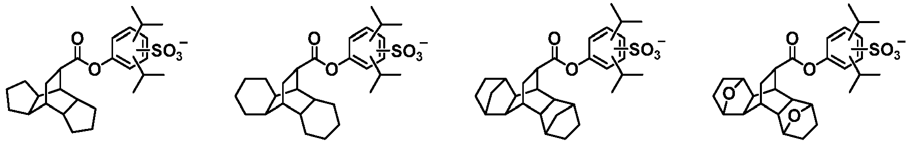

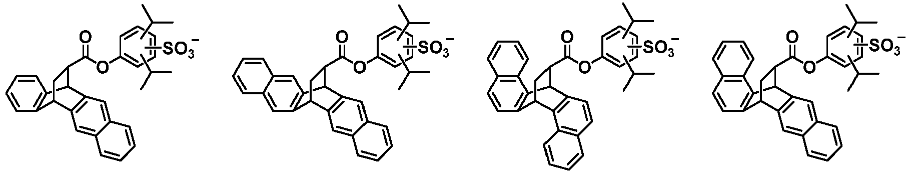

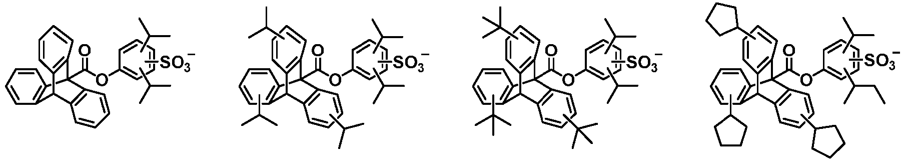

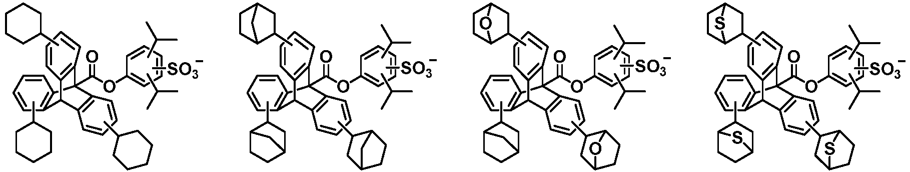

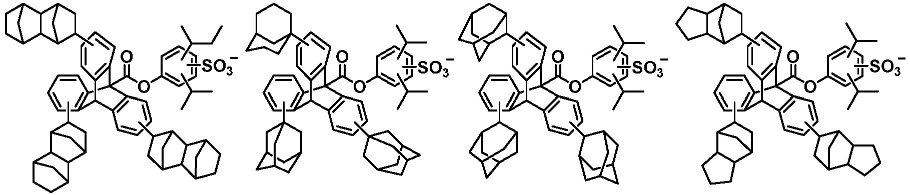

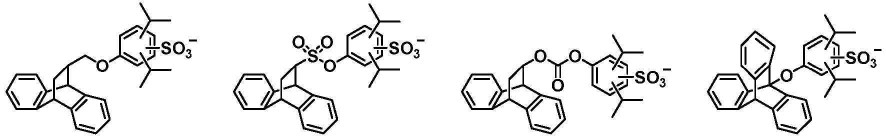

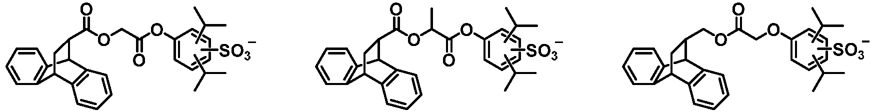

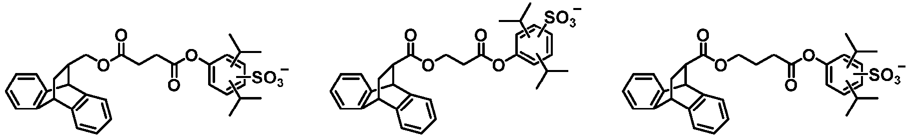

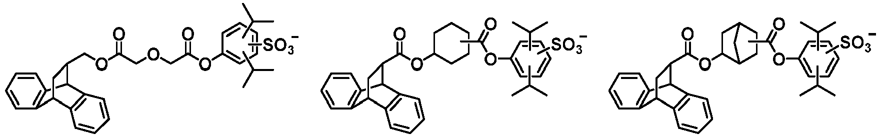

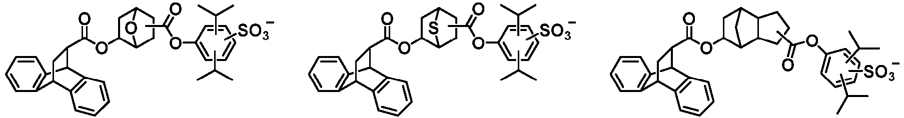

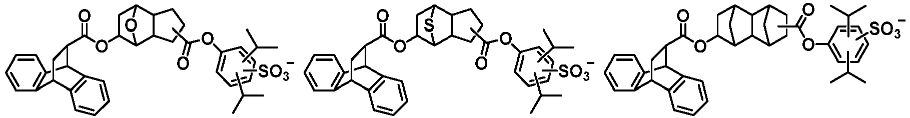

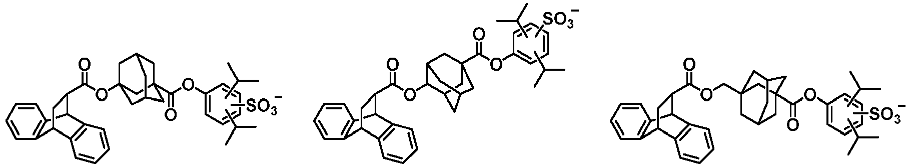

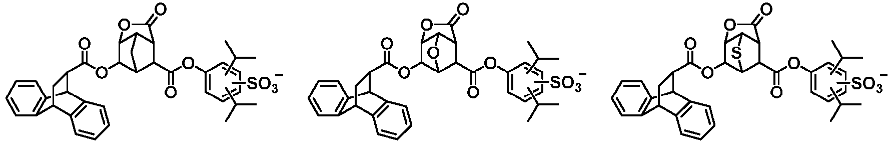

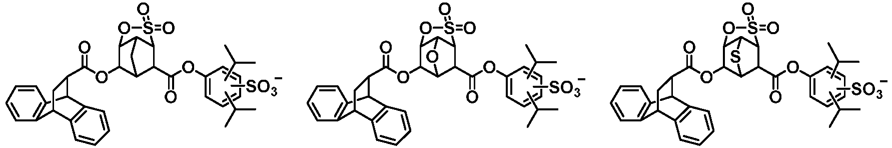

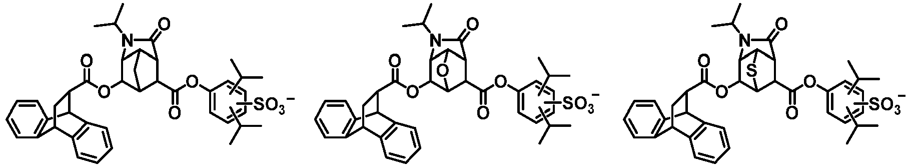

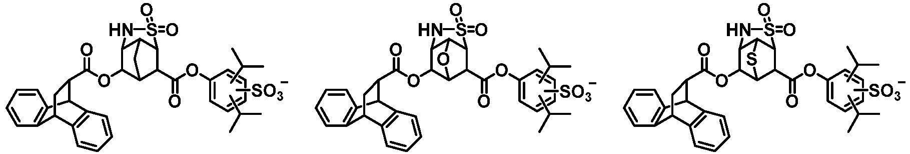

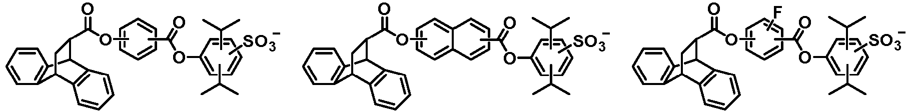

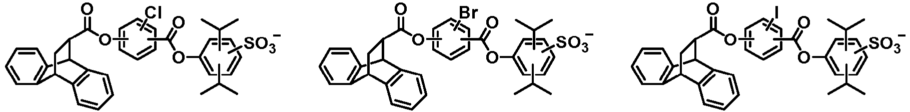

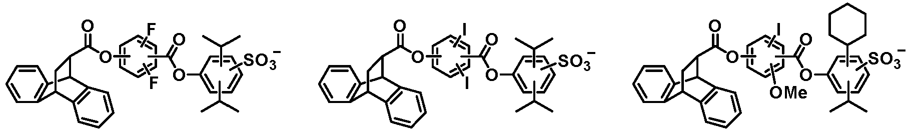

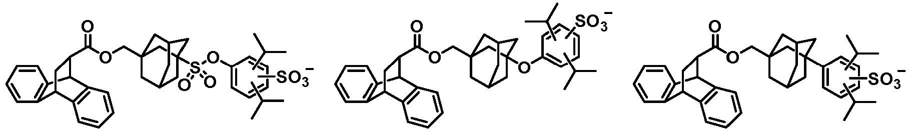

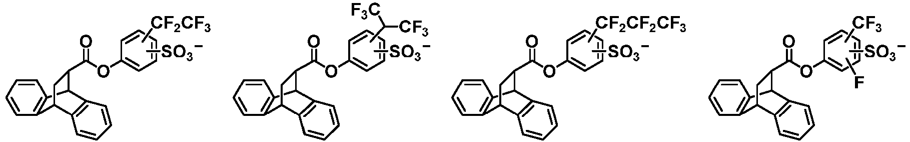

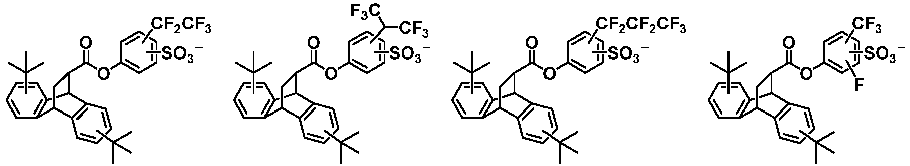

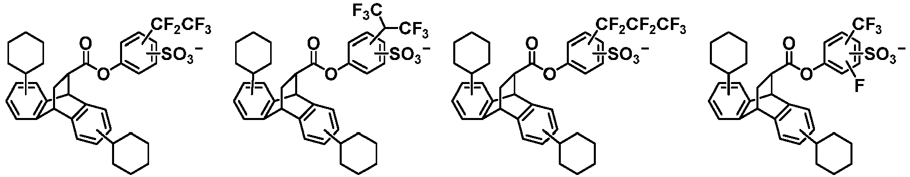

The onium salt as photoacid generator (A) contains a cation having the formula (A2).

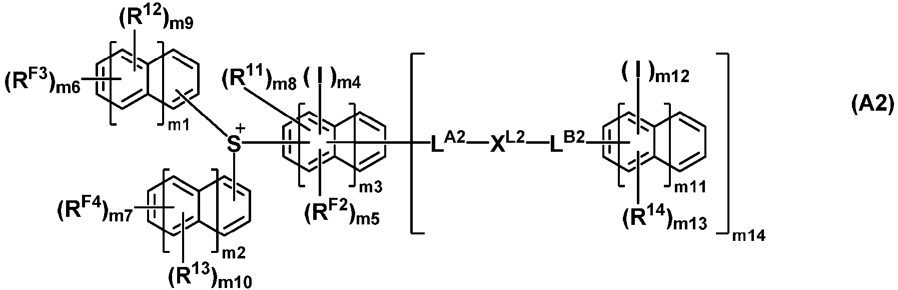

In formula (A2), m1 is 0 or 1. The relevant structure is a benzene ring when m1=0, and a naphthalene ring when m1=1. The benzene ring corresponding to m1=0 is preferred from the aspect of solvent solubility. The subscript m2 is 0 or 1. The relevant structure is a benzene ring when m2=0, and a naphthalene ring when m2=1. The benzene ring corresponding to m2=0 is preferred from the aspect of solvent solubility. The subscript m3 is 0 or 1. The relevant structure is a benzene ring when m3=0, and a naphthalene ring when m3=1. The benzene ring corresponding to m3=0 is preferred from the aspect of solvent solubility.

In formula (A2), m4 is 0, 1, 2, 3 or 4. As the number of iodine atoms in the cation structure increases, the compound becomes more absorptive to EUV, but so poor in solvent solubility that it may precipitate in a resist composition. For this reason, m4 is preferably 0, 1, 2 or 3, more preferably 0, 1 or 2.

In formula (A2), m5 is 0, 1, 2, 3 or 4. It is preferred from the aspect of reactant availability that m5 be 0, 1, 2 or 3, more preferably 0, 1 or 2. The subscript m6 is 0, 1, 2, 3, 4, 5 or 6. It is preferred from the aspect of reactant availability that m6 be 0, 1, 2 or 3, more preferably 0, 1 or 2. The subscript m7 is 0, 1, 2, 3, 4, 5 or 6. It is preferred from the aspect of reactant availability that m7 be 0, 1, 2 or 3, more preferably 0, 1 or 2.

In formula (A2), m8 is 0, 1 or 2. In view of reactant availability, m8 is preferably 0 or 1. The subscript m9 is 0, 1 or 2. In view of reactant availability, m9 is preferably 0 or 1. The subscript m10 is 0, 1 or 2. In view of reactant availability, m10 is preferably 0 or 1.

In formula (A2), m11 is 0 or 1. The relevant structure is a benzene ring when m11=0, and a naphthalene ring when m11=1. The benzene ring corresponding to m11=0 is preferred from the aspect of solvent solubility.

In formula (A2), m12 is 0, 1, 2, 3 or 4. As the number of iodine atoms in the cation structure increases, the compound becomes more absorptive to EUV, but so poor in solvent solubility that it may precipitate in a resist composition. For this reason, m12 is preferably 0, 1, 2 or 3, more preferably 0, 1 or 2.

In formula (A2), m13 is 0, 1 or 2. In view of reactant availability, m13 is preferably 0 or 1. The subscript m14 is 0, 1 or 2. In view of synthesis, m14 is preferably 0 or 1.

The subscripts m1 to m14 are in the range that when m1=0, 0 ≤ m6+m9 ≤ 4, and when m1=1, 0 ≤ m6+m9 ≤ 6; when m2=0, 0 ≤ m7+m10 ≤ 4, and when m2=1, 0 ≤ m7+m10 ≤ 6; when m3=0, 1 ≤ m4+m5+m8+m14 ≤ 4, and when m3=1, 1 ≤ m4+m5+m8+m14 ≤ 6; when ml 1=0, 0 ≤ m12+m13 ≤ 4, and when m11=1, 0 ≤ m12+m13 ≤ 6; and m4+m12 ≥ 1.

In formula (A2), R^{F2} to R^{F4} are each independently fluorine, a C₁-C₆ fluorinated saturated hydrocarbyl group, C₁-C₆ fluorinated saturated hydrocarbyloxy group, or C₁-C₆ fluorinated saturated hydrocarbylthio group. Of these, trifluoromethyl, trifluoromethoxy, and trifluorothiomethoxy are preferred. When m5 is 2 or more, a plurality of R^{F2} may be identical or different. When m6 is 2 or more, a plurality of R^{F3} may be identical or different. When m7 is 2 or more, a plurality of R^{F4} may be identical or different.

In formula (A2), R¹¹ to R¹⁴ are each independently halogen exclusive of iodine and fluorine, nitro group, cyano group, a C₁-C₂₀ hydrocarbyl group which may contain a heteroatom, C₁-C₂₀ hydrocarbyloxy group which may contain a heteroatom, C₁-C₂₀ hydrocarbylthio group which may contain a heteroatom. The hydrocarbyl group and hydrocarbyl moiety in the hydrocarbyloxy and hydrocarbylthio groups may be saturated or unsaturated and straight, branched or cyclic. Examples thereof are as exemplified above for the hydrocarbyl group R¹ in formula (A1). In the hydrocarbyl group and hydrocarbyl moiety in the hydrocarbyloxy and hydrocarbylthio groups, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy, cyano, fluorine, chlorine, bromine, iodine, carbonyl, ether bond, ester bond, sulfonate ester bond, carbonate bond, lactone ring, sultone ring, carboxylic anhydride (-C(=O)-O-C(=O)-) or haloalkyl moiety.

When m8=2, two R¹¹ may be identical or different and two R¹¹ may bond together to form a ring with the carbon atoms to which they are attached. When m9=2, two R¹² may be identical or different and two R¹² may bond together to form a ring with the carbon atoms to which they are attached. When m10=2, two R¹³ may be identical or different and two R¹³ may bond together to form a ring with the carbon atoms to which they are attached. When m13=2, two R¹⁴ may be identical or different and two R¹⁴ may bond together to form a ring with the carbon atoms to which they are attached. Examples of the ring thus formed include cyclopropane, cyclobutane, cyclopentane, cyclohexane, norbornane, and adamantane rings. In the ring, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the ring may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-) or haloalkyl moiety.

The aromatic rings directly bonded to S⁺ in the sulfonium cation having formula (A2) may bond together to form a ring with S⁺. Exemplary structures of the ring are shown below.

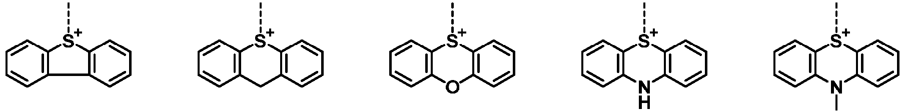

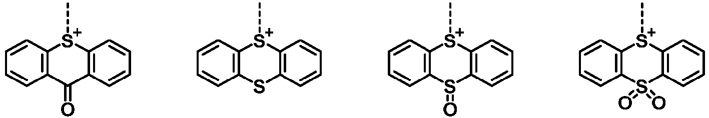

In formula (A2), L¹² and L¹² are each independently a single bond, ether bond, ester bond, amide bond, sulfonate ester bond, sulfonamide bond, carbonate bond or carbamate bond. L^{A2} is preferably a single bond, ether bond, ester bond or sulfonate ester bond, more preferably an ester bond or sulfonate ester bond. L^{B2} is preferably a single bond, ether bond or ester bond, more preferably a single bond.

In formula (A2), X^{L2} is a single bond or a C₁-C₄₀ hydrocarbylene group which may contain a heteroatom. Examples of the C₁-C₄₀ hydrocarbylene group which may contain a heteroatom are as exemplified above for the C₁-C₄₀ hydrocarbylene group X^{L1}, but not limited thereto.

Preferably, the sulfonium cation having formula (A2) has the formula (A2-1): 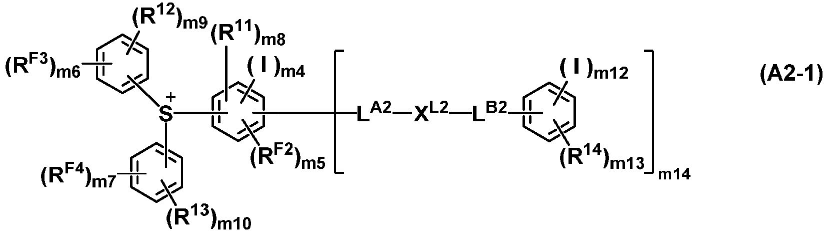 wherein m4 to m10, m12 to m14, R^{F2} to R^{F4} R¹¹ to R¹⁴, L^{A2}, L^{B2}, and X^{L2} are as defined above.

More preferably, the sulfonium cation having formula (A2-1) has the formula (A2-2): 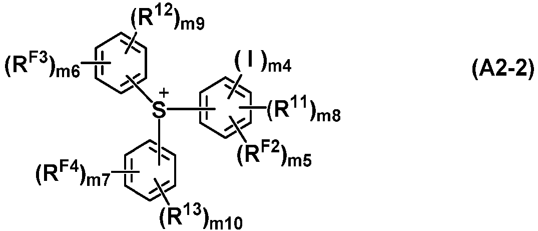 wherein m4 to m10, R^{F2} to R^{F4}, and R¹¹ to R¹³ are as defined above.

Examples of the sulfonium cation having formula (A2) are shown below, but not limited thereto.

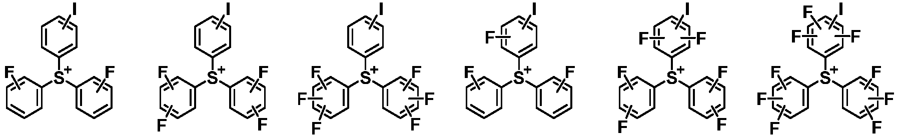

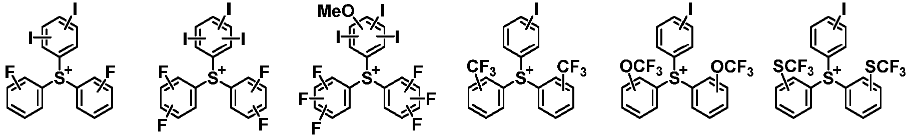

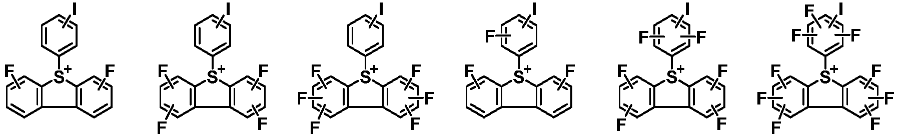

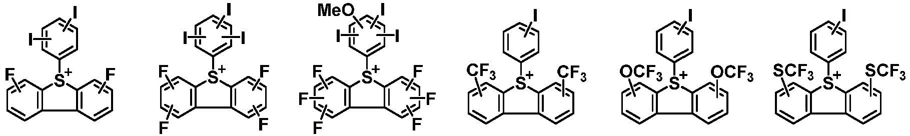

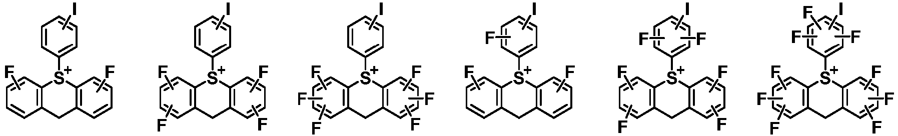

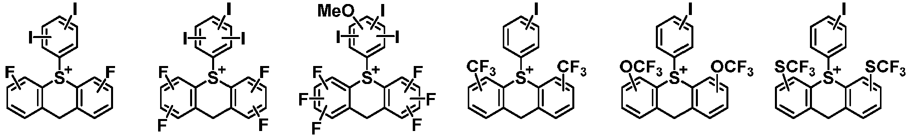

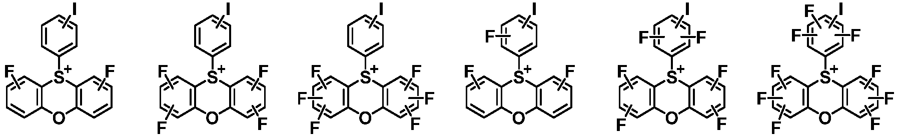

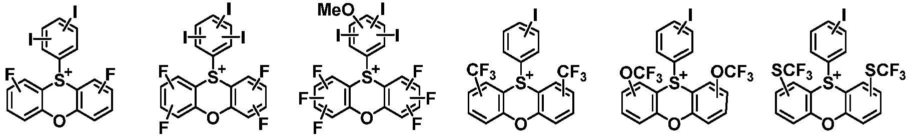

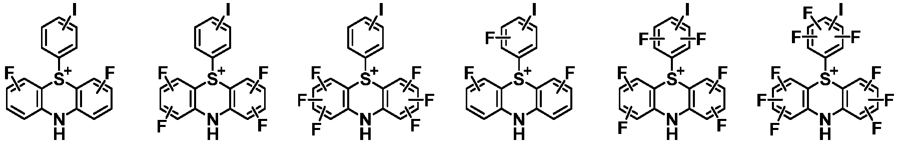

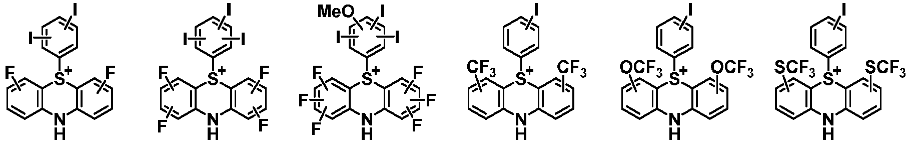

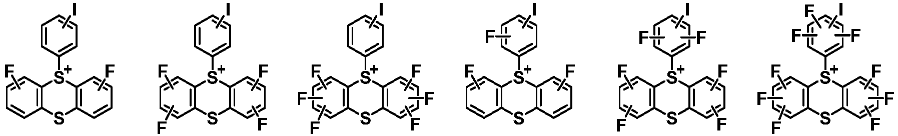

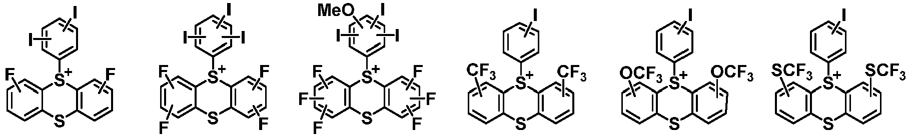

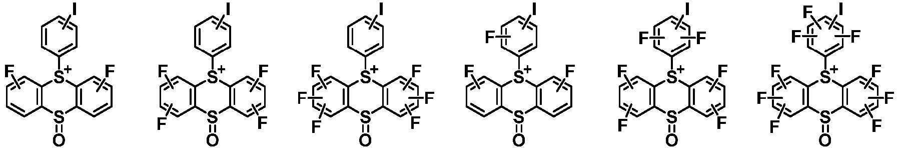

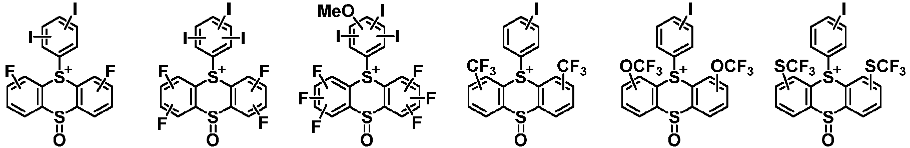

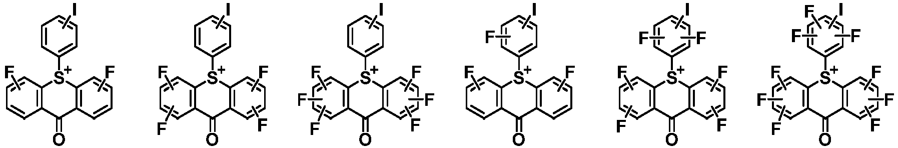

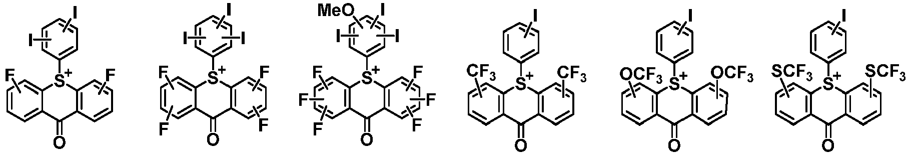

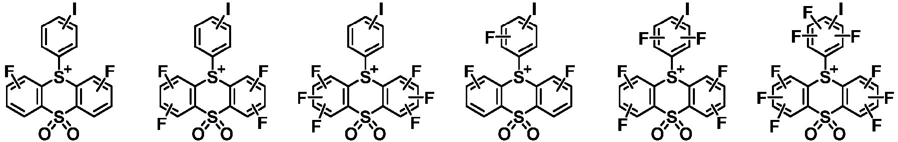

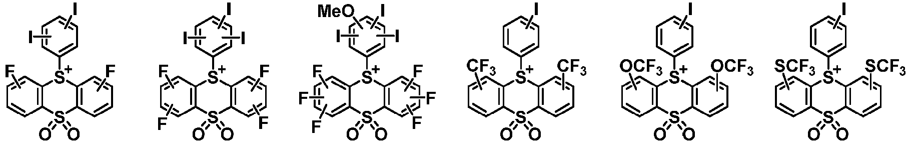

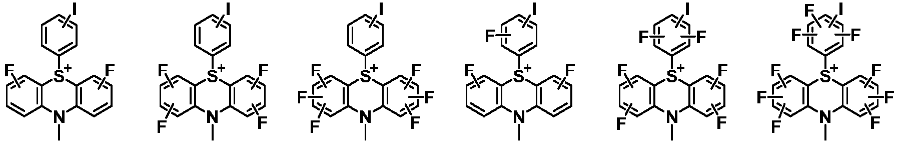

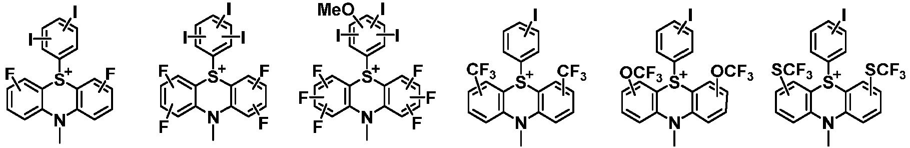

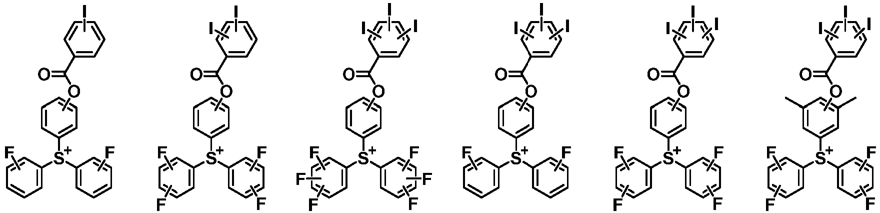

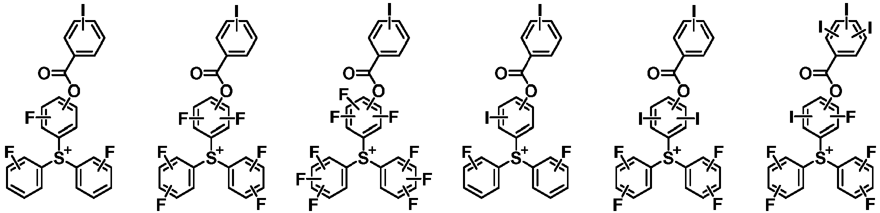

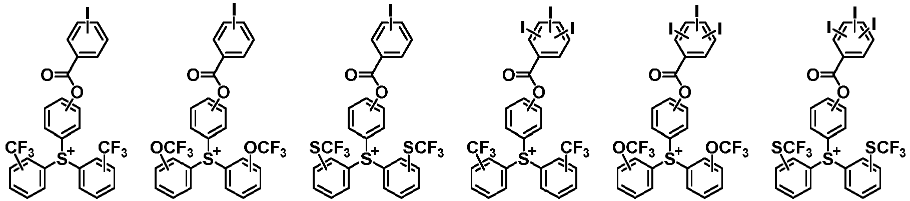

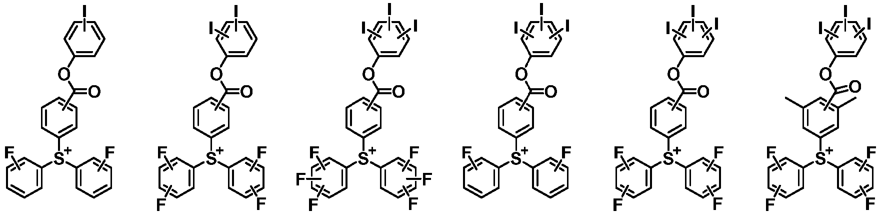

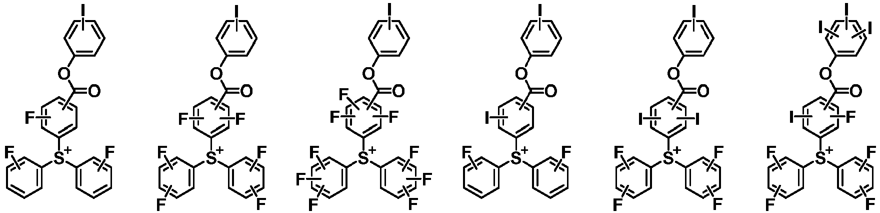

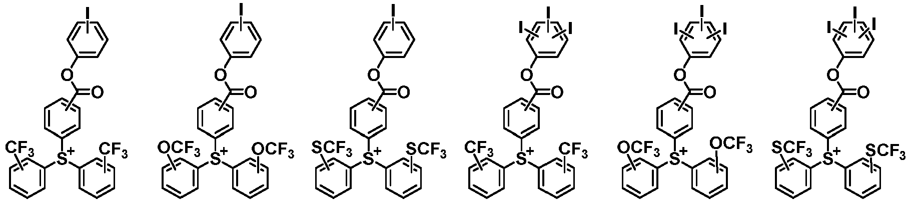

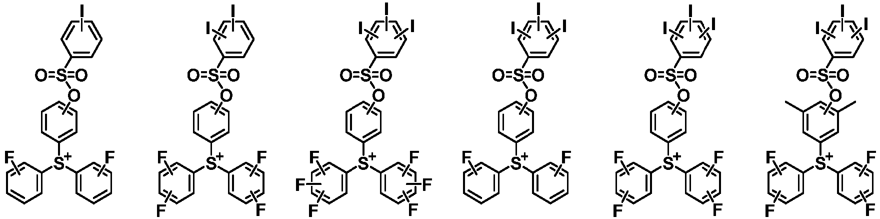

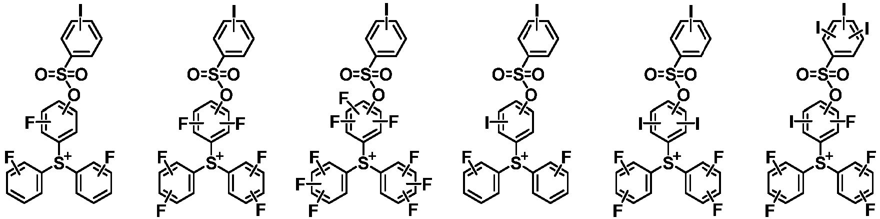

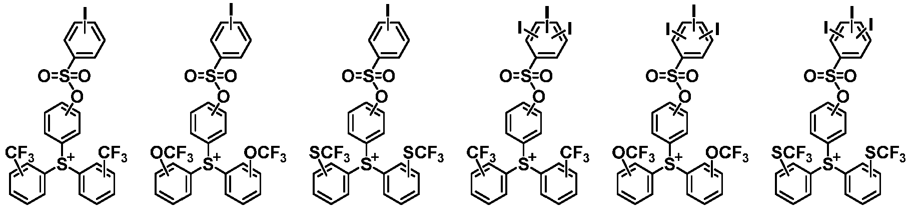

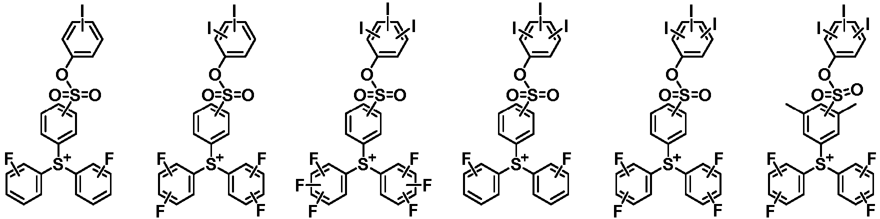

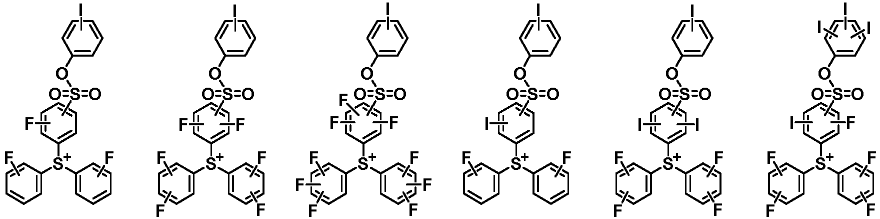

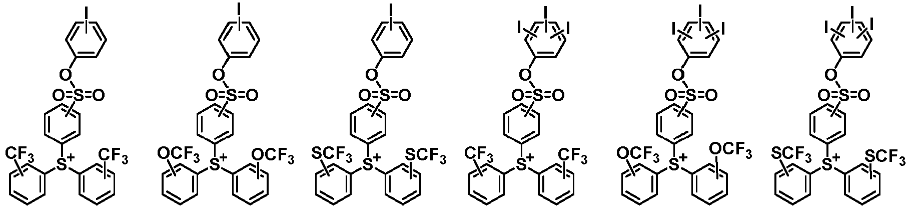

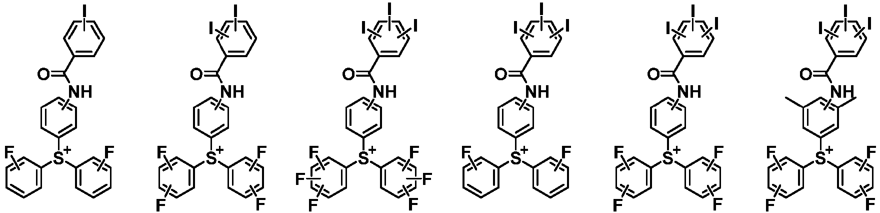

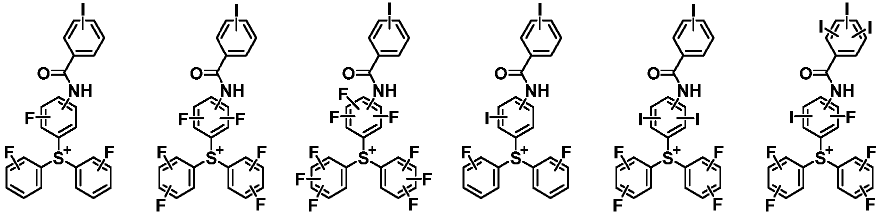

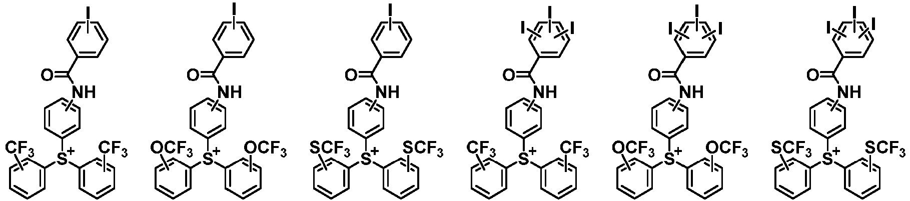

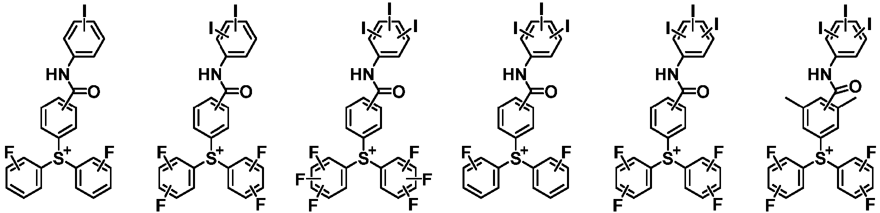

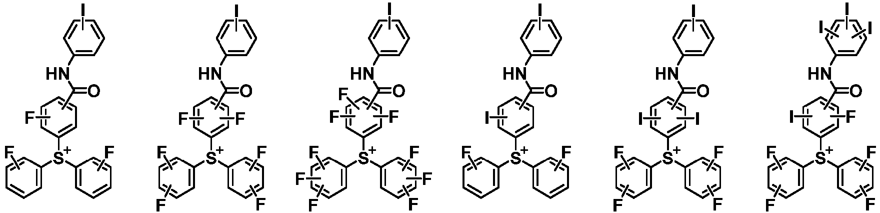

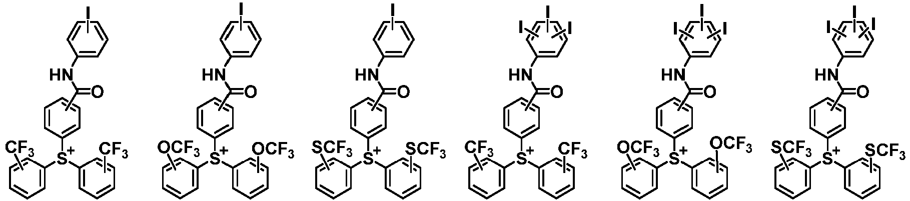

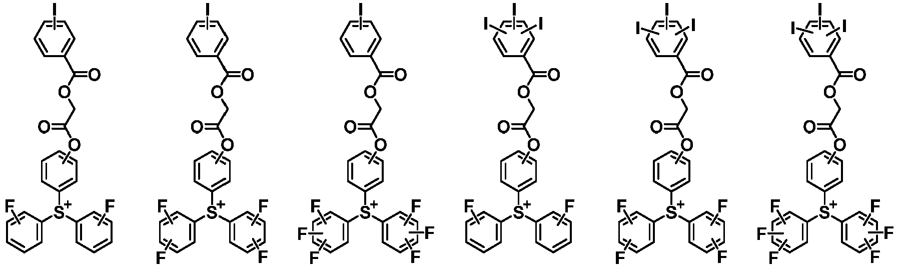

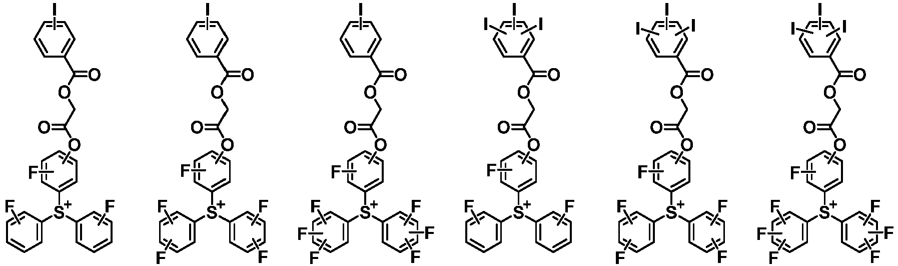

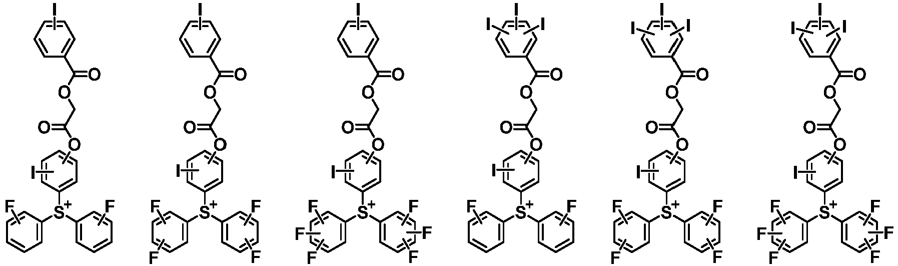

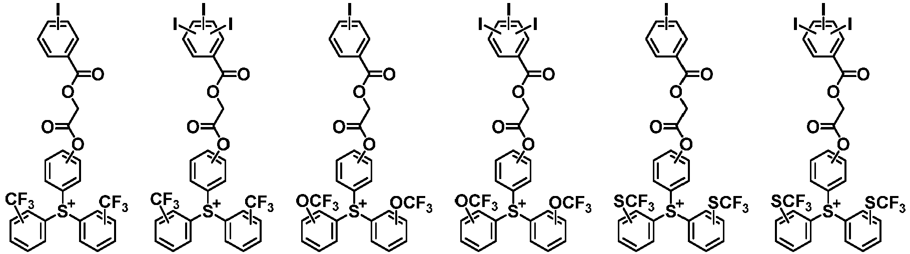

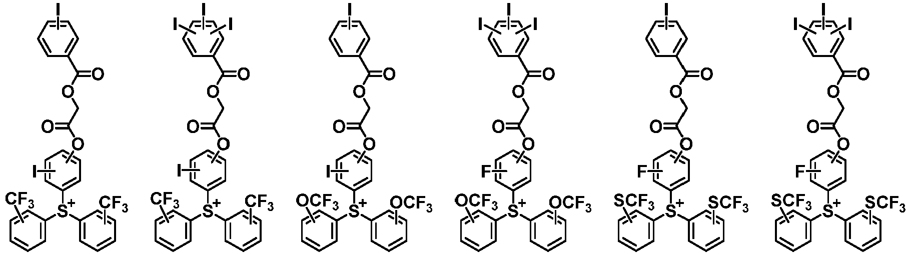

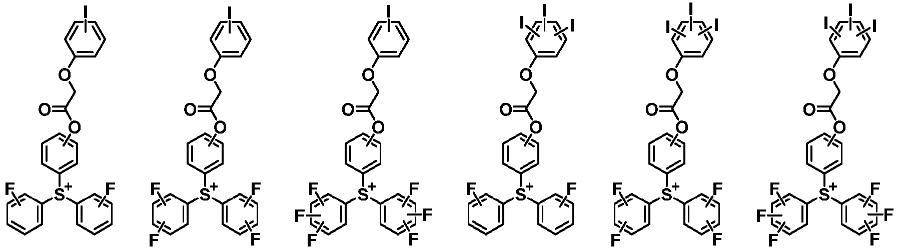

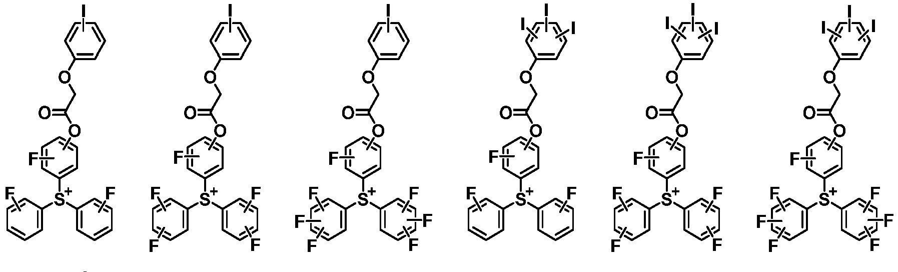

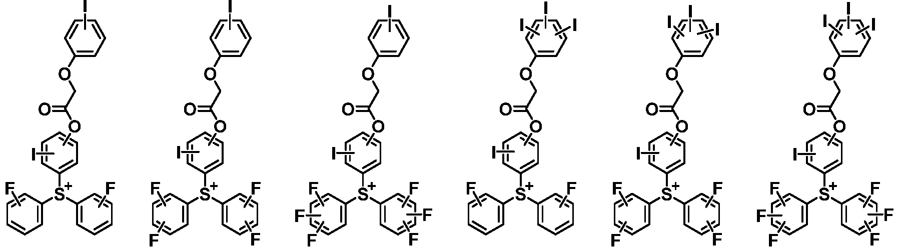

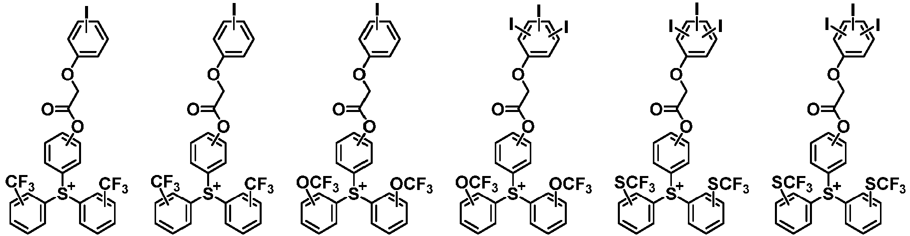

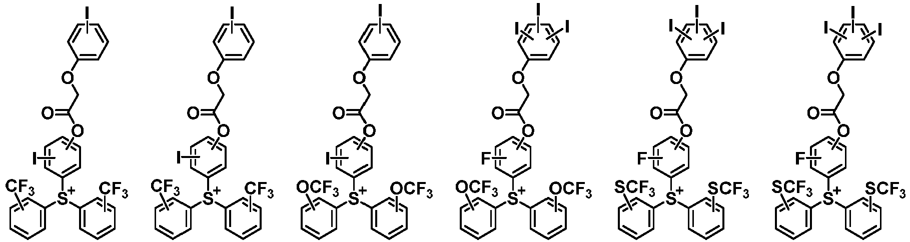

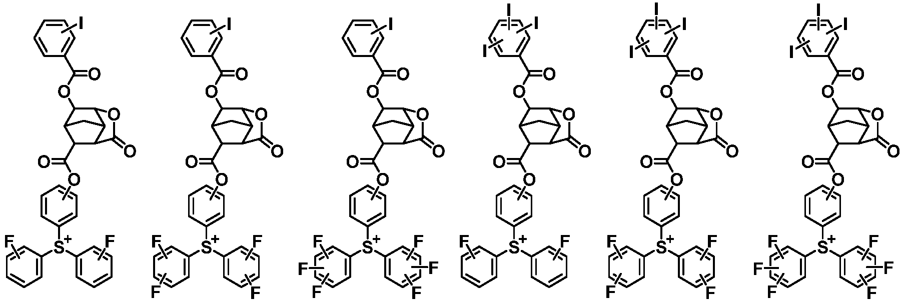

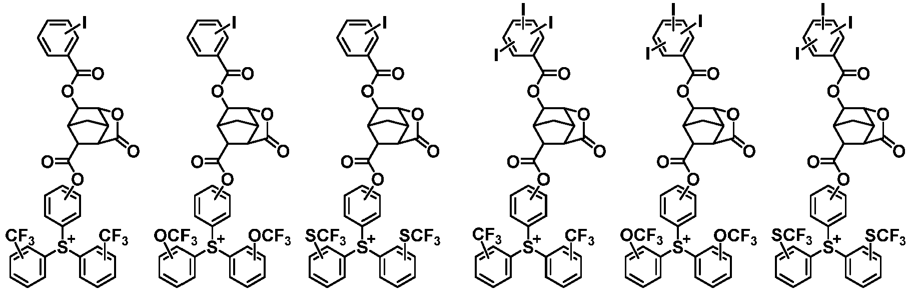

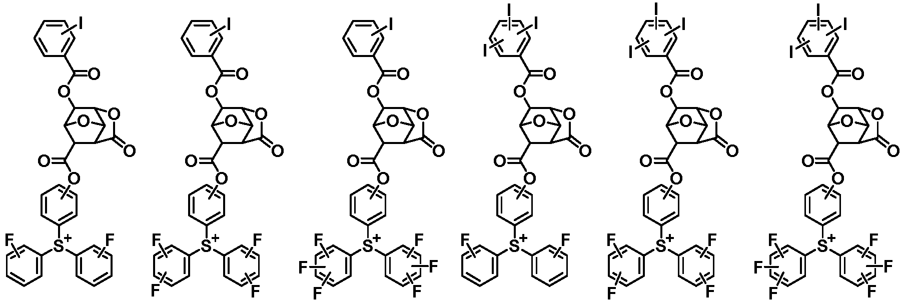

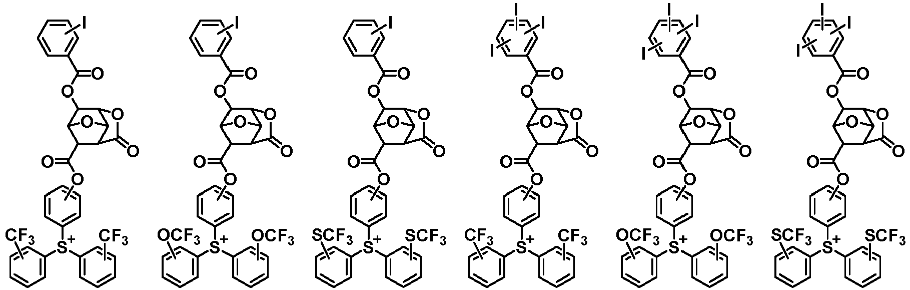

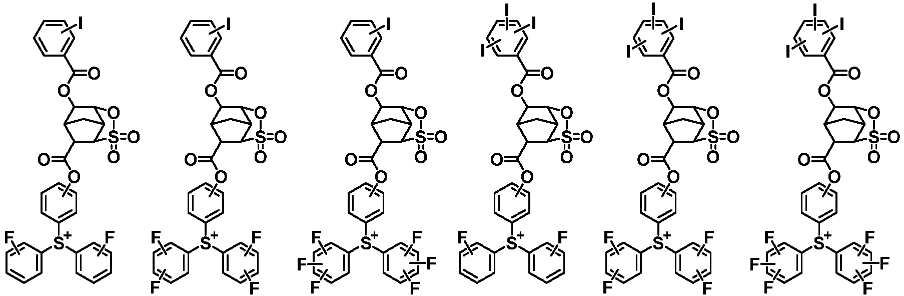

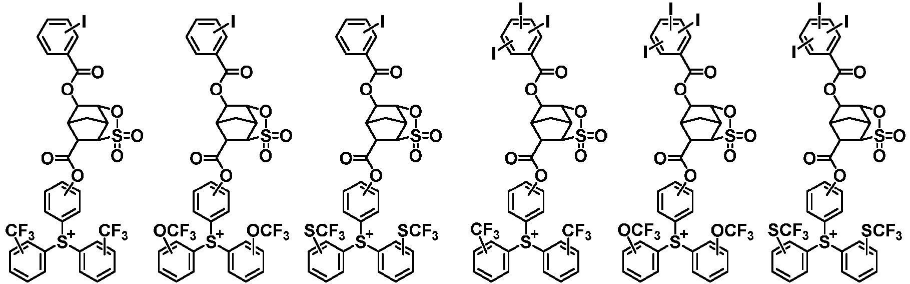

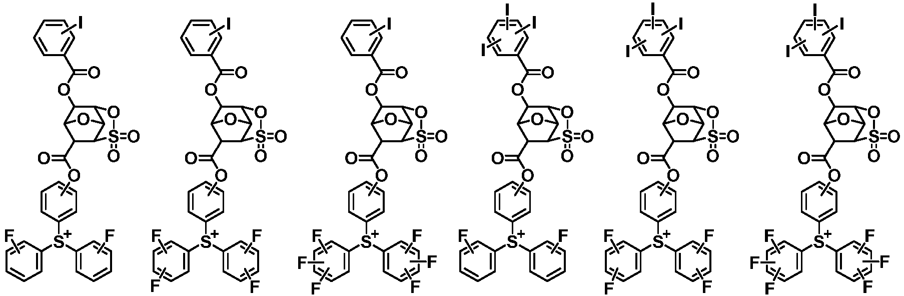

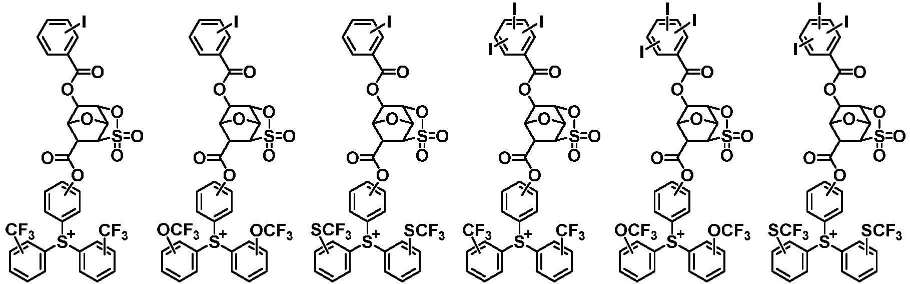

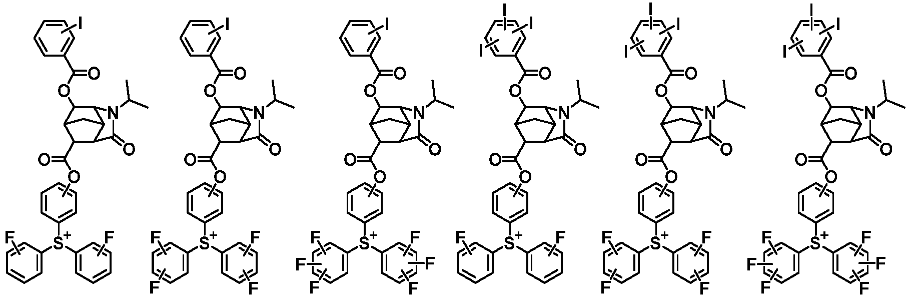

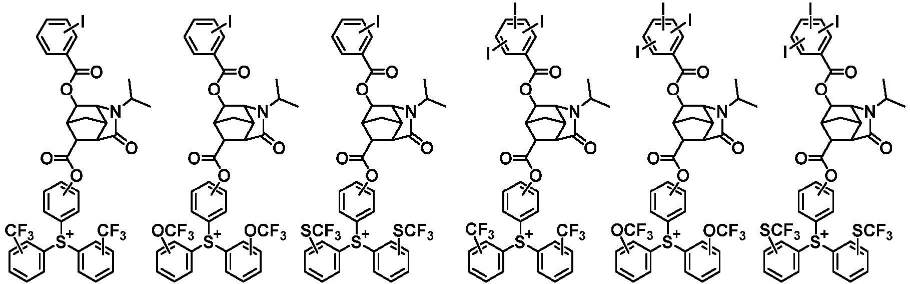

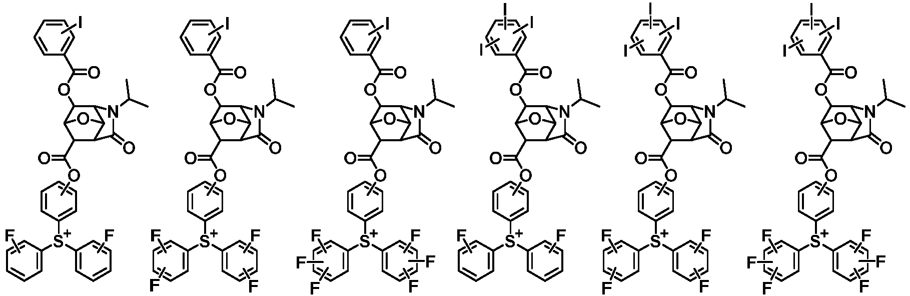

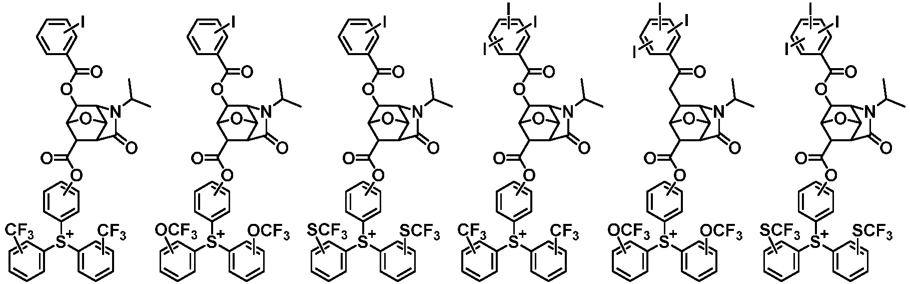

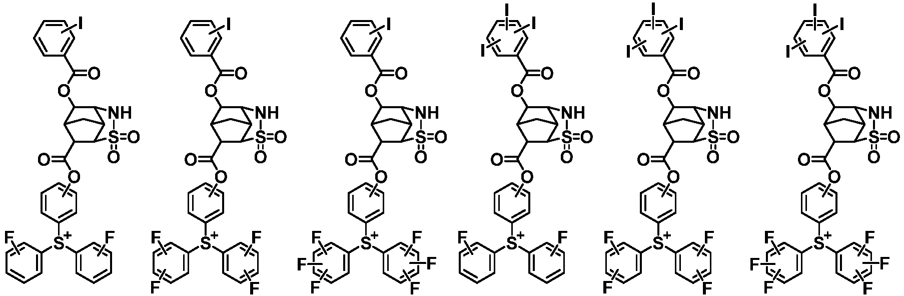

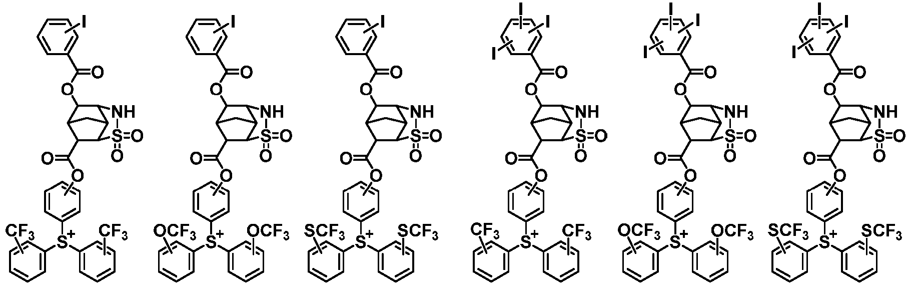

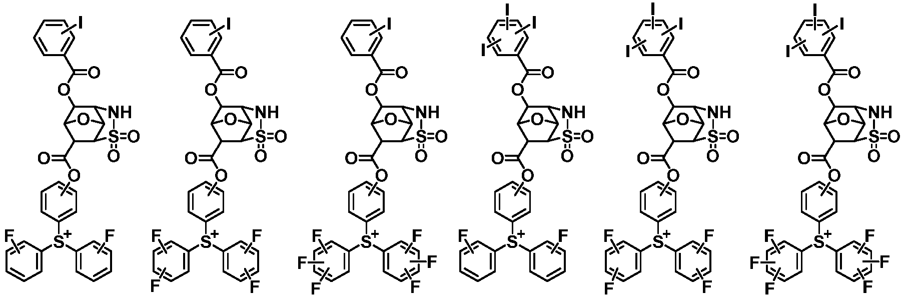

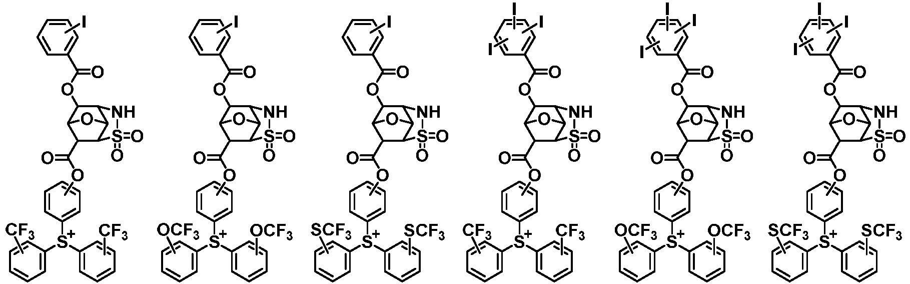

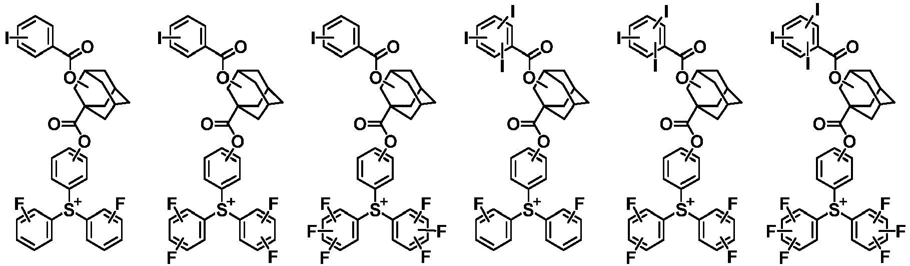

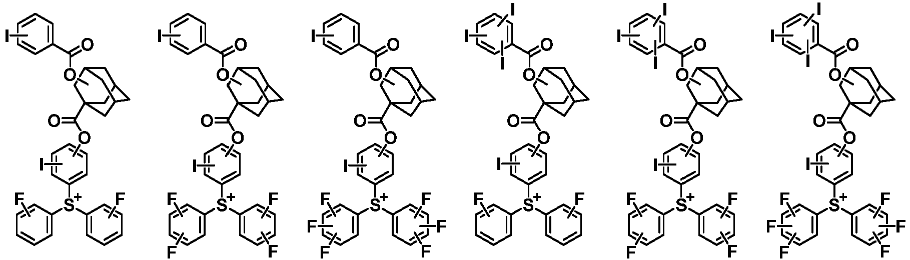

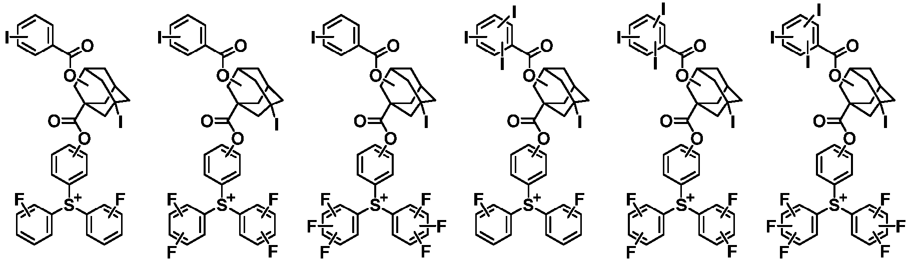

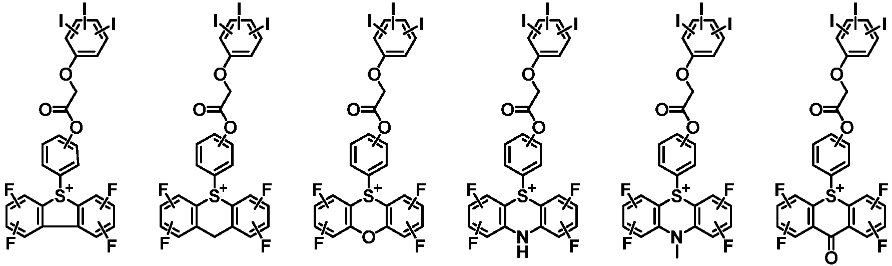

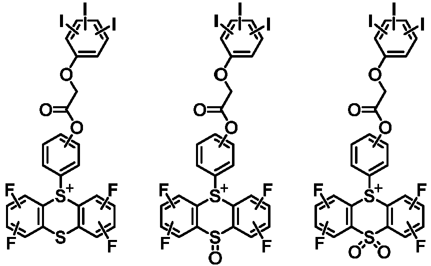

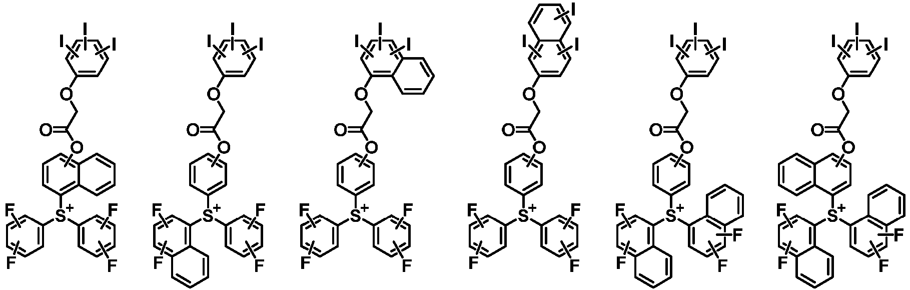

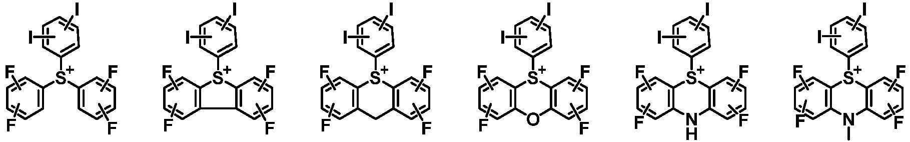

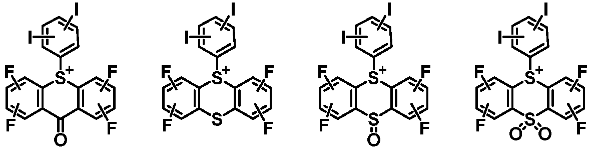

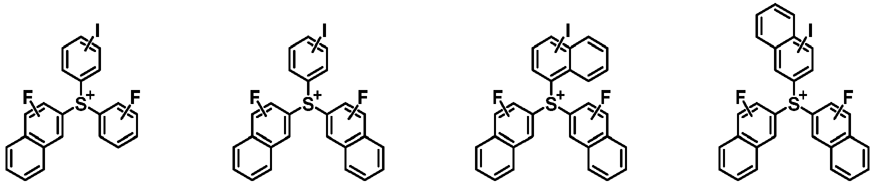

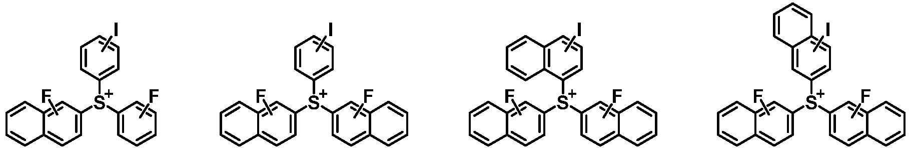

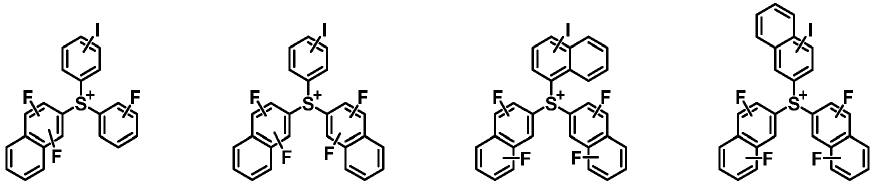

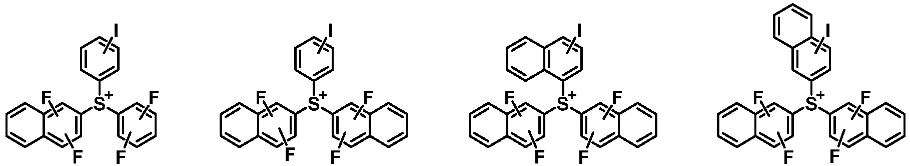

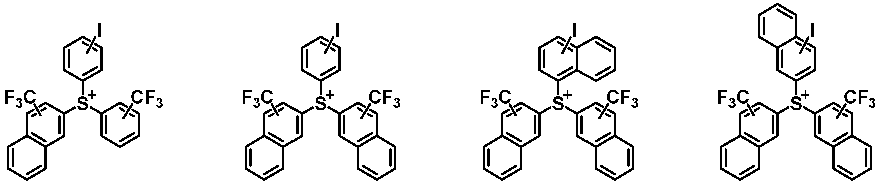

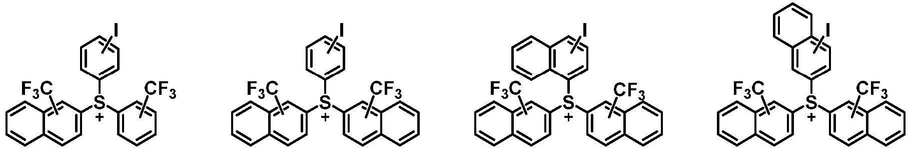

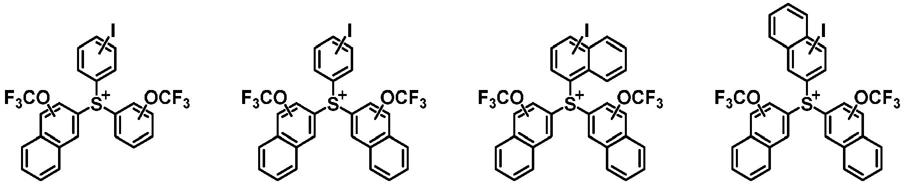

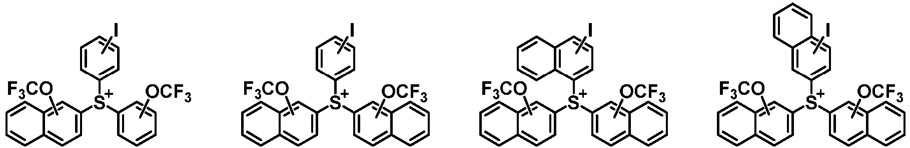

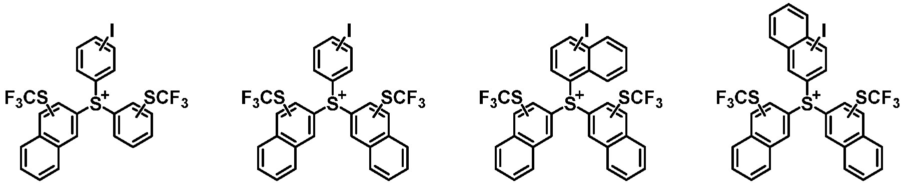

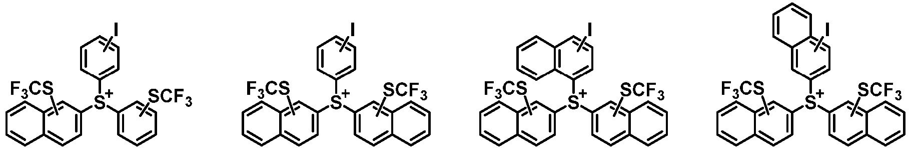

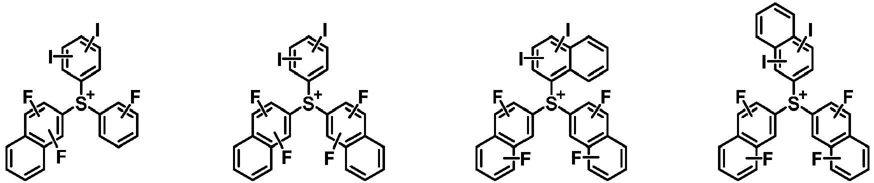

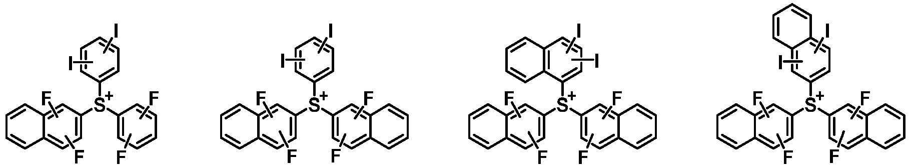

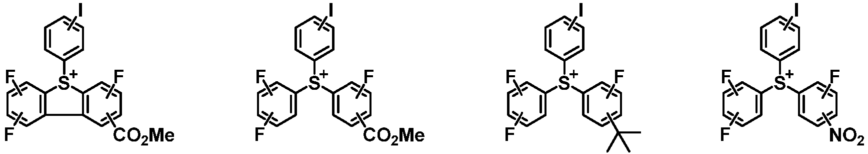

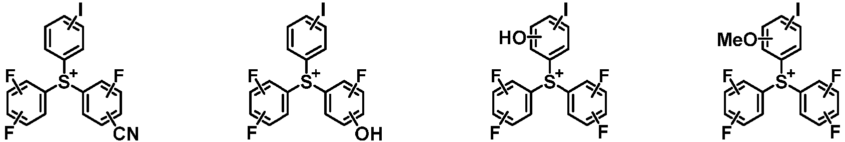

Examples of the onium salt include arbitrary combinations of anions with cations, both as mentioned above.

For the synthesis of the inventive onium salt, reference should be made to JP-A 2010-155824 and JP 7067271, for example. These preparation methods are merely exemplary and the method of preparing the onium salt is not limited thereto.

The inventive onium salt is structurally characterized in that it consists of an aromatic sulfonic acid anion substituted with a hydrocarbyl group containing at least one aromatic ring and a triarylsulfonium cation containing iodine and fluorine. The hydrocarbyl group containing at least one aromatic ring has a large excluded volume and thus serves as a bulky substituent to effectively inhibit the generated acid from diffusing. This effect is readily obtained particularly when the hydrocarbyl group containing at least one aromatic ring is an aromatic ring structure having a substituent as represented by formula (W-1) or a fused ring structure having a substituent as represented by formula (W-2). The structure is also enough resistant to alkaline developer to prevent the pattern in unexposed region from losing a film thickness. The aromatic sulfonic acid structure, on the other hand, generates an acid of robust structure, which is effective for restraining acid diffusion.

It is preferred that the aromatic ring that forms the aromatic sulfonic acid structure contain fluorine or an electron attractive sulfonate ester bond as a linking group. Then the generated acid has a higher acidity enough to efficiently deprotect the acid labile groups in the base polymer. In the triarylsulfonium cation containing iodine and fluorine, the fluorine atom, which is a substituent for promoting solvent solubility, acts to increase the solvent solubility of the onium salt so that the onium salt may be uniformly dissolved in the solvent. In addition, iodine atom has a moderate dissolution inhibition relative to alkaline developer and prevents the resist pattern from collapsing. By virtue of the synergy of these effects, the positive resist composition comprising the onium salt can form a resist pattern having a high sensitivity, low acid diffusion, and improved LWR of line patterns or improved CDU of hole patterns. The pattern is fully resistant to collapse. The resist composition is effective for forming small-size patterns.

In the chemically amplified positive resist composition, the amount of the photoacid generator (A) is preferably 0.1 to 20 parts by weight, more preferably 1 to 10 parts by weight per 80 parts by weight of a base polymer (B) to be described just below. As long as the amount of photoacid generator (A) is in the range, an acid is generated in an amount necessary for deprotection of acid labile groups so that a pattern of satisfactory profile may be formed. **In** addition, the composition has storage stability. The photoacid generator (A) may be used alone or in admixture of two or more.

**In** the chemically amplified positive resist composition, an acid generator other than the onium salt consisting of an anion having formula (A1) and a cation having formula (A2) may be added for the purpose of correcting the pattern profile or the like. The other acid generator may be selected from well-known acid generators for use in resist compositions. The amount of the other acid generator is preferably 0 to 40 parts by weight, more preferably 0 to 30 parts by weight per 80 parts by weight of base polymer (B). The other acid generator may be used alone or in admixture.

### (B) Base polymer

The resist composition further comprises (B) a base polymer containing a polymer which is decomposed under the action of acid to increase its solubility in alkaline developer.

The polymer contains repeat units having the formula (B 1), which are also referred to as repeat units B1.

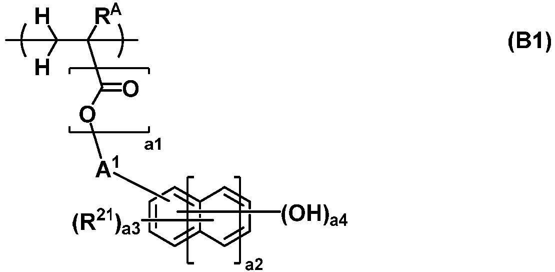

In formula (B1), a1 is 0 or 1. The subscript a2 is 0, 1 or 2. The relevant structure is a benzene ring when a2=0, a naphthalene ring when a2=1, and an anthracene ring when a2=2. The subscript a3 is an integer meeting 0 ≤ a3 ≤ 5+2(a2)-a4, and a4 is 1, 2 or 3. When a2 is 0, preferably a3 is 0, 1, 2 or 3 and a4 is 1, 2 or 3. When a2 is 1 or 2, preferably a3 is 0, 1, 2, 3 or 4 and a4 is 1, 2 or 3.

In formula (B1), R^{A} is hydrogen, fluorine, methyl or trifluoromethyl.

In formula (B1), R²¹ is halogen, nitro, carboxy, an optionally halogenated C₁-C₆ saturated hydrocarbyl group, optionally halogenated C₁-C₆ saturated hydrocarbyloxy group or optionally halogenated C₂-C₈ saturated hydrocarbylcarbonyloxy group. The saturated hydrocarbyl group and saturated hydrocarbyl moiety in the saturated hydrocarbyloxy and saturated hydrocarbylcarbonyloxy groups may be straight, branched or cyclic. Examples thereof include alkyl groups such as methyl, ethyl, n-propyl, isopropyl, butyl, pentyl, hexyl, and structural isomers thereof; cycloalkyl groups such as cyclopropyl, cyclobutyl, cyclopentyl and cyclohexyl; and combinations thereof. A carbon count within the upper limit ensures a satisfactory solubility in alkaline developer. A plurality of R²¹ may be identical or different when a3 is 2 or more.

In formula (B1), A¹ is a single bond or C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of a1=1 in formula (B1), the ether bond may be incorporated at any position excluding the position between the α- and β-carbons relative to the ester oxygen. In case of a1=0, the atom bonding to the backbone becomes an ether oxygen atom, and a second ether bond may be incorporated at any position excluding the position between the α- and β-carbons relative to the ether oxygen. Saturated hydrocarbylene groups having no more than 10 carbon atoms are desirable because of a sufficient solubility in alkaline developer.

Preferred examples of the repeat units B1 wherein a1 =0 and A¹ is a single bond (meaning that the aromatic ring is directly bonded to the backbone of the polymer), that is, repeat units free of a linker: -C(=O)-O-A¹- include units derived from 3-hydroxystyrene, 4-hydroxystyrene, 5-hydroxy-2-vinylnaphthalene, and 6-hydroxy-2-vinylnaphthalene. Exemplary units are shown below, but not limited thereto. Herein R^{A} is as defined above.

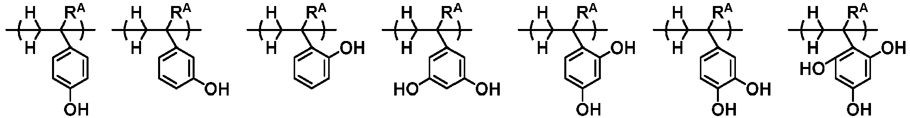

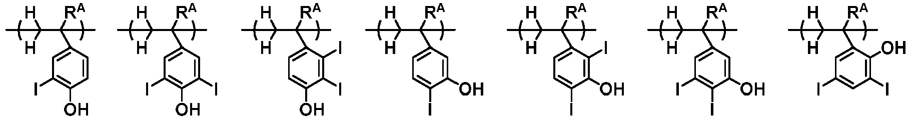

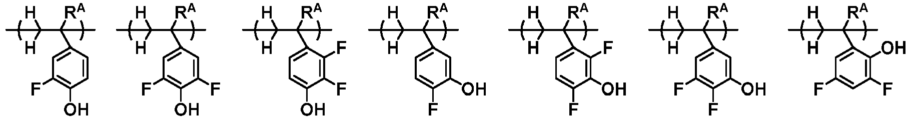

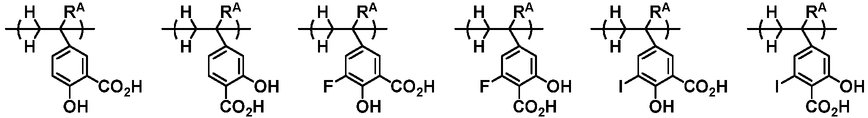

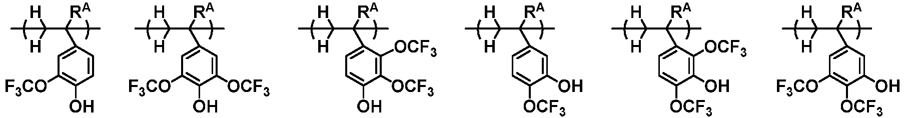

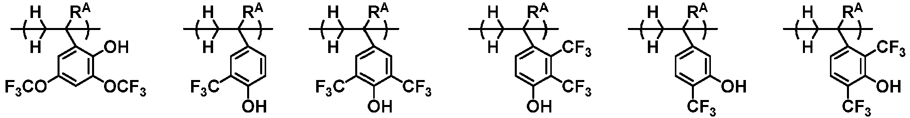

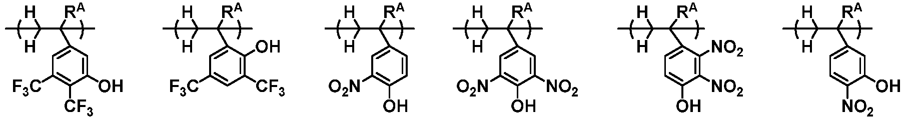

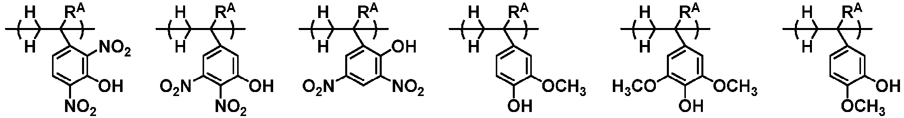

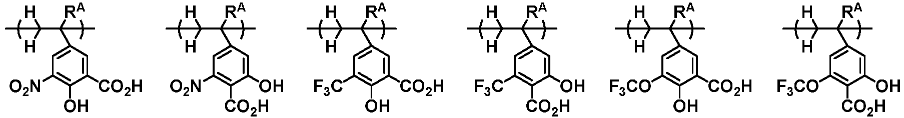

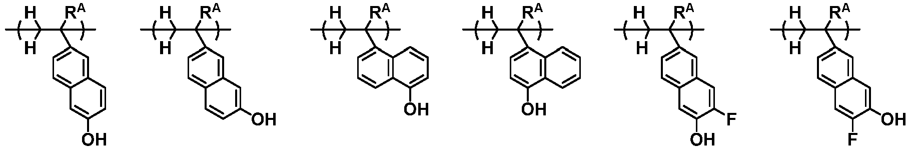

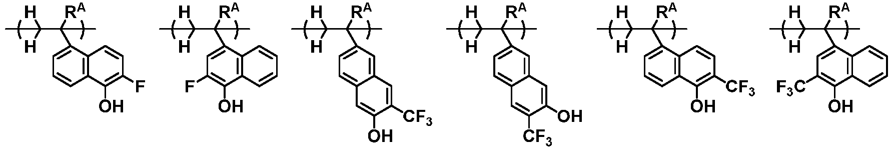

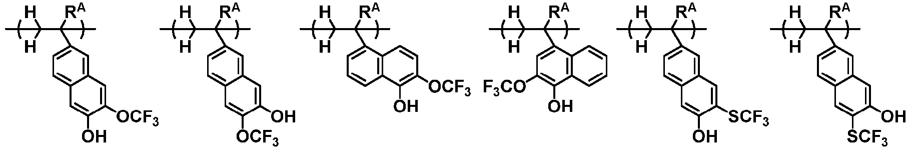

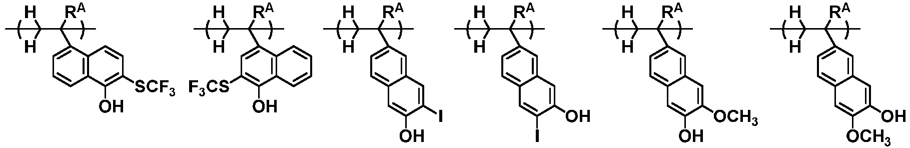

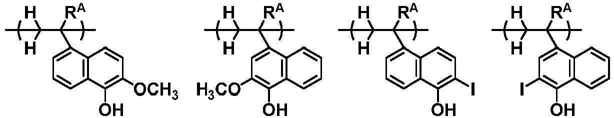

Preferred examples of the repeat units B1 wherein a1=1, that is, having a linker: -C(=O)-O-A¹- are shown below, but not limited thereto. Herein R^{A} is as defined above.

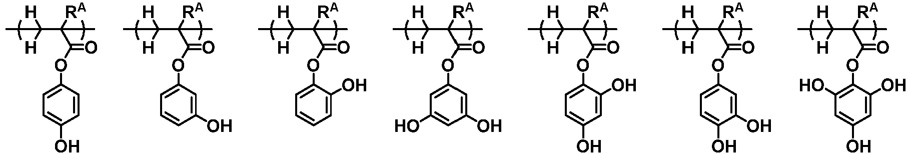

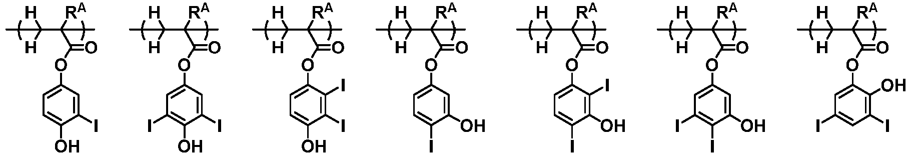

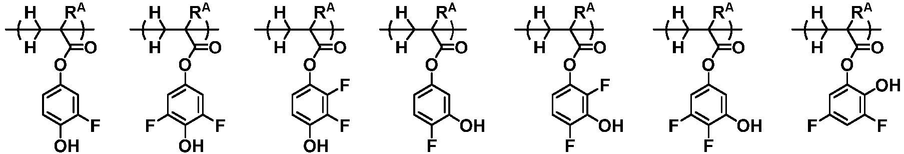

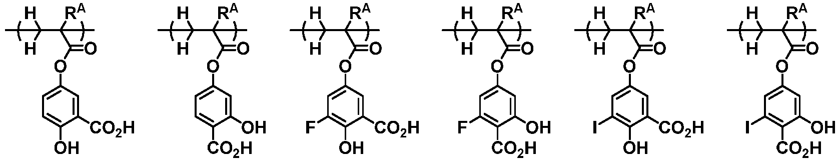

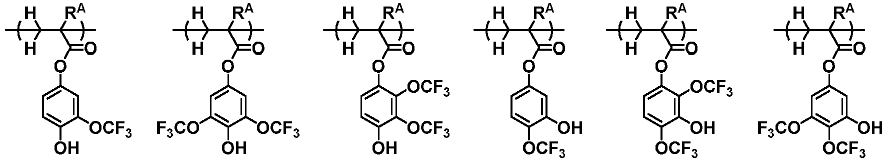

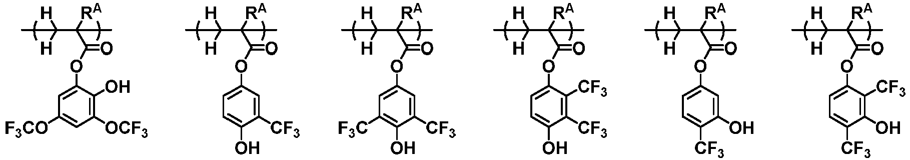

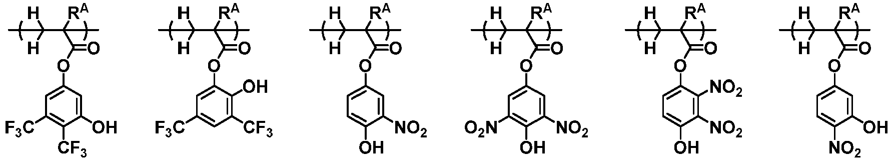

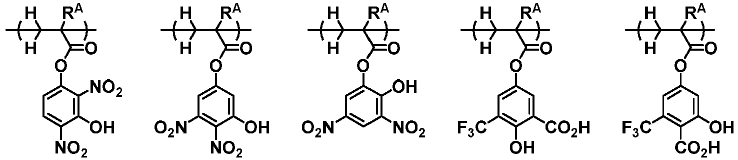

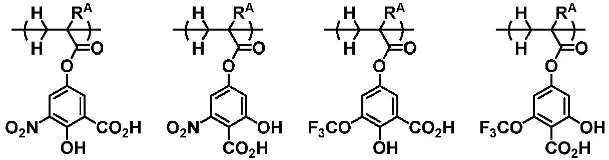

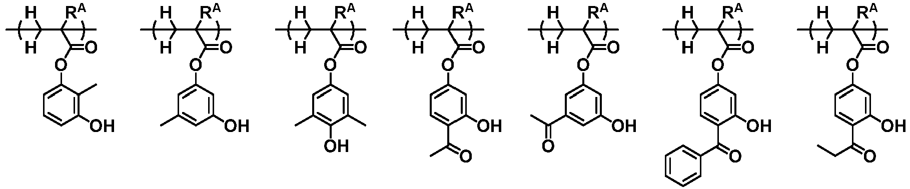

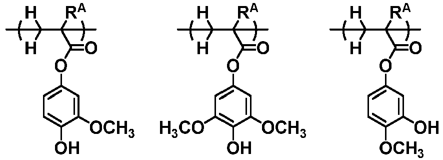

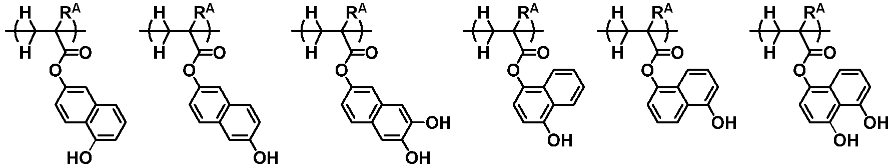

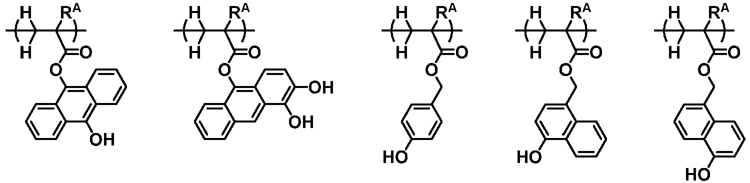

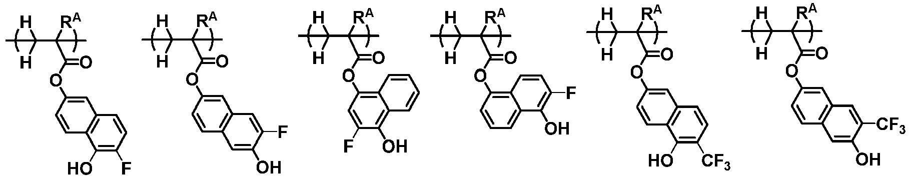

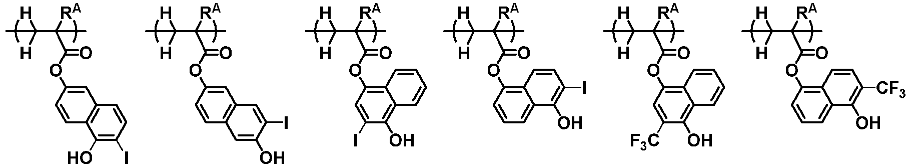

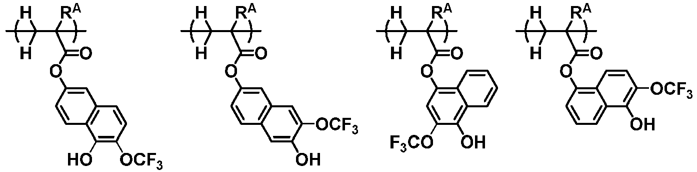

The content of repeat units B1 is preferably 15 to 90 mol%, more preferably 15 to 80 mol% of the overall units of the polymer. When the polymer further comprises repeat units of at least one type selected from repeat units having formula (B3) and repeat units having formula (B4), which provide the polymer with higher etch resistance, the repeat units containing a phenolic hydroxy group as a substituent, the total content of repeat units B1 and repeat units B3 and/or B4 should preferably fall in the range. The repeat units B1 may be of one type or a combination of plural types.

In a preferred embodiment, the polymer further contains repeat units B2 having an acidic functional group protected with an acid labile group (i.e., repeat units protected with an acid labile group and adapted to turn alkali soluble under the action of acid) in order that the positive resist composition in an exposed region turn soluble in alkaline developer.

Typical of the repeat unit B2 is a unit having the formula (B2-1), also referred to as repeat unit B2-1.

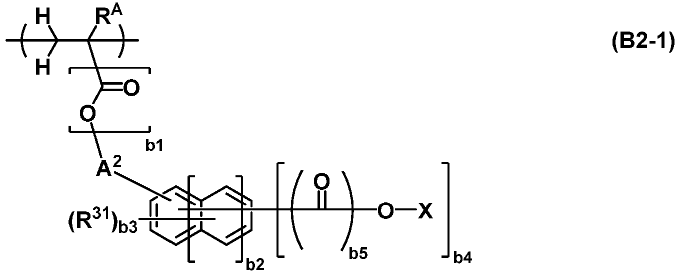

In formula (B2-1), b1 is 0 or 1. The subscript b2 is 0, 1 or 2. The structure represents a benzene skeleton when b2=0, a naphthalene skeleton when b2=1, and an anthracene skeleton when b2=2. The subscript b3 is an integer meeting 0 ≤ b3 ≤ 5+2(b2)-b4. The subscript b4 is 1, 2 or 3, and b5 is 0 or 1. When b2=0, preferably b3 is 0, 1, 2 or 3 and b4 is 1, 2 or 3. When b2=1 or 2, preferably b3 is 0, 1, 2, 3 or 4 and b4 is 1, 2 or 3.

In formula (B2-1), R^{A} is hydrogen, fluorine, methyl or trifluoromethyl.

In formula (B2-1), R³¹ is halogen, an optionally halogenated C₁-C₆ saturated hydrocarbyl group, optionally halogenated C₁-C₆ saturated hydrocarbyloxy group or optionally halogenated C₂-C₈ saturated hydrocarbylcarbonyloxy group. The saturated hydrocarbyl group and saturated hydrocarbyl moiety in the saturated hydrocarbyloxy group and saturated hydrocarbylcarbonyloxy group may be straight, branched or cyclic, and examples thereof include alkyl groups such as methyl, ethyl, n-propyl, isopropyl, butyl, pentyl, hexyl, and structural isomers thereof, cycloalkyl groups such as cyclopropyl, cyclobutyl, cyclopentyl and cyclohexyl, and combinations thereof. A carbon count within the upper limit ensures good solubility in alkaline developer. A plurality of R³¹ may be identical or different when b3 is 2 or more.

In formula (B2-1), A² is a single bond or a C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of b1=1 in formula (B2-1), the ether bond may be incorporated at any position excluding the position between the α-carbon and β-carbon relative to the ester oxygen. In case of b1=0, the atom that bonds with the backbone becomes an ethereal oxygen, and a second ether bond may be incorporated at any position excluding the position between the α-carbon and β-carbon relative to that ethereal oxygen. Saturated hydrocarbylene groups having no more than 10 carbon atoms are desirable because of a sufficient solubility in alkaline developer.

In formula (B2-1), X is an acid labile group when b4=1, and hydrogen or an acid labile group, at least one X being an acid labile group, when b4=2 or 3. That is, repeat units B2-1 have phenolic hydroxy groups bonded to an aromatic ring, at least one of which is protected with an acid labile group, or repeat units B2-1 have a carboxy group bonded to an aromatic ring, which is protected with an acid labile group. The acid labile group used herein is not particularly limited as long as it is commonly used in a number of well-known chemically amplified resist compositions and eliminated under the action of acid to release an acidic group.

Typical of the acid labile group is a tertiary saturated hydrocarbyl group. The tertiary saturated hydrocarbyl group is preferably of 4 to 18 carbon atoms because a monomer for use in polymerization is recoverable by distillation.

The saturated hydrocarbyl group bonded to the tertiary carbon atom in the tertiary saturated hydrocarbyl group is preferably of 1 to 15 carbon atoms. The C₁-C₁₅ saturated hydrocarbyl group may be straight, branched or cyclic and contain an oxygen-containing functional group such as an ether bond or carbonyl group in its carbon-carbon bond. The saturated hydrocarbyl groups bonded to the tertiary carbon atom may bond together to form a ring with the tertiary carbon atom to which they are attached.

Examples of the alkyl substituent include methyl, ethyl, propyl, adamantyl, norbornyl, tetrahydrofuran-2-yl, 7-oxanorbornan-2-yl, cyclopentyl, 2-tetrahydrofuryl, tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, tetracyclo[4.4.0.1^{2,5}.1^{7,10}]dodecyl, and 3-oxo-1-cyclohexyl.

Examples of the tertiary saturated hydrocarbyl group include, but are not limited to, tert-butyl, tert-pentyl, 1-ethyl-1-methylpropyl, 1,1-diethylpropyl, 1,1,2-trimethylpropyl, 1-adamantyl-1-methylethyl, 1-methyl-1-(2-norbornyl)ethyl, 1-methyl-1-(tetrahydrofuran-2-yl)ethyl, 1-methyl-1-(7-oxanorbornan-2-yl)ethyl, 1-methylcyclopentyl, 1-ethylcyclopentyl, 1-propylcyclopentyl, 1-cyclopentylcyclopentyl, 1-cyclohexylcyclopentyl, 1-(2-tetrahydrofuryl)cyclopentyl, 1-(7-oxanorbornan-2-yl)cyclopentyl, 1-methylcyclohexyl, 1-ethylcyclohexyl, 1-cyclopentylcyclohexyl, 1-cyclohexylcyclohexyl, 2-methyl-2-norbornyl, 2-ethyl-2-norbornyl, 8-methyl-8-tricyclo[5 .2.1.0^{2,6}]decyl, 8-ethyl-8-tricyclo[5.2.1.0^{2,6}]decyl, 3-methyl-3-tetracyclo[4.4.0.1^{2,5} 1^{7,10}]dodecyl, 3-ethyl-3-tetracyclo[4.4.0.1^{2,5}.1^{7,10}]dodecyl, 2-methyl-2-adamantyl, 2-ethyl-2-adamantyl, 1-methyl-3-oxo-1-cyclohexyl, 1-methyl-1-(tetrahydrofuran-2-yl)ethyl, 5-hydroxy-2-methyl-2-adamantyl, and 5-hydroxy-2-ethyl-2-adamantyl.

A group having the following formula (B2-1-1) is also suitable as the acid labile group. The group having formula (B2-1-1) is often used as the acid labile group. It is a good choice of the acid labile group that ensures to form a pattern having a relatively rectangular pattern-substrate interface in a consistent manner. An acetal structure is formed when X is a group having formula (B2-1-1).

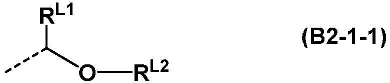

In formula (B2-1-1), R^{L1} is hydrogen or a C₁-C₁₀ saturated hydrocarbyl group. The saturated hydrocarbyl group may be straight, branched or cyclic.

A choice of R^{L1} may depend on the designed sensitivity of labile group to acid. For example, hydrogen or a group in which the carbon atom bonded to acetal carbon is tertiary is selected when the acid labile group is designed to ensure relatively high stability and to be decomposed with strong acid. Examples of R^{L1} bonded to acetal carbon via tertiary carbon include tert-butyl, tert-pentyl, and 1-adamantyl, but are not limited thereto. A straight alkyl group is selected when the acid labile group is designed to have relatively high reactivity and high sensitivity to pH changes. Although the choice varies with a particular combination of acid generator and quencher in the resist composition, R^{L1} is preferably a group in which the carbon in bond with acetal carbon is secondary, when R^{L2} is a relatively large alkyl group substituted at the end and the acid labile group is designed to undergo a substantial change of solubility by decomposition. Examples of R^{L1} bonded to acetal carbon via secondary carbon include isopropyl, sec-butyl, cyclopentyl, and cyclohexyl, but are not limited thereto.

In formula (B2-1-1), R^{L2} is a C₁-C₃₀ hydrocarbyl group. The hydrocarbyl group may be saturated or unsaturated and straight, branched or cyclic. Some constituent -CH₂-in the hydrocarbyl group may be replaced by a heteroatom such as oxygen or sulfur so that the group may contain an ether bond or sulfide bond. Illustrative are C₁-C₃₀ saturated hydrocarbyl groups and C₆-C₃₀ aryl groups. R^{L2} is preferably a C₁-C₆ hydrocarbyl group for acquiring a higher resolution in forming small-size patterns. When R^{L2} is a C₁-C₆ hydrocarbyl group, the alcohol created after a progress of acid-aided deprotection reaction is water soluble. Then, when a positive pattern is formed using an alkaline developer, the alcohol is dissolved in the developer so that defects remaining in the exposed region are minimized.

Preferred examples of the group having formula (B2-1-1) are given below, but not limited thereto. Herein R^{L1} is as defined above.

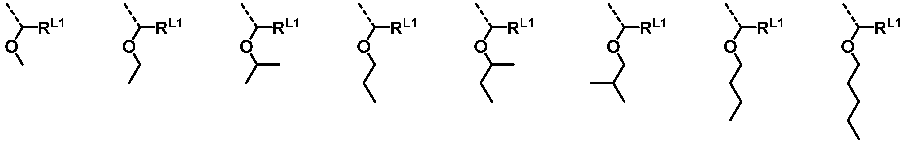

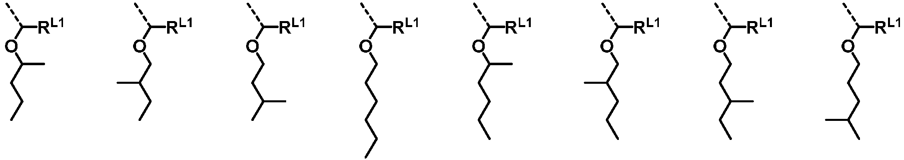

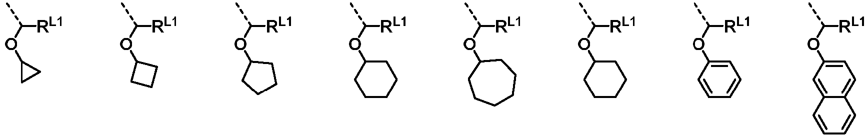

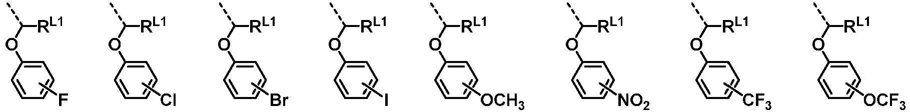

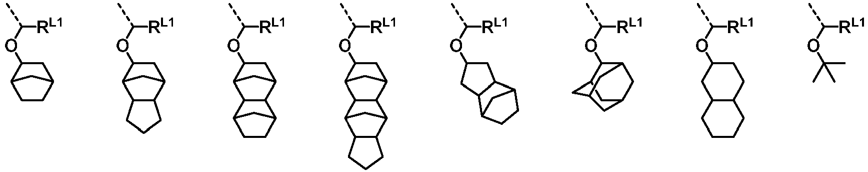

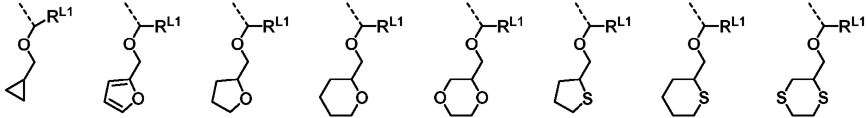

Another acid labile group which can be used herein is a phenolic hydroxy group whose hydrogen is substituted by -CH₂COO-(tertiary saturated hydrocarbyl group). The tertiary saturated hydrocarbyl group may be the same as the foregoing tertiary saturated hydrocarbyl group used for the protection of a phenolic hydroxy group.

Another example of repeat unit B2 is a repeat unit having the following formula (B2-2), referred to as repeat unit B2-2. The repeat unit B2-2 which enables to increase the dissolution rate in the exposed region is a useful choice of the acid labile group-containing unit which affords satisfactory performance against line width variations during develop loading.

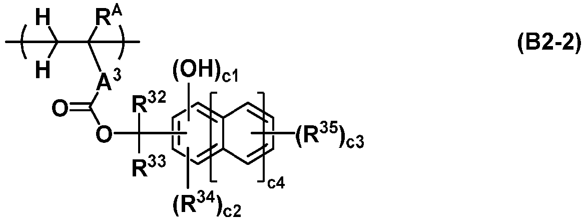

In formula (B2-2), c1 is 0, 1 or 2, c2 is 0, 1 or 2, c3 is 0, 1, 2, 3, 4 or 5, and c4 is 0, 1 or 2.

In formula (B2-2), R^{A} is hydrogen, fluorine, methyl or trifluoromethyl.

In formula (B2-2), R³² and R³³ are each independently a C₁-C₁₀ hydrocarbyl group which may contain a heteroatom. R³² and R³³ may bond together to form a ring with the carbon atom to which they are attached.

In formula (B2-2), R³⁴ is each independently fluorine, C₁-C₅ fluorinated alkyl group or C₁-C₅ fluorinated alkoxy group.

In formula (B2-2), R³⁵ is each independently a C₁-C₁₀ hydrocarbyl group which may contain a heteroatom.

In formula (B2-2), A³ is a single bond, phenylene group, naphthylene group, or *-C(=O)-O-A³¹-. A³¹ is a C₁-C₂₀ aliphatic hydrocarbylene group which may contain hydroxy, ether bond, ester bond or lactone ring, or phenylene or naphthylene group, and * is a point of attachment to the carbon atom in the backbone.

Preferred examples of the repeat unit B2-2 are shown below, but not limited thereto. Herein R^{A} is as defined above.

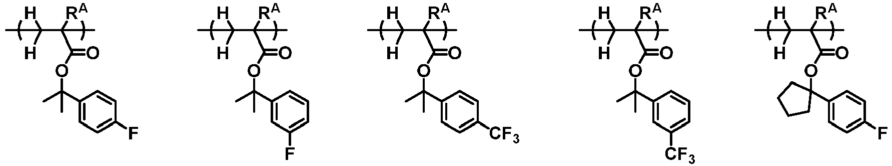

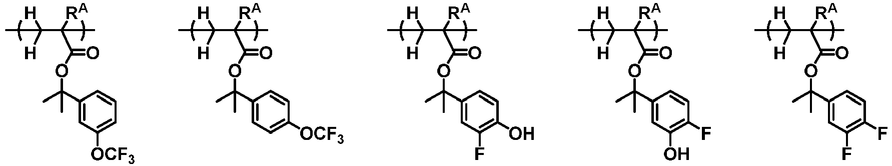

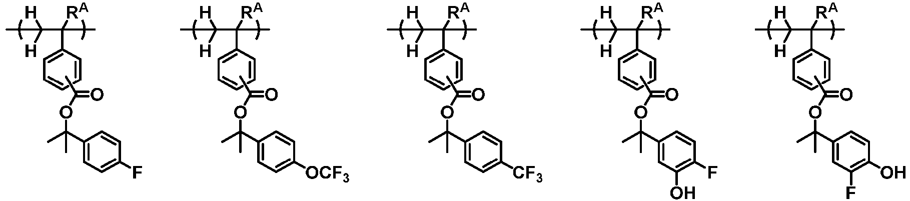

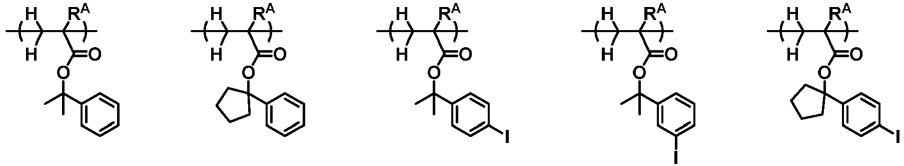

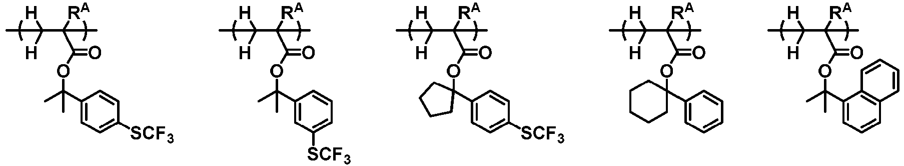

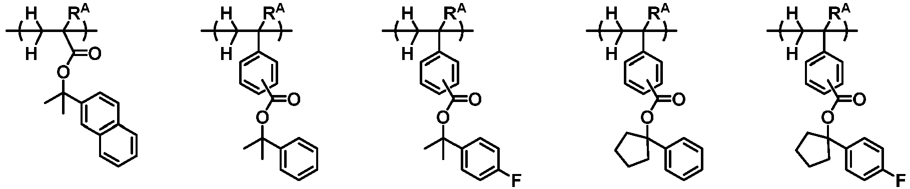

The content of repeat units B2 is preferably 5 to 95 mol%, more preferably 20 to 80 mol% based on the overall repeat units of the polymer. Each of repeat units B2 may be of one type or a mixture of two or more types.

In a preferred embodiment, the polymer further comprises repeat units of at least one type selected from units having the formulae (B3), (B4) and (B5). These repeat units are simply referred to as repeat units B3, B4 and B5, respectively.

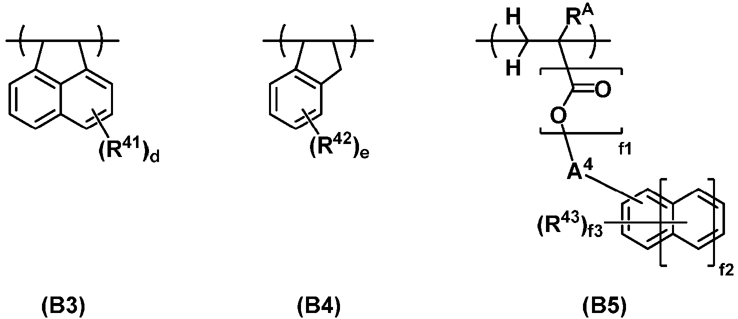

In formulae (B3) and (B4), d is 0, 1, 2, 3, 4, 5 or 6 and e is 0, 1, 2, 3 or 4.

In formulae (B3) and (B4), R⁴¹ and R⁴² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group. The saturated hydrocarbyl group, saturated hydrocarbyloxy group and saturated hydrocarbylcarbonyloxy group may be straight, branched or cyclic. When d is 2 or more, a plurality of R⁴¹ may be identical or different. When e is 2 or more, a plurality of R⁴² may be identical or different.

In formula (B5), f1 is 0 or 1. The subscript f2 is 0, 1 or 2. The relevant structure represents a benzene skeleton when f2=0, a naphthalene skeleton when f2=1, and an anthracene skeleton when f2=2. The subscript f3 is 0, 1, 2, 3, 4 or 5. When f2=0, preferably f3 is 0, 1, 2 or 3. When f2=1 or 2, preferably f3 is 0, 1, 2, 3 or 4.

In formula (B5), R^{A} is hydrogen, fluorine, methyl or trifluoromethyl.

In formula (B5), R⁴³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen atom, nitro group, or cyano group. When f2 is 1 or 2, R⁴³ may also be hydroxy. The saturated hydrocarbyl group, saturated hydrocarbyloxy group, saturated hydrocarbylcarbonyloxy group, saturated hydrocarbyloxyhydrocarbyl group, and saturated hydrocarbylthiohydrocarbyl group may be straight, branched or cyclic. When f3 is 2 or more, a plurality of R⁴³ may be identical or different.

In formula (B5), A⁴ is a single bond or a C₁-C₁₀ saturated hydrocarbylene group in which some constituent -CH₂- may be replaced by -O-. The saturated hydrocarbylene group may be straight, branched or cyclic. Examples thereof are as exemplified above for A¹ in formula (B1).

When repeat units of at least one type selected from repeat units B3 to B5 are incorporated, better performance is obtained because not only the aromatic ring possesses etch resistance, but the cyclic structure incorporated into the backbone also exerts the effect of improving etch resistance and resistance to EB irradiation during pattern inspection step.

The content of repeat units B3 to B5 is preferably at least 5 mol% based on the overall repeat units of the polymer for obtaining the effect of improving etch resistance. Also, the content of repeat units B3 to B5 is preferably up to 25 mol%, more preferably up to 20 mol% based on the overall repeat units of the polymer. When the relevant units are free of functional groups or have a functional group other than hydroxy, their content of up to 25 mol% is preferred because the risk of forming development defects is eliminated. Each of the repeat units B3 to B5 may be of one type or a combination of plural types.

It is preferred that the polymer comprise repeat units B1, repeat units B2, and repeat units of at least one type selected from repeat units B3 to B5, because both etch resistance and high resolution are achievable. The total content of these repeat units is preferably at least 60 mol%, more preferably at least 70 mol%, even more preferably at least 80 mol%, most preferably at least 90 mol% based on the overall repeat units of the polymer.

In another preferred embodiment, the polymer further comprises repeat units of at least one type selected from repeat units having the formula (B6), repeat units having the formula (B7), repeat units having the formula (B8), repeat units having the formula (B9), and repeat units having the formula (B10), shown below. Notably these repeat units are also referred to as repeat units B6 to B 10.

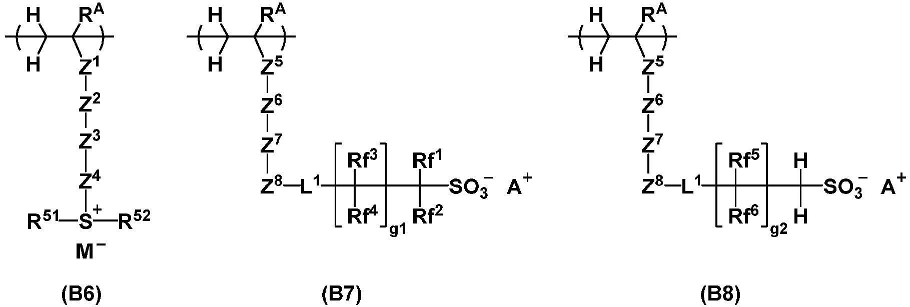

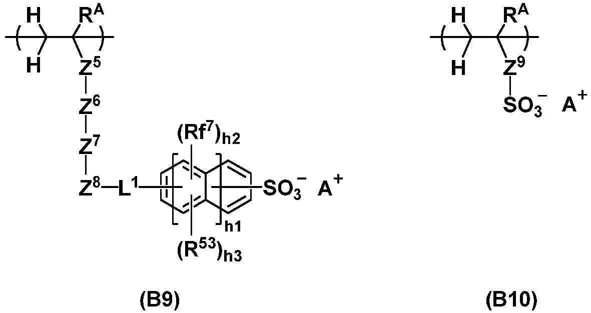

In formulae (B6) to (B10), R^{A} is each independently hydrogen, fluorine, methyl or trifluoromethyl. Z¹ is a single bond or an optionally substituted phenylene group. Z² is a single bond, **-C(=O)-O-Z²¹-, **-C(=O)-NH-Z²¹-, or **-O-Z²¹-, wherein Z²¹ is a C₁-C₆ aliphatic hydrocarbylene group, phenylene group or a divalent group obtained by combining the foregoing, which may contain halogen, carbonyl moiety, ester bond, ether bond or hydroxy moiety. Z³ is a single bond, ether bond, ester bond, amide bond, sulfonate ester bond, sulfonamide bond, carbonate bond or carbamate bond. Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety. Z⁵ is each independently a single bond, an optionally substituted phenylene, naphthylene, or *-C(=O)-O-Z⁵¹-, wherein Z⁵¹ is a C₁-C₁₀ aliphatic hydrocarbylene group which may contain halogen, hydroxy moiety, ether bond, ester bond or lactone ring, or phenylene or naphthylene group. Z⁶ is a single bond, ether bond, ester bond, amide bond, sulfonate ester bond, sulfonamide bond, carbonate bond or carbamate bond. Z⁷ is each independently a single bond, ***-Z⁷¹-C(=O)-O-, ***-C(=O)-NH-Z⁷¹-, or ***-O-Z⁷¹-, wherein Z⁷¹ is a C₁-C₂₀ hydrocarbylene group which may contain a heteroatom. Z⁸ is each independently a single bond, ****-Z⁸¹-C(=O)-O-, ****-C(=O)-NH-Z⁸¹-, or ****-O-Z⁸¹-, wherein Z⁸¹ is a C₁-C₂₀ hydrocarbylene group which may contain a heteroatom. Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, wherein Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety. The asterisk * designates a point of attachment to the carbon atom in the backbone, ** is a point of attachment to Z¹, *** is a point of attachment to Z⁶, and **** is a point of attachment to Z⁷.

The aliphatic hydrocarbylene group represented by Z²¹, Z⁵¹ and Z⁹¹ may be straight, branched or cyclic. Examples thereof include alkanediyl groups such as methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, 1,1-dimethylethane-1,2-diyl, pentane-1,5-diyl, 2-methylbutane-1,2-diyl, and hexane-1,6-diyl; cycloalkanediyl groups such as cyclopropanediyl, cyclobutanediyl, cyclopentanediyl, and cyclohexanediyl; and combinations thereof.

The hydrocarbylene group represented by Z⁷¹ and Z⁸¹ may be saturated or unsaturated and straight, branched or cyclic. Examples thereof are shown below, but not limited thereto.

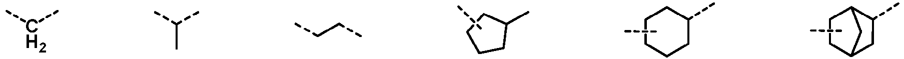

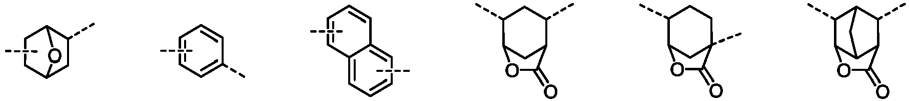

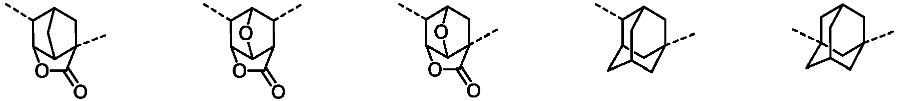

In formula (B6), R⁵¹ and R⁵² are each independently a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, and tert-butyl; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl, and adamantyl; C₂-C₂₀ alkenyl groups such as vinyl, 1-propenyl, 2-propenyl, butenyl, and hexenyl; C₃-C₂₀ cyclic unsaturated hydrocarbyl groups such as cyclohexenyl; C₆-C₂₀ aryl groups such as phenyl, naphthyl and thienyl; C₇-C₂₀ aralkyl groups such as benzyl, 1-phenylethyl and 2-phenylethyl, and combinations thereof. Inter alia, aryl groups are preferred. In the hydrocarbyl group, some or all of the hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-), or haloalkyl moiety.

R⁵¹ and R⁵² may bond together to form a ring with the sulfur atom to which they are attached. Examples of the ring are shown below.

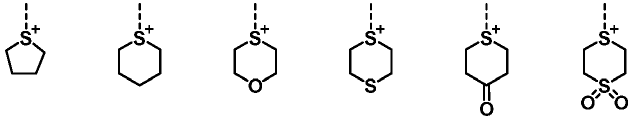

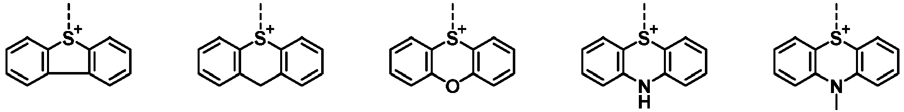

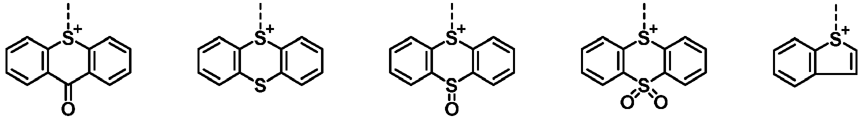

Herein the broken line designates a point of attachment to Z⁴.

Examples of the cation in repeat units B6 are shown below, but not limited thereto. Herein R^{A} is as defined above.

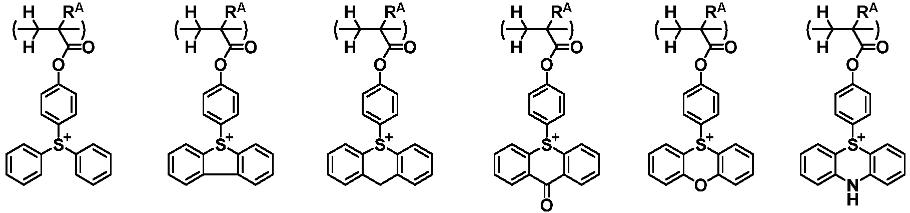

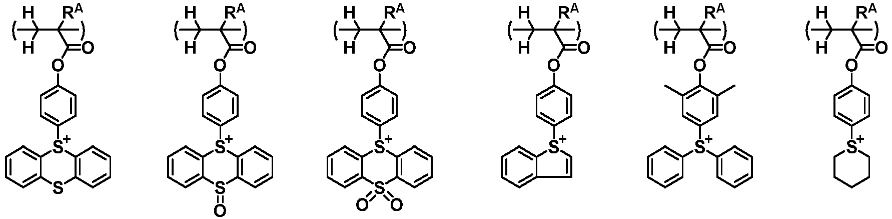

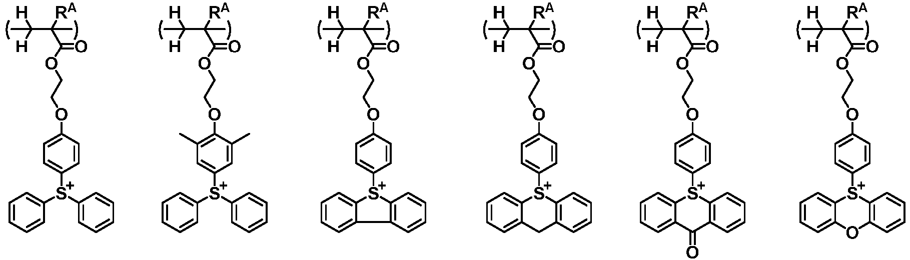

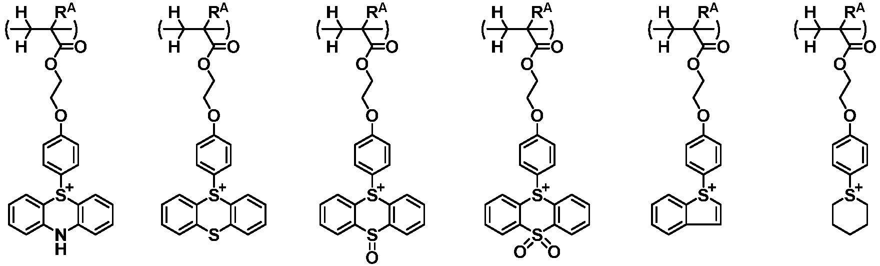

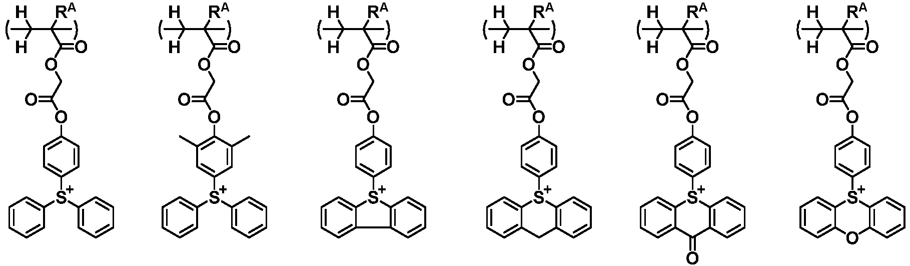

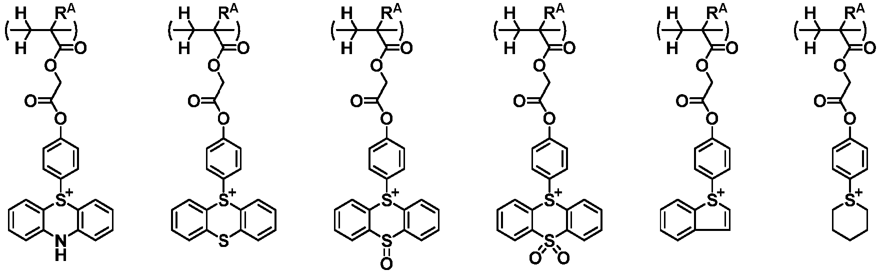

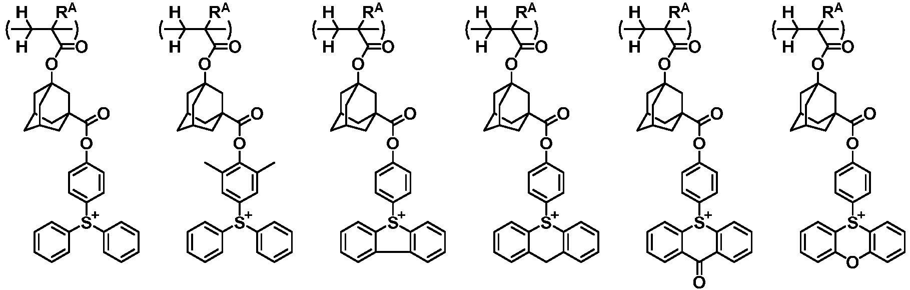

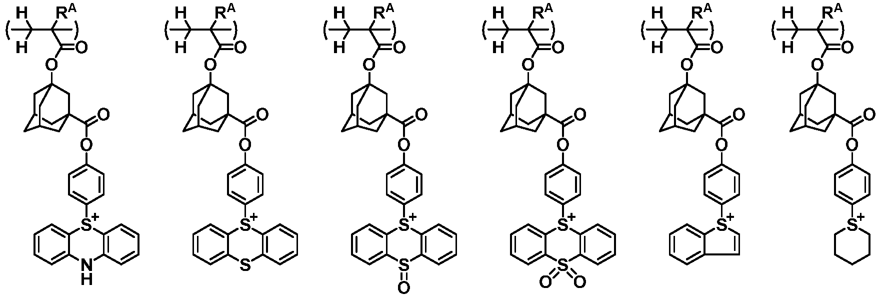

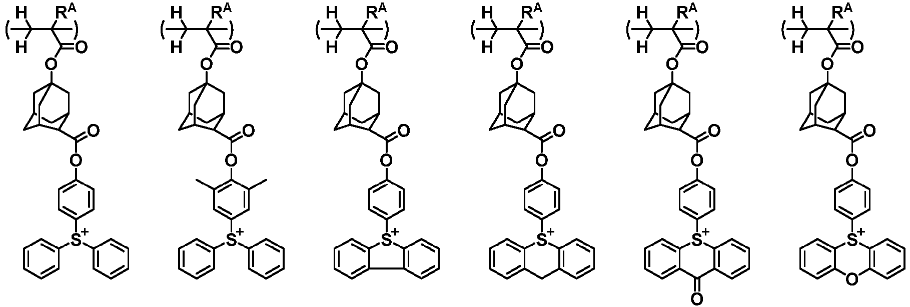

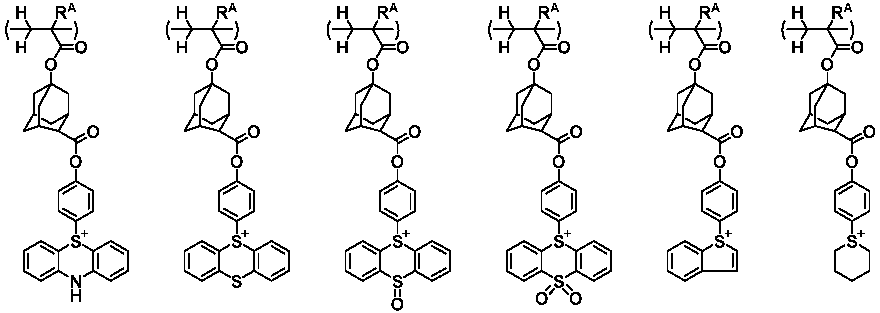

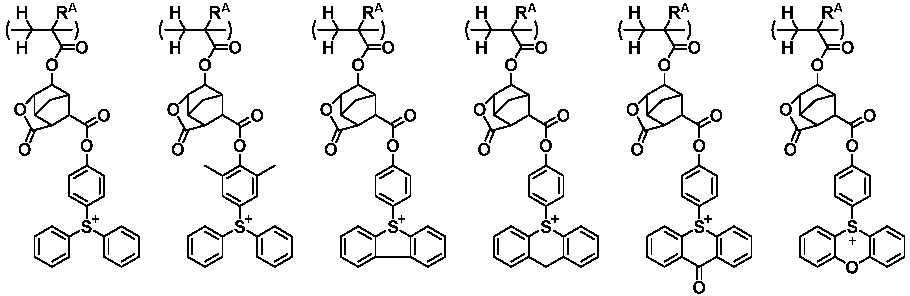

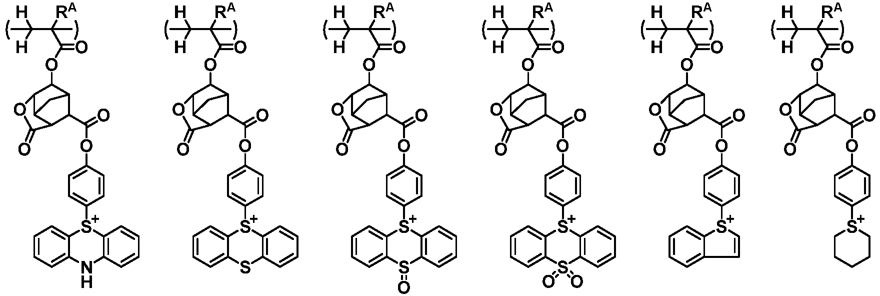

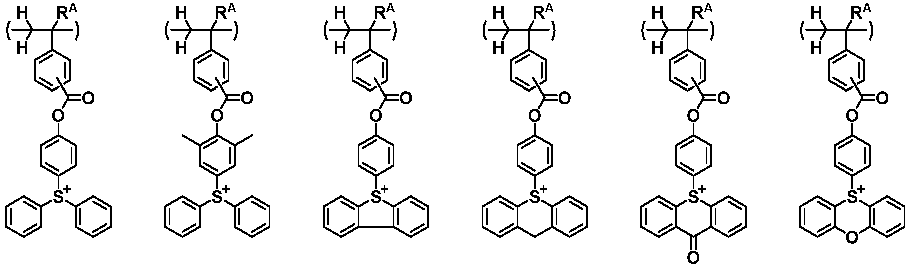

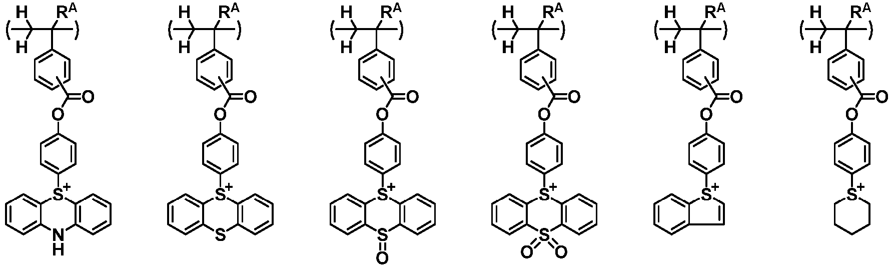

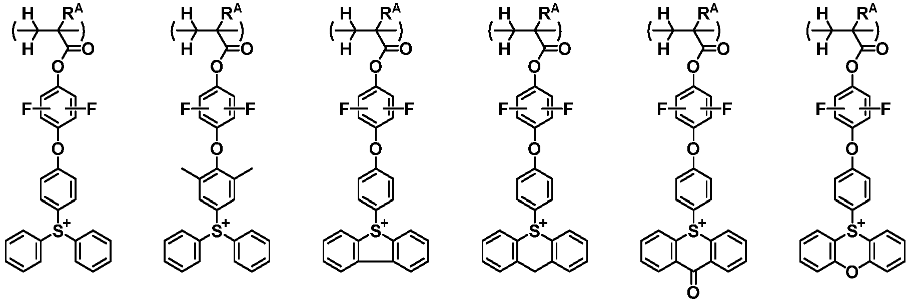

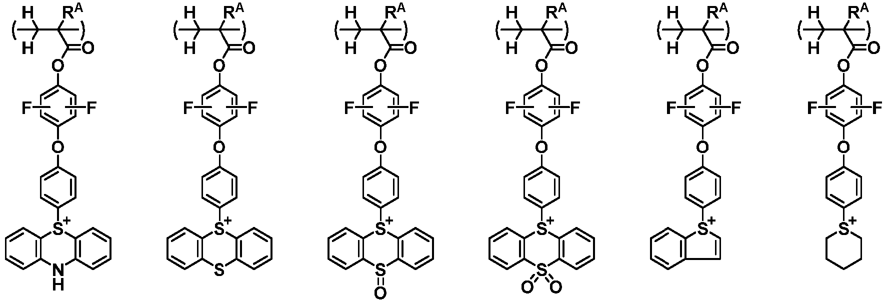

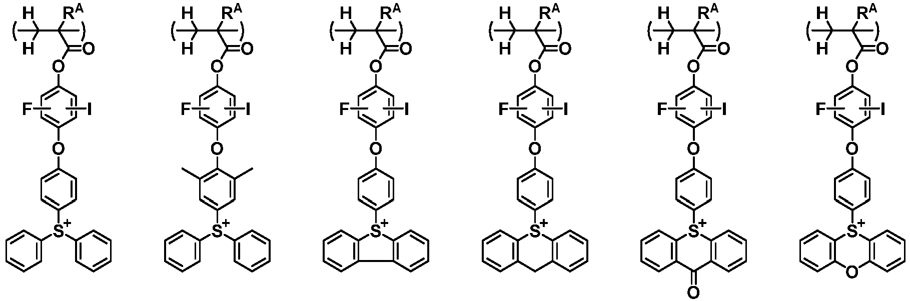

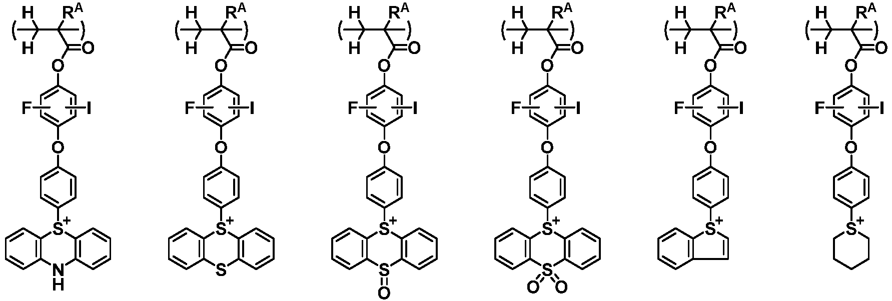

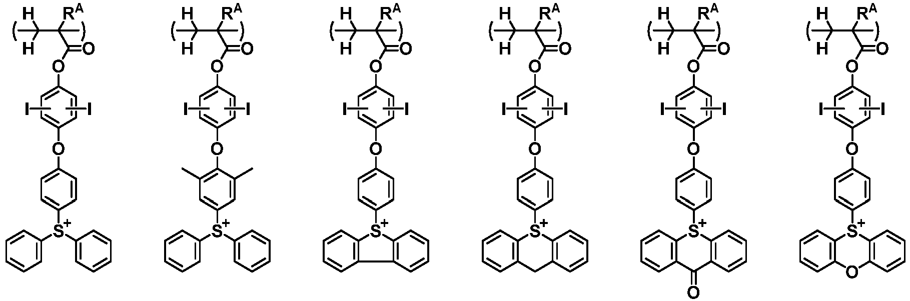

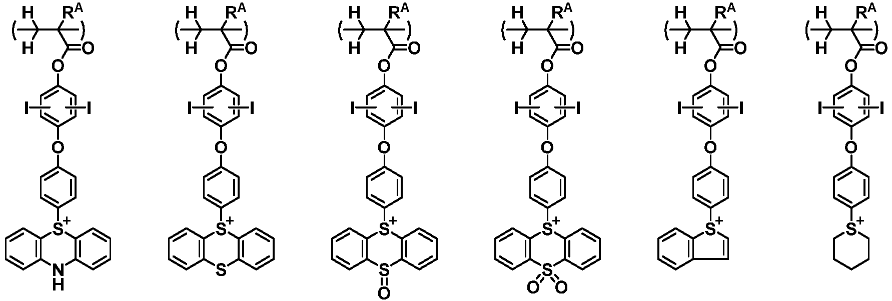

In formula (B6), M⁻ is a non-nucleophilic counter ion. Halide ions, sulfonate anions, imide anions, and methide anions are preferred. Examples of the non-nucleophilic counter ion include halide ions such as chloride and bromide ions; sulfonate anions, specifically fluoroalkylsulfonate ions such as triflate, 1,1,1-trifluoroethanesulfonate, and nonafluorobutanesulfonate, arylsulfonate ions such as tosylate, benzenesulfonate, 4-fluorobenzenesulfonate, and 1,2,3,4,5-pentafluorobenzenesulfonate, alkylsulfonate ions such as mesylate and butanesulfonate; imide ions such as bis(trifluoromethylsulfonyl)imide, bis(perfluoroethylsulfonyl)imide and bis(perfluorobutylsulfonyl)imide; and methide ions such as tris(trifluoromethylsulfonyl)methide and tris(perfluoroethylsulfonyl)methide.

Anions having the following formulae (B6-1) to (B6-4) are also useful as the non-nucleophilic counter ion.

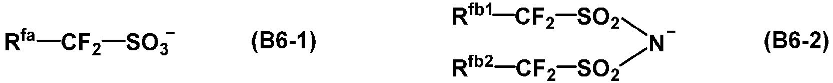

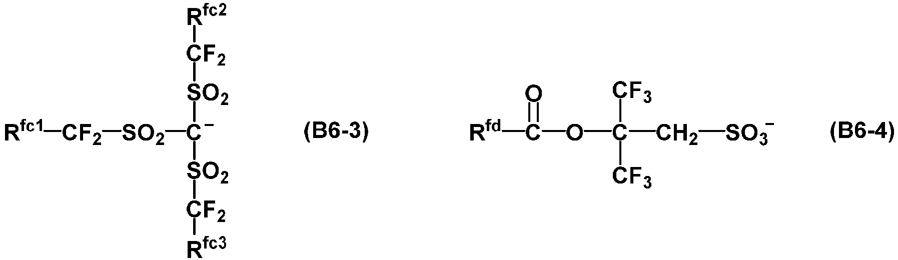

In formula (B6-1), R^{fa} is fluorine or a C₁-C₄₀ hydrocarbyl group which may contain a heteroatom. The hydrocarbyl group may be saturated or unsaturated and straight, branched or cyclic. Examples thereof are as will be exemplified below for the hydrocarbyl group R^{fa1} in formula (B6-1-1).

Of the anions of formula (B6-1), an anion having the formula (B6-1-1) is preferred.

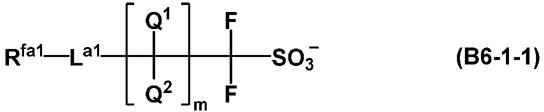

In formula (B6-1-1), Q¹ and Q² are each independently hydrogen, fluorine or a C₁-C₆ fluorinated saturated hydrocarbyl group. It is preferred for solvent solubility that at least one of Q¹ and Q² be trifluoromethyl. The subscript m is 0, 1, 2, 3 or 4, preferably 1.

R^{fa1} is a C₁-C₃₅ hydrocarbyl group which may contain a heteroatom. As the heteroatom, oxygen, nitrogen, sulfur and halogen atoms are preferred, with oxygen being most preferred. Of the hydrocarbyl groups, those groups of 6 to 30 carbon atoms are preferred from the aspect of achieving a high resolution in forming patterns of small feature size. The hydrocarbyl group may be saturated or unsaturated and straight, branched or cyclic. Examples thereof include C₁-C₃₅ alkyl groups such as methyl, ethyl, n-propyl, isopropyl, n-butyl, isobutyl, sec-butyl, tert-butyl, pentyl, neopentyl, hexyl, heptyl, 2-ethylhexyl, nonyl, undecyl, tridecyl, pentadecyl, heptadecyl, and icosyl; C₃-C₃₅ cyclic saturated hydrocarbyl groups such as cyclopentyl, cyclohexyl, 1-adamantyl, 2-adamantyl, 1-adamantylmethyl, norbornyl, norbornylmethyl, tricyclodecyl, tetracyclododecyl, tetracyclododecylmethyl, and dicyclohexylmethyl; C₂-C₃₅ unsaturated aliphatic hydrocarbyl groups such as 2-propenyl and 3-cyclohexenyl; C₆-C₃₅ aryl groups such as phenyl, 1-naphthyl, 2-naphthyl and 9-fluorenyl; and C₇-C₃₅ aralkyl groups such as benzyl and diphenylmethyl, and combinations thereof.

In the foregoing hydrocarbyl groups, some or all hydrogen may be substituted by a moiety containing a heteroatom such as oxygen, sulfur, nitrogen or halogen, or some constituent -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonate ester bond, carbonate bond, lactone ring, sultone ring, carboxylic anhydride (-C(=O)-O-C(=O)-) or haloalkyl moiety. Examples of the heteroatom-containing hydrocarbyl group include tetrahydrofuryl, methoxymethyl, ethoxymethyl, methylthiomethyl, acetamidomethyl, trifluoroethyl, (2-methoxyethoxy)methyl, acetoxymethyl, 2-carboxy-1-cyclohexyl, 2-oxopropyl, 4-oxo-1-adamantyl, and 3-oxocyclohexyl.

In formula (B6-1-1), L^{a1} is a single bond, ether bond, ester bond, sulfonate ester bond, carbonate bond or carbamate bond. From the aspect of synthesis, an ether bond or ester bond is preferred, with the ester bond being more preferred.

Examples of the anion having formula (B6-1) are shown below, but not limited thereto. Herein Q¹ is as defined above.

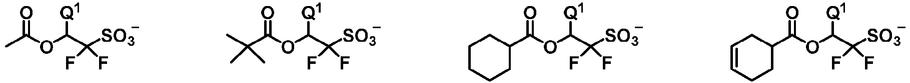

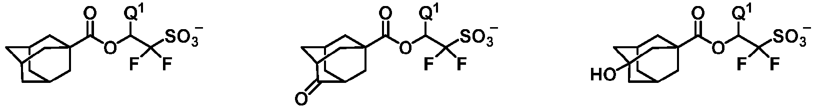

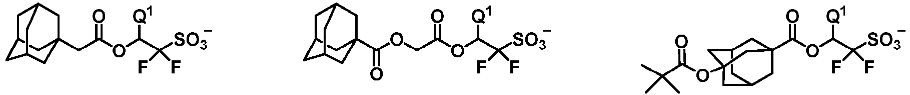

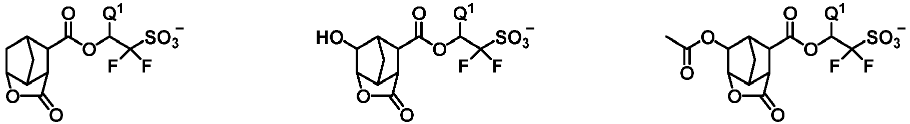

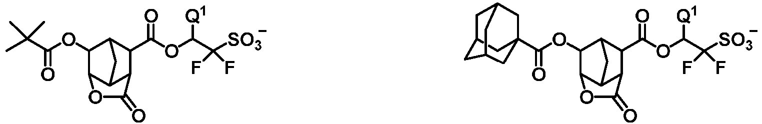

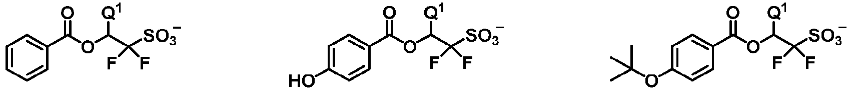

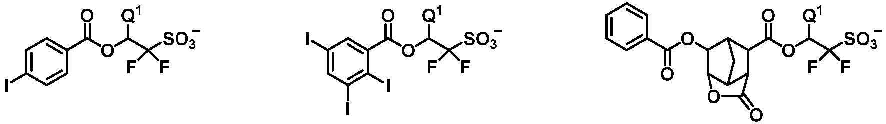

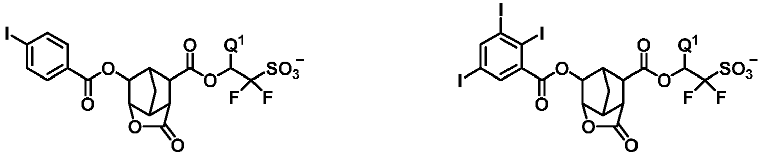

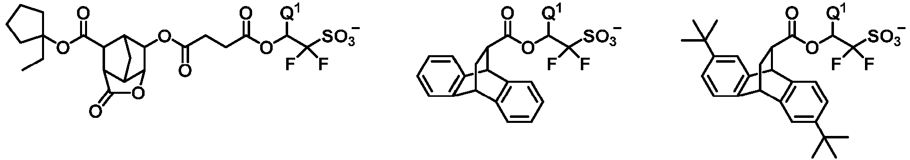

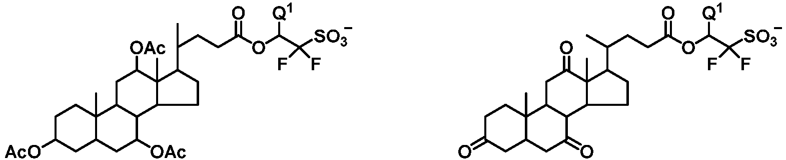

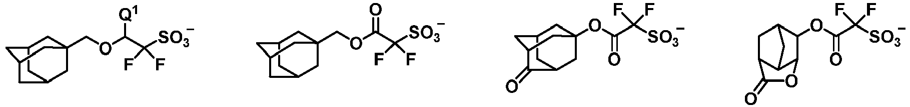

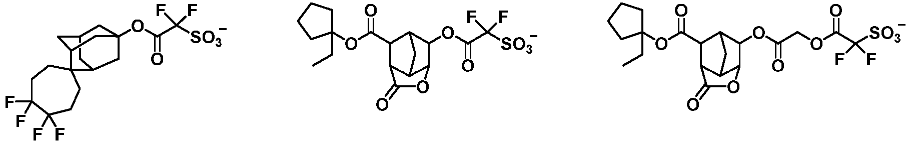

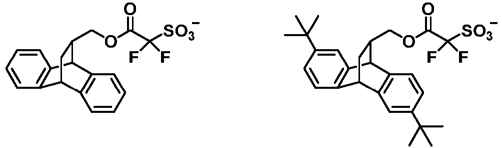

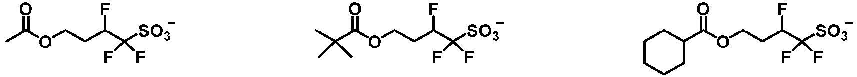

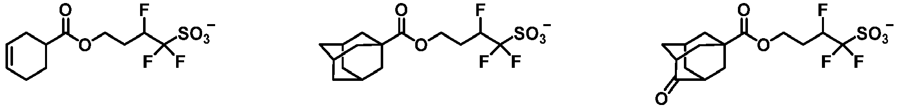

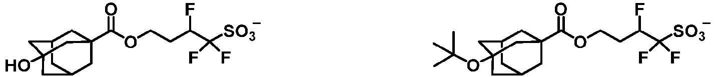

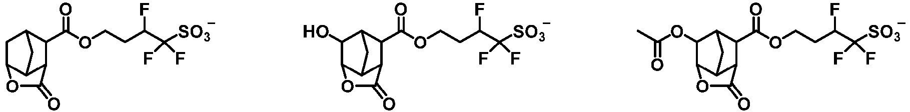

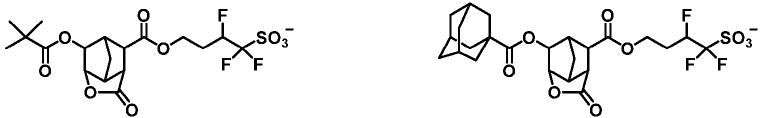

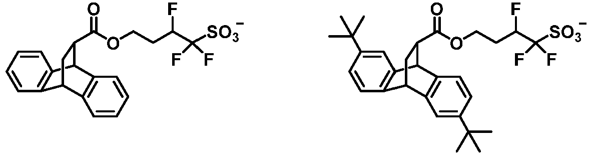

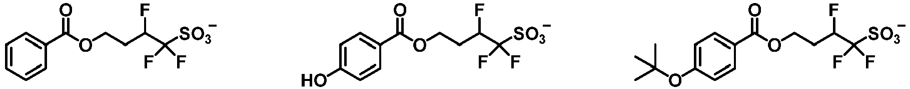

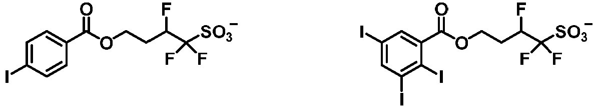

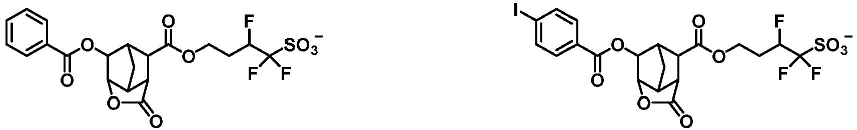

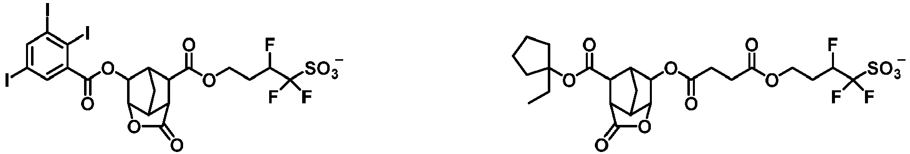

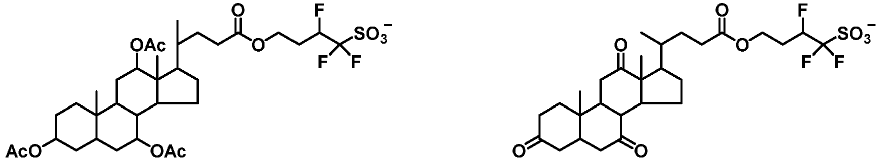

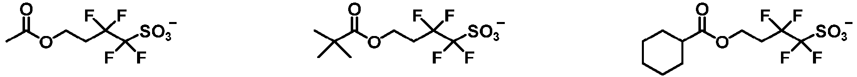

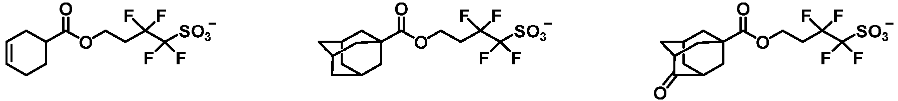

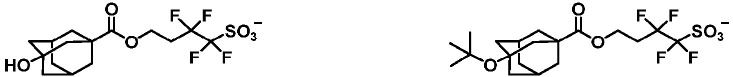

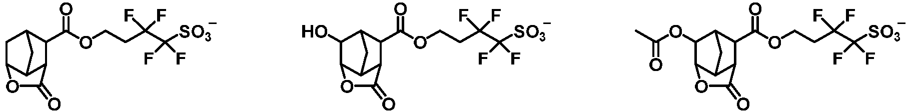

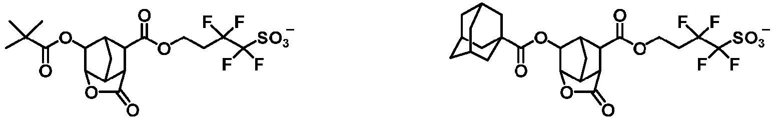

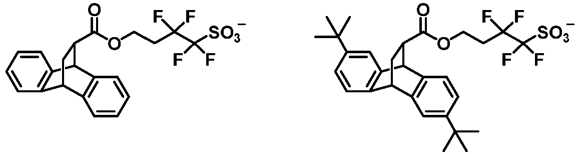

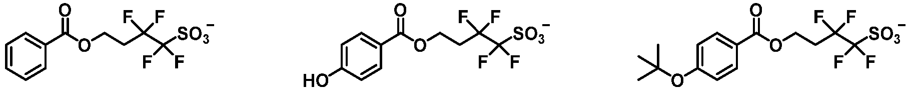

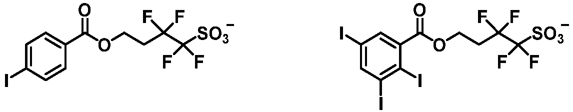

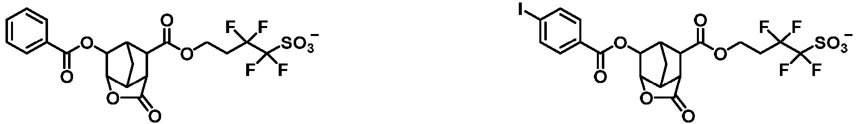

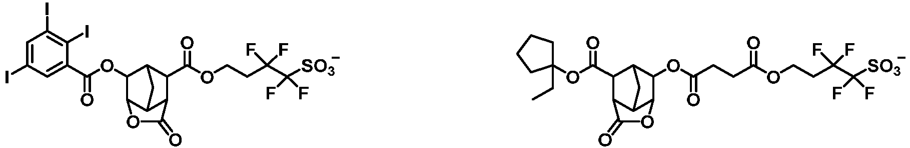

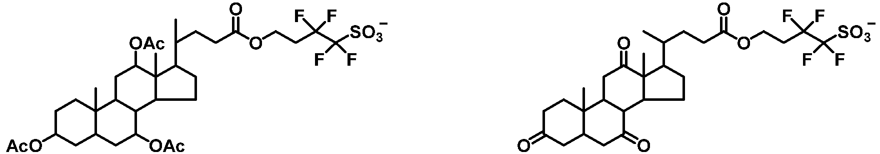

In formula (B6-2), R^{fb1} and R^{fb2} are each independently fluorine or a C₁-C₄₀ hydrocarbyl group which may contain a heteroatom. The hydrocarbyl group may be saturated or unsaturated and straight, branched or cyclic. Examples thereof are as exemplified above for R^{fa1} in formula (B6-1-1). Preferably R^{fb1} and R^{fb2} are fluorine or C₁-C₄ straight fluorinated alkyl groups. Also, R^{fb1} and R^{fb2} may bond together to form a ring with the linkage: -CF₂-SO₂-N⁻-SO₂-CF₂- to which they are attached. It is preferred that a combination of R^{fb1} and R^{fb2} be a fluorinated ethylene or fluorinated propylene group.

In formula (B6-3), R^{fc1}, R^{fc2} and R^{fc3} are each independently fluorine or a C₁-C₄₀ hydrocarbyl group which may contain a heteroatom. The hydrocarbyl group may be saturated or unsaturated and straight, branched or cyclic. Examples thereof are as exemplified for R^{fa1} in formula (B6-1-1). Preferably R^{fc1}, R^{fc2} and R^{fc3} are fluorine or C₁-C₄ straight fluorinated alkyl groups. Also, R^{fc1} and R^{fc2} may bond together to form a ring with the linkage: -CF₂-SO₂-C⁻-SO₂-CF₂- to which they are attached. It is preferred that a combination of R^{fc1} and R^{fc2} be a fluorinated ethylene or fluorinated propylene group.

In formula (B6-4), R^{fd} is a C₁-C₄₀ hydrocarbyl group which may contain a heteroatom. The hydrocarbyl group may be saturated or unsaturated and straight, branched or cyclic. Examples thereof are as exemplified above for R^{fa1} in formula (B6-1-1).

Examples of the anion having formula (B6-4) are shown below, but not limited thereto.

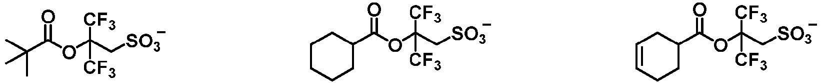

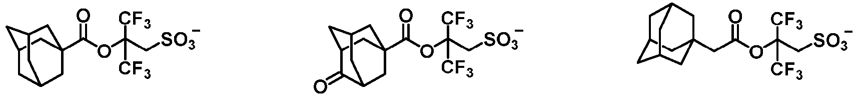

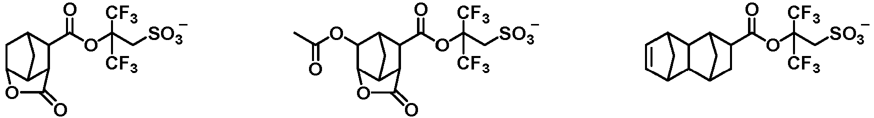

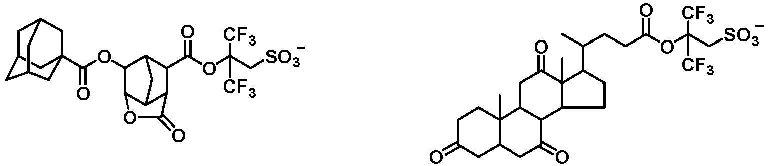

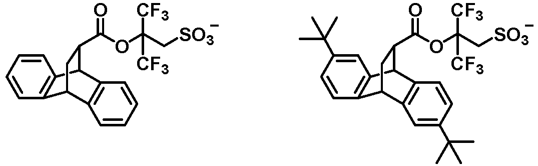

Anions having an iodized or brominated aromatic ring are also useful as the non-nucleophilic counter ion. These anions have the formula (B6-5).

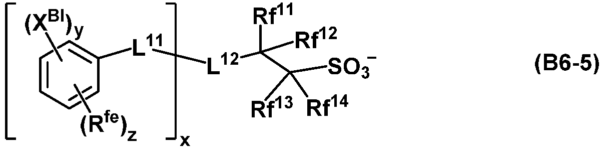

In formula (B6-5), x is 1, 2 or 3, y is 1, 2, 3, 4 or 5, z is 0, 1, 2 or 3, and y+z is from 1 to 5. Preferably, y is 1, 2 or 3, more preferably 2 or 3, and z is 0, 1 or 2.

In formula (B6-5), X^{BI} is iodine or bromine. A plurality of X^{BI} may be identical or different when x and/or y is 2 or more.

In formula (B6-5), L¹¹ is a single bond, ether bond, ester bond, or a C₁-C₆ saturated hydrocarbylene group which may contain an ether bond or ester bond. The saturated hydrocarbylene group may be straight, branched or cyclic.

In formula (B6-5), L¹² is a single bond or a C₁-C₂₀ divalent linking group when x=1, or a C₁-C₂₀ (x+1)-valent linking group when x=2 or 3. The linking group may contain an oxygen, sulfur or nitrogen atom.

In formula (B6-5), R^{fe} is hydroxy, carboxy, fluorine, chlorine, bromine, amino group, or a C₁-C₂₀ hydrocarbyl, C₁-C₂₀ hydrocarbyloxy, C₂-C₂₀ hydrocarbylcarbonyl, C₂-C₂₀ hydrocarbyloxycarbonyl, C₂-C₂₀ hydrocarbylcarbonyloxy, or C₁-C₂₀ hydrocarbylsulfonyloxy group, which may contain fluorine, chlorine, bromine, hydroxy, amino or ether bond, or -N(R^{feA})(R^{feB}), -N(R^{feC})-C(=O)-R^{feD} or -N(R^{feC})-C(=O)-O-R^{feD}. R^{feA} and R^{feB} are each independently hydrogen or a C₁-C₆ saturated hydrocarbyl group. R^{feC} is hydrogen, or a C₁-C₆ saturated hydrocarbyl group which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. R^{feD} is a C₁-C₁₆ aliphatic hydrocarbyl group, C₆-C₁₂ aryl group or C₇-C₁₅ aralkyl group, which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. The aliphatic hydrocarbyl group may be saturated or unsaturated and straight, branched or cyclic. The hydrocarbyl, hydrocarbyloxy, hydrocarbylcarbonyl, hydrocarbyloxycarbonyl, hydrocarbylcarbonyloxy, and hydrocarbylsulfonyloxy groups may be straight, branched or cyclic. A plurality of R^{fe} may be identical or different when x and/or z is 2 or more.

Of these, R^{fe} is preferably hydroxy, -N(R^{feC})-C(=O)-R^{feD}, -N(R^{feC})-C(=O)-O-R^{feD}, fluorine, chlorine, bromine, methyl or methoxy.

In formula (B6-5), Rf¹¹ to Rf¹⁴ are each independently hydrogen, fluorine or trifluoromethyl, at least one of Rf¹¹ to Rf¹⁴ is fluorine or trifluoromethyl. Rf¹¹ and Rf¹², taken together, may form a carbonyl group. More preferably, both Rf¹³ and Rf¹⁴ are fluorine.

Examples of the anion having formula (B6-5) are shown below, but not limited thereto. X^{BI} is as defined above.

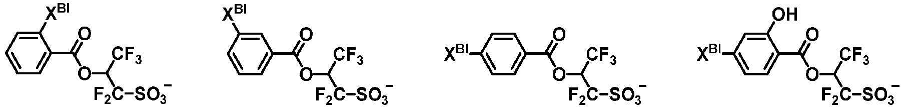

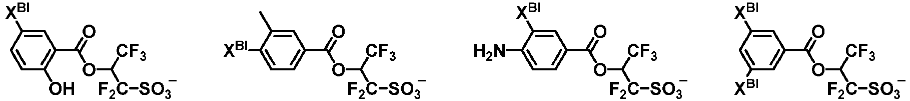

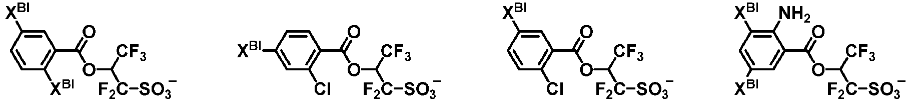

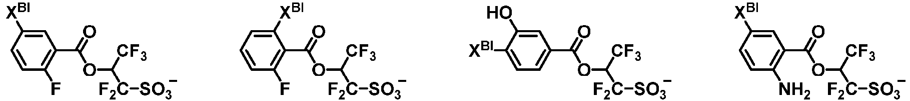

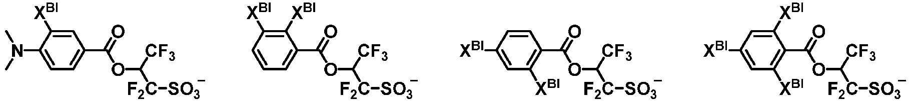

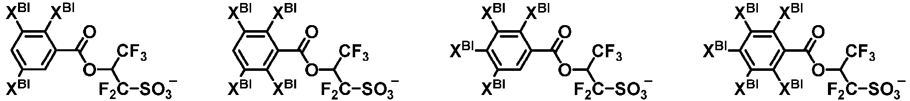

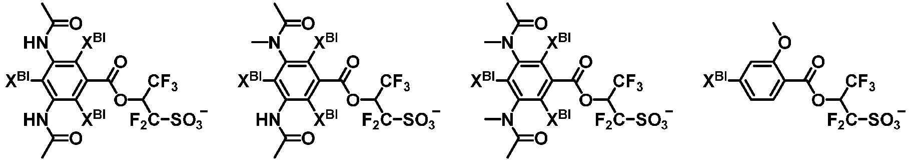

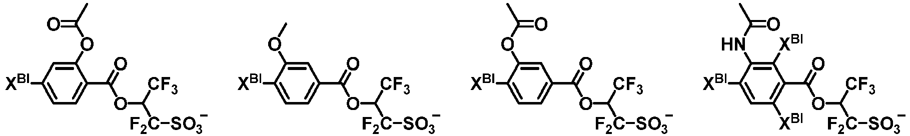

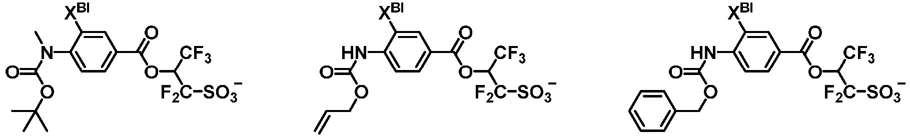

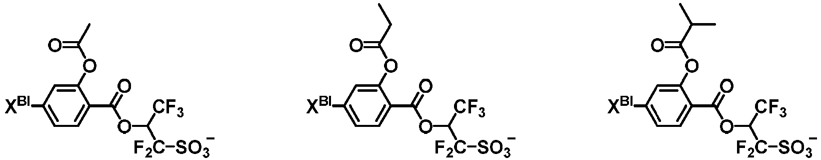

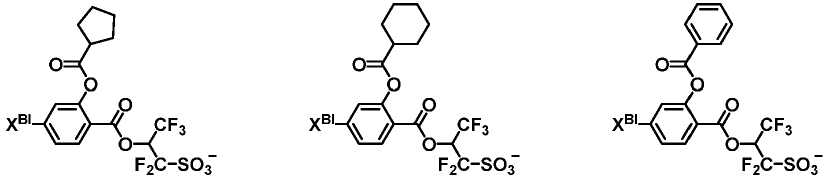

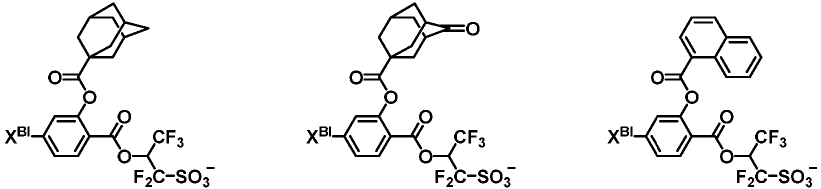

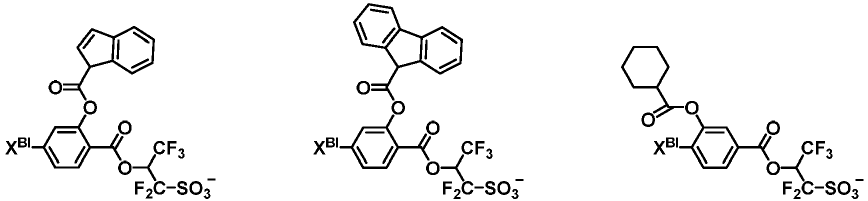

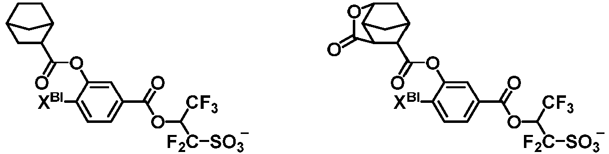

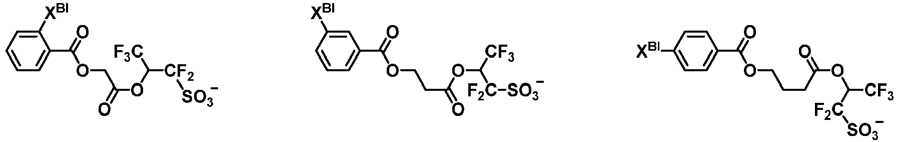

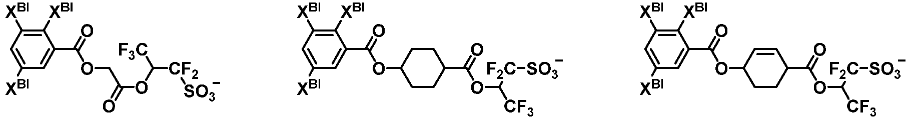

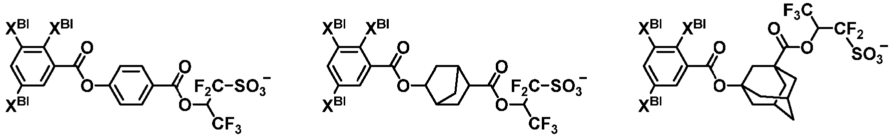

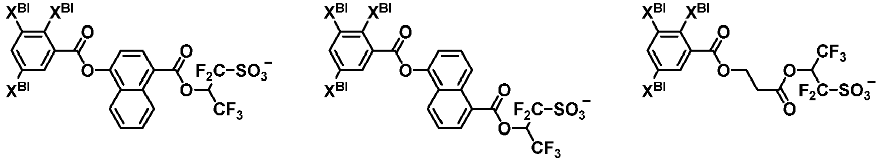

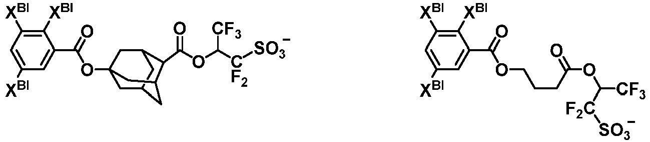

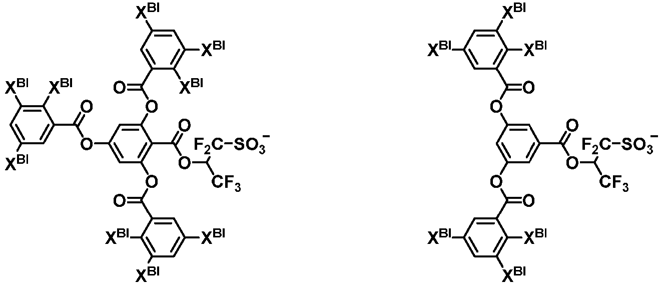

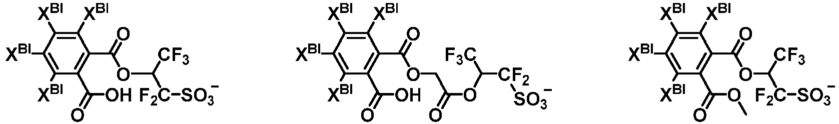

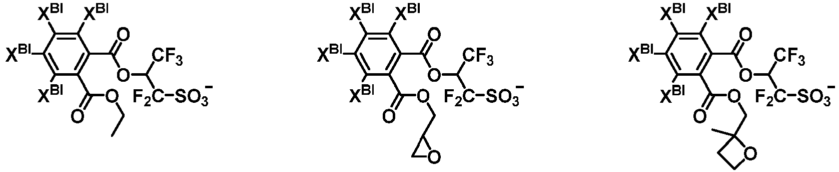

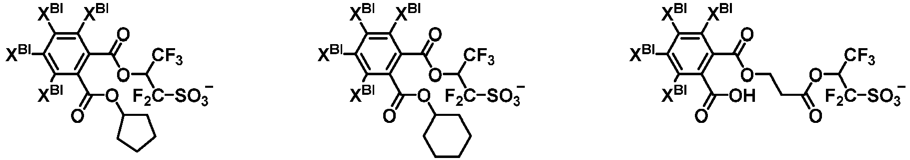

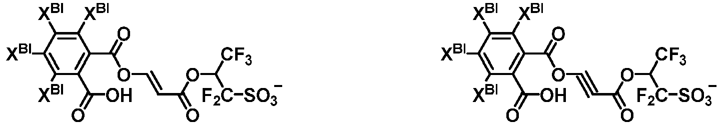

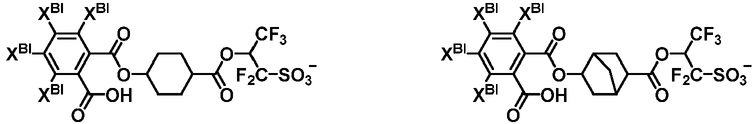

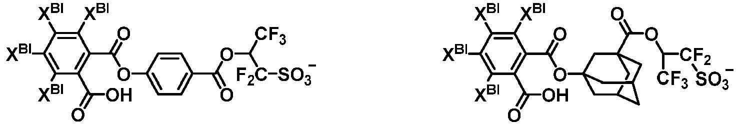

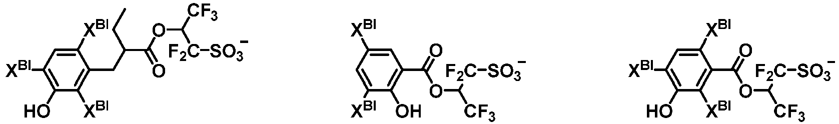

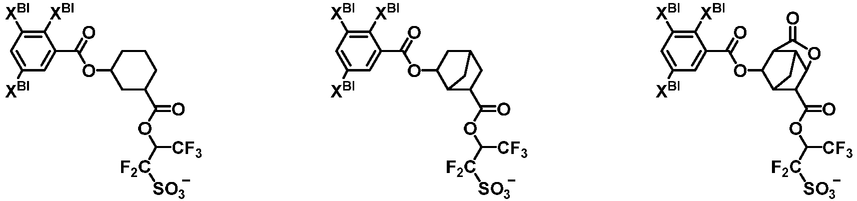

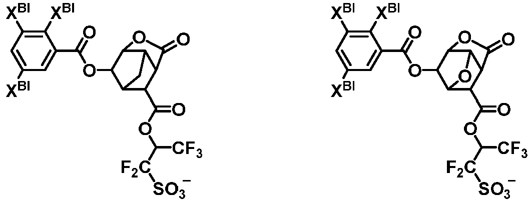

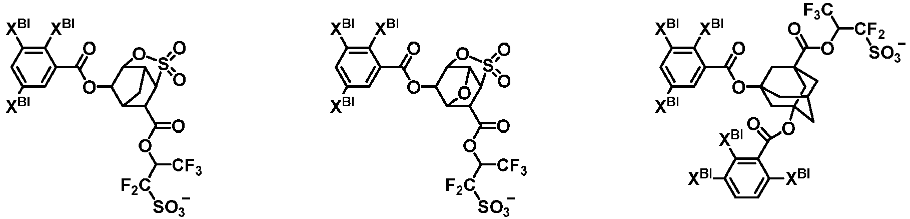

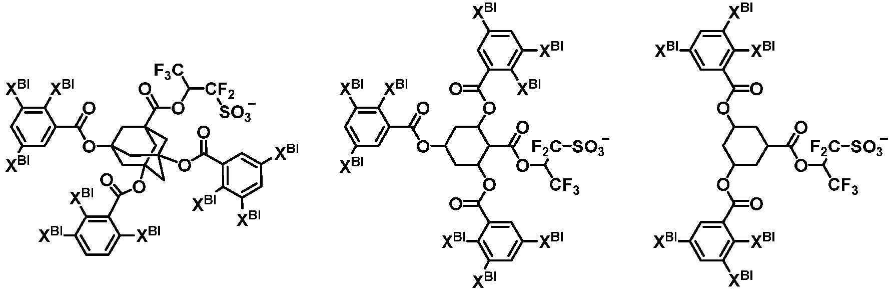

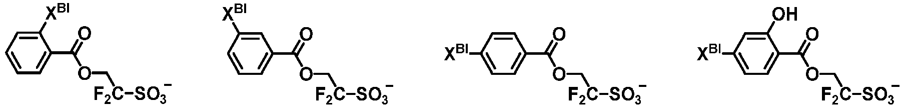

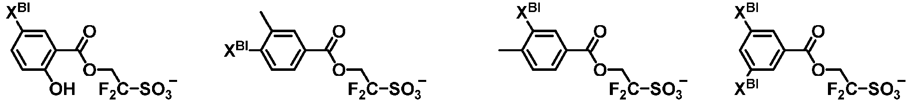

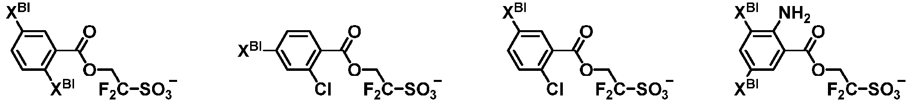

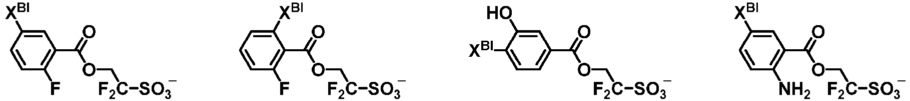

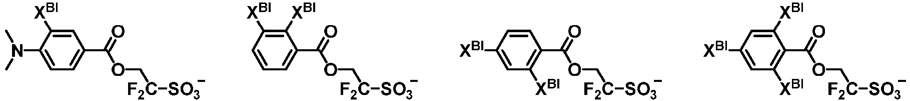

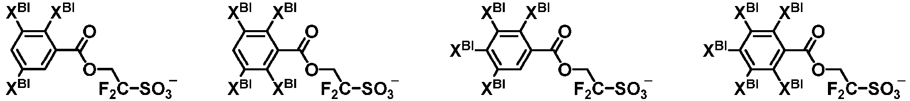

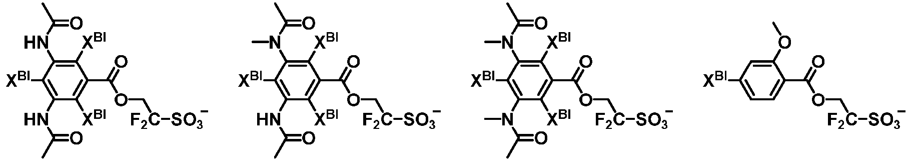

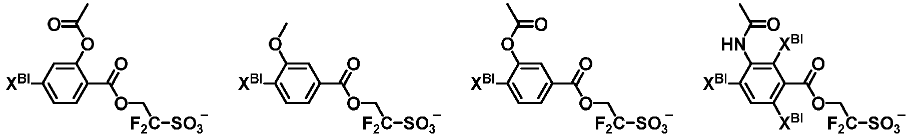

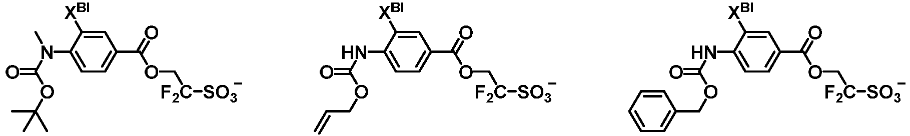

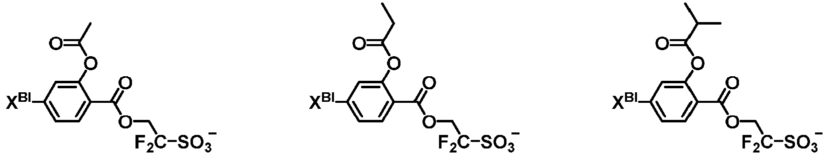

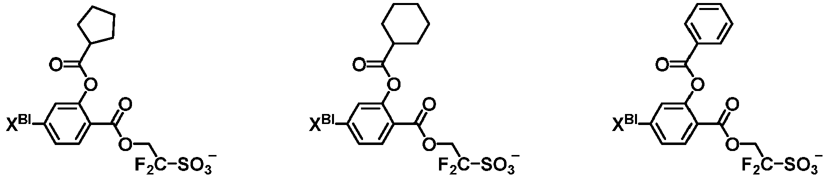

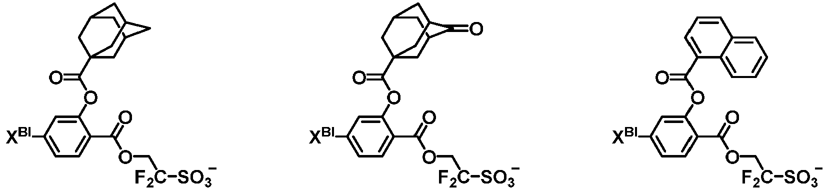

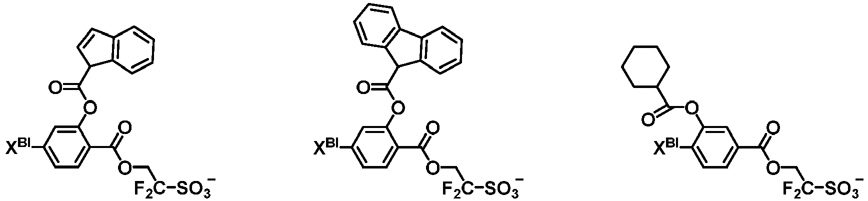

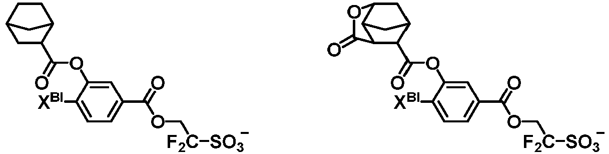

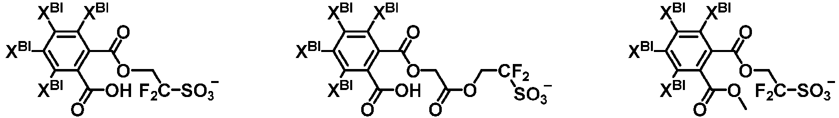

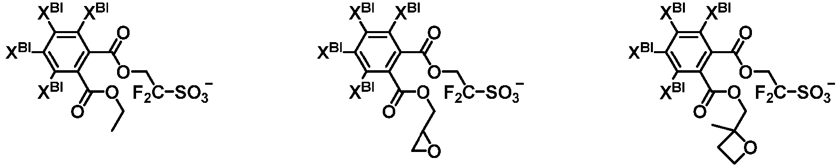

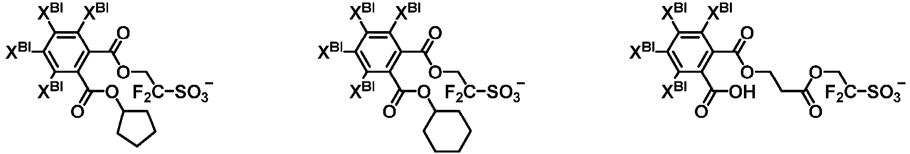

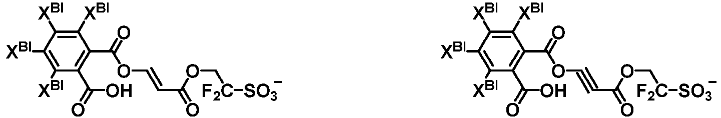

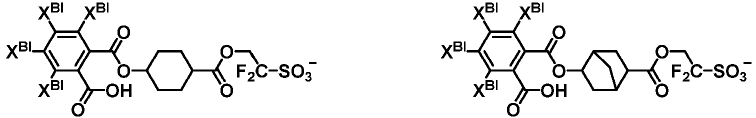

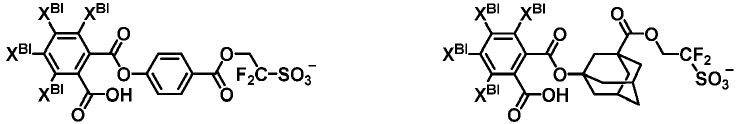

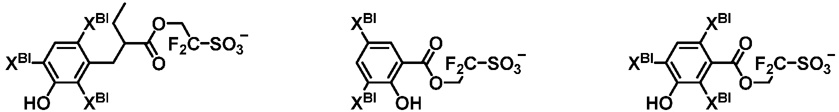

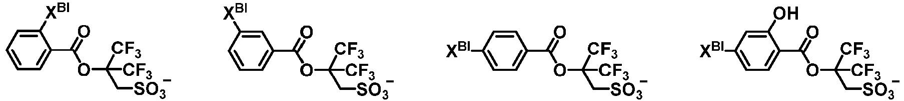

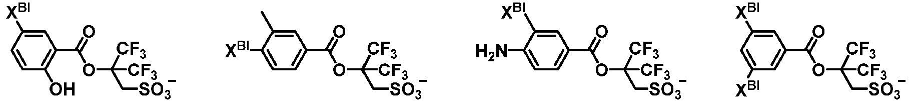

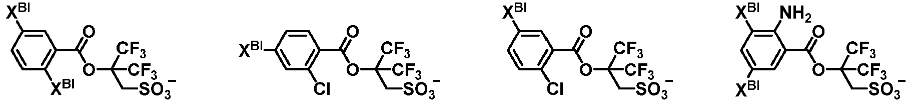

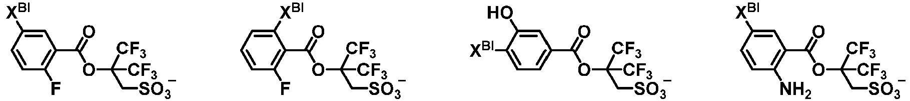

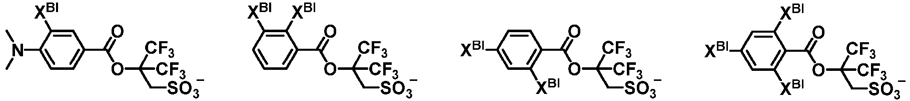

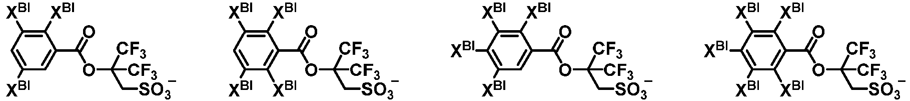

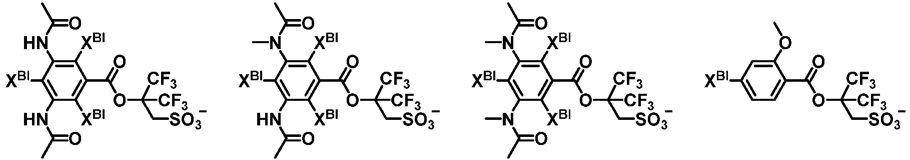

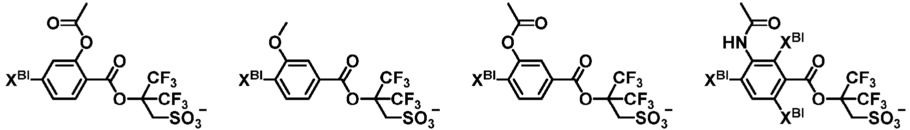

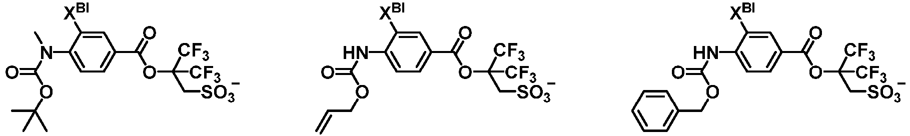

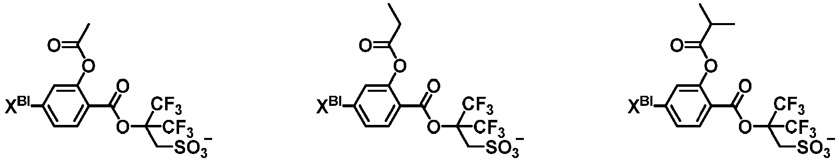

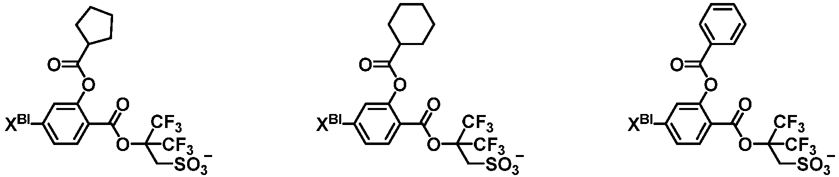

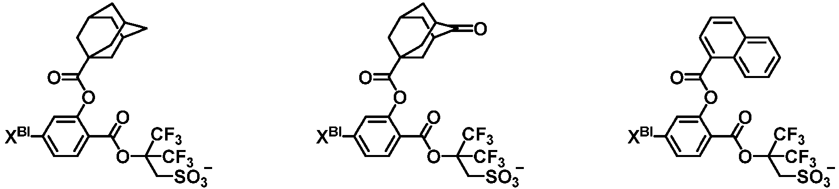

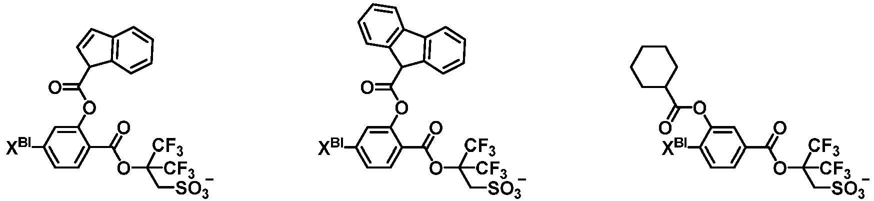

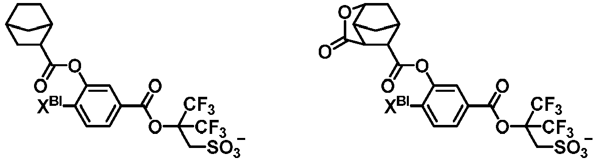

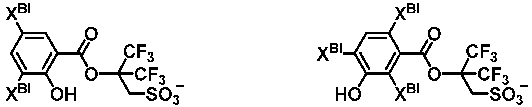

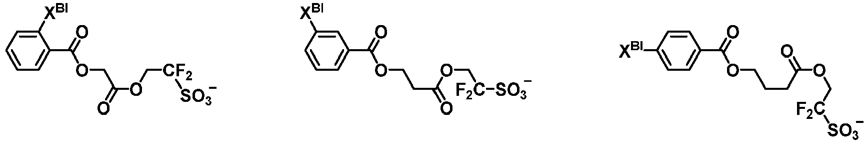

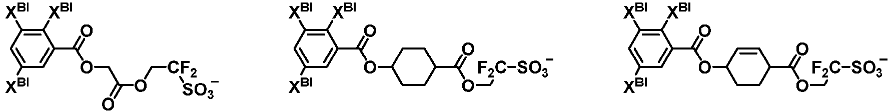

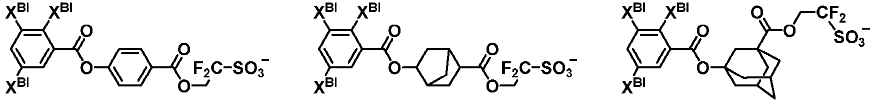

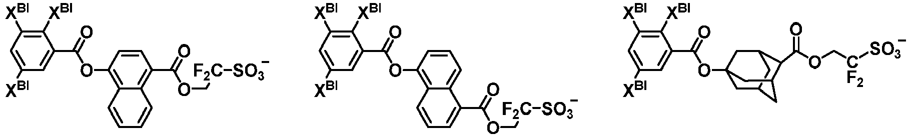

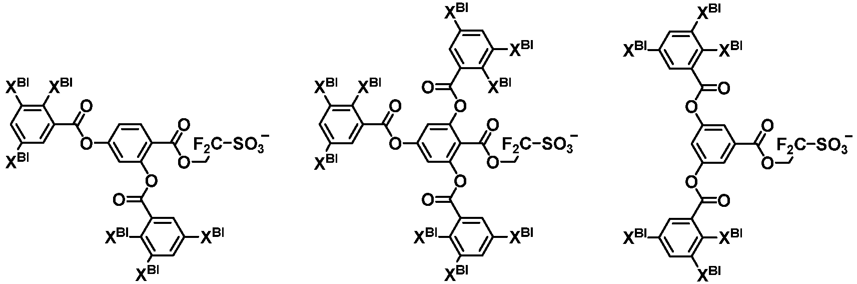

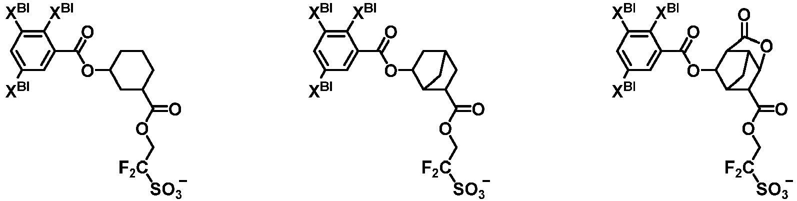

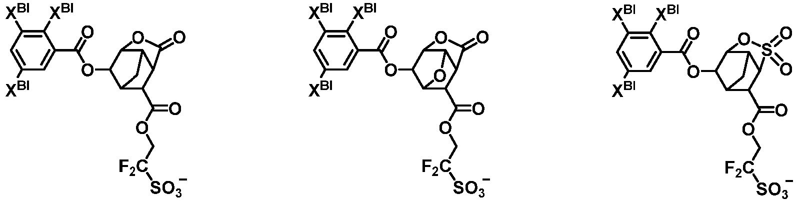

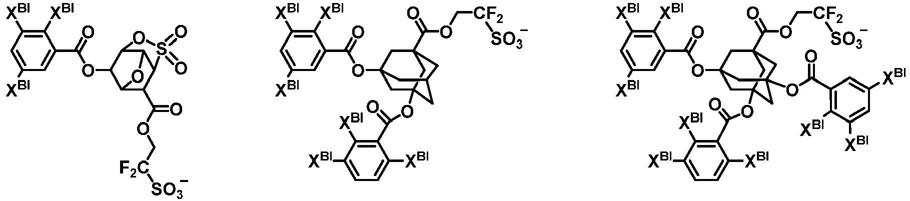

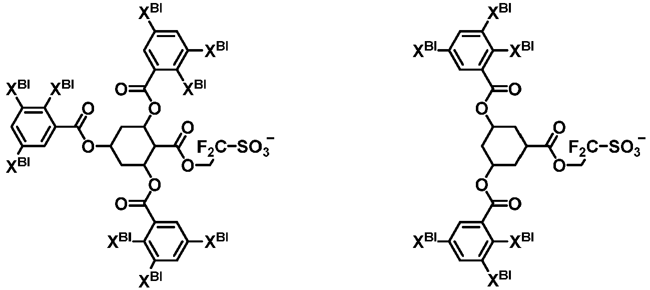

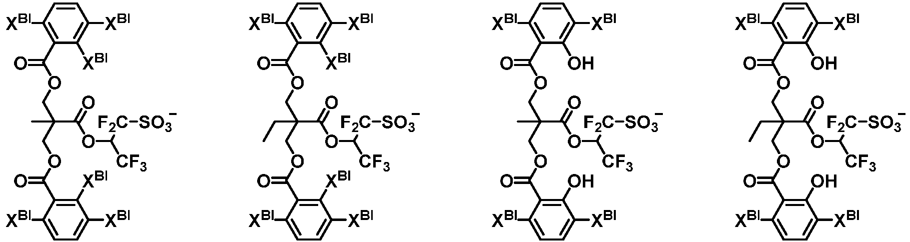

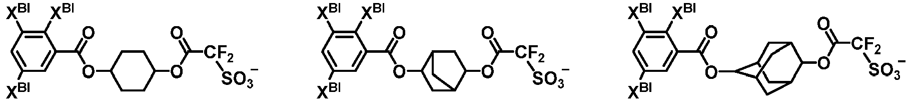

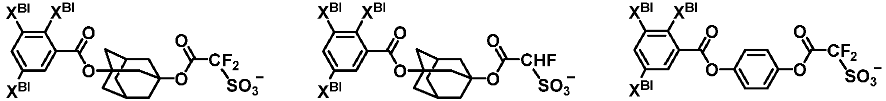

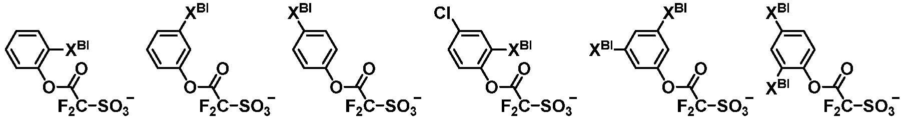

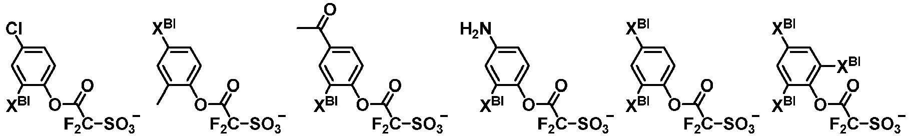

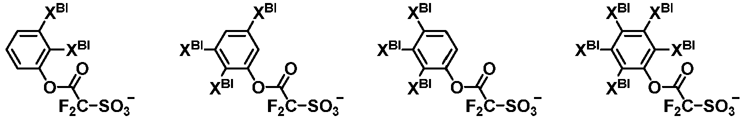

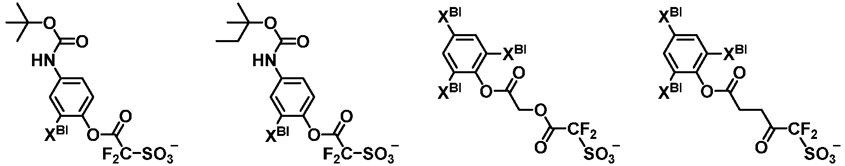

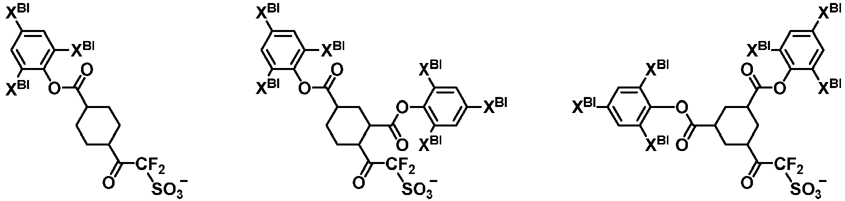

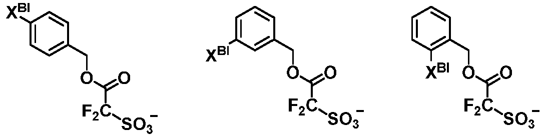

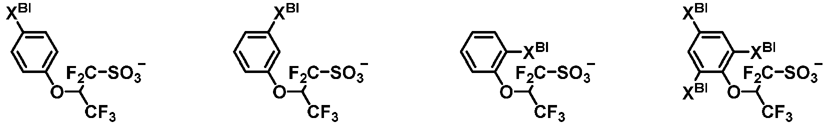

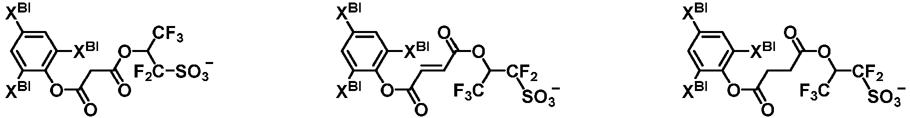

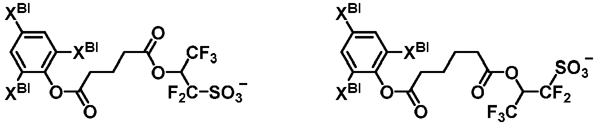

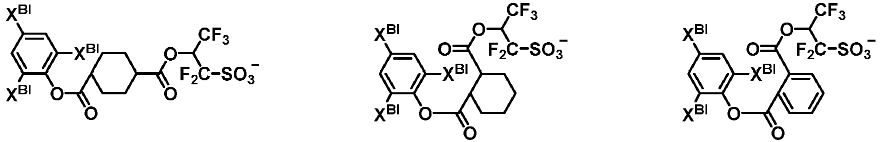

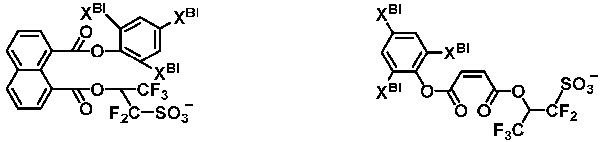

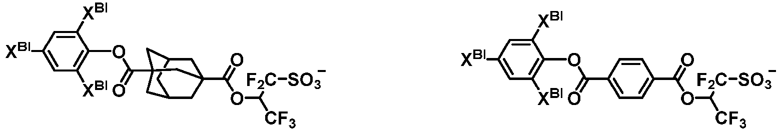

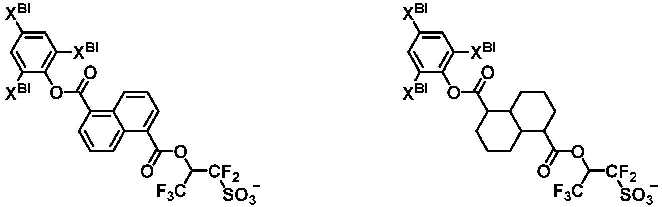

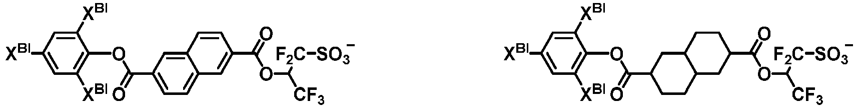

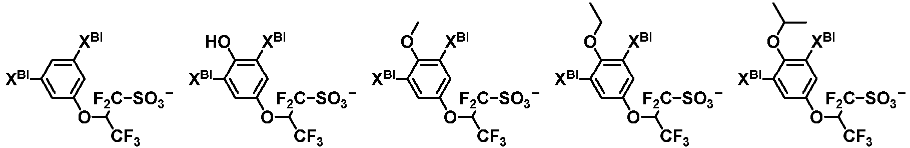

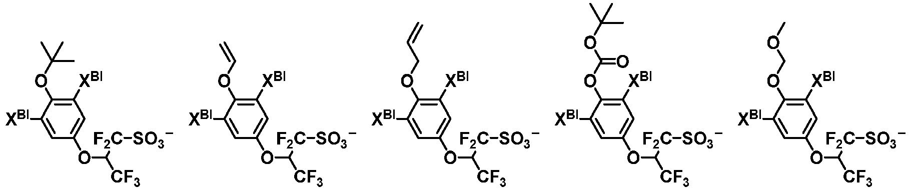

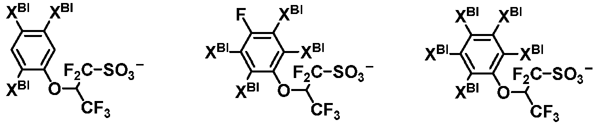

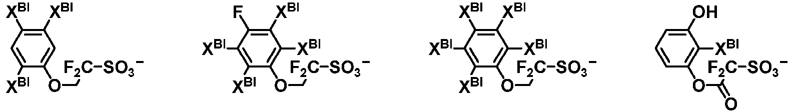

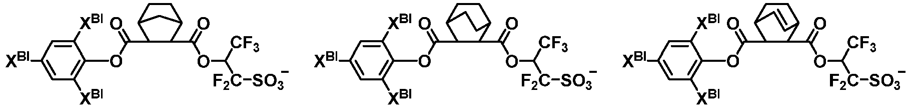

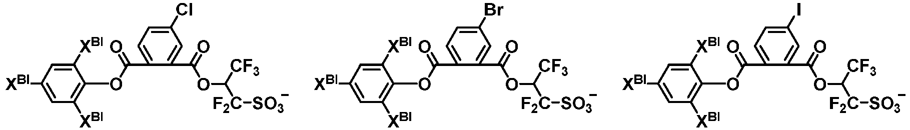

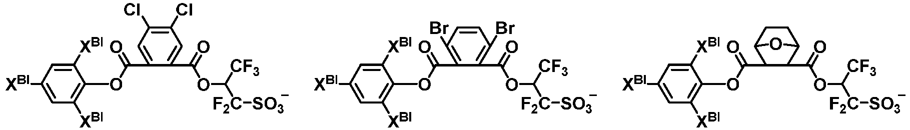

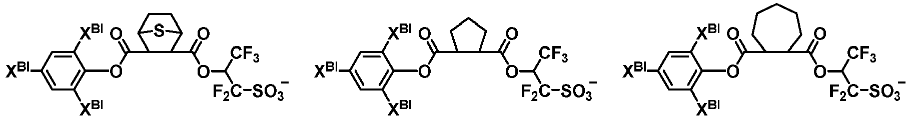

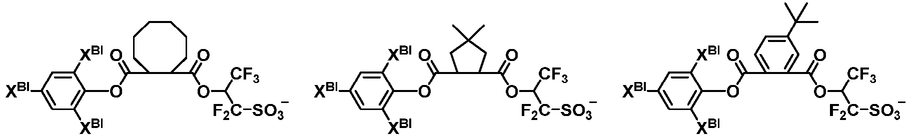

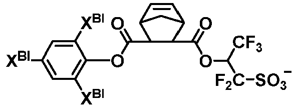

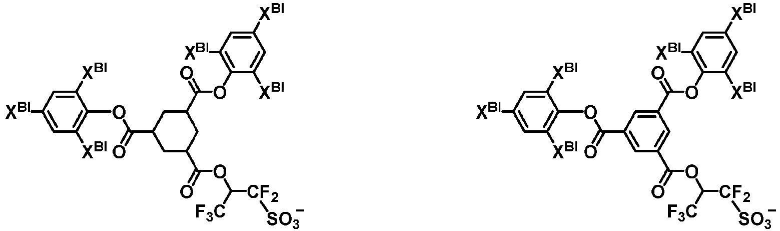

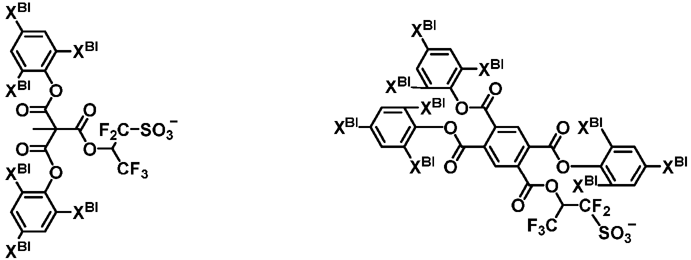

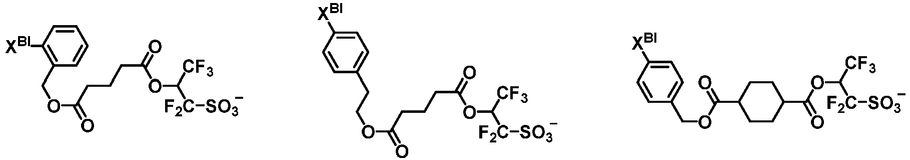

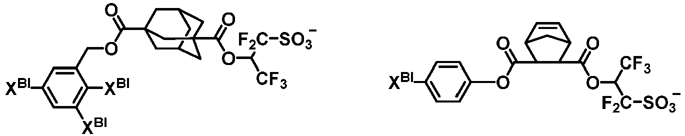

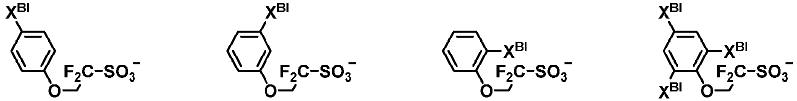

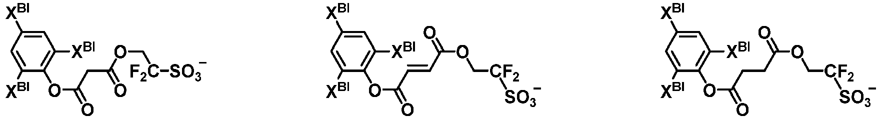

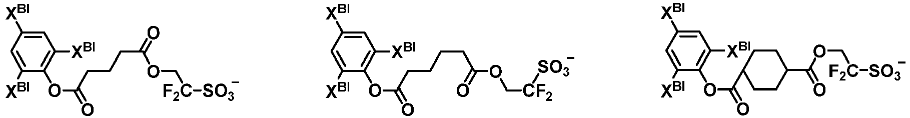

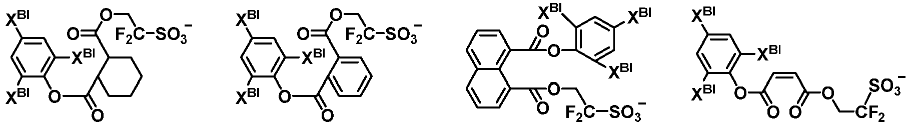

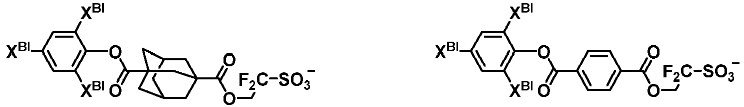

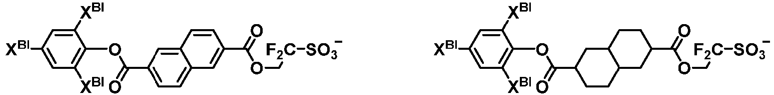

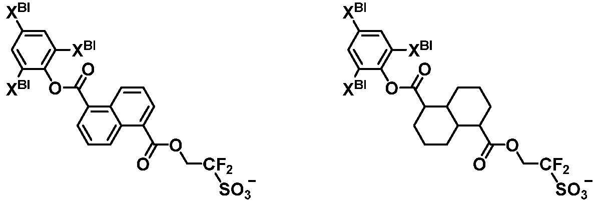

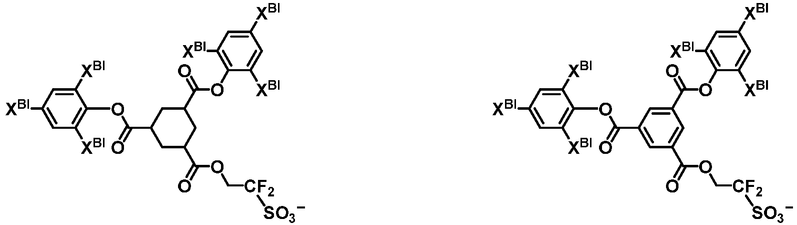

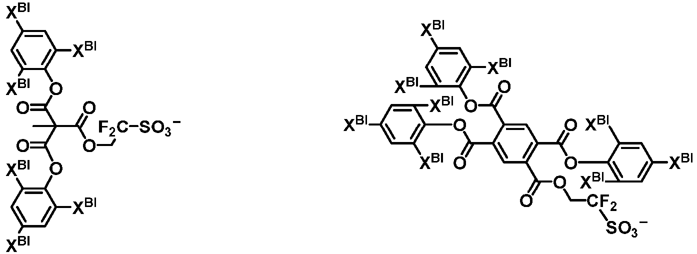

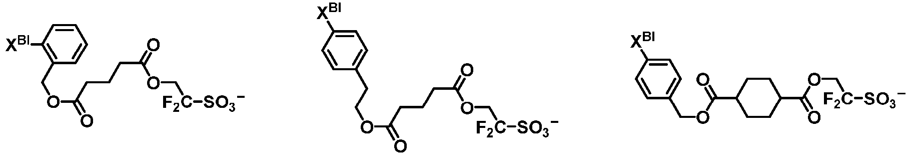

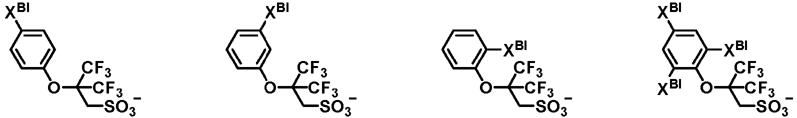

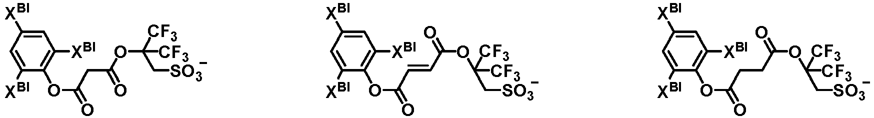

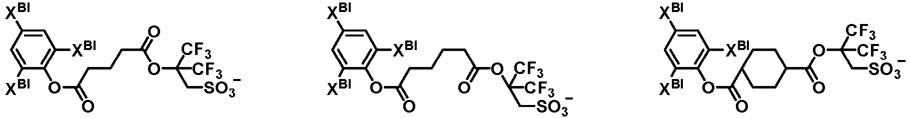

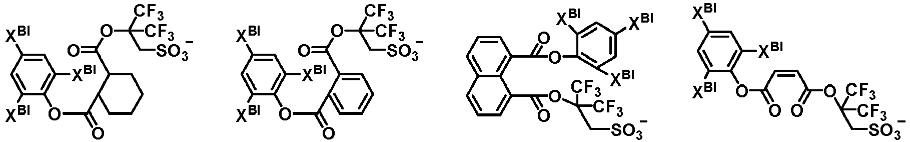

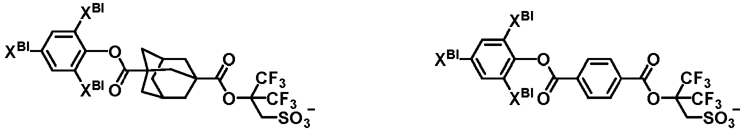

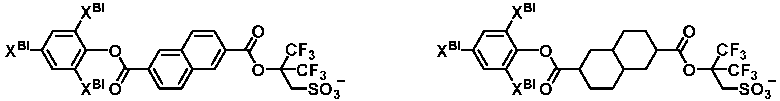

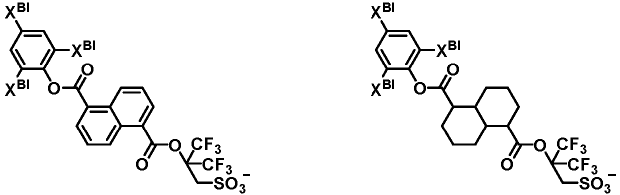

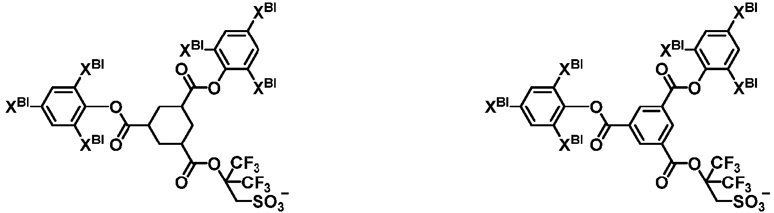

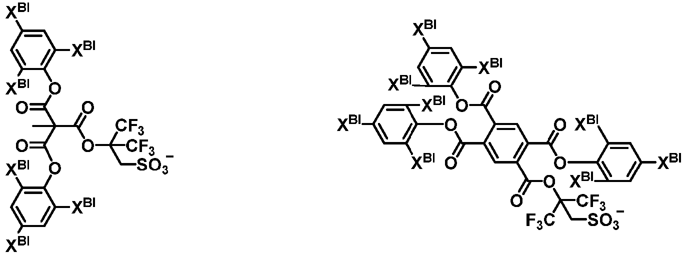

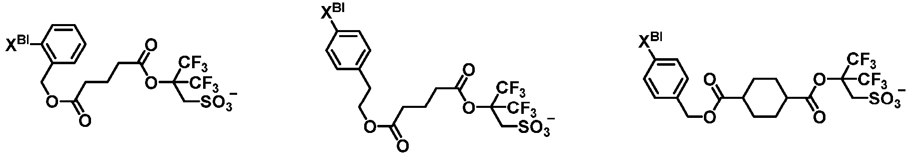

Other useful examples of the non-nucleophilic counter ion include fluorobenzenesulfonic acid anions having an iodized aromatic ring bonded thereto as described in JP 6648726, anions having an acid-catalyzed decomposition mechanism as described in WO 2021/200056 and JP-A 2021-070692, anions having a cyclic ether group as described in JP-A 2018-180525 and JP-A 2021-035935, and anions as described in JP-A 2018-092159.

Further useful examples of the non-nucleophilic counter ion include fluorine-free bulky benzenesulfonic acid anions as described in JP-A 2006-276759, JP-A 2015-117200, JP-A 2016-065016, and JP-A 2019-202974; fluorine-free benzenesulfonic acid or alkylsulfonic acid anions having an iodized aromatic group bonded thereto as described in JP 6645464.

Also useful are bissulfonic acid anions as described in JP-A 2015-206932, sulfonamide or sulfonimide anions having sulfonic acid side and different side as described in WO 2020/158366, and anions having a sulfonic acid side and a carboxylic acid side as described in JP-A 2015-024989.

In formulae (B7) and (B8), g1 and g2 are each independently 0, 1, 2 or 3, preferably 1.

In formula (B9), h1 is 0 or 1, h2 is 0, 1, 2, 3 or 4, h3 is 0, 1, 2, 3 or 4, with the proviso that when h1=0, 0 ≤ h2+h3 ≤ 4, and when h1=1, 0 ≤ h2+h3 ≤ 6.

In formulae (B7), (B8) and (B9), L¹ is a single bond, ether bond, ester bond, carbonyl, sulfonate ester bond, carbonate bond or carbamate bond. From the aspect of synthesis, an ether bond, ester bond or carbonyl is preferred, with the ester bond or carbonyl being more preferred.

In formula (B7), Rf¹ and Rf² are each independently fluorine or a C₁-C₆ fluorinated saturated hydrocarbyl group. It is preferred that both Rf¹ and Rf² be fluorine because the generated acid has a higher acid strength. Rf³ and Rf⁴ are each independently hydrogen, fluorine or a C₁-C₆ fluorinated saturated hydrocarbyl group. It is preferred for solvent solubility that at least one of Rf³ and Rf⁴ be trifluoromethyl.

In formula (B8), Rf⁶ and Rf⁶ are each independently hydrogen, fluorine or a C₁-C₆ fluorinated saturated hydrocarbyl group. It is excluded that all Rf⁵ and Rf⁶ are hydrogen at the same time. It is preferred for solvent solubility that at least one of Rf⁶ and Rf⁶ be trifluoromethyl.

In formula (B9), Rf⁷ is fluorine, a C₁-C₆ fluorinated alkyl group, C₁-C₆ fluorinated alkoxy group, or C₁-C₆ fluorinated alkylthio group. Rf⁷ is preferably fluorine, trifluoromethyl, difluoromethyl, trifluoromethoxy, difluoromethoxy, trifluoromethylthio or difluoromethylthio, more preferably fluorine, trifluoromethyl or trifluoromethoxy. When h2 is 2, 3 or 4, a plurality of Rf⁷ may be identical or different.

In formula (B9), R⁵³ is a C₁-C₂₀ hydrocarbyl group which may contain a heteroatom. The hydrocarbyl group may be saturated or unsaturated and straight, branched or cyclic. Examples thereof are as exemplified above for the hydrocarbyl group R¹ in formula (A1), but not limited thereto. When h3 is 2, 3 or 4, a plurality of R⁵³ may be identical or different.

When h3 is 2, 3 or 4, a plurality of R⁵³ may bond together to form a ring with the carbon atom to which they are attached. Examples of the ring thus formed include cyclopropane, cyclobutane, cyclopentane, cyclohexane, norbornane, and adamantane rings. In the ring, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the ring may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-) or haloalkyl moiety.

Examples of the anion in repeat unit B7 are shown below, but not limited thereto. Herein R^{A} is as defined above.

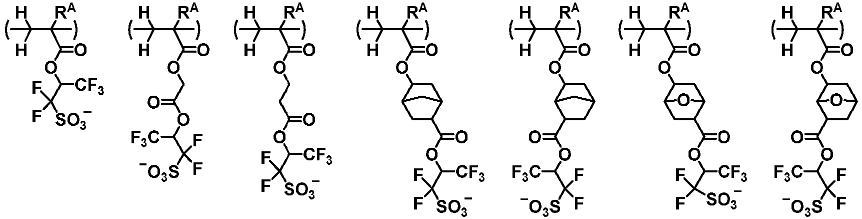

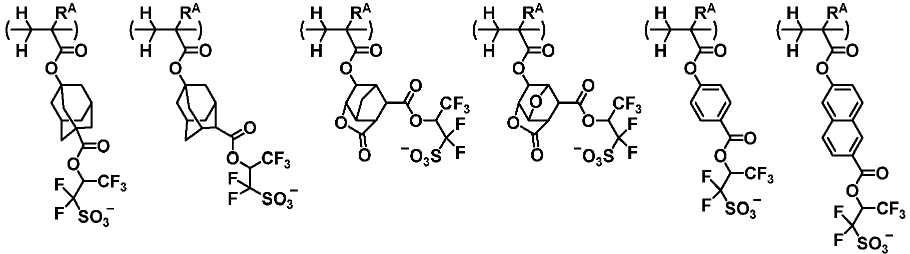

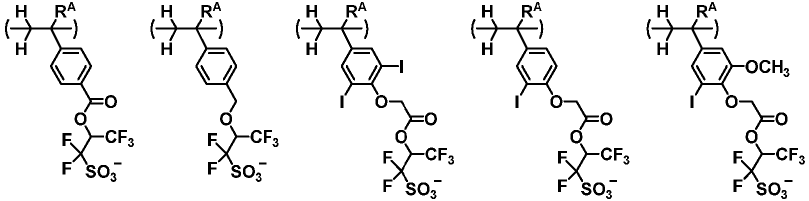

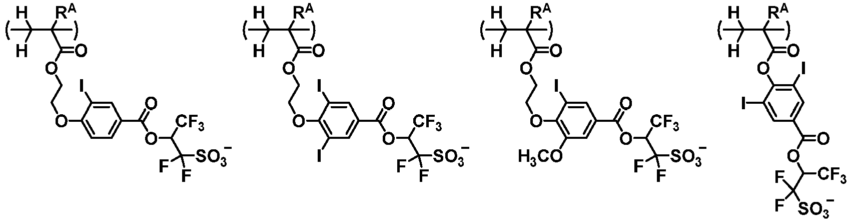

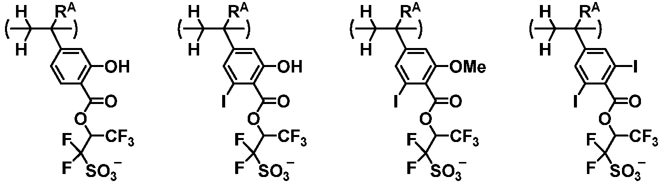

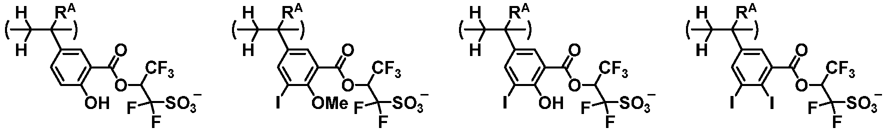

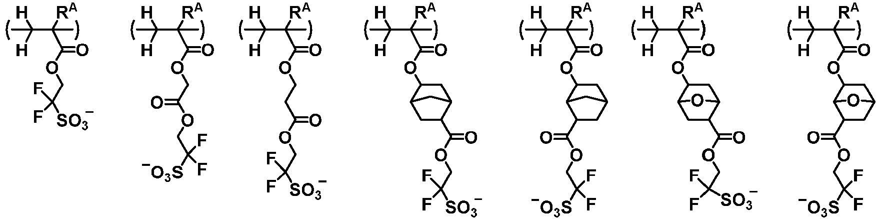

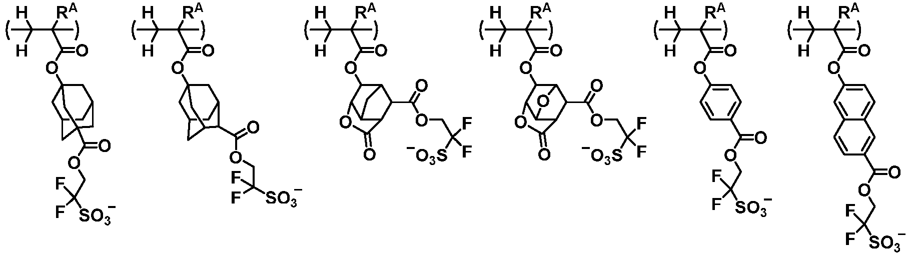

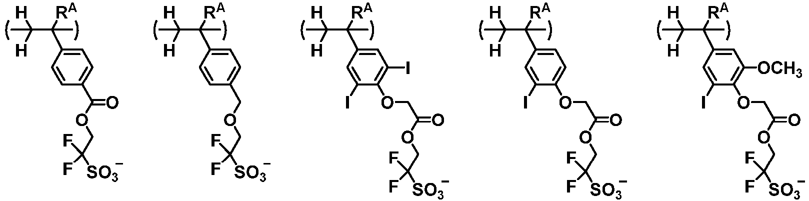

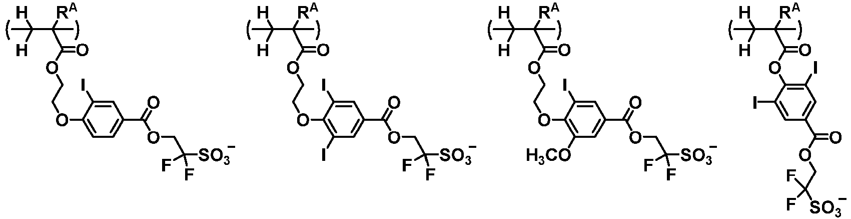

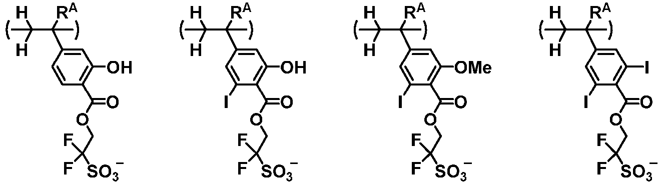

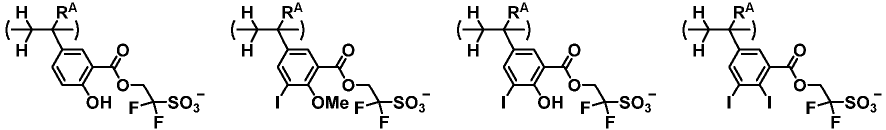

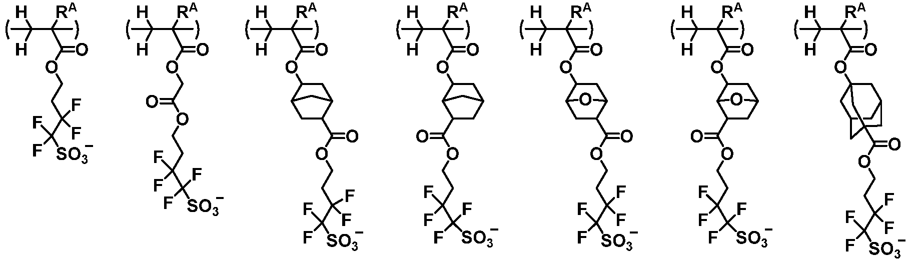

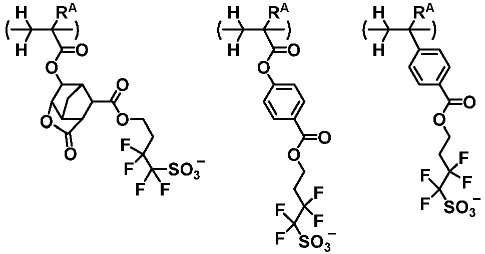

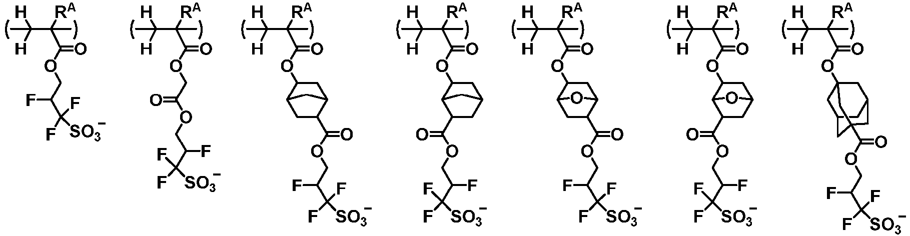

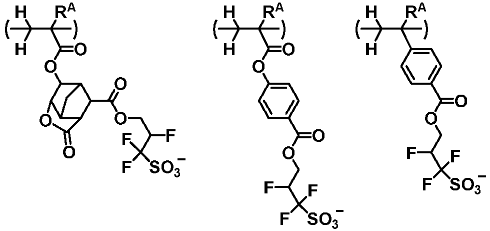

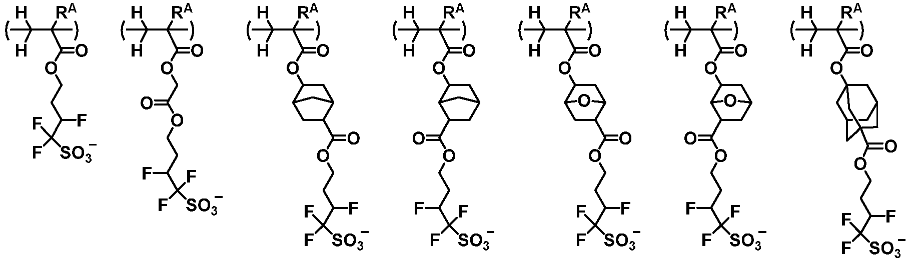

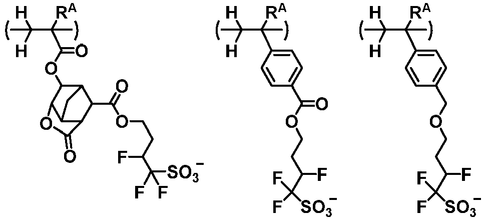

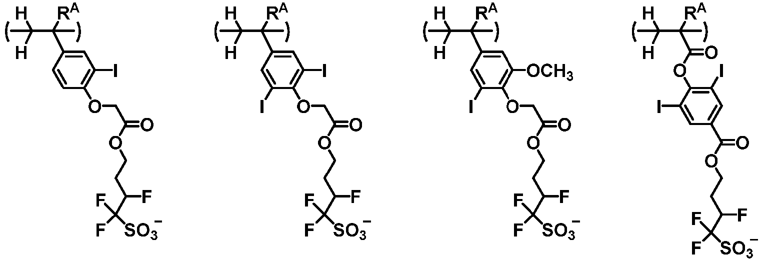

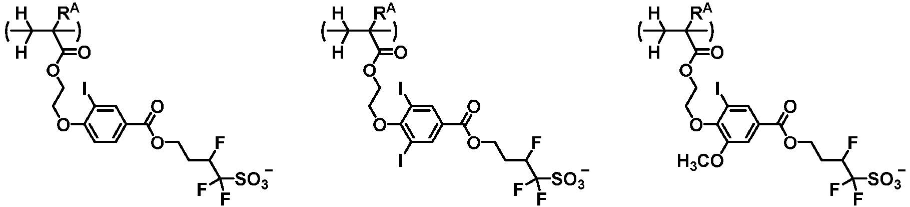

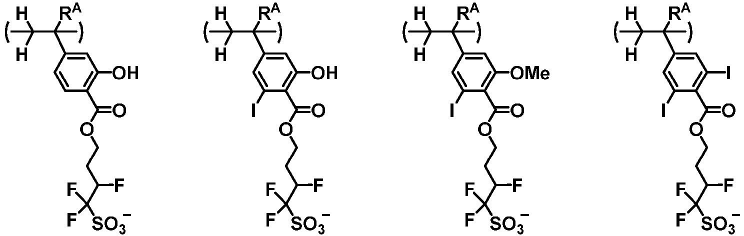

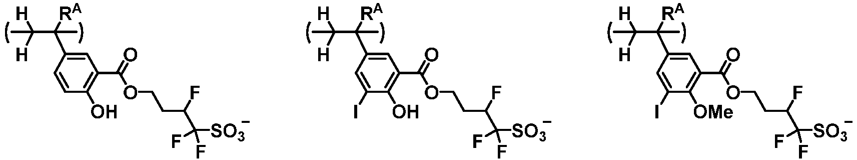

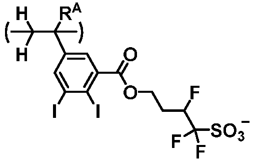

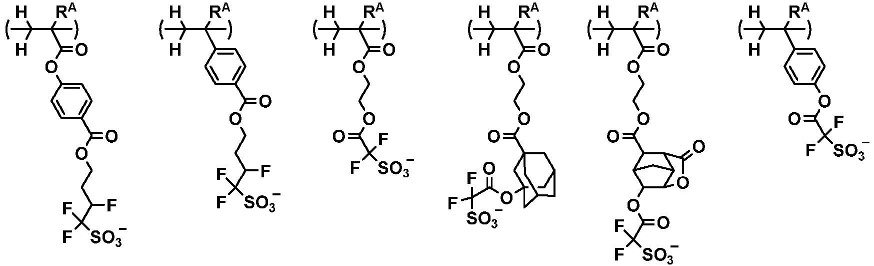

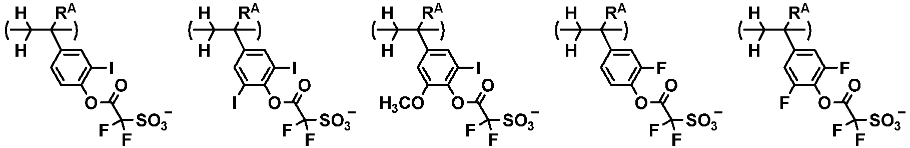

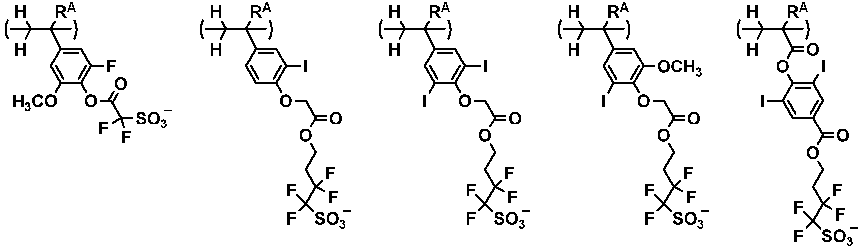

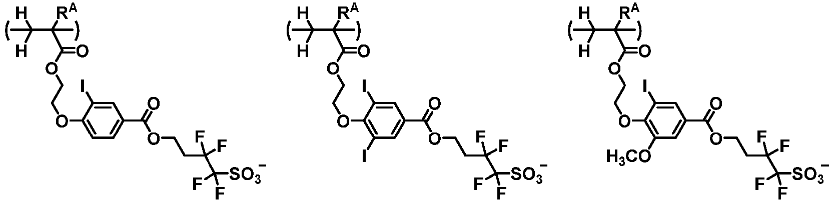

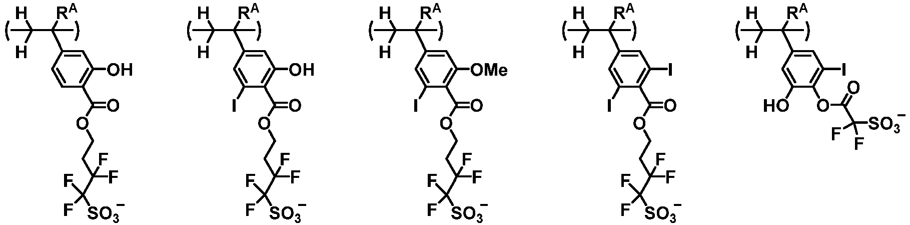

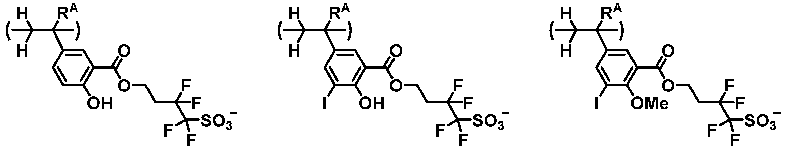

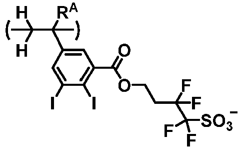

Examples of the anion in repeat unit B8 are shown below, but not limited thereto. Herein R^{A} is as defined above.

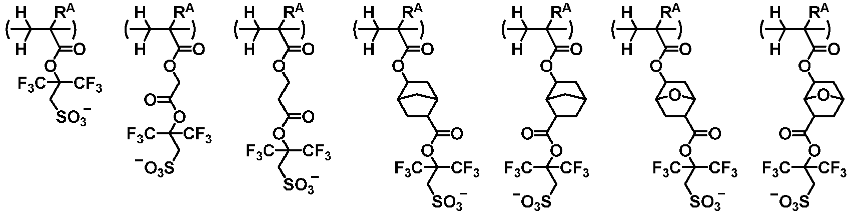

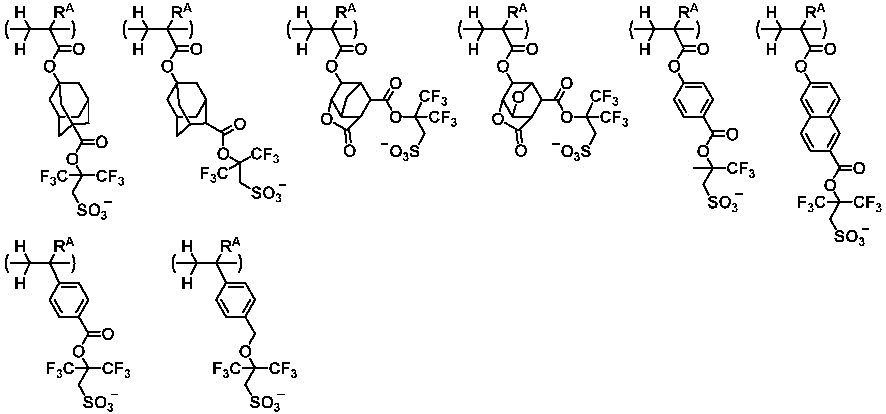

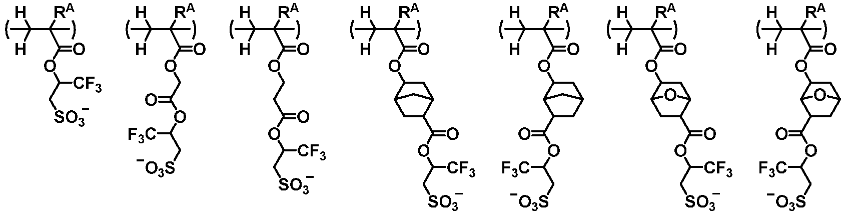

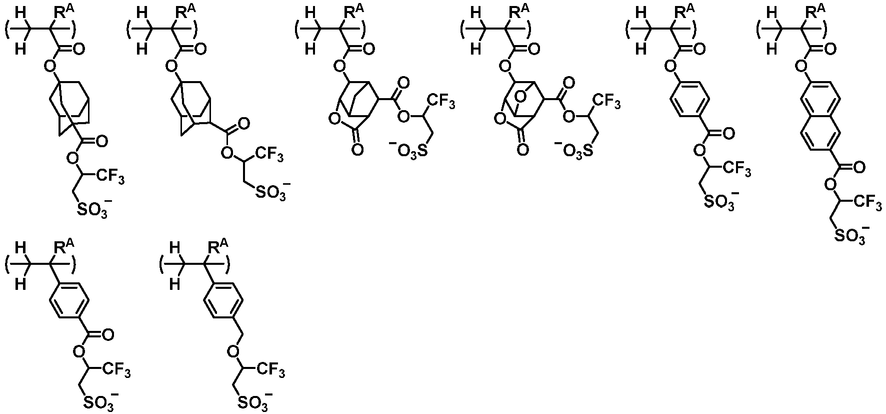

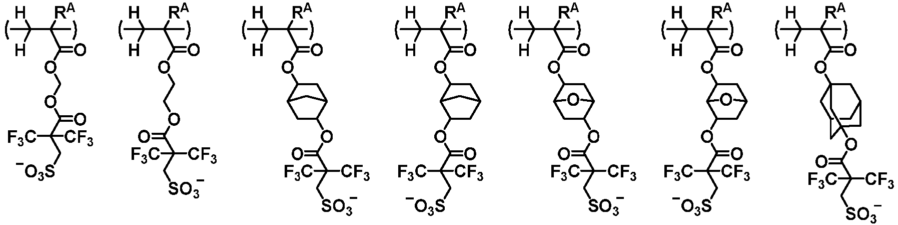

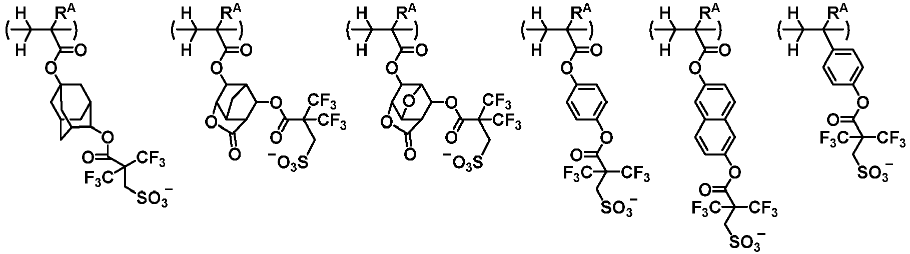

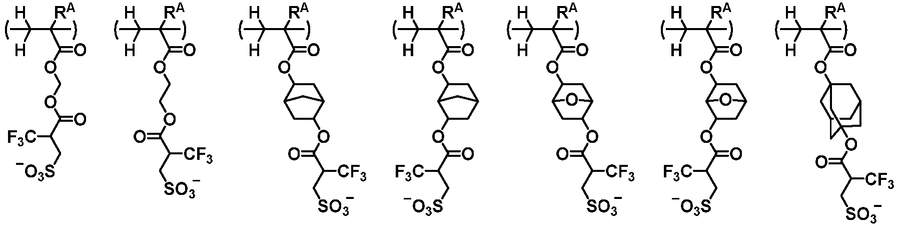

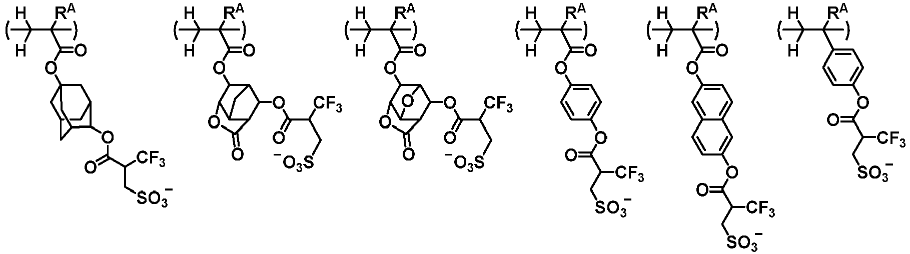

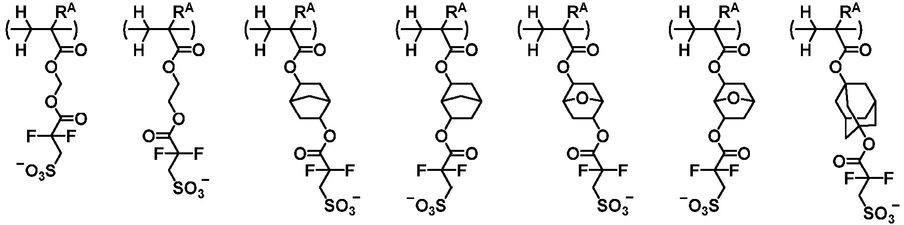

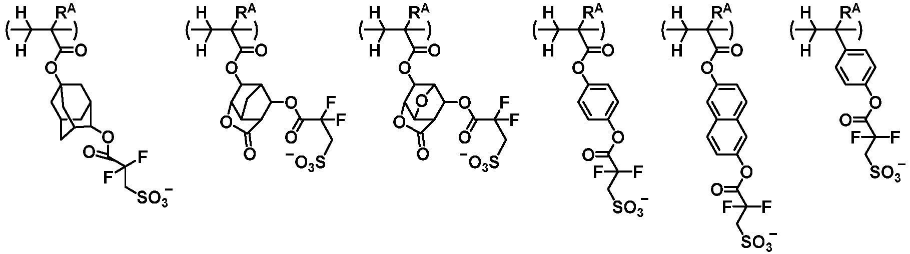

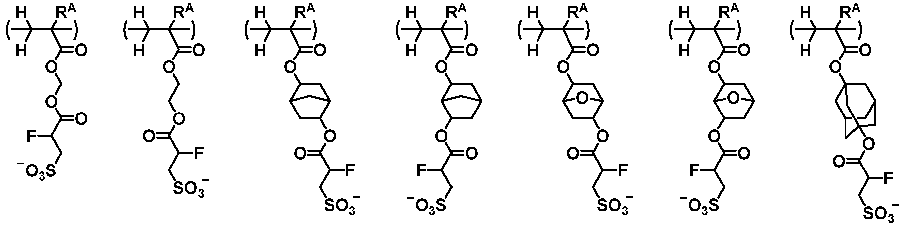

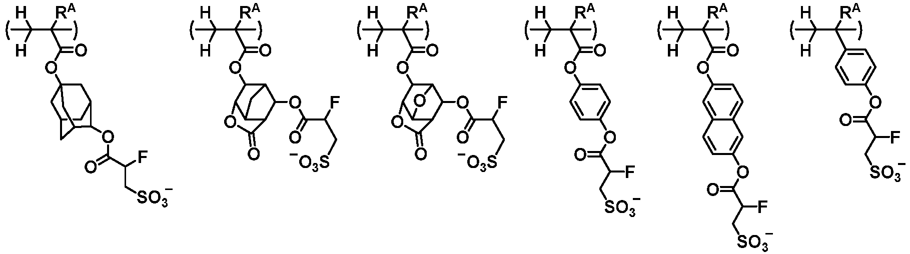

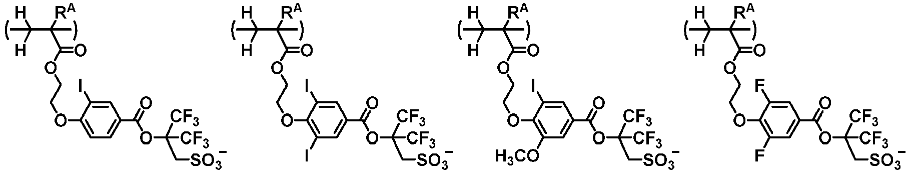

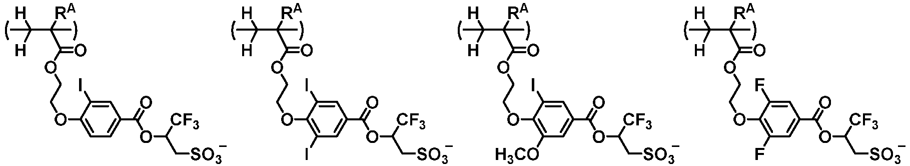

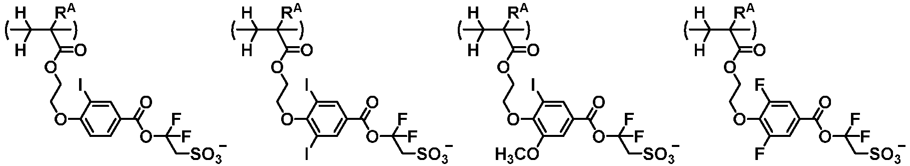

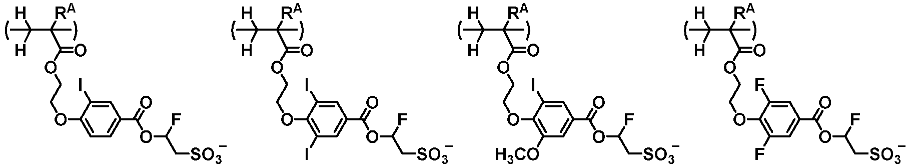

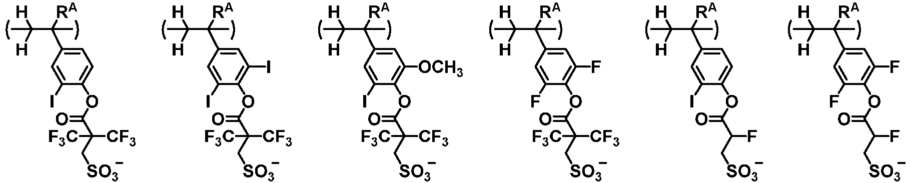

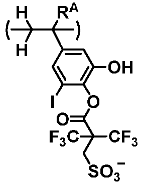

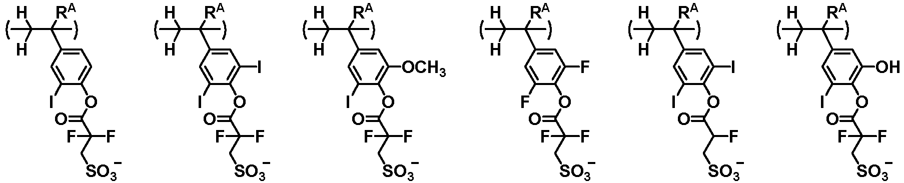

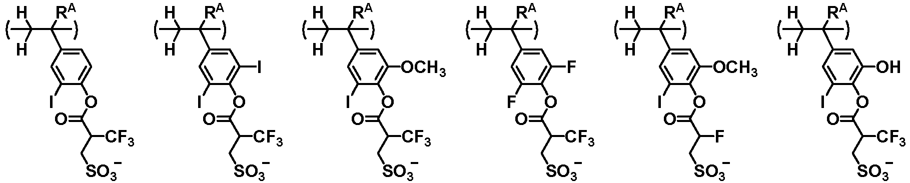

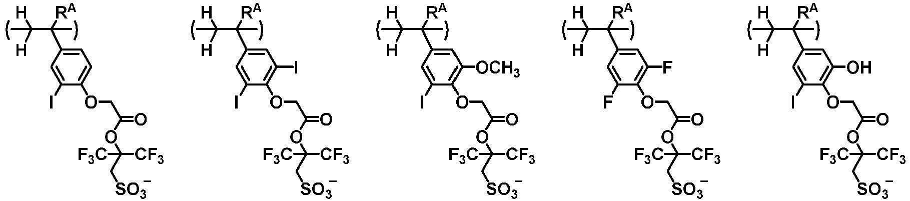

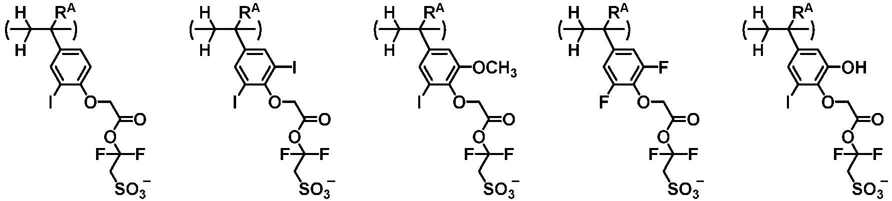

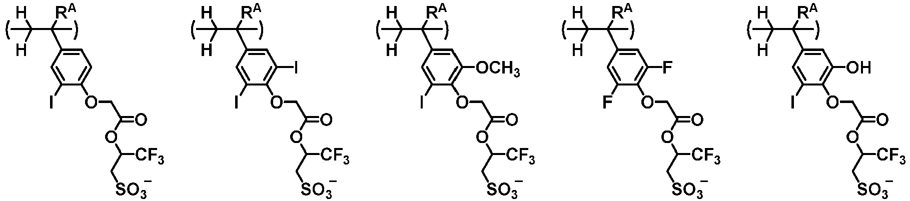

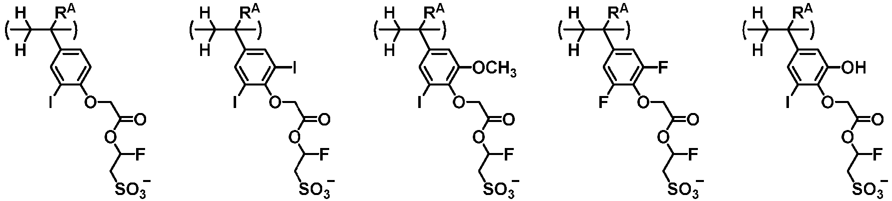

Examples of the anion in repeat unit B9 are shown below, but not limited thereto. Herein R^{A} is as defined above.

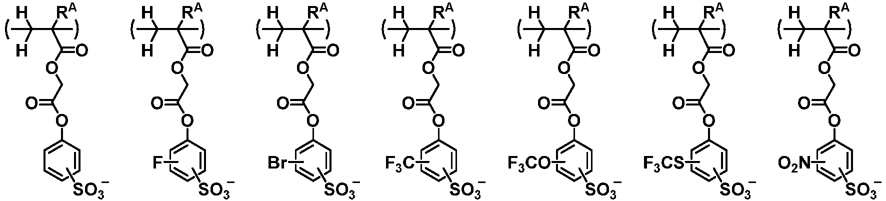

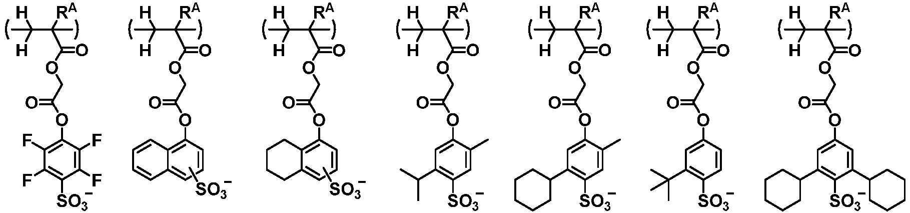

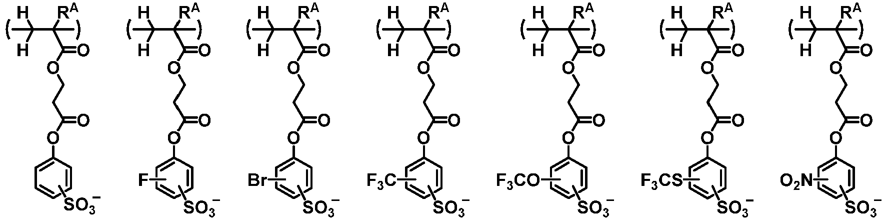

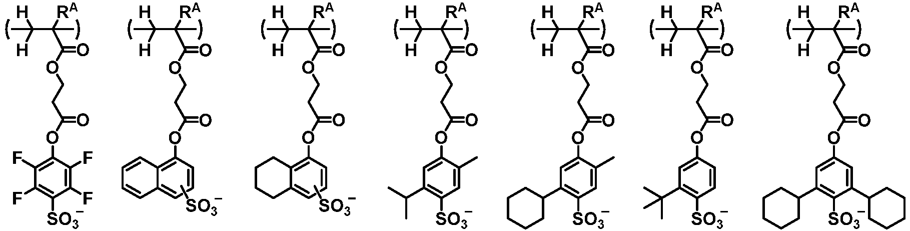

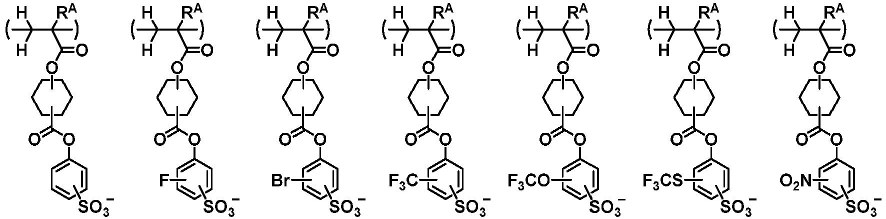

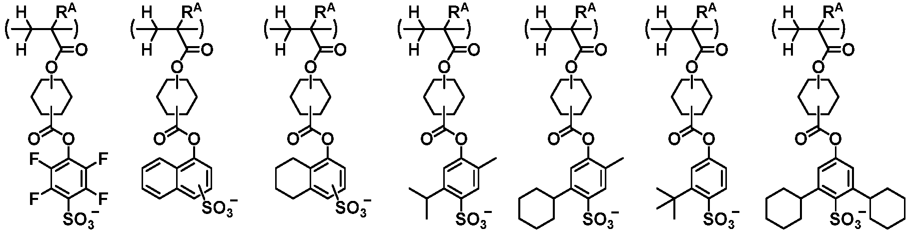

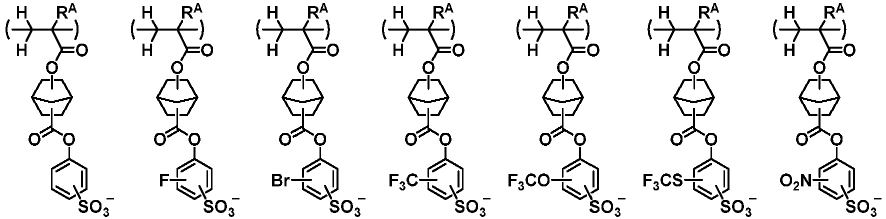

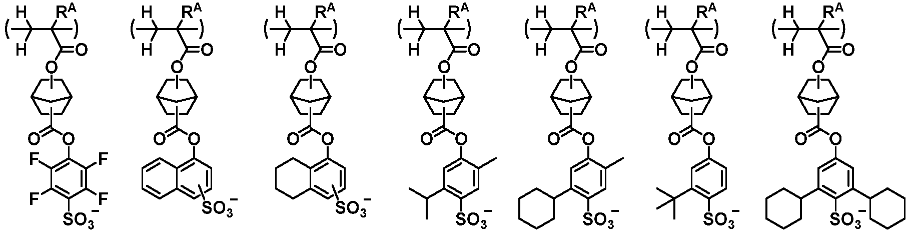

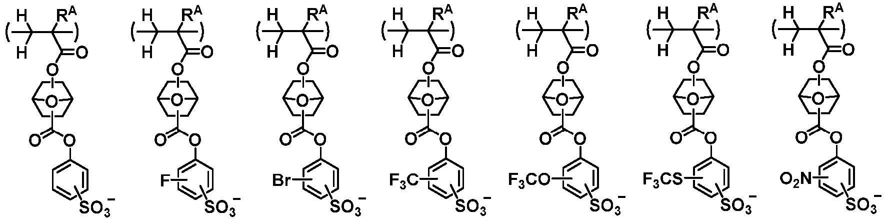

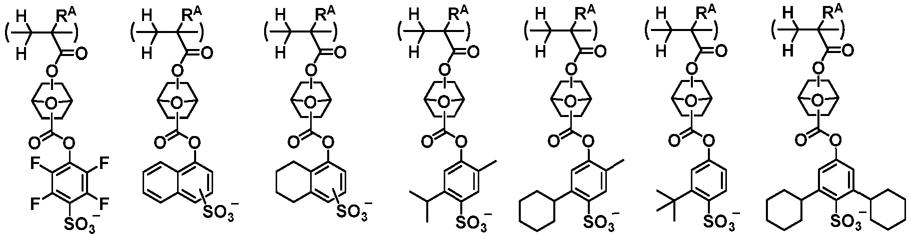

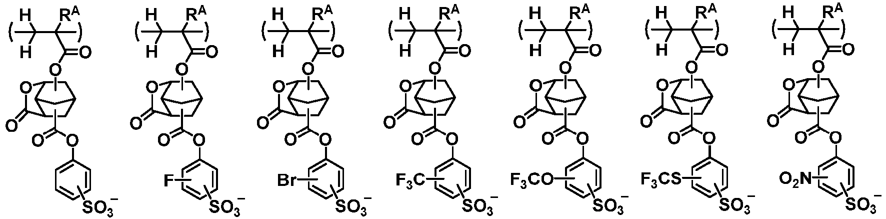

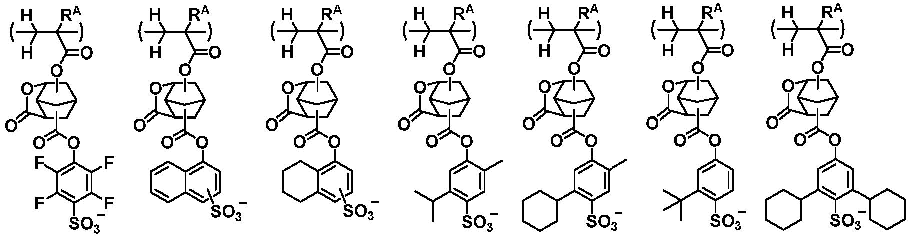

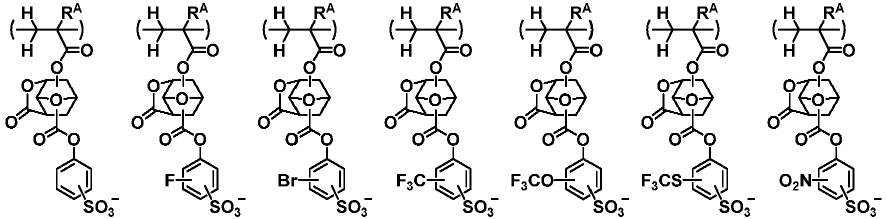

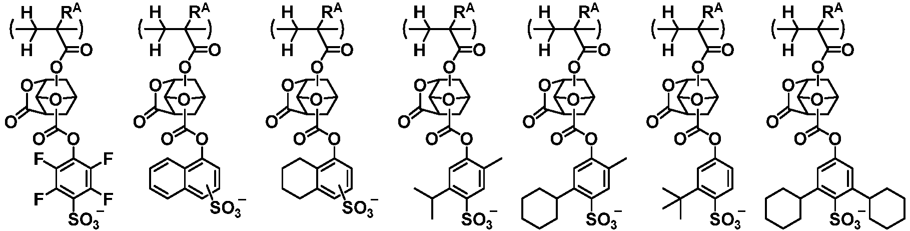

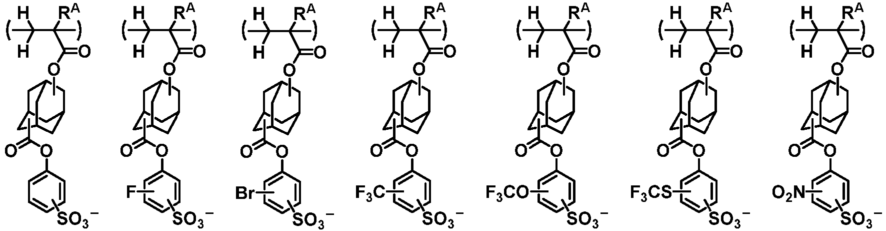

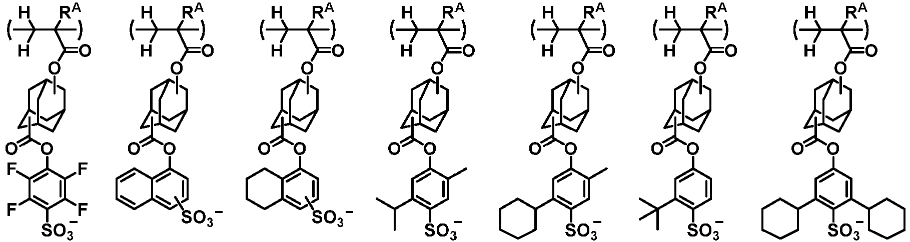

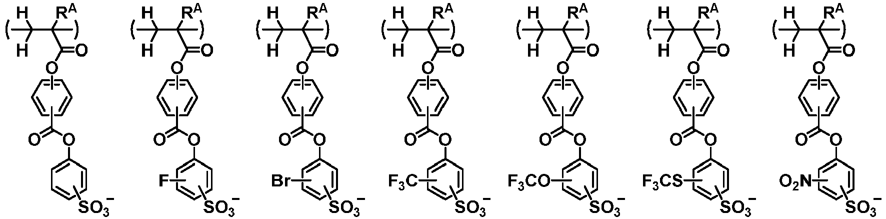

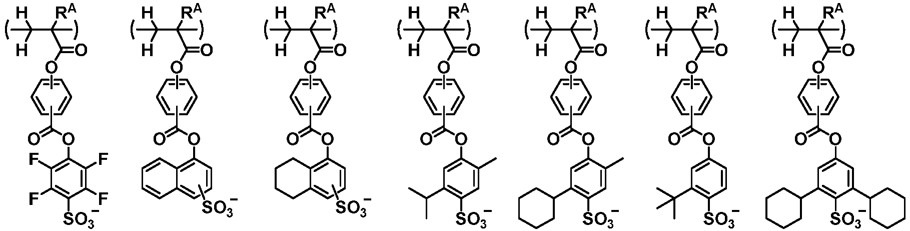

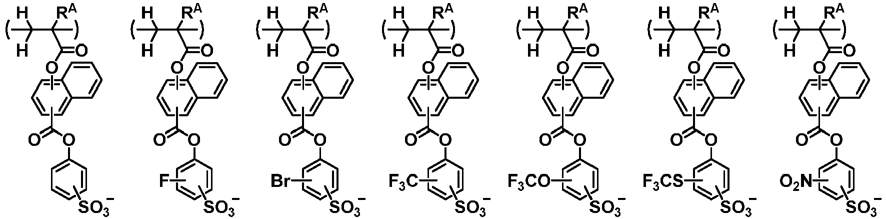

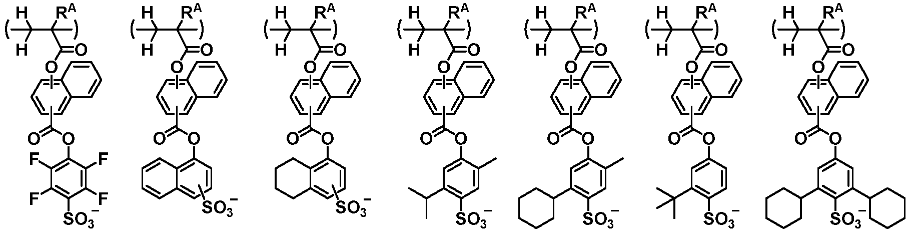

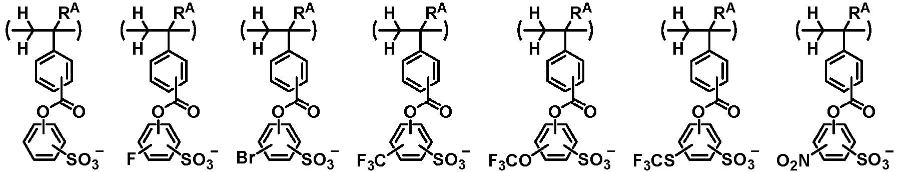

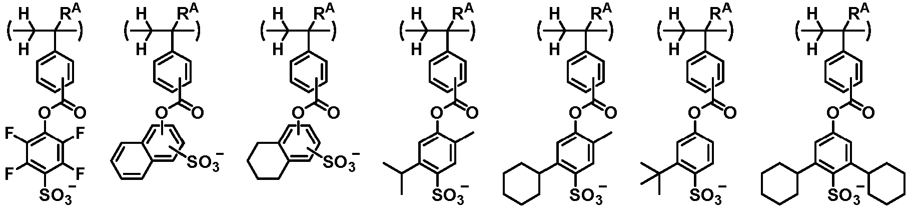

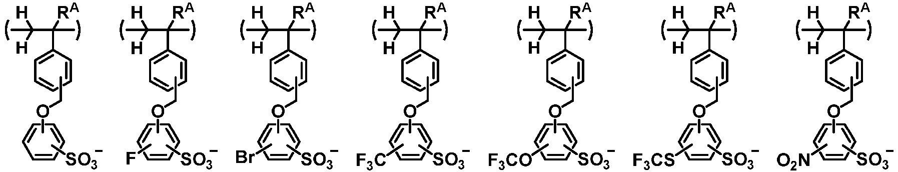

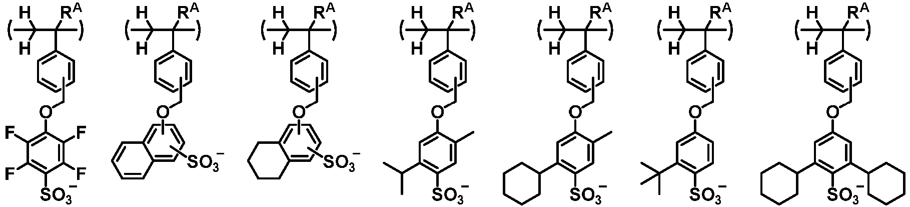

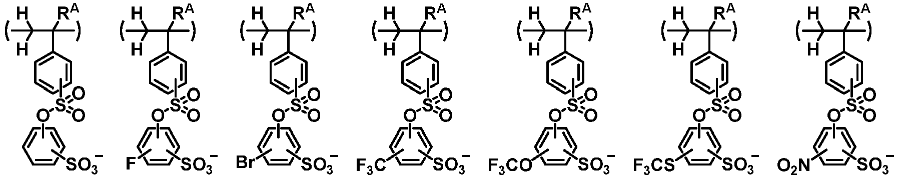

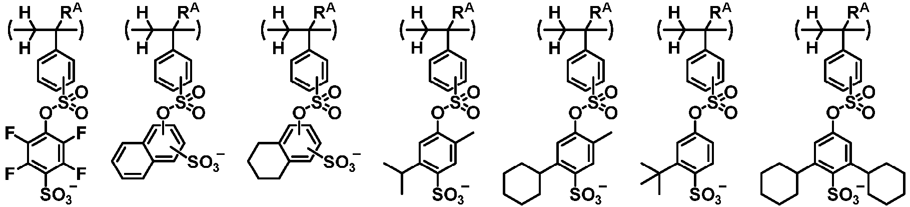

Examples of the anion in repeat unit B 10 are shown below, but not limited thereto. Herein R^{A} is as defined above.

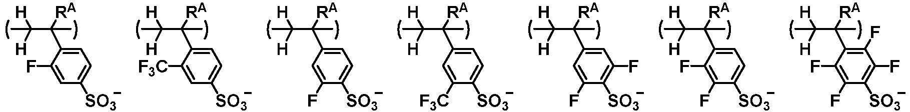

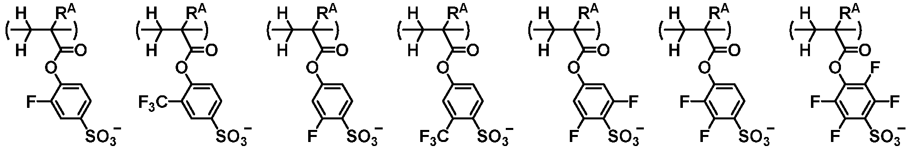

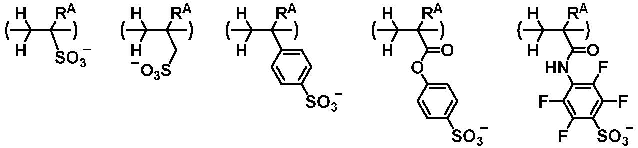

In formulae (B7) to (B10), A⁺ is an onium cation. Suitable onium cations include sulfonium, iodonium and ammonium cations, with the sulfonium and iodonium cations being preferred. Examples of the sulfonium cation include those described in JP-A 2024-003744, paragraphs [0102]-[0125], and JP-A 2023-169812, paragraphs [0070]-[0085], and those having formula (A2), but are not limited thereto. Examples of the iodonium cation include those described in JP-A 2024-000259, paragraph [0181], but are not limited thereto. Examples of the ammonium cation are as will be exemplified later as the ammonium cation having formula (am-1), but not limited thereto.

Illustrative structures of repeat units B6 to B10 include arbitrary combinations of anions with cations, both as mentioned above.

The repeat units B6 to B10 are capable of generating an acid upon exposure to high-energy radiation. It is believed that binding of the relevant units to a polymer enables to appropriately control acid diffusion and to form a pattern with reduced LER. Since the acid-generating unit is bound to a polymer, the phenomenon that acid volatilizes from the exposed region and re-deposits on the unexposed region during bake in vacuum is suppressed. This is effective for reducing LER and for suppressing profile degradation due to unwanted film thickness loss in the unexposed region.

Of repeat units B6 to B10, repeat units B7 to B10 are preferred for the processing of photomask blanks because an optimum acid strength is available for the suppression of acid diffusion and the design of an acid labile group on the polymer. The repeat units B7, B8 and B9 are more preferred

When repeat units B6 to B10 are included, their content is preferably 0.1 to 30 mol%, more preferably 0.5 to 20 mol% based on the overall repeat units of the polymer. Each of repeat units B6 to B10 may be of one type or a combination of plural types.

The content of repeat units having an aromatic ring structure is preferably at least 65 mol%, more preferably at least 75 mol%, even more preferably at least 85 mol% based on the overall repeat units of the polymer. When the polymer does not contain repeat units B6 to B10, it is preferred that all units have an aromatic ring structure.

The polymer may further comprise (meth)acrylate units protected with an acid labile group or (meth)acrylate units having an adhesive group such as lactone structure or hydroxy group other than phenolic hydroxy as commonly used in the art. These repeat units are effective for fine adjustment of properties of a resist film, but not essential.

Examples of the (meth)acrylate unit having an adhesive group include repeat units having the following formulae (B11) to (B13), which are also referred to as repeat units B11 to B13. While these units do not exhibit acidity, they may be used as auxiliary units for providing adhesion to substrates or adjusting solubility.

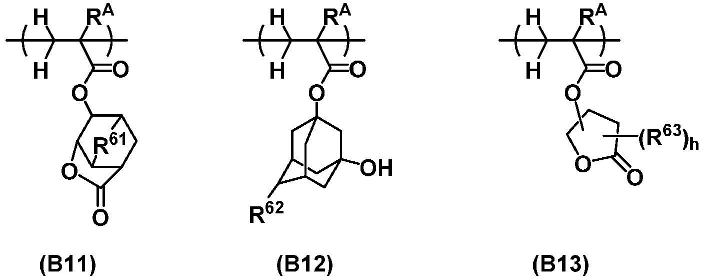

In formulae (B11) to (B13), R^{A} is each independently hydrogen, fluorine, methyl or trifluoromethyl. R⁶¹ is -O- or methylene. R⁶² is hydrogen or hydroxy. R⁶³ is a C₁-C₄ saturated hydrocarbyl group, and h is 0, 1, 2 or 3.

When repeat units B11 to B13 are included, their content is preferably 0 to 20 mol%, more preferably 0 to 10 mol% based on the overall repeat units of the polymer. Each of repeat units B11 to B13 may be of one type or a combination of plural types.

The polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630, for example.

The polymer should preferably have a Mw of 1,000 to 50,000, and more preferably 2,000 to 20,000. A Mw of at least 1,000 eliminates the risk that pattern features are rounded at their top to invite degradations of resolution and LER. A Mw of up to 50,000 eliminates the risk that LER is degraded when a pattern with a line width of up to 100 nm is formed. As used herein, Mw is measured by GPC versus polystyrene standards using tetrahydrofuran (THF) or dimethylformamide (DMF) solvent.

The polymer preferably has a narrow molecular weight distribution or dispersity (Mw/Mn) of 1.0 to 2.0, more preferably 1.0 to 1.9. A polymer with such a narrow dispersity eliminates the risk that foreign particles are left on the pattern after development and the pattern profile is aggravated.

The base polymer is designed such that the dissolution rate in alkaline developer is preferably up to 10 nm/min, more preferably up to 7 nm/min, even more preferably up to 5 nm/min. In the advanced generation of lithography wherein the coating film on the substrate is in a thin film range of up to 100 nm, the influence of pattern film thickness loss during alkaline development becomes strong. When the polymer has an alkaline dissolution rate of greater than 10 nm/min, pattern collapse occurs, i.e., a small-size pattern cannot be formed. The problem becomes outstanding in the fabrication of photomasks requiring to be defectless and having a tendency of strong development process. It is noted that the dissolution rate of a base polymer in alkaline developer is computed by spin coating a 16.7 wt% solution of a polymer in propylene glycol monomethyl ether acetate (PGMEA) solvent onto a 8-inch silicon wafer, baking at 100°C for 90 seconds to form a film of 1,000 nm thick, developing the film in a 2.38 wt% aqueous solution of tetramethylammonium hydroxide (TMAH) at 23°C for 100 seconds, and measuring a loss of film thickness.

In addition to the polymer defined above, the base polymer (B) may contain another polymer. The other polymer may be any of prior art well-known base polymers used in resist compositions. The content of the other polymer is not particularly limited as long as the benefits of the invention are not impaired.

### (C) Organic solvent

The chemically amplified positive resist composition may comprise an organic solvent as component (C). The organic solvent used herein is not particularly limited as long as the components are soluble therein. Examples of the organic solvent are described in JP-A 2008-111103, paragraphs [0144] to [0145] (USP 7,537,880). Specifically, exemplary solvents include ketones such as cyclohexanone, cyclopentanone, methyl-2-n-pentyl ketone, and 2-heptanone; alcohols such as 3-methoxybutanol, 3-methyl-3-methoxybutanol, 1-methoxy-2-propanol, 1-ethoxy-2-propanol, and diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate (EL), ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactones such as γ-butyrolactone (GBL), and mixtures thereof. Where an acid labile group of acetal form is used, a high boiling alcohol solvent such as diethylene glycol, propylene glycol, glycerol, 1,4-butanediol or 1,3-butanediol may be added to accelerate deprotection reaction of acetal.

Of the above organic solvents, it is recommended to use 1-ethoxy-2-propanol, PGMEA, PGME, cyclohexanone, EL, GBL, and mixtures thereof.

In the resist composition, the organic solvent (C) is preferably used in an amount of 200 to 10,000 parts, more preferably 400 to 5,000 parts by weight per 80 parts by weight of the base polymer (B). The organic solvent may be used alone or in admixture.

### (D) Fluorinated polymer

The chemically amplified positive resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. The fluorinated polymer contains repeat units of at least one type selected from repeat units having the formula (D1), repeat units having the formula (D2), repeat units having the formula (D3), and repeat units having the formula (D4), and may contain repeat units of at least one type selected from repeat units having the formula (D5) and repeat units having the formula (D6). It is noted that repeat units having formulae (D1), (D2), (D3), (D4), (D5), and (D6) are also referred to as repeat units D1, D2, D3, D4, D5, and D6, respectively, hereinafter. Since the fluorinated polymer also has a surface-active function, it can prevent insoluble residues from re-depositing onto the substrate during the development step and is thus effective for preventing development defects.

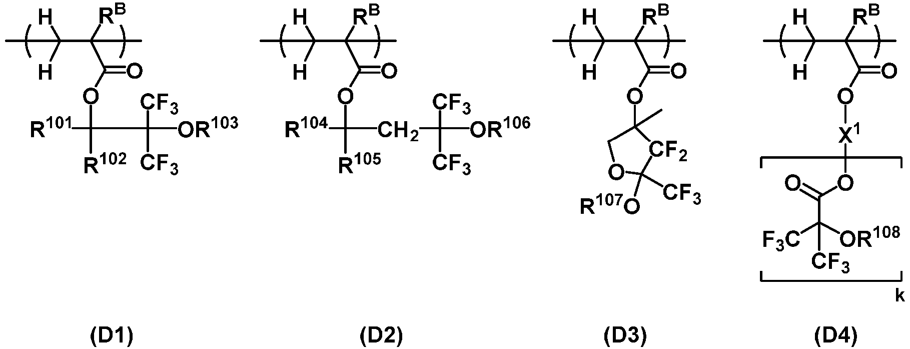

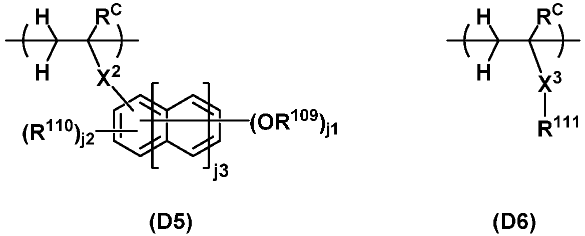

In formulae (D1) to (D6), j1 is 1, 2 or 3, j2 is an integer meeting: 0 ≤ j2 ≤ 5+2(j3)-j1, j3 is 0 or 1, and k is 1, 2 or 3. R^{B} is each independently hydrogen, fluorine, methyl or trifluoromethyl. R^{C} is each independently hydrogen or methyl. R¹⁰¹, R¹⁰², R¹⁰⁴ and R¹⁰⁵ are each independently hydrogen or a C₁-C₁₀ saturated hydrocarbyl group. R¹⁰³, R¹⁰⁶, R¹⁰⁷ and R¹⁰⁸ are each independently hydrogen, a C₁-C₁₅ hydrocarbyl group or fluorinated hydrocarbyl group, or an acid labile group. An ether bond or carbonyl moiety may intervene in a carbon-carbon bond in the hydrocarbyl groups or fluorinated hydrocarbyl groups represented by R¹⁰³, R¹⁰⁶, R¹⁹⁷ and R¹⁰⁸. R¹⁰⁹ is hydrogen or a C₁-C₅ straight or branched hydrocarbyl group in which a heteroatom-containing moiety may intervene in a carbon-carbon bond. R¹¹⁰ is a C₁-C₅ straight or branched hydrocarbyl group in which a heteroatom-containing moiety may intervene in a carbon-carbon bond. R¹¹¹ is a C₁-C₂₀ saturated hydrocarbyl group in which at least one hydrogen is substituted by fluorine and some constituent -CH₂- may be replaced by an ester bond or ether bond. X¹ is a C₁-C₂₀ (k+1)-valent hydrocarbon group or C₁-C₂₀ (k+1)-valent fluorinated hydrocarbon group. X² is a single bond, *-C(=O)-O- or *-C(=O)-NH- wherein * designates a point of attachment to the carbon atom in the backbone. X³ is a single bond, -O-, *-C(=O)-O-X³¹-X³²- or *-C(=O)-NH-X³¹-X³²-, wherein X³¹ is a single bond or a C₁-C₁₀ saturated hydrocarbylene group, X³² is a single bond, ester bond, ether bond or sulfonamide bond, and * designates a point of attachment to the carbon atom in the backbone.

In formulae (D1) and (D2), the C₁-C₁₀ saturated hydrocarbyl group represented by R¹⁰¹, R¹⁰², R¹⁰⁴ and R¹⁰⁵ may be straight, branched or cyclic and examples thereof include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl, and n-decyl, and C₃-C₁₀ cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

In formulae (D1) to (D4), the C₁-C₁₅ hydrocarbyl group represented by R¹⁰³, R¹⁰⁶, R¹⁰⁷ and R¹⁰⁸ may be straight, branched or cyclic and examples thereof include C₁-C₁₅ alkyl, C₂-C₁₅ alkenyl and C₂-C₁₅ alkynyl groups, with the alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl. The fluorinated hydrocarbyl groups correspond to the foregoing hydrocarbyl groups in which some or all carbon-bonded hydrogen atoms are substituted by fluorine atoms.

In formula (D4), examples of the C₁-C₂₀ (k+1)-valent hydrocarbon group X¹ include the foregoing C₁-C₂₀ alkyl groups and C₃-C₂₀ cyclic saturated hydrocarbyl groups, with k number of hydrogen atoms being eliminated. Examples of the C₁-C₂₀ (k+1)-valent fluorinated hydrocarbon group X¹ include the foregoing (k+1)-valent hydrocarbon groups in which at least one hydrogen atom is substituted by fluorine.

Examples of the repeat units D1 to D4 are given below, but not limited thereto. Herein R^{B} is as defined above.

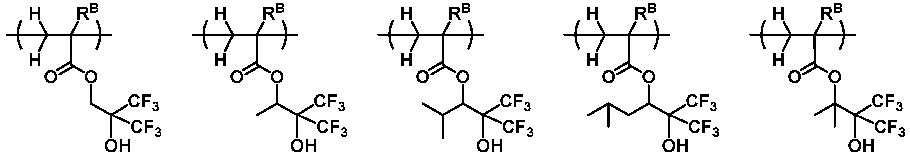

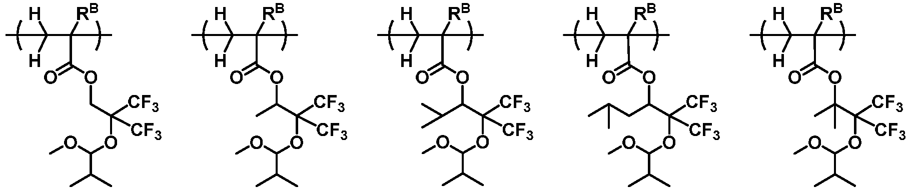

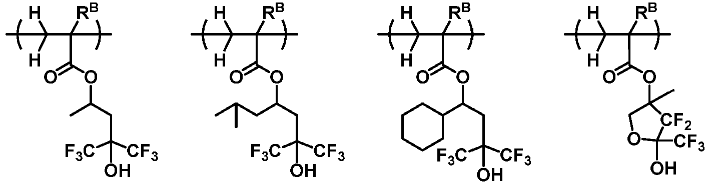

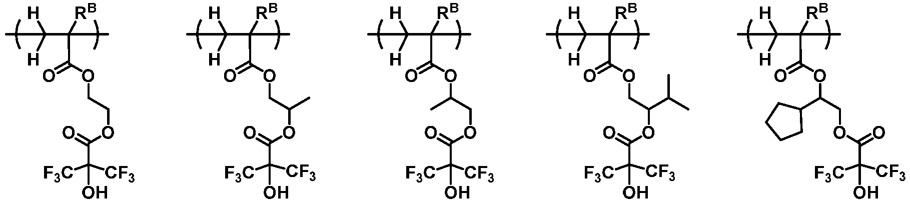

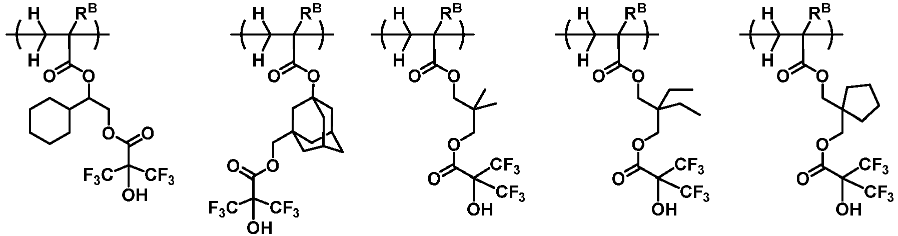

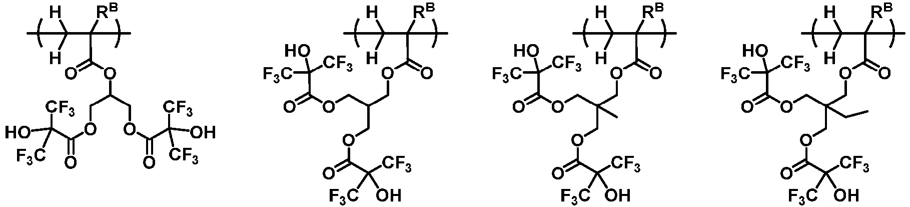

In formula (D5), examples of the C₁-C₅ hydrocarbyl groups R¹⁰⁹ and R¹¹⁰ include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In these groups, a moiety containing a heteroatom such as oxygen, sulfur or nitrogen may intervene in a carbon-carbon bond.

In formula (D5), -OR¹⁰⁹ is preferably a hydrophilic group. In this case, R¹⁰⁹ is preferably hydrogen or a C₁-C₅ alkyl group in which oxygen intervenes in a carbon-carbon bond.

In formula (D5), X² is preferably *-C(=O)-O- or *-C(=O)-NH-. Also preferably R^{C} is methyl. The inclusion of carbonyl in X² enhances the ability to trap the acid originating from the anti-charging film. A polymer wherein R^{C} is methyl is a robust polymer having a high glass transition temperature (Tg) which is effective for suppressing acid diffusion. As a result, the resist film is improved in stability with time, and neither resolution nor pattern profile is degraded.

Examples of the repeat unit D5 are given below, but not limited thereto. Herein R^{C} is as defined above.

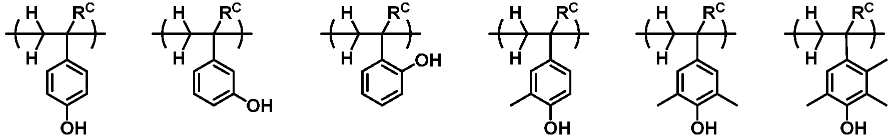

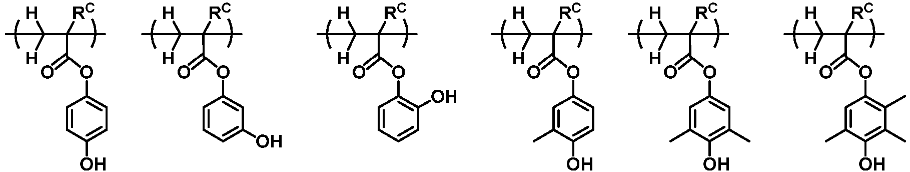

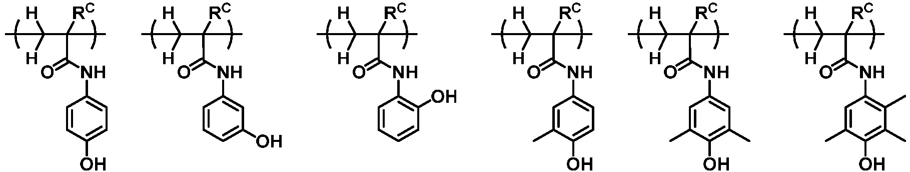

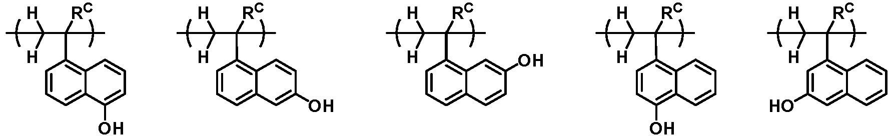

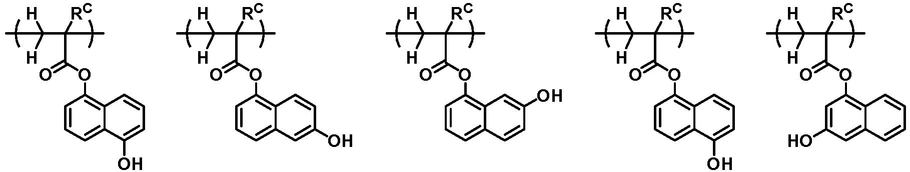

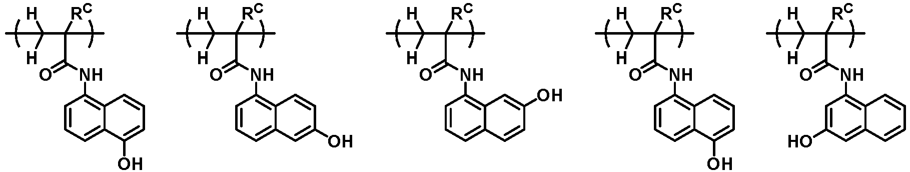

In formula (D6), the C₁-C₁₀ saturated hydrocarbylene group X³ may be straight, branched or cyclic and examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl.

The C₁-C₂₀ saturated hydrocarbyl group having at least one hydrogen substituted by fluorine, represented by R¹¹¹, may be straight, branched or cyclic and examples thereof include C₁-C₂₀ alkyl groups and C₃-C₂₀ cyclic saturated hydrocarbyl groups in which at least one hydrogen is substituted by fluorine.

Examples of the repeat unit D6 are given below, but not limited thereto. Herein R^{C} is as defined above.

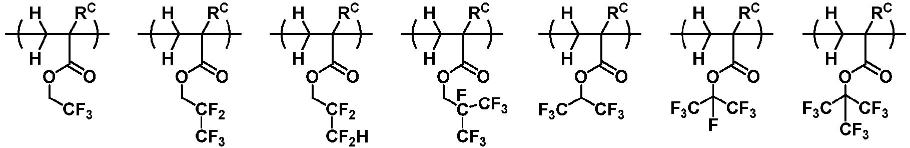

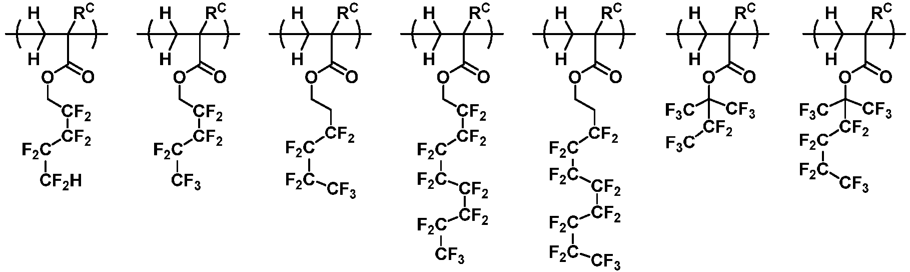

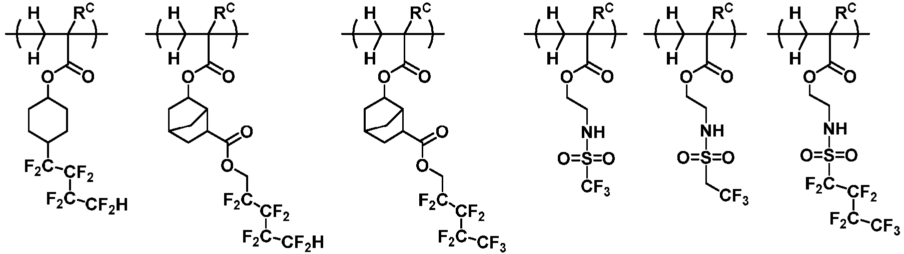

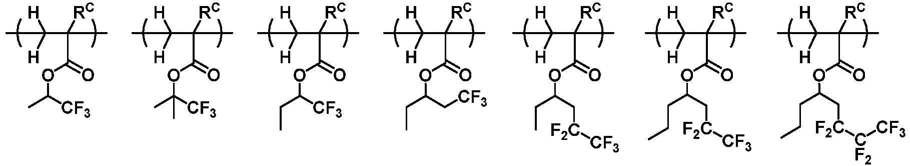

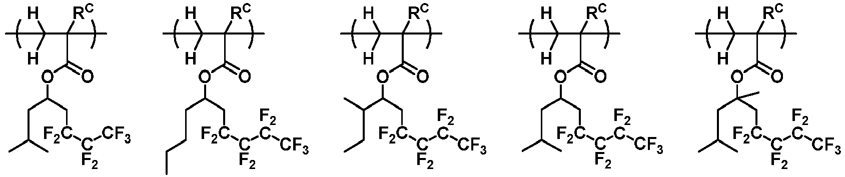

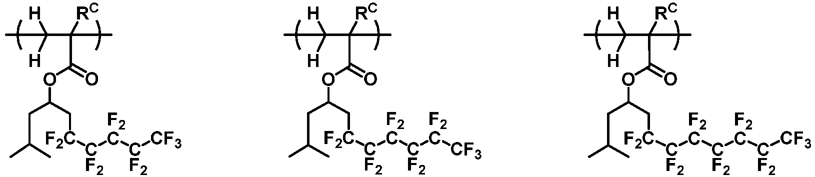

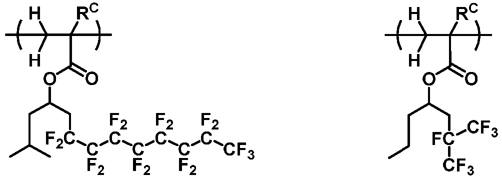

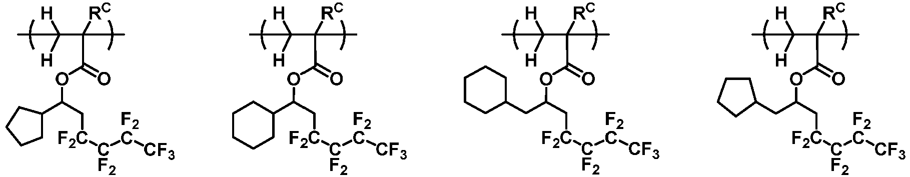

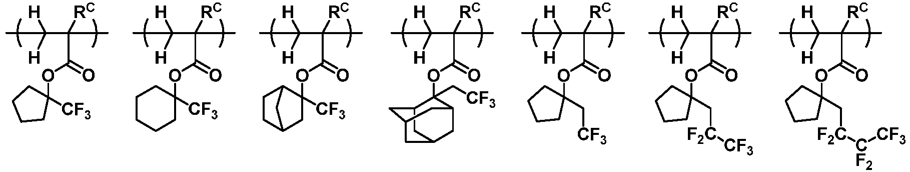

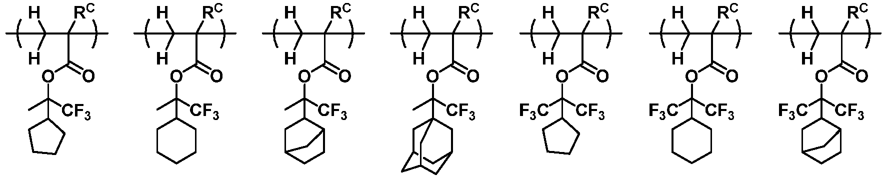

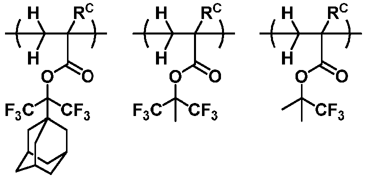

The content of repeat units D1 to D4 is preferably 15 to 95 mol%, more preferably 20 to 85 mol% based on the overall repeat units of the fluorinated polymer. The content of repeat unit D5 and/or D6 is preferably 5 to 85 mol%, more preferably 15 to 80 mol% based on the overall repeat units of the fluorinated polymer. Each of repeat units D1 to D6 may be used alone or in admixture.

The fluorinated polymer may comprise additional repeat units as well as the repeat units D1 to D6. Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

The fluorinated polymer should preferably have a Mw of 2,000 to 50,000, and more preferably 3,000 to 20,000. A fluorinated polymer with a Mw of less than 2,000 helps acid diffusion, degrading resolution and detracting from age stability. A polymer with too high Mw has a reduced solubility in solvent, with a risk of leaving coating defects. The fluorinated polymer preferably has a dispersity (Mw/Mn) of 1.0 to 2.2, more preferably 1.0 to 1.7.

In the resist composition, the fluorinated polymer (D) is preferably used in an amount of 0.01 to 30 parts by weight, more preferably 0.1 to 20 parts by weight per 80 parts by weight of the base polymer (B). The fluorinated polymer may be used alone or in admixture.

### (E) Quencher

The chemically amplified positive resist composition preferably comprises a quencher as component (E). As used herein, the quencher refers to a compound capable of trapping an acid generated from the acid generator upon exposure. The quencher is effective for holding down the rate of diffusion of the acid (generated by the acid generator) in the resist film. Even when a substrate whose outermost surface is made of a chromium-containing material is used, the quencher is effective for suppressing the influence of the acid (generated in the resist film) on the chromium-containing material.

The quencher is typically selected from conventional basic compounds. Conventional basic compounds include primary, secondary, and tertiary aliphatic amines, mixed amines, aromatic amines, heterocyclic amines, nitrogen-containing compounds with carboxy group, nitrogen-containing compounds with sulfonyl group, nitrogen-containing compounds with hydroxy group, nitrogen-containing compounds with hydroxyphenyl group, alcoholic nitrogen-containing compounds, amide derivatives, imide derivatives, and carbamate derivatives. Also included are primary, secondary, and tertiary amine compounds, specifically amine compounds having a hydroxy, ether bond, ester bond, lactone ring, cyano, or sulfonate ester group as described in JP-A 2008-111103, paragraphs [0146]-[0164], and compounds having a carbamate group as described in JP 3790649. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound may be effective for further suppressing the diffusion rate of acid in the resist film or correcting the pattern profile.

Onium salts such as sulfonium, iodonium and ammonium salts of carboxylic acids which are not fluorinated at α-position as described in USP 8,795,942 (JP-A 2008-158339) may also be used as the quencher. While an α-fluorinated sulfonic acid, imide acid, and methide acid are necessary to deprotect the acid labile group, an α-non-fluorinated carboxylic acid is released by salt exchange with an α-non-fluorinated onium salt. The α-non-fluorinated carboxylic acid functions as a quencher because it does not induce substantial deprotection reaction.

Examples of the onium salt of α-non-fluorinated carboxylic acid include compounds having the formula (E1).

**R²⁰¹-CO₂⁻ MqA⁺** **(E1)**

In formula (E1), R²⁰¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl.

The hydrocarbyl group R²⁰¹ may be saturated or unsaturated and straight, branched or cyclic. Examples thereof include C₁-C₄₀ alkyl groups such as methyl, ethyl, n-propyl, isopropyl, n-butyl, sec-butyl, tert-butyl, n-pentyl, tert-pentyl, n-hexyl, n-octyl, 2-ethylhexyl, n-nonyl, n-decyl; C₃-C₄₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₂-C₄₀ alkenyl groups such as vinyl, allyl, propenyl, butenyl and hexenyl; C₃-C₄₀ cyclic unsaturated aliphatic hydrocarbyl groups such as cyclohexenyl; C₆-C₄₀ aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), di- or trialkylphenyl groups (e.g., 2,4-dimethylphenyl and 2,4,6-triisopropylphenyl), alkylnaphthyl groups (e.g., methylnaphthyl and ethylnaphthyl), dialkylnaphthyl groups (e.g., dimethylnaphthyl and diethylnaphthyl); and C₇-C₄₀ aralkyl groups such as benzyl, 1-phenylethyl and 2-phenylethyl.

In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy moiety, cyano moiety, carbonyl moiety, ether bond, thioether bond, ester bond, sulfonate ester bond, carbonate bond, lactone ring, sultone ring, carboxylic anhydride (-C(=O)-O-C(=O)-), or haloalkyl moiety. Suitable heteroatom-containing hydrocarbyl groups include heteroaryl groups such as thienyl; alkoxyphenyl groups such as 4-hydroxyphenyl, 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl and n-butoxynaphthyl; dialkoxynaphthyl groups such as dimethoxynaphthyl and diethoxynaphthyl; and aryloxoalkyl groups, typically 2-aryl-2-oxoethyl groups such as 2-phenyl-2-oxoethyl, 2-(1-naphthyl)-2-oxoethyl and 2-(2-naphthyl)-2-oxoethyl.

In formula (E1), Mq_{A}⁺ is an onium cation. The onium cation is preferably selected from sulfonium, iodonium and ammonium cations, more preferably sulfonium and iodonium cations. Exemplary sulfonium cations include those described in JP-A 2024-003744, paragraphs [0102]-[0125] and JP-A 2023-169812, paragraphs [0070]-[0085], and those having formula (A2), but are not limited thereto. Exemplary iodonium cations include those described in JP-A 2024-000259, paragraph [0181], but are not limited thereto.

The ammonium cation preferably has the formula (am-1).

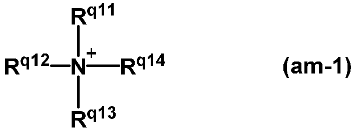

In formula (am-1), R^{q11} to R^{q14} are each independently a C₁-C₄₀ hydrocarbyl group which may contain a heteroatom. Any two of R^{q11} to R^{q14} may bond together to form a ring with the nitrogen atom to which they are attached. Examples of the hydrocarbyl group are as exemplified above for the hydrocarbyl group R¹ in formula (A1).

Examples of the ammonium cation having formula (am-1) are shown below, but not limited thereto.

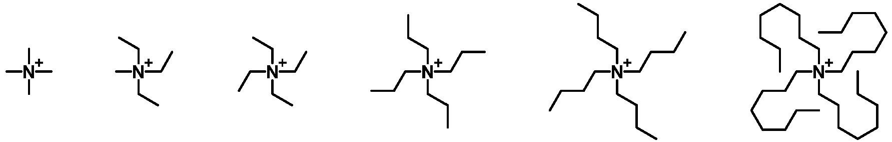

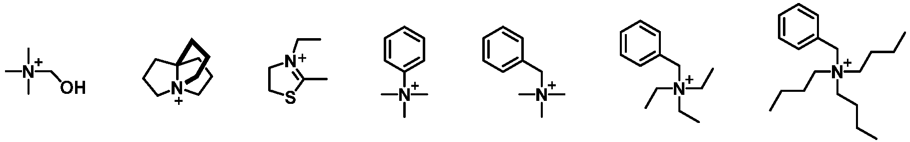

Examples of the anion in the onium salt having formula (E1) are shown below, but not limited thereto.

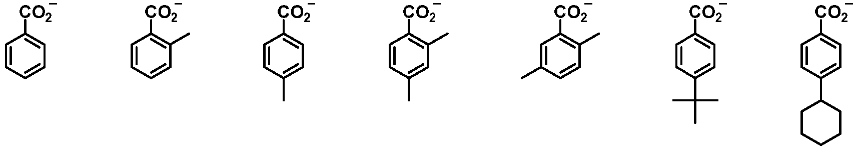

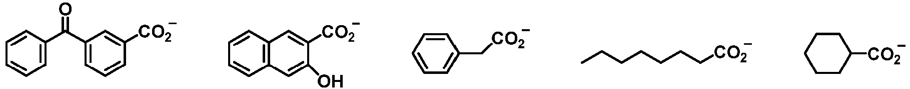

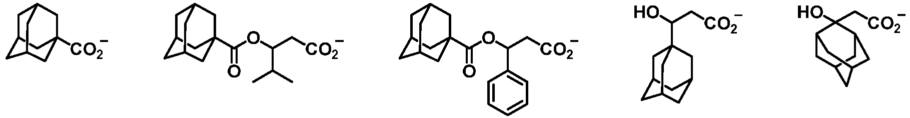

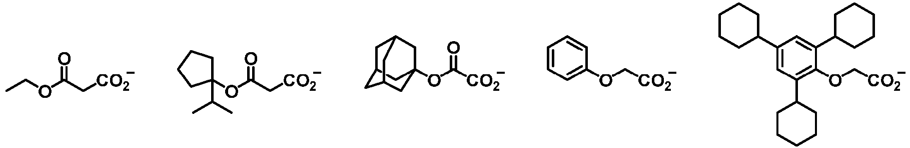

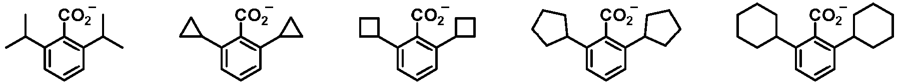

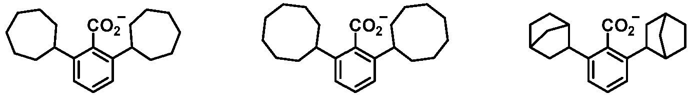

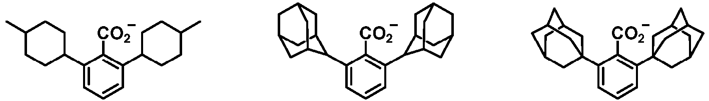

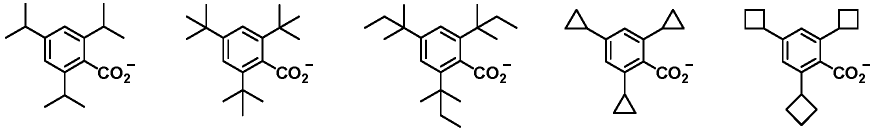

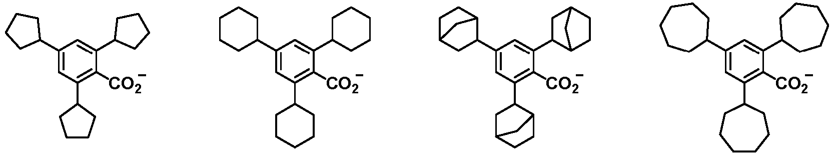

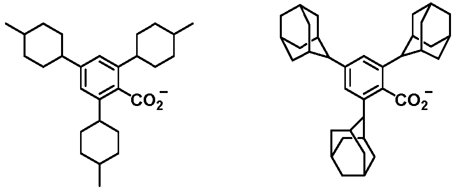

A sulfonium salt of iodized benzene ring-containing carboxylic acid having the formula (E2) is also useful as the quencher.

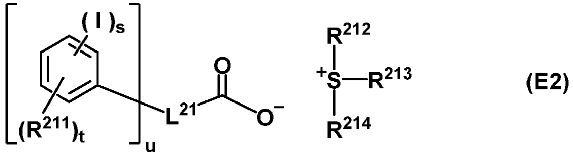

In formula (E2), s is 1, 2, 3, 4 or 5, t is 0, 1, 2 or 3, s+t is from 1 to 5, and u is 1, 2 or 3.

In formula (E2), R²¹¹ is hydroxy, fluorine, chlorine, bromine, amino, nitro, cyano, or a C₁-C₆ saturated hydrocarbyl, C₁-C₆ saturated hydrocarbyloxy, C₂-C₆ saturated hydrocarbylcarbonyloxy or C₁-C₄ saturated hydrocarbylsulfonyloxy group, in which some or all hydrogen may be substituted by halogen, or -N(R^{211A})-C(=O)-R^{211B} or -N(R^{211A})-C(=O)-O-R^{211B}. R^{211A} is hydrogen or a C₁-C₆ saturated hydrocarbyl group. R^{211B} is a C₁-C₆ saturated hydrocarbyl or C₂-C₈ unsaturated aliphatic hydrocarbyl group. A plurality of R²¹¹ may be identical or different when t and/or u is 2 or 3.

In formula (E2), L²¹ is a single bond or a C₁-C₂₀ (u+1)-valent linking group which may contain at least one moiety selected from ether bond, carbonyl moiety, ester bond, amide bond, sultone ring, lactam ring, carbonate bond, halogen, hydroxy moiety, and carboxy moiety. The saturated hydrocarbyl, saturated hydrocarbyloxy, saturated hydrocarbylcarbonyloxy, and saturated hydrocarbylsulfonyloxy groups may be straight, branched or cyclic.

In formula (E2), R²¹², R²¹³ and R²¹⁴ are each independently halogen, or a C₁-C₂₀ hydrocarbyl group which may contain a heteroatom. The hydrocarbyl group may be saturated or unsaturated and straight, branched or cyclic. Examples thereof include C₁-C₂₀ alkyl, C₂-C₂₀ alkenyl, C₆-C₂₀ aryl, and C₇-C₂₀ aralkyl groups. In these groups, some or all hydrogen may be substituted by hydroxy, carboxy, halogen, oxo, cyano, nitro, sultone ring, sulfo, or sulfonium salt-containing moiety, or some -CH₂- may be replaced by an ether bond, ester bond, carbonyl moiety, amide bond, carbonate bond or sulfonate ester bond. Also, R²¹² and R²¹³ may bond together to form a ring with the sulfur atom to which they are attached.

Examples of the compound having formula (E2) include those described in USP 10,295,904 (JP-A 2017-219836). These compounds exert a sensitizing effect due to remarkable absorption and an acid diffusion-controlling effect.

A nitrogen-containing carboxylic acid salt compound having the formula (E3) is also useful as the quencher.

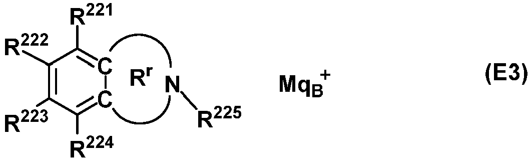

In formula (E3), R²²¹ to R²²⁴ are each independently hydrogen, -L²²-CO₂⁻, or a C₁-C₂₀ hydrocarbyl group which may contain a heteroatom. R²²¹ and R²²², R²²² and R²²³, or R²²³ and R²²⁴ may bond together to form a ring with the carbon atom to which they are attached. L²² is a single bond or a C₁-C₂₀ hydrocarbylene group which may contain a heteroatom. R²²⁵ is hydrogen or a C₁-C₂₀ hydrocarbyl group which may contain a heteroatom.

In formula (E3), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L²²-CO₂⁻ and in which some carbon may be replaced by sulfur, oxygen or nitrogen. The ring may be alicyclic or aromatic and is preferably a 5- or 6-membered ring. Suitable rings include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (E3) has at least one -L²²-CO₂⁻. That is, at least one of R²²¹ to R²²⁴ is -L²²-CO₂⁻, and/or at least one of hydrogen atoms bonded to carbon atoms in the ring R^{r} is substituted by -L²²-CO₂⁻.

In formula (E3), Mq_{B}⁺ is a sulfonium, iodonium or ammonium cation, with the sulfonium cation being preferred. Examples of the sulfonium cation include those described in JP-A 2024-003744, paragraphs [0102]-[0125], and JP-A 2023-169812, paragraphs [0070]-[0085], and those having formula (A2), but are not limited thereto.

Examples of the anion in the compound having formula (E3) are shown below, but not limited thereto.

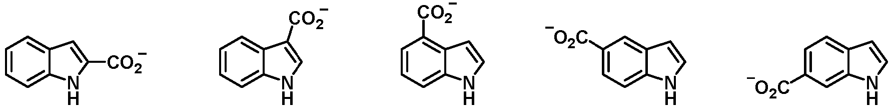

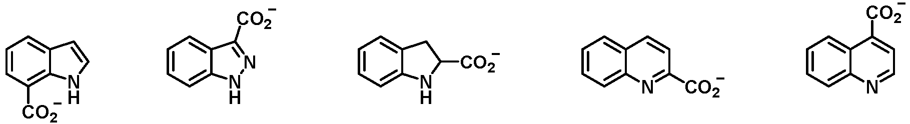

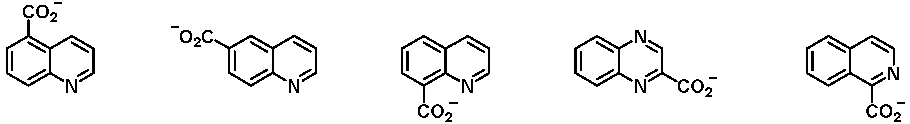

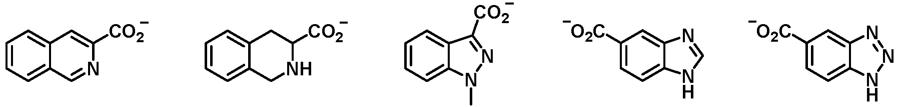

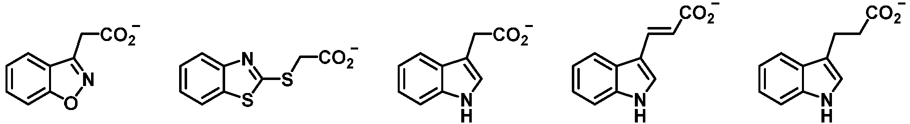

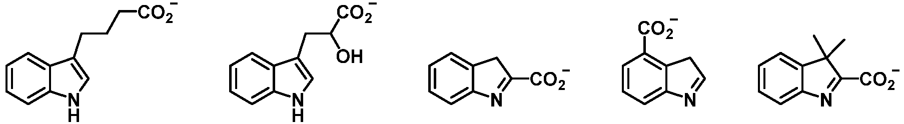

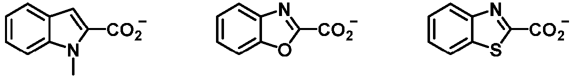

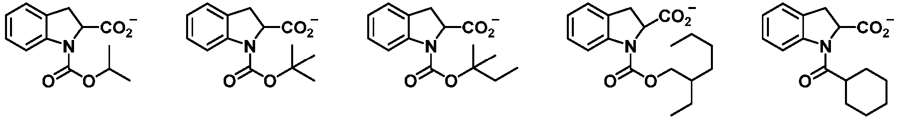

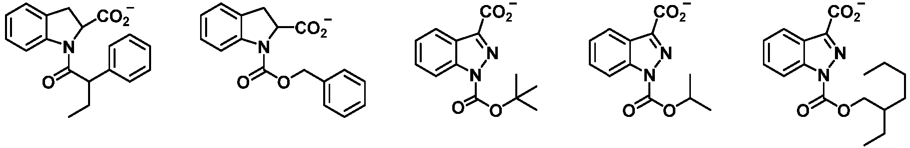

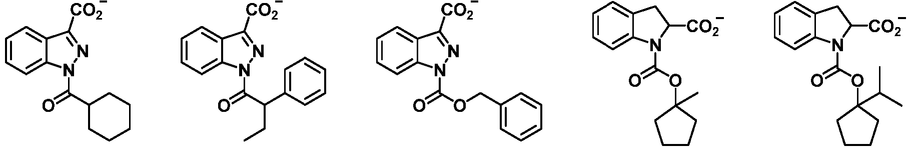

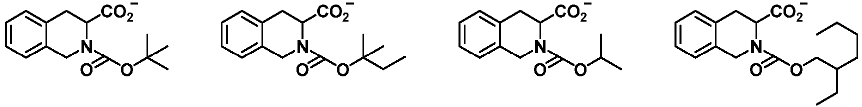

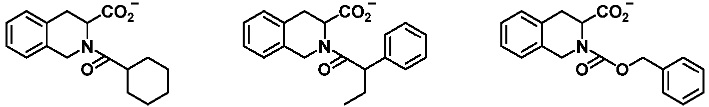

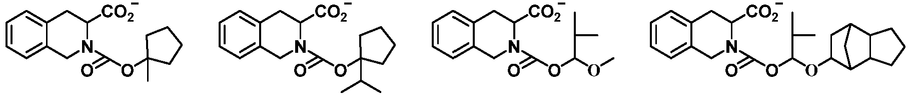

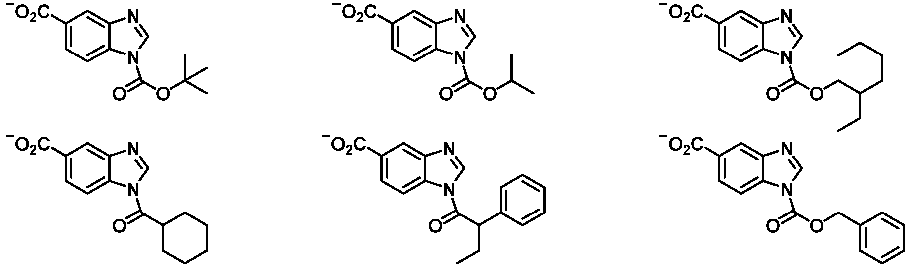

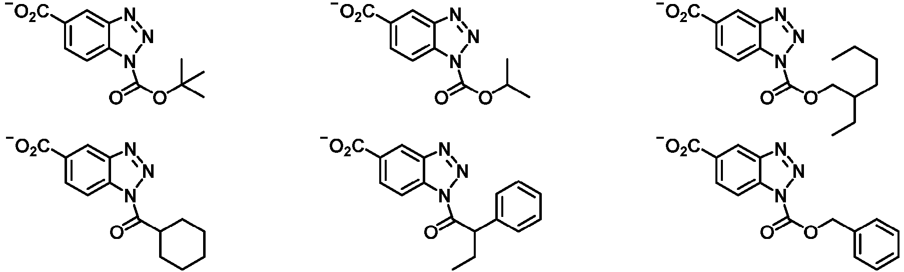

Weak acid betaine compounds are also useful as the quencher. Non-limiting examples thereof are shown below.

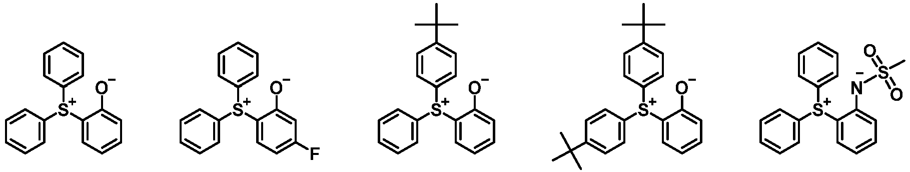

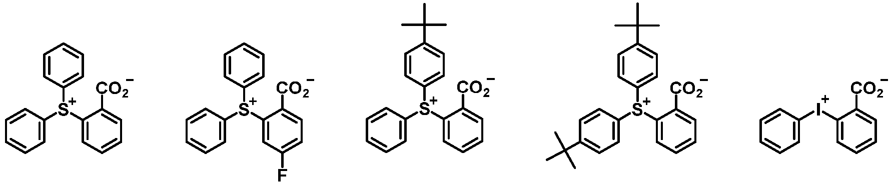

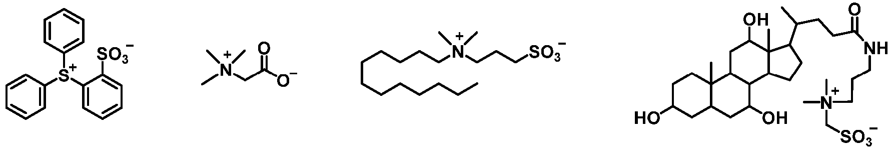

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

When used, the quencher (E) is preferably added in an amount of 0 to 50 parts, more preferably 0.1 to 40 parts by weight per 80 parts by weight of the base polymer (B). The quencher may be used alone or in admixture.

When the chemically amplified positive resist composition contains both the PAG (A) and the quencher (E), the weight ratio of the PAG to the quencher, (A)/(E) is preferably less than 3/1, more preferably less than 2.5/1, even more preferably less than 2/1. As long as the weight ratio of the PAG to the quencher is in the range, the resist composition is able to fully suppress acid diffusion, leading to improved resolution and dimensional uniformity.

### (F) Surfactant

The resist composition may contain any conventional surfactants for facilitating to coat the composition to the substrate. A number of surfactants are known in the art as described in WO 2006/121096, JP-A 2008-102383, JP-A 2008-304590, JP-A 2004-115630, and JP-A 2005-008766, and any suitable one may be chosen therefrom.

When the resist composition contains the surfactant (G), the amount of the surfactant (G) added is preferably up to 2 parts by weight, more preferably up to 1 part by weight and preferably at least 0.01 part by weight per 80 parts by weight of the base polymer (B).

### Process

A further embodiment of the invention is a process of forming a pattern from the chemically amplified positive resist composition defined above by lithography. The preferred process includes the steps of applying the resist composition onto a substrate to form a resist film thereon, exposing the resist film to high-energy radiation, and developing the exposed resist film in a developer. Any desired steps may be added to the process if necessary.

The substrate used herein may be a substrate for integrated circuitry fabrication, e.g., Si, SiO₂, SiN, SiON, TiN, WSi, BPSG, SOG, organic antireflective film, etc. or a substrate for mask circuitry fabrication, e.g., Cr, CrO, CrON, MoSi₂, SiO₂, etc.

The resist composition is applied onto a substrate by a suitable coating technique such as spin coating. The coating is prebaked on a hot plate preferably at a temperature of 60 to 150°C for 1 to 20 minutes, more preferably at 80 to 140°C for 1 to 10 minutes. The resulting resist film preferably has a thickness of 0.03 to 2 µm.

Then the resist film is exposed to a pattern of high-energy radiation, typically UV, deep UV, excimer (KrF or ArF) laser, EUV of wavelength 3 to 15 nm, EB, X-ray, γ-ray or synchrotron radiation. On use of UV, deep UV, excimer laser, EUV, X-ray, γ-ray or synchrotron radiation, the resist film is exposed through a mask having a desired pattern, preferably in a dose of 1 to 300 mJ/cm², more preferably 10 to 200 mJ/cm². On use of EB, a pattern may be written directly, preferably in a dose of 1 to 300 µC/cm², more preferably 10 to 200 µC/cm². The inventive resist composition is quite effective in the EUV and EB lithography.

The exposure may be performed by conventional lithography whereas the immersion lithography of holding a liquid between the resist film and the mask may be employed if desired. The liquid is typically water, and in this case, a protective film which is insoluble in water may be formed on the resist film.

After the exposure, the resist film may be baked (PEB), for example, on a hotplate preferably at 60 to 150°C for 1 to 20 minutes, more preferably at 80 to 140°C for 1 to 10 minutes.

The resist film is then developed with a developer in the form of an aqueous base solution, for example, 0.1 to 5 wt%, preferably 2 to 3 wt% aqueous solution of tetramethylammonium hydroxide (TMAH) for 0.1 to 3 minutes, preferably 0.5 to 2 minutes by conventional techniques such as dip, puddle and spray techniques. In this way, the exposed region of the resist film is dissolved away, and a desired resist pattern is formed on the substrate.

The chemically amplified positive resist composition has high etch resistance and is useful when used to meet the requirement that even when the duration from exposure to PEB is prolonged, the pattern experiences little changes of line width and has reduced LER. The resist composition is effectively applicable to a substrate, specifically a substrate having a surface layer of material to which a resist film is less adherent and which is likely to invite pattern stripping or pattern collapse, and particularly a substrate having sputter deposited on its outermost surface metallic chromium or a chromium compound containing at least one light element selected from oxygen, nitrogen and carbon. The resist composition is useful particularly when a photomask blank is used as the substrate.

### EXAMPLES

Examples of the invention are given below by way of illustration and not by way of limitation. The abbreviation "pbw" is parts by weight. Analysis is made by time-of-flight mass spectrometry (TOF-MS) using MALDI TOF-MS S3000 (JEOL Ltd.).

### [1] Synthesis of onium salts

### Synthesis Example 1-1

### Synthesis of onium salt PAG-1

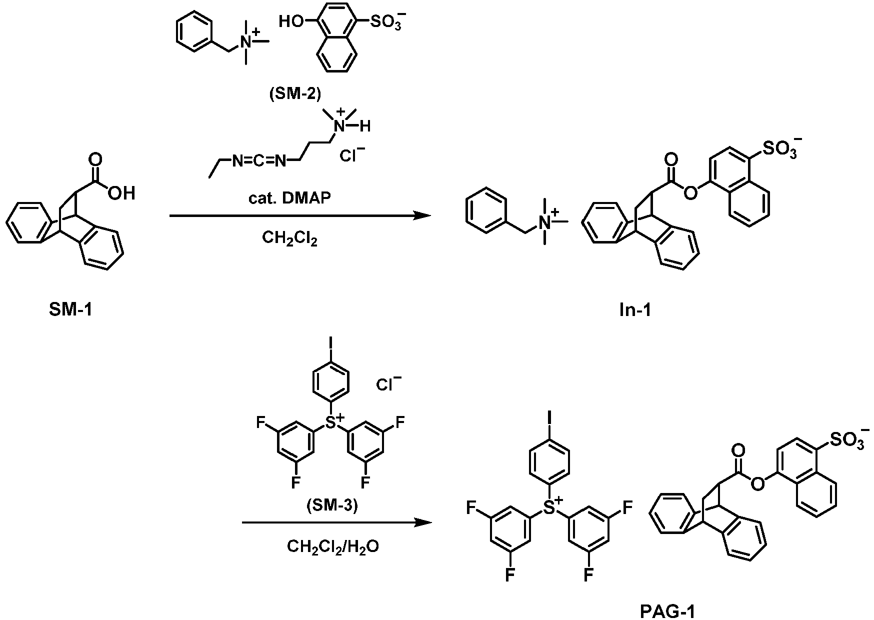

### (1) Synthesis of Intermediate In-1

In nitrogen atmosphere, a reactor was charged with 6.3 g of reactant SM-1, 9.8 g of reactant SM-2, 0.3 g of DMAP, and 50 g of methylene chloride and cooled in an ice bath. While the internal temperature of the reactor was kept below 20°C, 5.8 g of 1-ethyl-3-(3-dimethylaminopropyl)carbodiimide hydrochloride in powder form was added. Thereafter, the reactor was allowed to reach room temperature, at which the reaction solution was aged for 12 hours. At the end of aging, water was added to quench the reaction, followed by ordinary aqueous work-up. The subsequent steps of distilling off the solvent and washing the residue with diisopropyl ether gave 13.9 g of Intermediate In-1 as oily matter (yield 92%).

### (2) Synthesis of onium salt PAG-1

In nitrogen atmosphere, a reactor was charged with 13.9 g of Intermediate In-1, 12.6 g of reactant SM-3, 50 g of methylene chloride, and 30 g of water, which were stirred for 15 minutes. The organic layer was taken out, washed with water, and concentrated under reduced pressure. To the concentrate, 50 g of methyl isobutyl ketone was added, followed by azeotropic dewatering. Diisopropyl ether was then added for recrystallization, obtaining 20.0 g of the target onium salt, PAG-1 as white crystals (yield 95%).

PAG-1 was analyzed by TOF-MS, with the data shown below.

### MALDI TOF-MS:

positive M⁺ 461 (corresponding to C₁₈H₁₀F₄IS⁺)
negative M⁻ 455 (corresponding to C₂₇H₁₉O₅S⁻)

### Synthesis Examples 1-2 to 1-10

### Synthesis of onium salts PAG-2 to PAG-10

Onium salts PAG-2 to PAG-10 shown below were synthesized using corresponding reactants and well-known organic synthesis reactions.

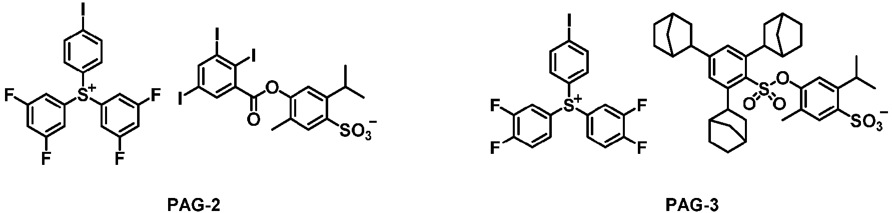

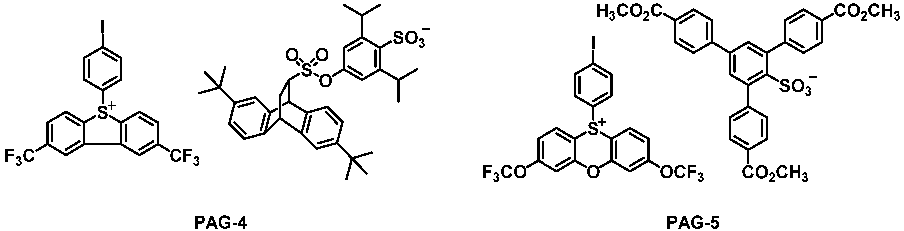

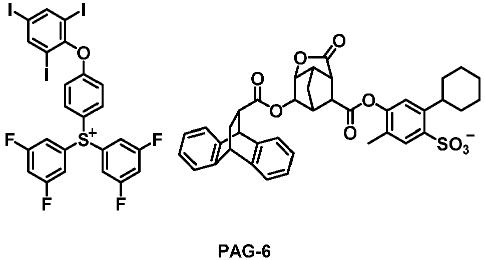

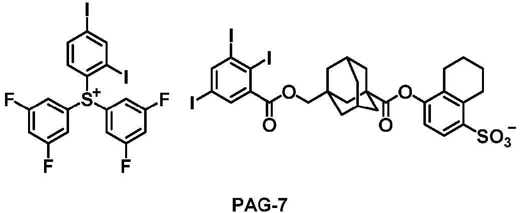

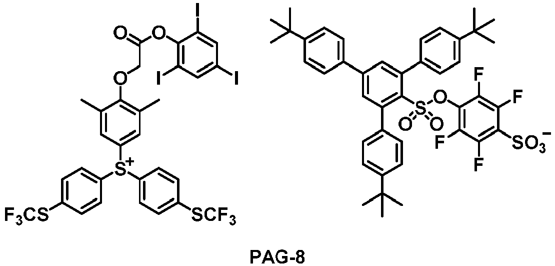

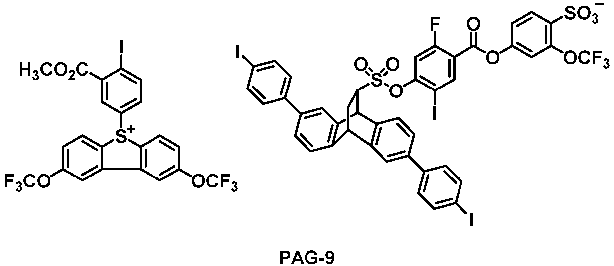

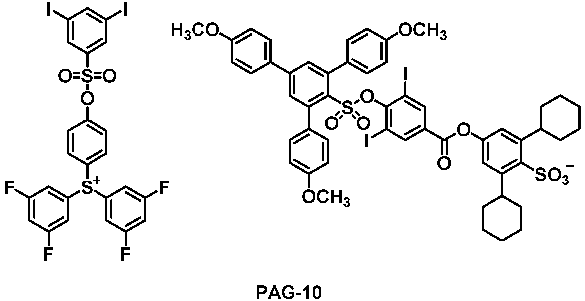

### [2] Synthesis of base polymers

### Synthesis Examples 2-1 to 2-6

### Synthesis of Base Polymers P-1 to P-6

Base polymers P-1 to P-6 were synthesized in a standard way by combining monomers, performing copolymerization reaction in a solvent, pouring the reaction solution to hexane for precipitation, washing the solid precipitate with hexane, isolation and drying. The polymer was analyzed for composition by ¹H-NMR and ¹³C-NMR spectroscopy and for Mw and Mw/Mn by GPC versus polystyrene standards using THF solvent.

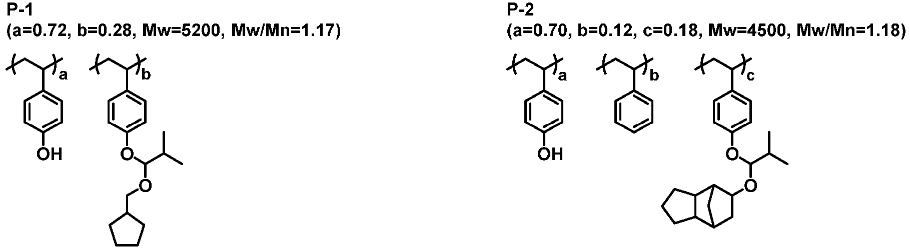

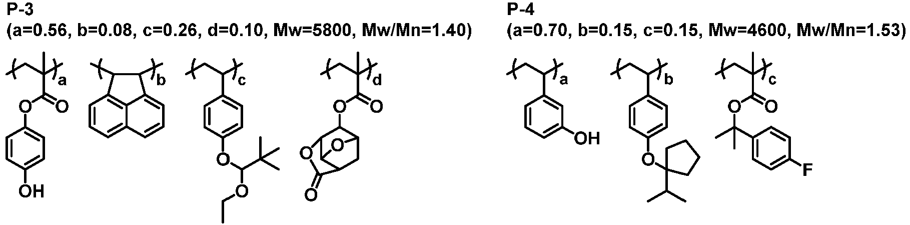

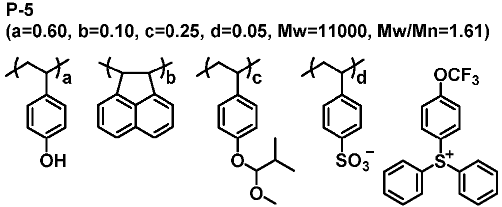

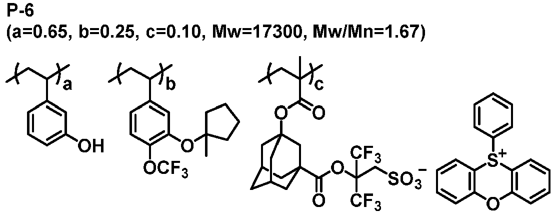

### [3] Preparation of chemically amplified positive resist compositions

### Examples 1-1 to 1-53 and Comparative Examples 1-1 to 1-28

A chemically amplified positive resist composition was prepared by dissolving selected components in an organic solvent in accordance with the formulation shown in Tables 1 to 3, and filtering the solution through a nylon filter with a pore size of 5 nm and a UPE filter with a pore size of 1 nm. The organic solvent was a mixture of 940 pbw of PGMEA, 1,870 pbw of EL and 1,870 pbw of PGME.

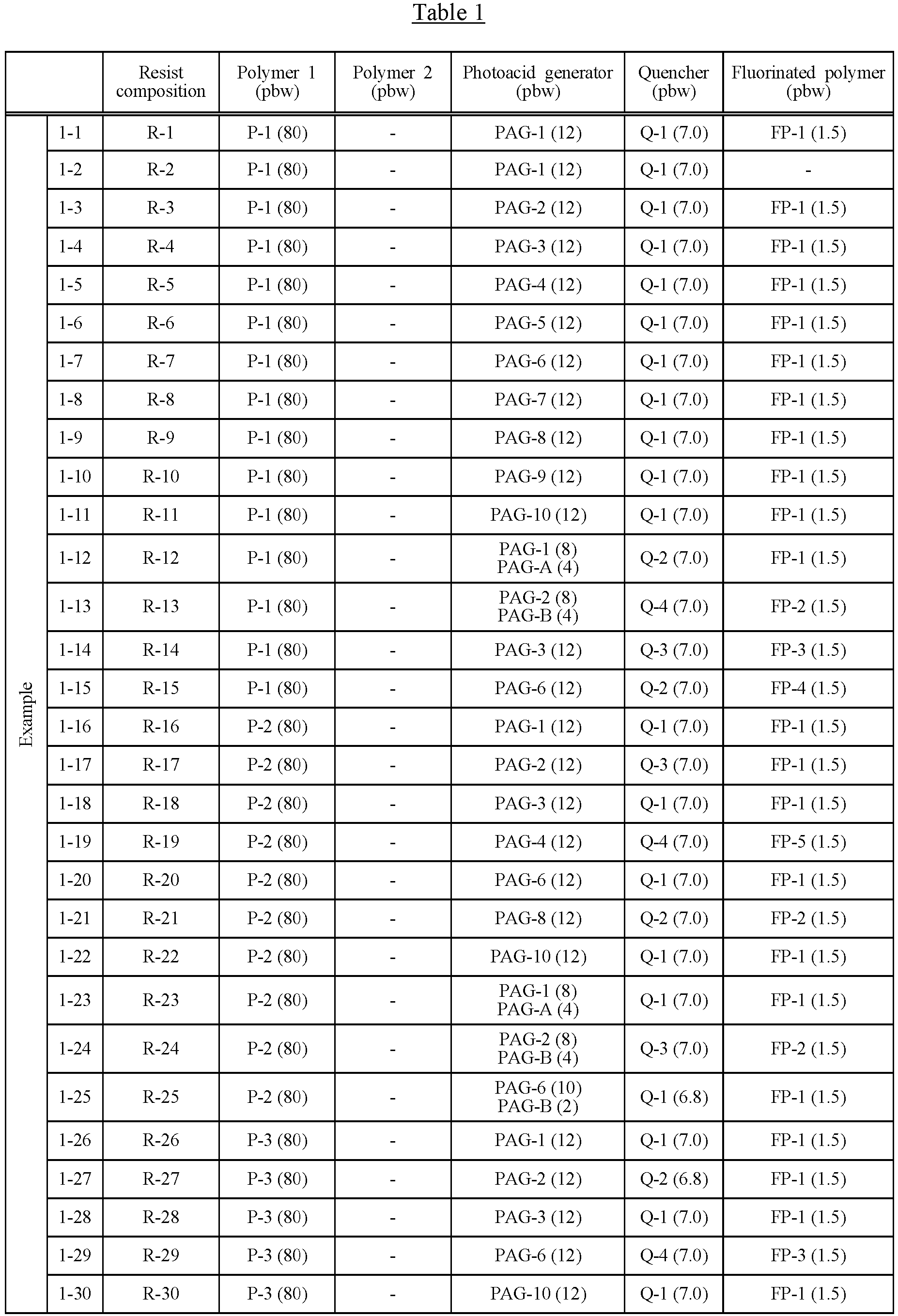

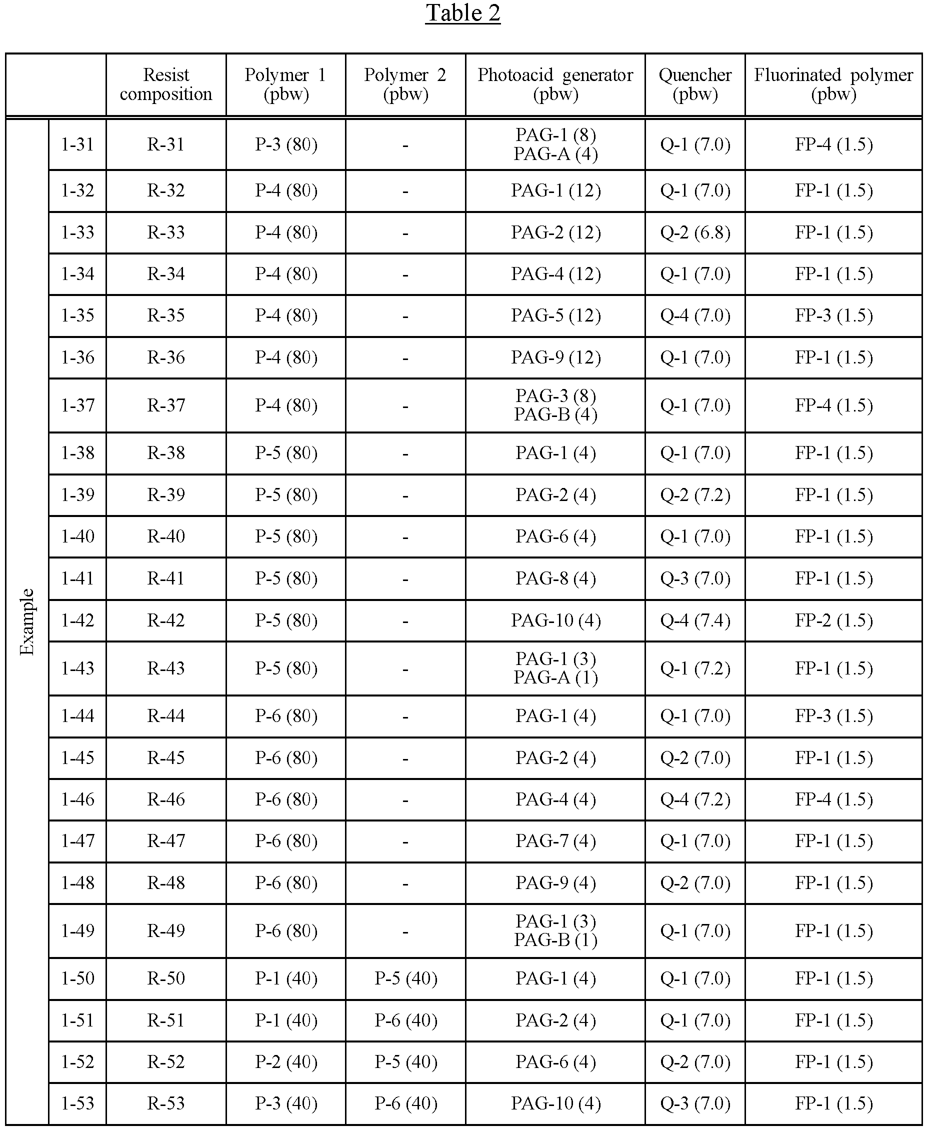

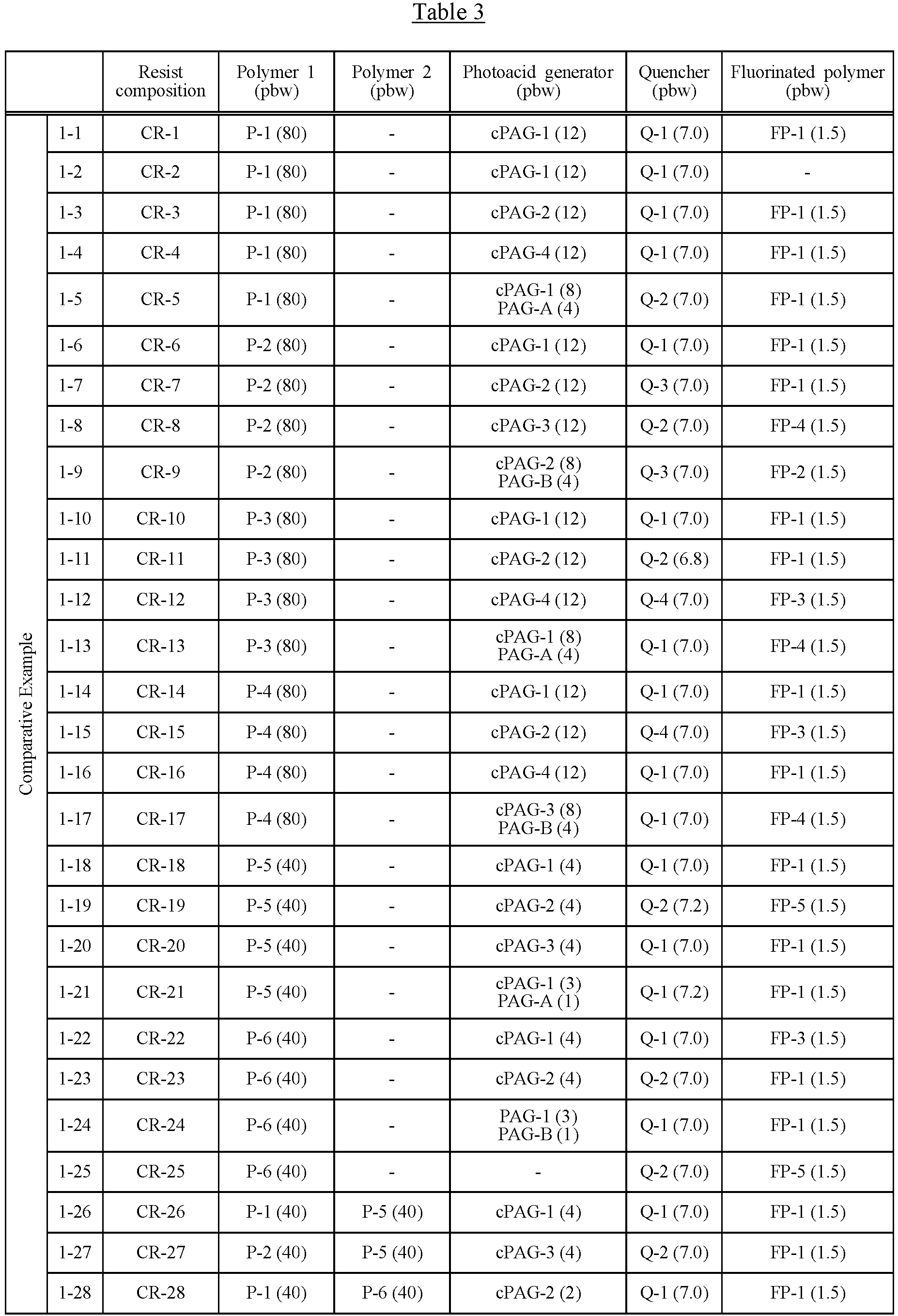

Comparative photoacid generators cPAG-1 to cPAG-4, photoacid generators PAG-A and PAG-B, Quenchers Q-1 to Q-4, and Fluorinated Polymers FP-1 to FP-5 in Tables 1 to 3 are identified below.

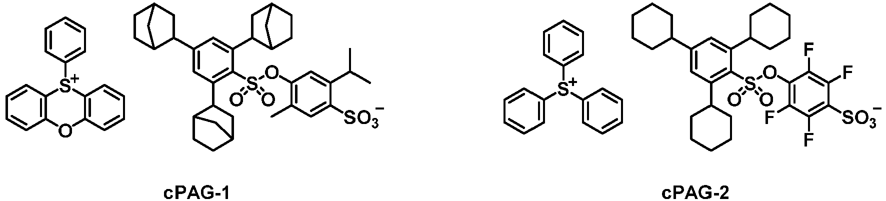

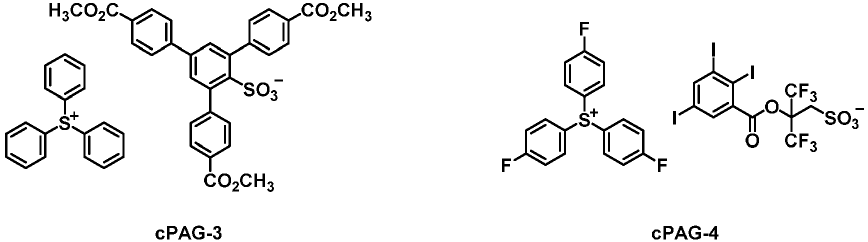

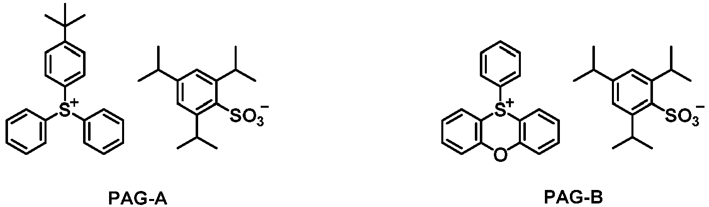

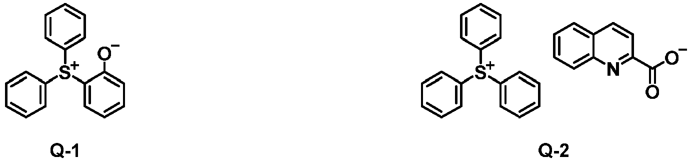

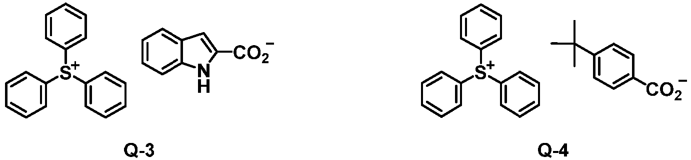

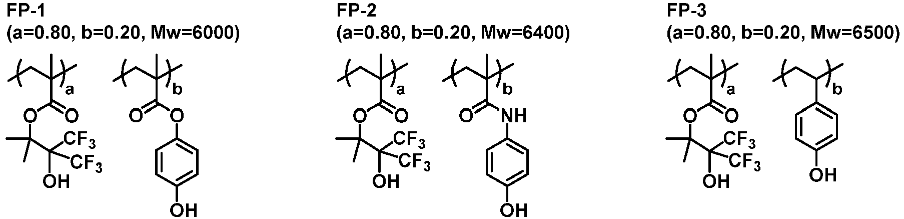

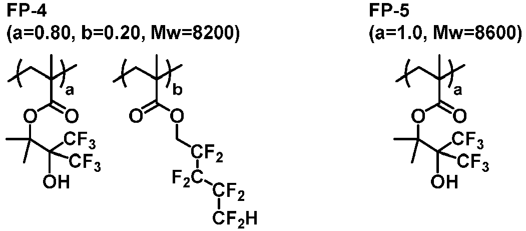

### [4] EB lithography test

### Examples 2-1 to 2-53 and Comparative Examples 2-1 to 2-28

A photomask blank of reflection type for an EUV lithography mask was furnished by starting with a low-coefficient-of-thermal-expansion glass substrate of 6 inches squares and depositing thereon a multilayer reflective film of 40 Mo/Si layers with a thickness of 284 nm, a Ru film of 3.5 nm thick as protective film, a TaN film of 70 nm thick as absorbing layer, and a CrN film of 6 nm thick as hard mask. Using a coater/developer system ACT-M (Tokyo Electron Ltd.), each of the resist compositions (R-1 to R-53, CR-1 to CR-28) was spin coated onto the photomask blank, and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The resist film was exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a positive pattern.

The resist pattern was evaluated as follows. The patterned mask blank was observed under a top-down scanning electron microscope (TD-SEM). The optimum dose (Eop) was defined as the exposure dose (µC/cm²) which provided a 1:1 resolution at the top and bottom of a 200-nm 1:1 line-and-space (LS) pattern. The resolution (or maximum IS resolution) was defined as the minimum size at the dose which provided a 9:1 resolution for an isolated space (IS) of 200 nm. The edge roughness (LER) of a 200-nm LS pattern was measured under SEM. The pattern was visually observed to judge whether or not the profile was rectangular. The results are shown in Tables 4 to 6.

**Table 4**

| | | Resist composition | Optimum dose (µC/cm²) | Maximum IS resolution (nm) | LER (nm) | Pattern profile |
|---|---|---|---|---|---|---|
| | 2-1 | R-1 | 210 | 15 | 4.0 | rectangular |
| | 2-2 | R-2 | 200 | 16 | 4.2 | rectangular |
| | 2-3 | R-3 | 205 | 15 | 4.1 | rectangular |
| | 2-4 | R-4 | 210 | 16 | 4.2 | rectangular |
| | 2-5 | R-5 | 215 | 15 | 4.3 | rectangular |
| | 2-6 | R-6 | 210 | 17 | 4.3 | rectangular |
| | 2-7 | R-7 | 215 | 16 | 4.1 | rectangular |
| | 2-8 | R-8 | 220 | 17 | 4.2 | rectangular |
| | 2-9 | R-9 | 210 | 16 | 4.2 | rectangular |
| | 2-10 | R-10 | 215 | 15 | 4.3 | rectangular |
| | 2-11 | R-11 | 215 | 15 | 4.4 | rectangular |
| | 2-12 | R-12 | 210 | 16 | 4.2 | rectangular |
| | 2-13 | R-13 | 215 | 17 | 4.3 | rectangular |
| | 2-14 | R-14 | 210 | 16 | 4.1 | rectangular |
| | 2-15 | R-15 | 215 | 16 | 4.3 | rectangular |
| Example | 2-16 | R-16 | 210 | 17 | 4.2 | rectangular |
| | 2-17 | R-17 | 215 | 16 | 4.1 | rectangular |
| | 2-18 | R-18 | 210 | 15 | 4.4 | rectangular |
| | 2-19 | R-19 | 210 | 16 | 4.2 | rectangular |
| | 2-20 | R-20 | 200 | 16 | 4.3 | rectangular |
| | 2-21 | R-21 | 210 | 18 | 4.3 | rectangular |
| | 2-22 | R-22 | 215 | 15 | 4.3 | rectangular |
| | 2-23 | R-23 | 210 | 17 | 4.5 | rectangular |
| | 2-24 | R-24 | 215 | 17 | 4.3 | rectangular |
| | 2-25 | R-25 | 210 | 16 | 4.2 | rectangular |
| | 2-26 | R-26 | 215 | 16 | 4.2 | rectangular |
| | 2-27 | R-27 | 215 | 16 | 4.2 | rectangular |
| | 2-28 | R-28 | 205 | 15 | 4.2 | rectangular |
| | 2-29 | R-29 | 200 | 16 | 4.1 | rectangular |
| | 2-30 | R-30 | 205 | 17 | 4.4 | rectangular |

**Table 5**

| | | Resist composition | Optimum dose (µC/cm²) | Maximum IS resolution (nm) | LER (nm) | Pattern profile |
|---|---|---|---|---|---|---|
| | 2-31 | R-31 | 210 | 15 | 4.2 | rectangular |
| | 2-32 | R-32 | 215 | 16 | 4.1 | rectangular |
| | 2-33 | R-33 | 210 | 15 | 4.2 | rectangular |
| | 2-34 | R-34 | 215 | 16 | 4.2 | rectangular |
| | 2-35 | R-35 | 220 | 16 | 4.3 | rectangular |
| | 2-36 | R-36 | 215 | 15 | 4.1 | rectangular |
| | 2-37 | R-37 | 210 | 17 | 4.2 | rectangular |
| | 2-38 | R-38 | 205 | 16 | 4.1 | rectangular |
| | 2-39 | R-39 | 215 | 17 | 4.0 | rectangular |
| | 2-40 | R-40 | 210 | 16 | 4.2 | rectangular |
| | 2-41 | R-41 | 215 | 17 | 4.2 | rectangular |
| Example | 2-42 | R-42 | 205 | 17 | 4.1 | rectangular |
| | 2-43 | R-43 | 200 | 15 | 4.2 | rectangular |
| | 2-44 | R-44 | 215 | 16 | 4.3 | rectangular |
| | 2-45 | R-45 | 215 | 16 | 4.4 | rectangular |
| | 2-46 | R-46 | 210 | 17 | 4.2 | rectangular |
| | 2-47 | R-47 | 215 | 16 | 4.1 | rectangular |
| | 2-48 | R-48 | 205 | 16 | 4.2 | rectangular |
| | 2-49 | R-49 | 215 | 17 | 4.3 | rectangular |
| | 2-50 | R-50 | 210 | 15 | 4.1 | rectangular |
| | 2-51 | R-51 | 215 | 16 | 4.3 | rectangular |
| | 2-52 | R-52 | 200 | 16 | 4.2 | rectangular |
| | 2-53 | R-53 | 215 | 16 | 4.1 | rectangular |

**Table 6**

| | | Resist composition | Optimum dose (µC/cm²) | Maximum IS resolution (nm) | LER (nm) | Pattern profile |
|---|---|---|---|---|---|---|
| | 2-1 | CR-1 | 215 | 19 | 4.9 | rectangular |
| | 2-2 | CR-2 | 200 | 20 | 4.9 | footing |
| | 2-3 | CR-3 | 200 | 22 | 5.1 | rectangular |
| | 2-4 | CR-4 | 215 | 20 | 4.8 | rectangular |
| | 2-5 | CR-5 | 205 | 18 | 5.1 | footing |
| | 2-6 | CR-6 | 210 | 22 | 5 | rectangular |
| | 2-7 | CR-7 | 200 | 21 | 4.9 | footing |
| | 2-8 | CR-8 | 210 | 22 | 4.7 | rectangular |
| | 2-9 | CR-9 | 210 | 21 | 4.8 | rectangular |
| | 2-10 | CR-10 | 210 | 21 | 4.9 | rectangular |
| | 2-11 | CR-11 | 215 | 20 | 5 | rectangular |
| | 2-12 | CR-12 | 210 | 20 | 4.9 | footing |
| | 2-13 | CR-13 | 210 | 22 | 4.7 | rectangular |
| Comparative Example | 2-14 | CR-14 | 210 | 21 | 4.8 | rectangular |
| | 2-15 | CR-15 | 215 | 22 | 5 | footing |
| | 2-16 | CR-16 | 210 | 22 | 4.8 | rectangular |
| | 2-17 | CR-17 | 215 | 20 | 4.7 | rectangular |
| | 2-18 | CR-18 | 200 | 21 | 4.9 | footing |
| | 2-19 | CR-19 | 210 | 21 | 4.8 | rectangular |
| | 2-20 | CR-20 | 215 | 21 | 5 | rectangular |
| | 2-21 | CR-21 | 200 | 20 | 4.9 | rectangular |
| | 2-22 | CR-22 | 205 | 23 | 5 | footing |
| | 2-23 | CR-23 | 215 | 22 | 4.8 | rectangular |
| | 2-24 | CR-24 | 200 | 23 | 4.7 | rectangular |
| | 2-25 | CR-25 | 205 | 22 | 5 | footing |
| | 2-26 | CR-26 | 215 | 20 | 4.8 | rectangular |
| | 2-27 | CR-27 | 200 | 19 | 4.9 | footing |
| | 2-28 | CR-28 | 205 | 20 | 4.7 | rectangular |

As is evident from Tables 4 to 6, the chemically amplified positive resist composition comprising the onium salt consisting of an anion having formula (A1) and a cation having formula (A2) as a photoacid generator and the resist pattern forming process within the scope of the invention are effective in photolithography for the fabrication of semiconductor devices and the processing of photomask blanks of transmission and reflection types.

## Claims

1. A chemically amplified positive resist composition comprising (A) a photoacid generator and (B) a base polymer containing a polymer adapted to be decomposed under the action of acid to increase its solubility in alkaline developer,
said photoacid generator (A) containing an onium salt consisting of an anion having the formula (A1) and a cation having the formula (A2): 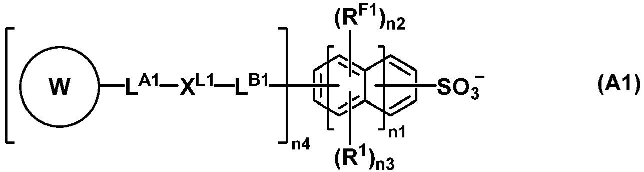 wherein n1 is 0 or 1, n2 is 0, 1, 2, 3 or 4, n3 is 0, 1, 2, 3 or 4, with the proviso that when n1=0, 0 ≤ n2+n3 ≤ 4, when n1=1, 0 ≤ n2+n3 ≤ 6, and n4 is 0 or 1,
W is a C₆-C₄₀ hydrocarbyl group containing at least one aromatic ring, which may contain a heteroatom,
R^{F1} is fluorine, a C₁-C₆ fluorinated saturated hydrocarbyl group, C₁-C₆ fluorinated saturated hydrocarbyloxy group, or C₁-C₆ fluorinated saturated hydrocarbylthio group,
R¹ is halogen exclusive of fluorine, nitro group, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom; when n3 is 2, 3 or 4, a plurality of R¹ may be identical or different, and a plurality of R¹ may bond together to form a ring with the carbon atoms to which they are attached,
L^{A1} and L^{B1} are each independently a single bond, ether bond, ester bond, amide bond, sulfonate ester bond, sulfonamide bond, carbonate bond or carbamate bond,
X^{L1} is a single bond or a C₁-C₄₀ hydrocarbylene group which may contain a heteroatom, 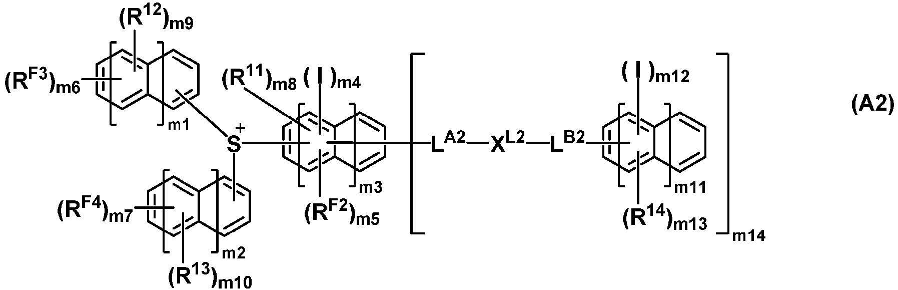 wherein m1 is 0 or 1, m2 is 0 or 1, m3 is 0 or 1, m4 is 0, 1, 2, 3 or 4, m5 is 0, 1, 2, 3 or 4, m6 is 0, 1, 2, 3, 4, 5 or 6, m7 is 0, 1, 2, 3, 4, 5 or 6, m8 is 0, 1 or 2, m9 is 0, 1 or 2, m10 is 0, 1 or 2, m11 is 0 or 1, m12 is 0, 1, 2, 3 or 4, m13 is 0, 1 or 2, m14 is 0, 1 or 2, with the proviso that when m1=0, 0 ≤ m6+m9 ≤ 4, and when m1=1, 0 ≤ m6+m9 ≤ 6; when m2=0, 0 ≤ m7+m10 ≤ 4, and when m2=1, 0 ≤ m7+m10 ≤ 6; when m3=0, 1 ≤ m4+m5+m8+m14 ≤ 4, and when m3=1, 1 ≤ m4+m5+m8+m14 ≤ 6; when m11=0, 0 ≤ m12+m13 ≤ 4, and when m11=1, 0 ≤ m12+m13 ≤ 6; m4+m12 ≥ 1,
R^{F2} to R^{F4} are each independently fluorine, a C₁-C₆ fluorinated saturated hydrocarbyl group, C₁-C₆ fluorinated saturated hydrocarbyloxy group, or C₁-C₆ fluorinated saturated hydrocarbylthio group; when m5 is 2 or more, a plurality of R^{F2} may be identical or different; when m6 is 2 or more, a plurality of R¹³ may be identical or different; when m7 is 2 or more, a plurality of R^{F4} may be identical or different,
R¹¹ to R¹⁴ are each independently halogen exclusive of iodine and fluorine, nitro group, cyano group, a C₁-C₂₀ hydrocarbyl group which may contain a heteroatom, C₁-C₂₀ hydrocarbyloxy group which may contain a heteroatom, C₁-C₂₀ hydrocarbylthio group which may contain a heteroatom; when m8=2, two R¹¹ may be identical or different and two R¹¹ may bond together to form a ring with the carbon atoms to which they are attached; when m9=2, two R¹² may be identical or different and two R¹² may bond together to form a ring with the carbon atoms to which they are attached; when m10=2, two R¹³ may be identical or different and two R¹³ may bond together to form a ring with the carbon atoms to which they are attached; when m13=2, two R¹⁴ may be identical or different and two R¹⁴ may bond together to form a ring with the carbon atoms to which they are attached;
the aromatic rings directly bonded to S⁺ in the sulfonium cation may bond together to form a ring with S⁺,
L^{A2} and L^{B2} are each independently a single bond, ether bond, ester bond, amide bond, sulfonate ester bond, sulfonamide bond, carbonate bond or carbamate bond,
X^{L2} is a single bond or a C₁-C₄₀ hydrocarbylene group which may contain a heteroatom,
said polymer comprising repeat units having the formula (B1): 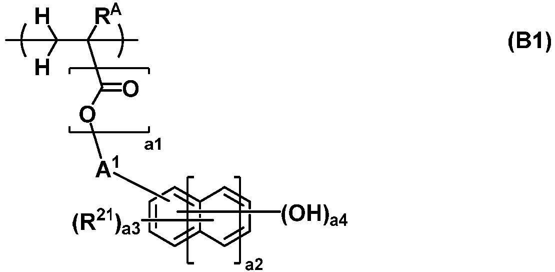 wherein a1 is 0 or 1, a2 is 0, 1 or 2, a3 is an integer meeting 0 ≤ a3 ≤ 5+2(a2)-a4, a4 is 1, 2 or 3,
R^{A} is hydrogen, fluorine, methyl or trifluoromethyl,
R²¹ is halogen, nitro group, carboxy group, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, and
A¹ is a single bond or C₁-C₁₀ saturated hydrocarbylene group in which some -CH₂-may be replaced by -O-.

2. The resist composition of claim 1 wherein W is a group having the formula (W-1) or (W-2): 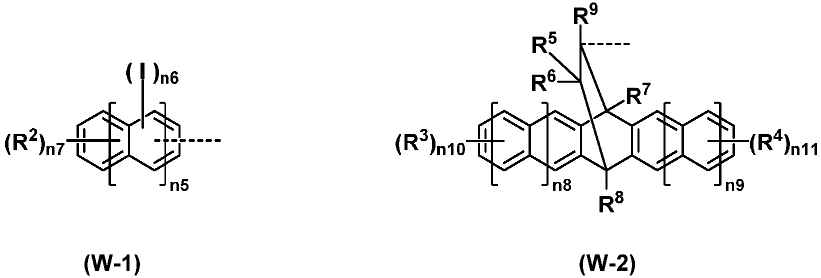 wherein n5 is 0 or 1, n6 is 0, 1, 2, 3 or 4, n7 is 0, 1, 2, 3 or 4, with the proviso that when n5=0, 1 ≤ n6+n7 ≤ 5, and when n5 =1, 1 ≤ n6+n7 ≤ 7, n8 is 0 or 1, n9 is 0 or 1, n10 is 0, 1, 2, 3 or 4, n11 is 0, 1, 2, 3 or 4,
R² is each independently hydrogen, halogen exclusive of iodine, hydroxy, or a C₁-C₂₀ hydrocarbyl group which may contain a heteroatom,
R³ and R⁴ are each independently hydrogen, halogen, or a C₁-C₂₀ hydrocarbyl group which may contain a heteroatom,
R⁵ to R⁹ are each independently hydrogen, halogen, or a C₁-C₄₀ hydrocarbyl group which may contain a heteroatom,
the broken line designates a point of attachment to L^{A1}

3. The resist composition of claim 1 or 2 wherein the anion having formula (A1) has the formula (A1-1): 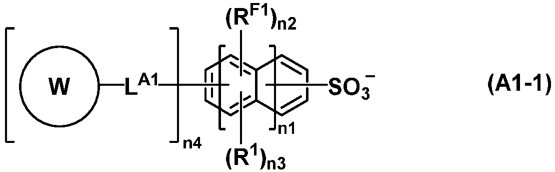 wherein n1 to n4, W, R^{F1}, R¹, and L^{A} are as defined above.

4. The resist composition of any one of claims 1 to 3 wherein the polymer further comprises repeat units having the formula (B2-1): 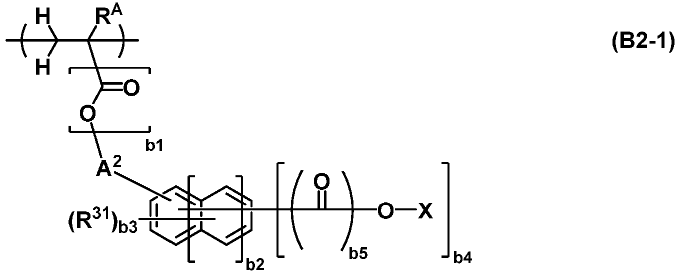 wherein R^{A} is hydrogen, fluorine, methyl or trifluoromethyl,
b1 is 0 or 1, b2 is 0, 1 or 2, b3 is an integer meeting 0 ≤ b3 ≤ 5+2(b2)-b4, b4 is 1, 2 or 3, b5 is 0 or 1,
R³¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
A² is a single bond or C₁-C₁₀ saturated hydrocarbylene group in which some -CH₂-may be replaced by -O-,
X is an acid labile group when b4=1, and X is hydrogen or an acid labile group, at least one being an acid labile group, when b4=2 or 3.

5. The resist composition of any one of claims 1 to 4 wherein the polymer further comprises repeat units having the formula (B2-2): 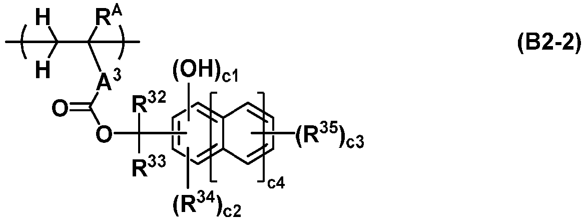 wherein c1 is 0, 1 or 2, c2 is 0, 1 or 2, c3 is 0, 1, 2, 3, 4 or 5, c4 is 0, 1 or 2,
R^{A} is hydrogen, fluorine, methyl or trifluoromethyl,
R³² and R³³ are each independently a C₁-C₁₀ hydrocarbyl group which may contain a heteroatom, R³² and R³³ may bond together to form a ring with the carbon atom to which they are attached,
R³⁴ is each independently fluorine, a C₁-C₅ fluorinated alkyl group or C₁-C₅ fluorinated alkoxy group,
R³⁵ is each independently a C₁-C₁₀ hydrocarbyl group which may contain a heteroatom, and
A³ is a single bond, phenylene, naphthylene or *-C(=O)-O-A³¹-, A³¹ is a C₁-C₂₀ aliphatic hydrocarbylene group which may contain a hydroxy moiety, ether bond, ester bond or lactone ring, a phenylene group or a naphthylene group, * designates a point of attachment to the carbon atom in the backbone.

6. The resist composition of any one of claims 1 to 5 wherein the polymer further comprises repeat units of at least one type selected from repeat units having the formula (B3), repeat units having the formula (B4), and repeat units having the formula (B5): 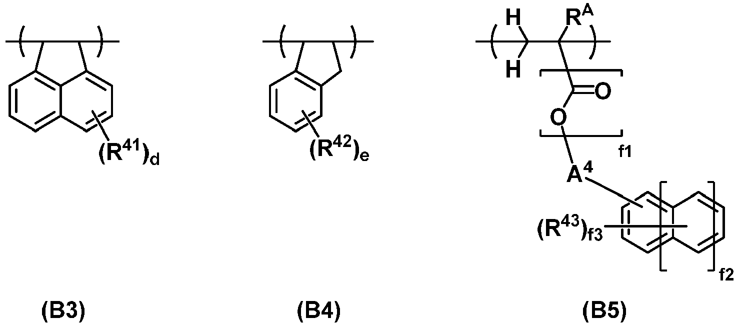 wherein d is 0, 1, 2, 3, 4, 5 or 6, e is 0, 1, 2, 3 or 4, f1 is 0 or 1, f2 is 0, 1 or 2, f3 is 0, 1, 2, 3, 4 or 5,
R^{A} is hydrogen, fluorine, methyl or trifluoromethyl,
R⁴¹ and R⁴² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
R⁴³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro, or cyano, R⁴³ may also be hydroxy when f2=1 or 2, and
A⁴ is a single bond or C₁-C₁₀ saturated hydrocarbylene group in which some -CH₂-may be replaced by -O-.

7. The resist composition of any one of claims 1 to 6 wherein the polymer further comprises repeat units of at least one type selected from repeat units having the formula (B6), repeat units having the formula (B7), repeat units having the formula (B8), repeat units having the formula (B9), and repeat units having the formula (B10): 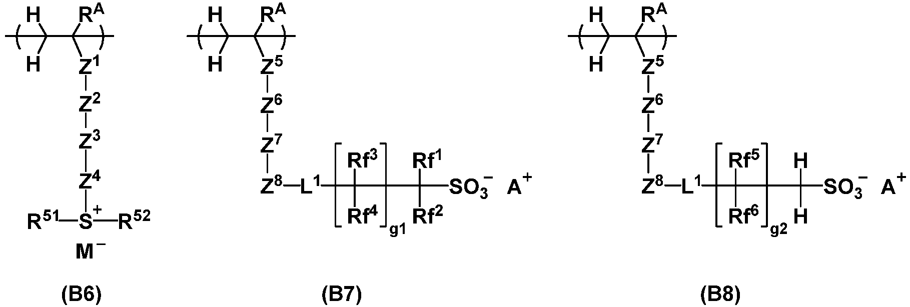 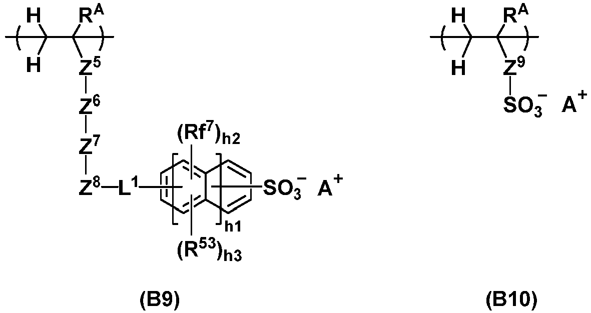 wherein g1 and g2 are each independently 0, 1, 2 or 3, h1 is 0 or 1, h2 is 0, 1, 2, 3 or 4, h3 is 0, 1, 2, 3 or 4, with the proviso that when h1=0, 0 ≤ h2+h3 ≤ 4, and when h1=1, 0 ≤ h2+h3 ≤ 6,
R^{A} is hydrogen, fluorine, methyl or trifluoromethyl,
Z¹ is a single bond or an optionally substituted phenylene group,
Z² is a single bond, **-C(=O)-O-Z²¹-, **-C(=O)-NH-Z²¹-, or **-O-Z²¹-, Z²¹ is a C₁-C₆ aliphatic hydrocarbylene group, phenylene group or a divalent group obtained by combining the foregoing, which may contain halogen, carbonyl moiety, ester bond, ether bond or hydroxy moiety,
Z³ is a single bond, ether bond, ester bond, amide bond, sulfonate ester bond, sulfonamide bond, carbonate bond or carbamate bond,
Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety,
Z⁵ is each independently a single bond, an optionally substituted phenylene, naphthylene, or *-C(=O)-O-Z⁵¹-, Z⁵¹ is a C₁-C₁₀ aliphatic hydrocarbylene group which may contain halogen, hydroxy moiety, ether bond, ester bond or lactone ring, or phenylene or naphthylene group,
Z⁶ is a single bond, ether bond, ester bond, amide bond, sulfonate ester bond, sulfonamide bond, carbonate bond or carbamate bond,
Z⁷ is each independently a single bond, ***-Z⁷¹-C(=O)-O-, ***-C(=O)-NH-Z7¹-, or ***-O-Z⁷¹-, Z⁷¹ is a C₁-C₂₀ hydrocarbylene group which may contain a heteroatom,
Z⁸ is each independently a single bond, ****-Z⁸¹-C(=O)-O-, ****-C(=O)-NH-Z⁸¹-, or ****-O-Z⁸¹-, Z⁸¹ is a C₁-C₂₀ hydrocarbylene group which may contain a heteroatom,
Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety,
* is a point of attachment to the carbon atom in the backbone, ** is a point of attachment to Z¹, *** is a point of attachment to Z⁶, **** is a point of attachment to Z⁷,
L¹ is a single bond, ether bond, ester bond, carbonyl group, sulfonate ester bond, sulfonamide bond, carbonate bond or carbamate bond,
Rf¹ and Rf² are each independently fluorine or a C₁-C₆ fluorinated saturated hydrocarbyl group,
Rf³ and Rf⁴ are each independently hydrogen, fluorine, or a C₁-C₆ fluorinated saturated hydrocarbyl group,
Rf⁶ and Rf⁶ are each independently hydrogen, fluorine, or a C₁-C₆ fluorinated saturated hydrocarbyl group, excluding that all Rf⁶ and Rf⁶ are hydrogen at the same time,
Rf⁷ is fluorine, a C₁-C₆ fluorinated alkyl group, C₁-C₆ fluorinated alkoxy group, or C₁-C₆ fluorinated alkylthio group,
R³¹ and R³² are each independently a C₁-C₂₀ hydrocarbyl group which may contain a heteroatom, R³¹ and R³² may bond together to form a ring with the sulfur atom to which they are attached,
R¹³ is halogen exclusive of fluorine, or a C₁-C₂₀ hydrocarbyl group which may contain a heteroatom; when h3 is 2, 3 or 4, a plurality of R⁵³ may bond together to form a ring with the carbon atoms to which they are attached,
M⁻ is a non-nucleophilic counter ion,
A⁺ is an onium cation.

8. The resist composition of any one of claims 1 to 7 wherein repeat units having an aromatic ring structure account for at least 60 mol% of the overall repeat units of the polymer in the base polymer.

9. The resist composition of any one of claims 1 to 8, further comprising (C) an organic solvent.

10. The resist composition of any one of claims 1 to 9, further comprising (D) a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 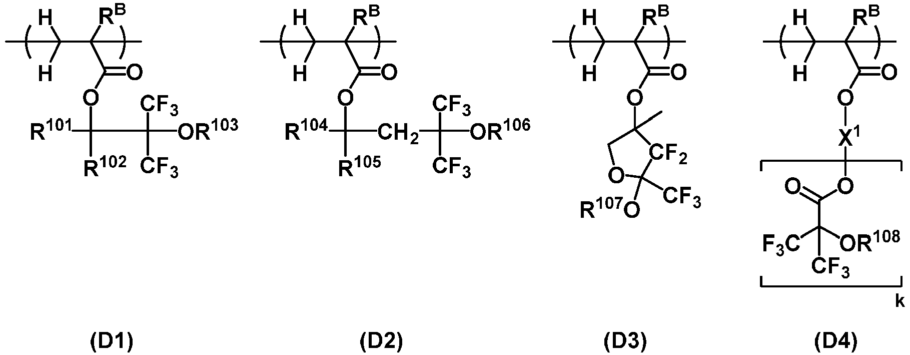 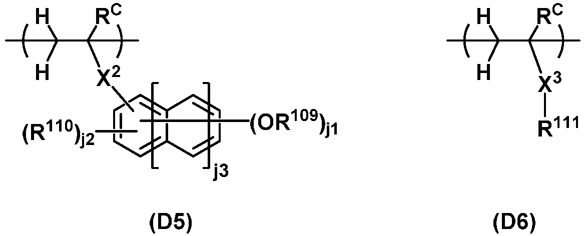 wherein j1 is 1, 2 or 3, j2 is an integer meeting 0 ≤ j2 ≤ 5+2(j3)-j 1, j3 is 0 or 1, k is 1, 2 or 3,
R^{B} is each independently hydrogen, fluorine, methyl or trifluoromethyl,
R^{C} is each independently hydrogen or methyl,
R¹⁰¹, R¹⁰², R¹⁰⁴ and R¹⁰⁵ are each independently hydrogen or a C₁-C₁₀ saturated hydrocarbyl group,
R¹⁰³, R¹⁰⁶, R¹⁰⁷ and R¹⁰⁸ are each independently hydrogen, a C₁-C₁₅ hydrocarbyl group, C₁-C₁₅ fluorinated hydrocarbyl group, or acid labile group, and when R¹⁰³, R¹⁰⁶, R¹⁰⁷ and R¹⁰⁸ each are a hydrocarbyl or fluorinated hydrocarbyl group, an ether bond or carbonyl moiety may intervene in a carbon-carbon bond,
R¹⁰⁹ is hydrogen or a C₁-C₅ straight or branched hydrocarbyl group in which a heteroatom-containing moiety may intervene in a carbon-carbon bond,
R¹¹⁰ is a C₁-C₅ straight or branched hydrocarbyl group in which a heteroatom-containing moiety may intervene in a carbon-carbon bond,
R¹¹¹ is a C₁-C₂₀ saturated hydrocarbyl group in which at least one hydrogen is substituted by fluorine, and in which some constituent -CH₂- may be replaced by an ester bond or ether bond,
X¹ is a C₁-C₂₀ (k+1)-valent hydrocarbon group or C₁-C₂₀ (k+1)-valent fluorinated hydrocarbon group,
X² is a single bond, *-C(=O)-O- or *-C(=O)-NH-, * designates a point of attachment to the carbon atom in the backbone,
X³ is a single bond, -O-, *-C(=O)=O-X³¹-X³²- or *-C(=O)-NH-X³¹-X³²-, X³¹ is a single bond or C₁-C₁₀ saturated hydrocarbylene group, X³² is a single bond, ester bond, ether bond, or sulfonamide bond, and * designates a point of attachment to the carbon atom in the backbone.

11. The resist composition of any one of claims 1 to 10, further comprising (E) a quencher.

12. The resist composition of claim 11 wherein the photoacid generator (A) and the quencher (E) are present in a weight ratio of less than 3/1.

13. The resist composition of any one of claims 1 to 12, further comprising a photoacid generator other than the photoacid generator set forth in claim 1.

14. A resist pattern forming process comprising the steps of:
applying the chemically amplified positive resist composition of any one of claims 1 to 13 onto a substrate to form a resist film thereon,
exposing the resist film patternwise to high-energy radiation, and
developing the exposed resist film in an alkaline developer.

15. The process of claim 14 wherein the substrate is a photomask blank.
